# EUROPÄISCHE PATENTSCHRIFT

(11) **EP 3 658 564 B1**
(45) Veröffentlichungstag und Bekanntmachung des Hinweises auf die Patenterteilung: **19.05.2021**
(21) Anmeldenummer: 18740862.0
(22) Anmeldetag: 23.07.2018
(51) Int. Cl.: C07F 15/00, H01L 51/50

(54) **METALLKOMPLEXE**
METAL COMPLEXES
COMPLEXES MÉTALLIQUES

(30) Priorität: 25.07.2017 EP 17182995
(43) Veröffentlichungstag der Anmeldung: 03.06.2020
(73) Patentinhaber: Merck Patent GmbH, 64293 Darmstadt (DE)
(72) Erfinder: STOESSEL, Philipp, 60389 Frankfurt Am Main (DE); AUCH, Armin, 64297 Darmstadt (DE)
(86) Internationale Anmeldenummer: PCT/EP2018/069854
(87) Internationale Veröffentlichungsnummer: WO 2019/020538

(56) Entgegenhaltungen:
- WO-A1-2004/085449
- WO-A1-2016/124304
- WO-A1-2017/032439

## Beschreibung

Die vorliegende Erfindung betrifft Iridiumkomplexe, welche sich für den Einsatz in organischen Elektrolumineszenzvorrichtungen, insbesondere als Emitter, eignen.

Gemäß dem Stand der Technik werden in phosphoreszierenden organischen Elektrolumineszenzvorrichtungen (OLEDs) als Triplettemitter vor allem Iridiumkomplexe eingesetzt, insbesondere bis- und tris-orthometallierte Komplexe mit aromatischen Liganden, wobei die Liganden über ein negativ geladenes Kohlenstoffatom und ein neutrales Stickstoffatom oder über ein negativ geladenes Kohlenstoffatom und ein neutrales Carben-Kohlenstoffatom an das Metall binden. Beispiele für solche Komplexe sind Tris(phenylpyridyl)iridium(III) und Derivate davon, sowie eine Vielzahl verwandter Komplexe bekannt, beispielsweise mit 1- oder 3-Phenylisochinolinliganden, mit 2-Phenylchinolinliganden oder mit Phenylcarbenliganden, wobei diese Komplexe auch Acetylacetonat als Hilfsliganden aufweisen können. Derartige Komplexe sind auch mit polypodalen Liganden bekannt, wie beispielsweise in WO 2016/124304 beschrieben. Auch wenn diese Komplexe mit polypodalen Liganden Vorteile gegenüber den Komplexen zeigen, die ansonsten die gleiche Ligandenstruktur aufweisen, deren einzelne Liganden jedoch nicht polypodal verbrückt sind, gibt es jedoch auch noch Verbesserungsbedarf. Dieser liegt insbesondere in der Effizienz der Lumineszenz der Verbindungen, sowie in der Sublimationstemperatur der Verbindungen. Eine geringere Sublimationstemperatur ermöglicht eine einfachere Aufreinigung der Komplexe in der Synthese sowie eine vereinfachte Verarbeitung, wenn diese durch Vakuumaufdamfpung in der OLED aufgebracht werden.

Aufgabe der vorliegenden Erfindung ist daher die Bereitstellung neuer und insbesondere verbesserter Metallkomplexe, welche sich als Emitter für die Verwendung in OLEDs eignen.

Überraschend wurde gefunden, dass Metallkomplexe mit einem hexadentaten tripodalen Liganden, der die nachfolgend beschriebene Struktur aufweist, diese Aufgabe lösen und sich sehr gut für die Verwendung in einer organischen Elektrolumineszenzvorrichtung eignen. Diese Metallkomplexe und organische Elektrolumineszenzvorrichtungen, welche diese Komplexe enthalten, sind daher der Gegenstand der vorliegenden Erfindung.

Gegenstand der Erfindung ist somit eine Verbindung der Formel (1), 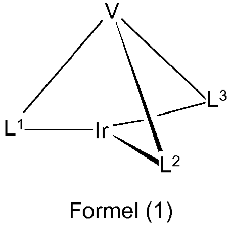 wobei für die verwendeten Symbole gilt:
- L¹: ist ein Teilligand der folgenden Formel (2), der über die beiden Gruppen Z an das Iridium koordiniert und der über die gestrichelte Bindung an V gebunden ist, 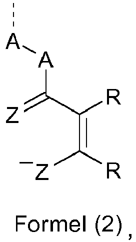 wobei gilt:
- A: ist bei jedem Auftreten gleich oder verschieden CR₂, O, S oder NR, wobei mindestens eine Gruppe A für CR₂ steht;
- Z: ist bei jedem Auftreten gleich oder verschieden O, S oder NR;
- L²: ist ein bidentater, monoanionischer Teilligand, der über ein Kohlenstoffatom und ein Stickstoffatom oder über zwei Kohlenstoffatome an das Iridium koordiniert;
- L³: ist ein bidentater, monoanionischer Teilligand, der über ein Kohlenstoffatom und ein Stickstoffatom oder über zwei Kohlenstoffatome an das Iridium koordiniert, oder ist ein Teilligand der oben aufgeführten Formel (2), der gleich oder verschieden L¹ sein kann;
- V: ist eine Gruppe der Formel (3), wobei die gestrichelten Bindungen jeweils die Position der Verknüpfung der Teilliganden L¹, L² und L³ darstellen,

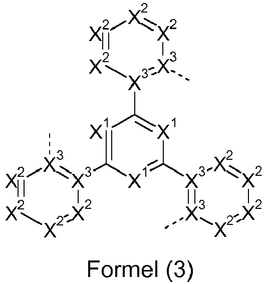

- X¹: ist bei jedem Auftreten gleich oder verschieden CR oder N;
- X²: ist bei jedem Auftreten gleich oder verschieden CR oder N, oder zwei benachbarte Gruppen X² stehen zusammen für NR, O oder S, so dass ein Fünfring entsteht; oder zwei benachbarte Gruppen X² stehen zusammen für CR oder N, wenn in dem Cyclus eine der Gruppen X³ für N steht, so dass ein Fünfring entsteht; mit der Maßgabe, dass maximal zwei benachbarte Gruppen X² in jedem Ring für N stehen;
- X³: ist bei jedem Auftreten in einem Cyclus C oder eine Gruppe X³ steht für N und die andere Gruppe X³ in demselben Cyclus steht für C, wobei die Gruppen X³ in den drei Cyclen unabhängig voneinander gewählt werden können; mit der Maßgabe, dass zwei benachbarte Gruppen X² zusammen für CR oder N stehen, wenn in dem Cyclus eine der Gruppen X³ für N steht;
- R: ist bei jedem Auftreten gleich oder verschieden H, D, F, Cl, Br, I, N(R¹)₂, OR¹, SR¹, CN, NO₂, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann und wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 40 aromatischen Ringatomen, das jeweils durch einen oder mehrere Reste R¹ substituiert sein kann; dabei können zwei Reste R auch miteinander ein Ringsystem bilden;
- R¹: ist bei jedem Auftreten gleich oder verschieden H, D, F, Cl, Br, I, N(R²)₂, OR², SR², CN, NO₂, Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann und wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein Ringsystem bilden;
- R²: ist bei jedem Auftreten gleich oder verschieden H, D, F oder ein aliphatischer, aromatischer und/oder heteroaromatischer organischer Rest, insbesondere ein Kohlenwasserstoffrest, mit 1 bis 20 C-Atomen, in dem auch ein oder mehrere H-Atome durch F ersetzt sein können;
dabei können die drei bidentaten Liganden L¹, L² und L³ außer durch die Brücke V auch noch durch eine weitere Brücke zu einem Kryptat geschlossen sein.

Bei dem Liganden handelt es sich erfindungsgemäß somit um einen hexadentaten, tripodalen Liganden mit drei bidentaten Teilliganden L¹, L² und L³. Dabei bedeutet bidentat, dass der jeweilige Teilligand im Komplex über zwei Koordinationsstellen an das Iridium koordiniert bzw. bindet. Tripodal bedeutet, dass der Ligand drei Teilliganden aufweist, die an die Brücke V bzw. die Brücke der Formel (3) gebunden sind. Da der Ligand drei bidentate Teilliganden aufweist, ergibt sich insgesamt ein hexadentater Ligand, also ein Ligand, der über sechs Koordinationsstellen an das Iridium koordiniert bzw. bindet. Der Begriff "bidentater Teilligand" bedeutet im Sinne dieser Anmeldung, dass es sich bei L¹, L² bzw. L³ jeweils um einen bidentaten Liganden handeln würde, wenn die Brücke V bzw. die Brücke der Formel (3) nicht vorhanden wäre. Durch die formale Abstraktion eines Wasserstoffatoms von diesem bidentaten Liganden und die Anknüpfung an die Brücke der Formel (3) ist dieser jedoch kein separater Ligand mehr, sondern ein Teil des so entstehenden hexadentaten Liganden, so dass hierfür der Begriff "Teilligand" verwendet wird.

Der Ligand der erfindungsgemäßen Verbindung weist somit die folgende Struktur (LIG) auf:

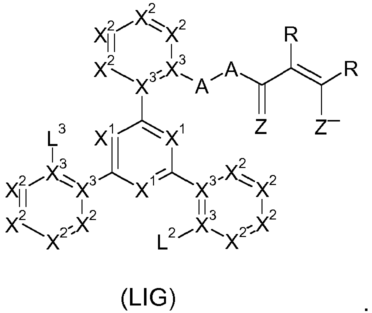

Die Bindung des Liganden an das Iridium kann sowohl eine Koordinationsbindung als auch eine kovalente Bindung sein bzw. der kovalente Anteil an der Bindung kann je nach Ligand variieren. Wenn in der vorliegenden Anmeldung die Rede davon ist, dass der Ligand bzw. der Teilligand an das Iridium koordiniert oder bindet, so bezeichnet dies im Sinne der vorliegenden Anmeldung jede Art der Bindung von dem Liganden bzw. Teilliganden an das Iridium, unabhängig vom kovalenten Anteil der Bindung.

Wenn zwei Reste R bzw. R¹ miteinander ein Ringsystem bilden, so kann dieses mono- oder polycyclisch, aliphatisch, heteroaliphatisch, aromatisch oder heteroaromatisch sein. Dabei können diese Reste, die miteinander ein Ringsystem bilden, benachbart sein, d.h. dass diese Reste an dasselbe Kohlenstoffatom oder an Kohlenstoffatome, die direkt aneinander gebunden sind, gebunden sind, oder sie können weiter voneinander entfernt sein. So ist beispielsweise auch die Ringbildung eines Restes R, der an der Gruppe X² gebunden ist, mit einem Rest R, der an der Gruppe X¹ gebunden ist, möglich. Wenn eine solche Ringbildung zwischen einem Rest R, der an der Gruppe X² gebunden ist, mit einem Rest R, der an der Gruppe X¹ gebunden ist, erfolgt, so erfolgt diese Ringbildung bevorzugt durch eine Gruppe mit drei Brückenatomen, bevorzugt mit drei Kohlenstoffatomen und besonders bevorzugt durch eine Gruppe -(CR₂)₃-.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

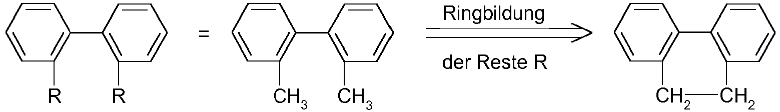

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

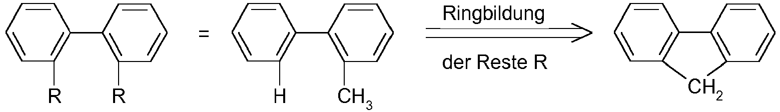

Wie oben beschrieben, kann diese Ringbildung an Resten erfolgen, die an direkt aneinander gebundene Kohlenstoffatome gebunden sind, oder an Resten, die an weiter entfernt liegende Kohlenstoffatome gebunden sind. Bevorzugt ist eine solche Ringbildung bei Resten, die an direkt aneinander gebundene Kohlenstoffatome gebunden sind.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei enthält die Heteroarylgruppe bevorzugt maximal drei Heteroatome. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₂₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer Gruppe OR¹ werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Im Folgenden werden bevorzugte Ausführungsformen des Brückenkopfes V, also der Struktur der Formel (3) ausgeführt.

Geeignete Ausführungsformen der Gruppe der Formel (3) sind die Strukturen der folgenden Formeln (4) bis (7), 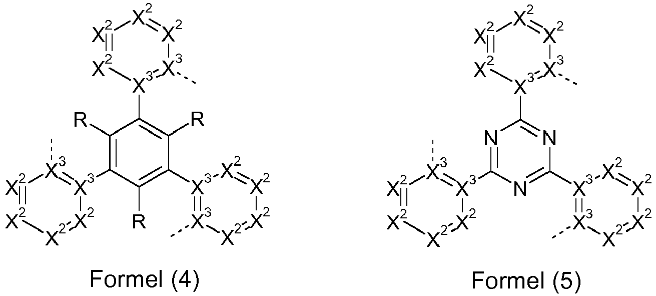 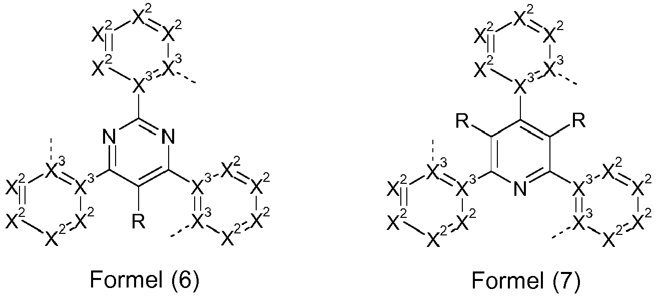 wobei die verwendeten Symbole die oben genannten Bedeutungen aufweisen.

In einer bevorzugten Ausführungsform der Erfindung stehen entweder alle Gruppen X¹ in der Gruppe der Formel (3) für CR, so dass der zentrale trivalente Cyclus der Formel (3) ein Benzol darstellt. Besonders bevorzugt stehen alle Gruppen X¹ in den Formeln (4), (6) und (7) für CH. In einer weiteren bevorzugten Ausführungsform der Erfindung stehen alle Gruppen X¹ für ein Stickstoffatom, so dass der zentrale trivalente Cyclus der Formel (3) ein Triazin darstellt. Bevorzugte Ausführungsformen der Formel (3) sind somit die Strukturen der Formeln (4) und (5).

Für bevorzugte Reste R am trivalenten zentralen Benzolring der Formel (4) bzw. am zentralen Pyrimidinring der Formel (6) bzw. am zentralen Pyridinring der Formel (7) gilt:
- R: ist bei jedem Auftreten gleich oder verschieden H, D, F, CN, OR¹, eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, die jeweils mit einem oder mehreren Resten R¹ substituiert sein kann, bevorzugt aber unsubstituiert ist, oder ein aromatisches oder heteroaromatisches Ringsystem mit 5 bis 24 aromatischen Ringatomen, das jeweils durch einen oder mehrere Reste R¹ substituiert sein kann; dabei kann der Rest R auch mit einem Rest R an X² ein Ringsystem bilden;
- R¹: ist bei jedem Auftreten gleich oder verschieden H, D, F, CN, OR², eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, die jeweils mit einem oder mehreren Resten R² substituiert sein kann, bevorzugt aber unsubstituiert ist, oder ein aromatisches oder heteroaromatisches Ringsystem mit 5 bis 24 aromatischen Ringatomen, das jeweils durch einen oder mehrere Reste R² substituiert sein kann; dabei können zwei oder mehrere benachbarte Reste R¹ miteinander ein Ringsystem bilden;
- R²: ist bei jedem Auftreten gleich oder verschieden H, D, F oder ein aliphatischer, aromatischer und/oder heteroaromatischer organischer Rest mit 1 bis 20 C-Atomen, in dem auch ein oder mehrere H-Atome durch F ersetzt sein können.

Für besonders bevorzugte Reste R am trivalenten zentralen Benzolring der Formel (4) bzw. am zentralen Pyrimidinring der Formel (6) bzw. am zentralen Pyridinring der Formel (7) gilt:
- R: ist bei jedem Auftreten gleich oder verschieden H, D, F, CN, eine geradkettige Alkylgruppe mit 1 bis 4 C-Atomen oder eine verzweigte oder cyclische Alkylgruppe mit 3 bis 6 C-Atomen, die jeweils mit einem oder mehreren Resten R¹ substituiert sein kann, bevorzugt aber unsubstituiert ist, oder ein aromatisches oder heteroaromatisches Ringsystem mit 6 bis 12 aromatischen Ringatomen, das jeweils durch einen oder mehrere Reste R¹ substituiert sein kann; dabei kann der Rest R auch mit einem Rest R an X² ein Ringsystem bilden;
- R¹: ist bei jedem Auftreten gleich oder verschieden H, D, F, CN, eine geradkettige Alkylgruppe mit 1 bis 4 C-Atomen oder eine verzweigte oder cyclische Alkylgruppe mit 3 bis 6 C-Atomen, die jeweils mit einem oder mehreren Resten R² substituiert sein kann, bevorzugt aber unsubstituiert ist, oder ein aromatisches oder heteroaromatisches Ringsystem mit 6 bis 12 aromatischen Ringatomen, das jeweils durch einen oder mehrere Reste R² substituiert sein kann; dabei können zwei oder mehrere benachbarte Reste R¹ miteinander ein Ringsystem bilden;
- R²: ist bei jedem Auftreten gleich oder verschieden H, D, F oder ein aliphatischer oder aromatischer Kohlenwasserstoffrest mit 1 bis 12 C-Atomen.

Besonders bevorzugt ist die Gruppe der Formel (4) eine Struktur der folgenden Formel (4'), 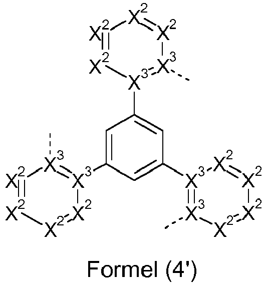 wobei die verwendeten Symbole die oben genannten Bedeutungen aufweisen.

Im Folgenden werden bevorzugte bivalente Arylen- bzw. Heteroaryleneinheiten beschrieben, wie sie in der Gruppe der Formeln (3) bis (7) vorkommen. Wie aus den Strukturen der Formeln (3) bis (7) ersichtlich, enthalten diese Strukturen drei ortho-verknüpfte bivalente Arylen- bzw. Heteroaryleneinheiten.

In einer bevorzugten Ausführungsform der Erfindung steht das Symbol X³ für C, so dass die Gruppen der Formeln (3) bis (7) durch die folgenden Formeln (3a) bis (7a) dargestellt werden können: 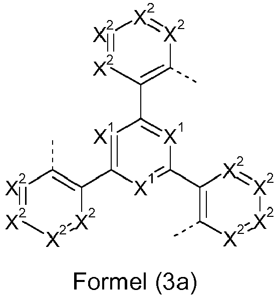 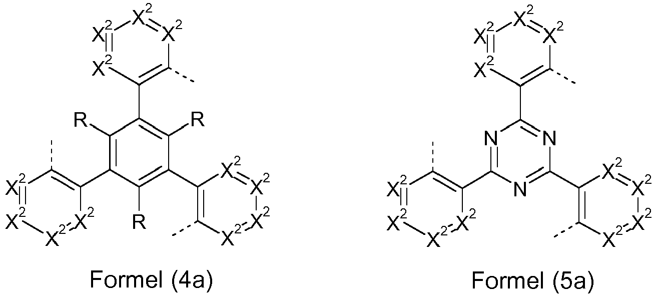 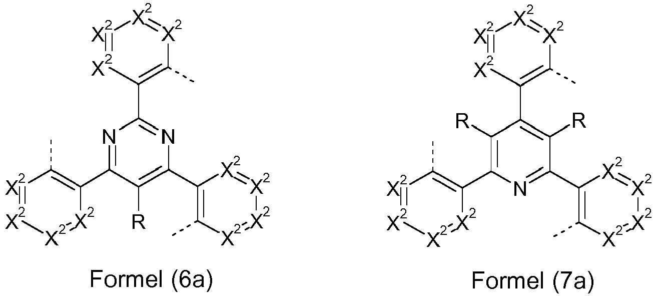 wobei die Symbole die oben aufgeführten Bedeutungen aufweisen.

Die Gruppe der Formel (3) kann formal durch die folgende Formel (3') dargestellt werden, wobei die Formeln (3) und (3') dieselben Strukturen umfassen: 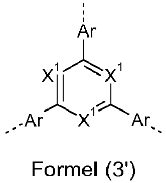 wobei Ar jeweils gleich oder verschieden für eine Gruppe der folgenden Formel (8) steht: 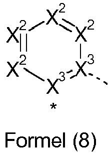 wobei die gestrichelte Bindung jeweils die Position der Bindung der bidentaten Teilliganden L¹, L² bzw. L³ an diese Struktur darstellt, * die Position der Verknüpfung der Einheit der Formel (8) mit der zentralen trivalenten Aryl- bzw. Heteroarylgruppe darstellt und X² die oben genannten Bedeutungen aufweist. Dabei sind bevorzugte Substituenten an der Gruppe der Formel (8) ausgewählt aus den oben beschriebenen Substituenten R.

Die Gruppe der Formel (8) kann einen heteroaromatischen Fünfring oder einen aromatischen oder heteroaromatischen Sechsring darstellen. In einer bevorzugten Ausführungsform der Erfindung enthält die Gruppe der Formel (8) maximal zwei Heteroatome in der Aryl- bzw. Heteroarylgruppe, besonders bevorzugt maximal ein Heteroatom. Dies schließt nicht aus, dass Substituenten, die gegebenenfalls an dieser Gruppe gebunden sind, auch Heteroatome enthalten können. Weiterhin schließt diese Definition nicht aus, dass durch die Ringbildung von Substituenten kondensierte aromatische oder heteroaromatische Strukturen entstehen, wie beispielsweise Naphthalin, Benzimidazol, etc.. Die Gruppe der Formel (8) ist bevorzugt ausgewählt aus Benzol, Pyridin, Pyrimidin, Pyrazin, Pyridazin, Pyrrol, Furan, Thiophen, Pyrazol, Imidazol, Oxazol und Thiazol.

Wenn beide Gruppen X³ in einem Cyclus für Kohlenstoffatome stehen, sind bevorzugte Ausführungsformen der Gruppe der Formel (8) die Strukturen der folgenden Formeln (9) bis (25), 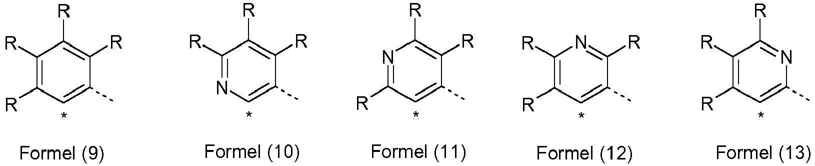 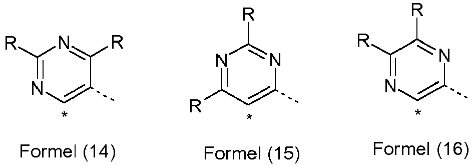 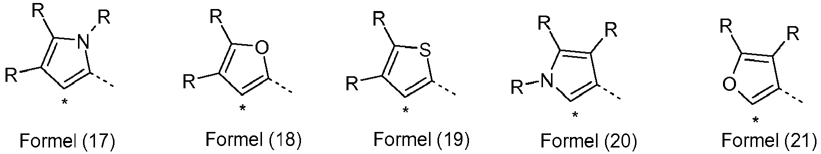 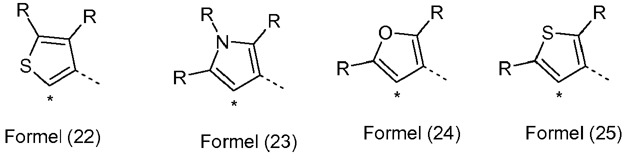 wobei die verwendeten Symbole die oben genannten Bedeutungen aufweisen.

Wenn eine Gruppe X³ in einem Cyclus für ein Kohlenstoffatom und die andere Grupp X³ in demselben Cyclus für ein Stickstoffatom steht, sind bevorzugte Ausführungsformen der Gruppe der Formel (8) die Strukturen der folgenden Formeln (26) bis (33), 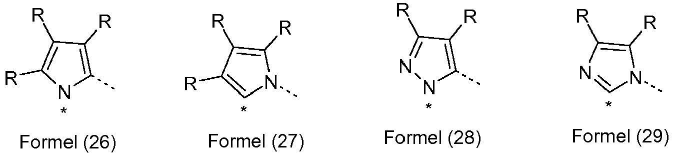 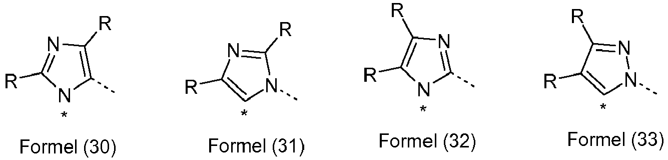 wobei die verwendeten Symbole die oben genannten Bedeutungen aufweisen.

Besonders bevorzugt sind die gegebenenfalls substituierten Sechsring-Aromaten und Sechsring-Heteroaromaten der oben abgebildeten Formeln (9) bis (13). Ganz besonders bevorzugt ist ortho-Phenylen, also eine Gruppe der oben genannten Formel (9).

Dabei können, wie auch oben bei der Beschreibung des Substituenten beschrieben, auch benachbarte Substituenten miteinander ein Ringsystem bilden, so dass kondensierte Strukturen, auch kondensierte Aryl- und Heteroarylgruppen, wie beispielsweise Naphthalin, Chinolin, Benzimidazol, Carbazol, Dibenzofuran oder Dibenzothiophen, entstehen können.

Dabei können die drei Gruppen der Formel (8), die der Gruppe der Formeln (3) bis (7) bzw. Formel (3') vorhanden sind, gleich oder verschieden sein. In einer bevorzugten Ausführungsform der Erfindung sind alle drei Gruppen der Formel (8) gleich und sind auch gleich substituiert.

Besonders bevorzugt sind die Strukturen der Formel (4) bis (7) ausgewählt aus den Strukturen der folgenden Formeln (4b) bis (7b), 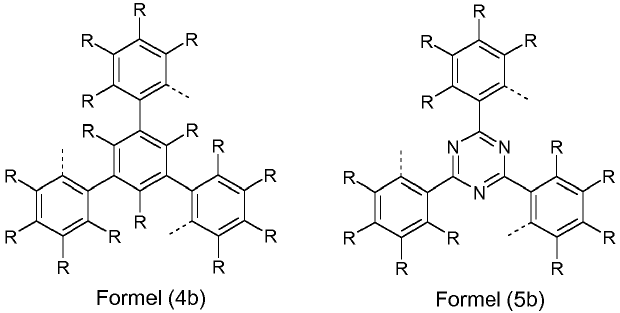 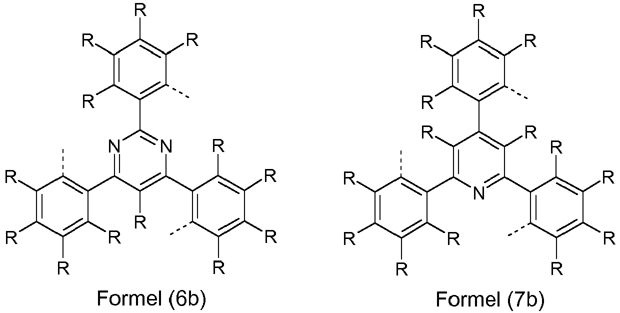 wobei die verwendeten Symbole die oben genannten Bedeutungen aufweisen.

Eine bevorzugte Ausführungsform der Formel (4b) ist die Struktur der folgenden Formel (4b'), 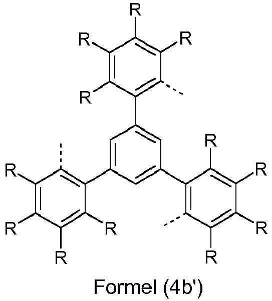 wobei die verwendeten Symbole die oben genannten Bedeutungen aufweisen.

Besonders bevorzugt sind die Gruppen R in den Formeln (3) bis (7) bei jedem Auftreten gleich oder verschieden H, D oder eine Alkylgruppe mit 1 bis 4 C-Atomen. Ganz besonders bevorzugt ist R = H. Ganz besonders bevorzugt sind also die Strukturen der folgenden Formeln (4c) bzw. (5c), 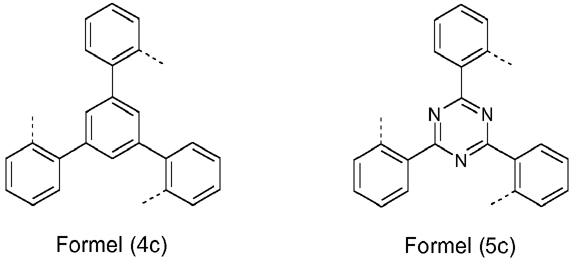 wobei die verwendeten Symbole die oben genannten Bedeutungen aufweisen.

Im Folgenden werden die bidentaten Teilliganden L¹ beschrieben. Wie oben beschrieben, weist der Teilligand L¹ eine Struktur der Formel (2) auf.

In einer bevorzugten Ausführungsform der Erfindung stehen beide Gruppen A für CR₂, besonders bevorzugt für CH₂.

In einer weiteren bevorzugten Ausführungsform der Erfindung steht mindestens eine Gruppe Z für O. Besonders bevorzugt stehen beide Gruppen Z für O.

Bevorzugt handelt es sich somit bei L¹ um einen Teilliganden der folgenden Formel (2a), 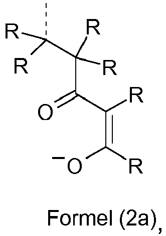 wobei die verwendeten Symbole die oben genannten Bedeutungen aufweisen.

Es kann auch bevorzugt sein, wenn zwei Reste R miteinander einen Ring bilden. Dies ist insbesondere dann bevorzugt, wenn die beiden Reste R an dasselbe Kohlenstoffatom binden, also in einer Gruppe CR₂, oder zwischen zwei Resten R, die innerhalb der Acetylacetonatgruppe direkt benachbart sind. Dabei handelt es sich bei dem Ring bevorzugt um eine aliphatischen Cyclus mit 5 oder 6 Ringatomen oder um einen aromatischen Cyclus. Beispiele für geeignete Ringbildung zwischen den Substituenten zeigen die folgenden Strukturen (L¹-A) bis (L¹-F), 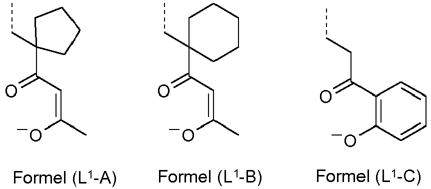 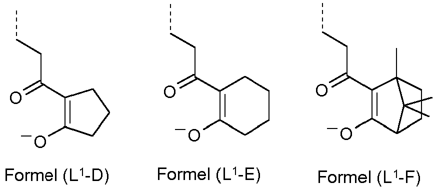 wobei diese Strukturen auch durch einen oder mehrere Reste R¹ substituiert sein können, bevorzugt aber unsubstituiert sind.

Besonders bevorzugt handelt es sich bei L¹ um einen Teilliganden der folgenden Formel (2b), 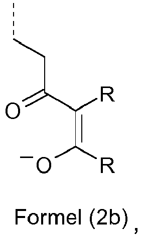 wobei die verwendeten Symbole die oben genannten Bedeutungen aufweisen.

Ganz besonders bevorzugt handelt es sich bei L¹ um einen Teilliganden der folgenden Formel (2c), 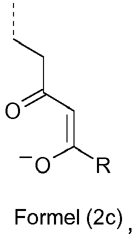 wobei die verwendeten Symbole die oben genannten Bedeutungen aufweisen.

Dabei ist R in Formel (2), (2a), (2b) und (2c) gleich oder verschieden bei jedem Auftreten bevorzugt ausgewählt aus der Gruppe bestehend aus H, D, OR¹, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R¹ substituiert sein kann, bevorzugt aber unsubstituiert ist, oder einem aromatischen oder heteroaromatischen Ringsystem mit 5 bis 24 aromatischen Ringatomen, das jeweils durch einen oder mehrere Reste R¹ substituiert sein kann; dabei können mehrere Reste R auch miteinander ein Ringsystem bilden. Besonders bevorzugte Reste R sind gleich oder verschieden bei jedem Auftreten ausgewählt aus der Gruppe bestehend aus H, D, einer geradkettigen Alkylgruppe mit 1 bis 4 C-Atomen oder einer verzweigten oder cyclischen Alkylgruppe mit 3 bis 6 C-Atomen, die jeweils mit einem oder mehreren Resten R¹ substituiert sein kann, bevorzugt aber unsubstituiert ist, oder einem aromatischen Ringsystem mit 6 bis 12 aromatischen Ringatomen, das jeweils durch einen oder mehrere Reste R¹ substituiert sein kann; dabei können mehrere Reste R auch miteinander ein Ringsystem bilden. Besonders bevorzugte Alkylgruppen sind Methyl, Ethyl, iso-Propyl, isoButyl, tert-Butyl und neo-Pentyl, insbesondere Methyl.

Weiterhin ist R¹ bevorzugt ausgewählt aus der Gruppe bestehend aus H, D, F, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R² substituiert sein kann, bevorzugt aber unsubstituiert ist, oder einem aromatischen oder heteroaromatischen Ringsystem mit 5 bis 24 aromatischen Ringatomen, das jeweils durch einen oder mehrere Reste R² substituiert sein kann; dabei können zwei oder mehrere benachbarte Reste R¹ miteinander ein Ringsystem bilden.

Im Folgenden werden die bidentaten Teilliganden L² und L³ beschrieben. Wie oben beschrieben koordiniert L² an das Iridium über ein Kohlenstoffatom und ein Stickstoffatom oder über zwei Kohlenstoffatome. Weiterhin ist L³ gleich oder verschieden L¹ oder L², stellt also einen Teilliganden der Formel (2) dar oder ist ein Teilligand, der an das Iridium über ein Kohlenstoffatom und ein Stickstoffatom oder über zwei Kohlenstoffatome koordiniert. In einer bevorzugten Ausführungsform der Erfindung sind L² und L³ beides Teilliganden, die jeweils an das Iridium über ein Kohlenstoffatom und ein Stickstoffatom oder über zwei Kohlenstoffatome koordinieren. Die beiden Teilliganden L² und L³ können gleich oder verschieden sein.

Bevorzugt weist mindestens einer der Teilliganden L² und L³ ein Kohlenstoffatom und ein Stickstoffatom als koordinierende Atome auf. Ganz besonders bevorzugt weisen beide Teilliganden L² und L³ ein Kohlenstoffatom und ein Stickstoffatom als koordinierende Atome auf.

Es ist weiterhin bevorzugt, wenn es sich bei dem Metallacyclus, der aus dem Iridium und dem Teilliganden L² bzw. L³ aufgespannt wird, um einen Fünfring handelt. Dies wird im Folgenden schematisch dargestellt: 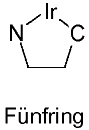 wobei N ein koordinierendes Stickstoffatom und C ein koordinierendes Kohlenstoffatom darstellen und die eingezeichneten Kohlenstoffatome Atome des Teilliganden L² bzw. L³ darstellen.

In einer bevorzugten Ausführungsform der Erfindung steht mindestens einer der Teilliganden L² bzw. L³, besonders bevorzugt beide Teilliganden L² und L³ gleich oder verschieden bei jedem Auftreten für eine Struktur gemäß den folgenden Formeln (L-1) oder (L-2), 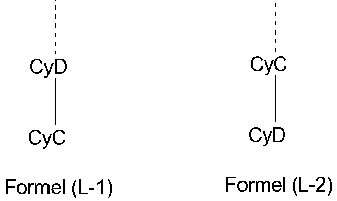 wobei die gestrichelte Bindung die Bindung des Teilliganden an die Brücke der Formel (3) darstellt und für die weiteren verwendeten Symbole gilt:
- CyC: ist gleich oder verschieden bei jedem Auftreten eine substituierte oder unsubstituierte Aryl- oder Heteroarylgruppe mit 5 bis 14 aromatischen Ringatomen, welche jeweils über ein Kohlenstoffatom an das Metall koordiniert und welche über eine kovalente Bindung mit CyD verbunden ist;
- CyD: ist gleich oder verschieden bei jedem Auftreten eine substituierte oder unsubstituierte Heteroarylgruppe mit 5 bis 14 aromatischen Ringatomen, welche über ein Stickstoffatom oder über ein Carben-Kohlenstoffatom an das Metall koordiniert und welche über eine kovalente Bindung mit CyC verbunden ist;
dabei können mehrere der optionalen Substituenten miteinander ein Ringsystem bilden; die optionalen Reste sind bevorzugt ausgewählt aus den oben genannten Resten R.

Dabei koordiniert CyD bevorzugt über ein neutrales Stickstoffatom oder über ein Carben-Kohlenstoffatom. Weiterhin koordiniert CyC über anionische Kohlenstoffatome.

Wenn mehrere der Substituenten, insbesondere mehrere Reste R, miteinander ein Ringsystem bilden, so ist die Bildung eines Ringsystems aus Substituenten, die an direkt benachbarten Kohlenstoffatomen gebunden sind, möglich. Weiterhin ist es auch möglich, dass die Substituenten an CyC und CyD miteinander einen Ring bilden, wodurch CyC und CyD auch zusammen eine einzige kondensierte Aryl- bzw. Heteroarylgruppe als bidentaten Liganden bilden können.

Dabei können beide Teilliganden L² und L³ eine Struktur der Formel (L-1) aufweisen, oder beide Teilliganden L² und L³ können eine Struktur der Formel (L-2) aufweisen, oder einer der Teilliganden L² und L³ weist eine Struktur der Formel (L-1), und der andere der Teillliganden weist eine Struktur der Formel (L-2) auf. In einer bevorzugten Ausführungsform der Erfindung weist einer der Teilliganden L² und L³ eine Struktur der Formel (L-1) auf, und der andere der Teilliganden L² und L³ weist eine Struktur der Formel (L-2) auf.

In einer bevorzugten Ausführungsform der vorliegenden Erfindung ist CyC eine Aryl- oder Heteroarylgruppe mit 6 bis 13 aromatischen Ringatomen, besonders bevorzugt mit 6 bis 10 aromatischen Ringatomen, ganz besonders bevorzugt mit 6 aromatischen Ringatomen, welche über ein Kohlenstoffatom an das Metall koordiniert, welche mit einem oder mehreren Resten R substituiert sein kann und welche über eine kovalente Bindung mit CyD verbunden ist.

Bevorzugte Ausführungsformen der Gruppe CyC sind die Strukturen der folgenden Formeln (CyC-1) bis (CyC-19), wobei die Gruppe CyC jeweils an der durch # gekennzeichneten Position an CyD bindet und an der durch * gekennzeichneten Position an das Iridium koordiniert, 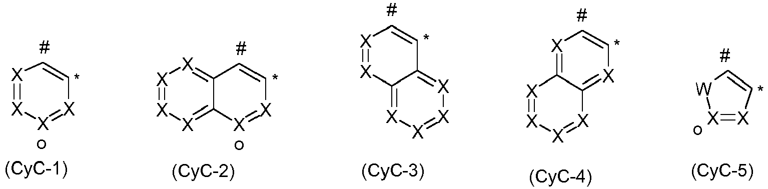 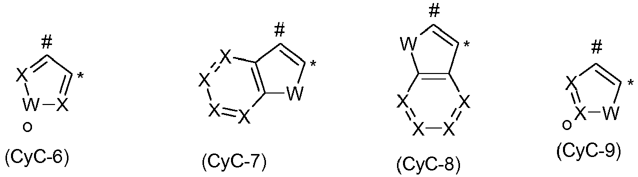 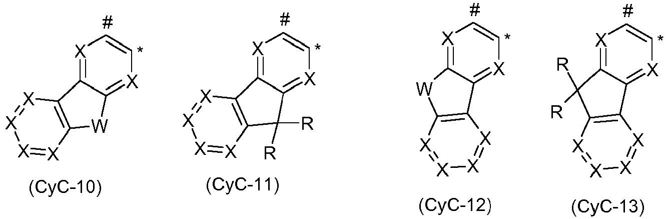 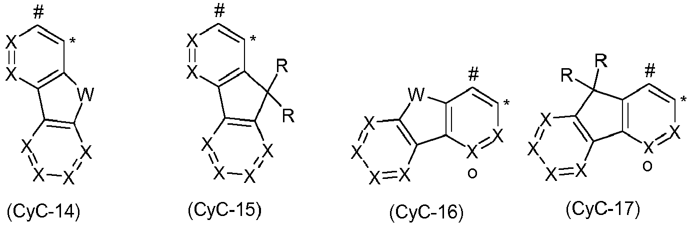 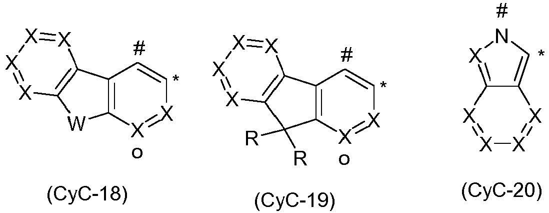 wobei R die oben genannten Bedeutungen aufweist und für die weiteren verwendeten Symbole gilt:
- X: ist bei jedem Auftreten gleich oder verschieden CR oder N mit der Maßgabe, dass maximal zwei Symbole X pro Cyclus für N stehen;
- W: ist bei jedem Auftreten gleich oder verschieden NR, O oder S;
mit der Maßgabe, dass, wenn die Brücke der Formel (3) an CyC gebunden ist, ein Symbol X für C steht und die Brücke der Formel (3) an dieses Kohlenstoffatom gebunden ist. Wenn die Gruppe CyC an die Brücke der Formel (3) gebunden ist, so erfolgt die Bindung bevorzugt über die mit "o" markierte Position der oben abgebildeten Formeln, so dass dann bevorzugt das mit "o" markierte Symbol X für C steht. Die oben abgebildeten Strukturen, die kein mit "o" markiertes Symbol X enthalten, sind bevorzugt nicht direkt an die Brücke der Formel (3) gebunden, da eine solche Bindung an die Brücke aus sterischen Gründen nicht vorteilhaft ist.

Bevorzugt stehen insgesamt maximal zwei Symbole X in CyC für N, besonders bevorzugt steht maximal ein Symbol X in CyC für N, ganz besonders bevorzugt stehen alle Symbole X für CR, mit der Maßgabe, dass, wenn die Brücke der Formel (3) an CyC gebunden ist, ein Symbol X für C steht und die Brücke der Formel (3) an dieses Kohlenstoffatom gebunden ist.

Besonders bevorzugte Gruppen CyC sind die Gruppen der folgenden Formeln (CyC-1a) bis (CyC-20a), 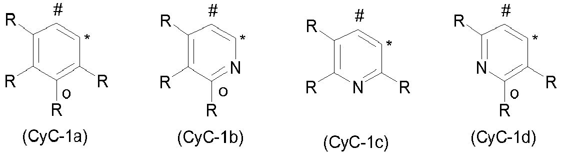 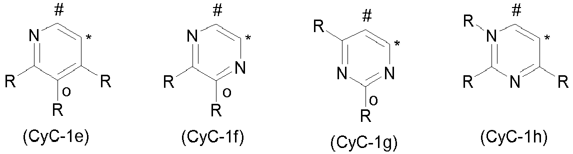 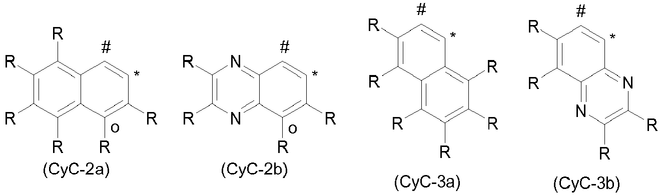 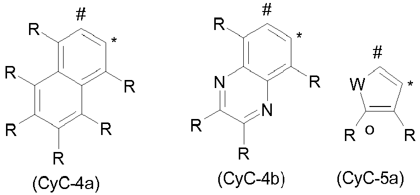 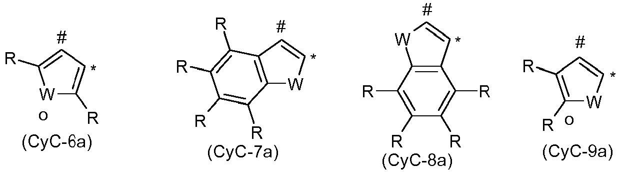 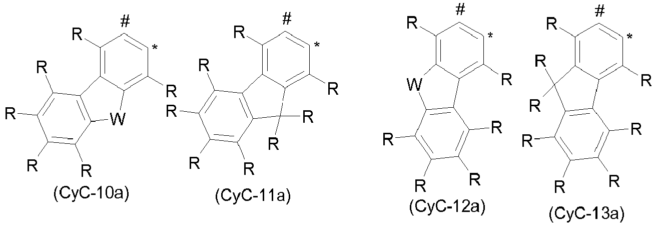 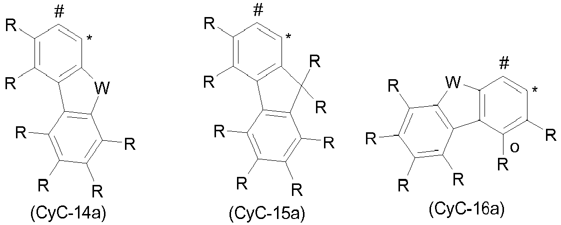 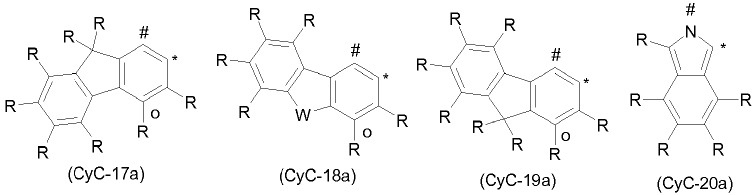 wobei die verwendeten Symbole die oben genannten Bedeutungen aufweisen und, wenn die Brücke der Formel (3) an CyC gebunden ist, ein Rest R nicht vorhanden ist und die Brücke der Formel (3) an das entsprechende Kohlenstoffatom gebunden ist. Wenn die Gruppe CyC an die Brücke der Formel (3) gebunden ist, so erfolgt die Bindung bevorzugt über die mit "o" markierte Position der oben abgebildeten Formeln, so dass dann bevorzugt in dieser Position der Rest R nicht vorhanden ist. Die oben abgebildeten Strukturen, die kein mit "o" markiertes Kohlenstoffatom enthalten, sind bevorzugt nicht direkt an die Brücke der Formel (3) gebunden.

Bevorzugte Gruppen unter den Gruppen (CyC-1) bis (CyC-19) sind die Gruppen (CyC-1), (CyC-3), (CyC-8), (CyC-10), (CyC-12), (CyC-13) und (CyC-16), und besonders bevorzugt sind die Gruppen (CyC-1a), (CyC-3a), (CyC-8a), (CyC-10a), (CyC-12a), (CyC-13a) und (CyC-16a).

In einer weiteren bevorzugten Ausführungsform der Erfindung ist CyD eine Heteroarylgruppe 5 bis 13 aromatischen Ringatomen, besonders bevorzugt mit 6 bis 10 aromatischen Ringatomen, welche über ein neutrales Stickstoffatom oder über ein Carben-Kohlenstoffatom an das Metall koordiniert und welche mit einem oder mehreren Resten R substituiert sein kann und welche über eine kovalente Bindung mit CyC verbunden ist.

Bevorzugte Ausführungsformen der Gruppe CyD sind die Strukturen der folgenden Formeln (CyD-1) bis (CyD-12), wobei die Gruppe CyD jeweils an der durch # gekennzeichneten Position an CyC bindet und an der durch * gekennzeichneten Position an das Iridium koordiniert, 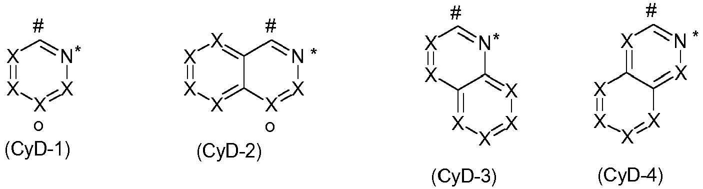 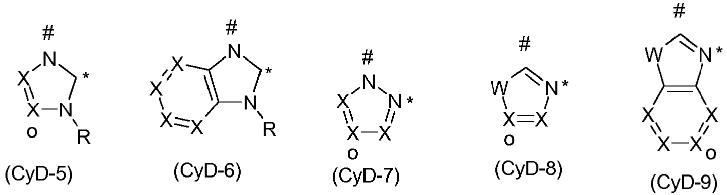 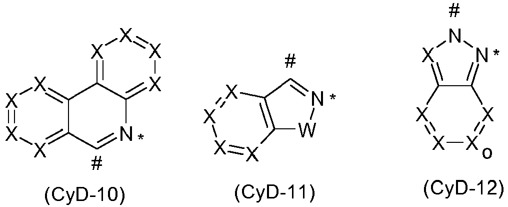 wobei X, W und R die oben genannten Bedeutungen aufweisen, mit der Maßgabe, dass, wenn die Brücke der Formel (3) an CyD gebunden ist, ein Symbol X für C steht und die Brücke der Formel (3) an dieses Kohlenstoffatom gebunden ist. Wenn die Gruppe CyD an die Brücke der Formel (3) gebunden ist, so erfolgt die Bindung bevorzugt über die mit "o" markierte Position der oben abgebildeten Formeln, so dass dann bevorzugt das mit "o" markierte Symbol X für C steht. Die oben abgebildeten Strukturen, die kein mit "o" markiertes Symbol X enthalten, sind bevorzugt nicht direkt an die Brücke der Formel (3) gebunden, da eine solche Bindung an die Brücke aus sterischen Gründen nicht vorteilhaft ist.

Dabei koordinieren die Gruppen (CyD-1) bis (CyD-4) und (CyD-7) bis (CyD-12) über ein neutrales Stickstoffatom und (CyD-5) und (CyD-6) über ein Carben-Kohlenstoffatom an das Metall.

Bevorzugt stehen insgesamt maximal zwei Symbole X in CyD für N, besonders bevorzugt steht maximal ein Symbol X in CyD für N, insbesondere bevorzugt stehen alle Symbole X für CR, mit der Maßgabe, dass, wenn die Brücke der Formel (3) an CyD gebunden ist, ein Symbol X für C steht und die Brücke der Formel (3) an dieses Kohlenstoffatom gebunden ist.

Besonders bevorzugte Gruppen CyD sind die Gruppen der folgenden Formeln (CyD-1a) bis (CyD-12b), 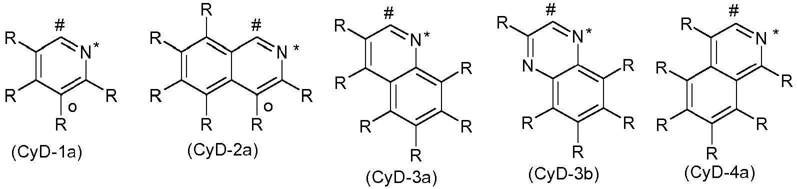 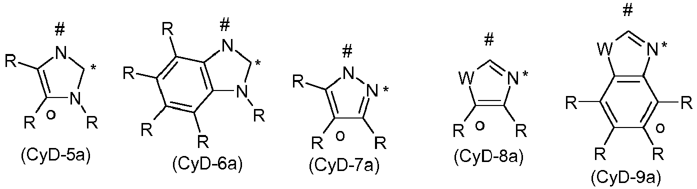 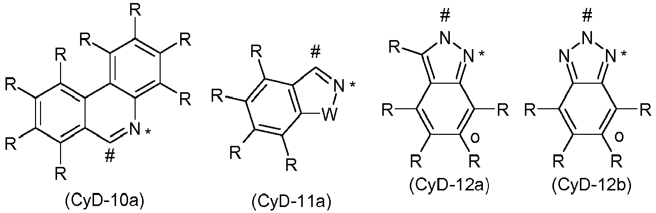 wobei die verwendeten Symbole die oben genannten Bedeutungen aufweisen und, wenn die Brücke der Formel (3) an CyD gebunden ist, ein Rest R nicht vorhanden ist und die Brücke der Formel (3) an das entsprechende Kohlenstoffatom gebunden ist. Wenn die Gruppe CyD an die Brücke der Formel (3) gebunden ist, so erfolgt die Bindung bevorzugt über die mit "o" markierte Position der oben abgebildeten Formeln, so dass dann bevorzugt in dieser Position der Rest R nicht vorhanden ist. Die oben abgebildeten Strukturen, die kein mit "o" markiertes Kohlenstoffatom enthalten, sind bevorzugt nicht direkt an die Brücke der Formel gebunden.

Bevorzugte Gruppen unter den Gruppen (CyD-1) bis (CyD-12) sind die Gruppen (CyD-1), (CyD-2), (CyD-3), (CyD-4), (CyD-5) und (CyD-6), insbesondere (CyD-1), (CyD-2) und (CyD-3), und besonders bevorzugt sind die Gruppen (CyD-1a), (CyD-2a), (CyD-3a), (CyD-4a), (CyD-5a) und (CyD-6a), insbesondere (CyD-1a), (CyD-2a) und (CyD-3a).

In einer bevorzugten Ausführungsform der vorliegenden Erfindung ist CyC eine Aryl- oder Heteroarylgruppe mit 6 bis 13 aromatischen Ringatomen, und gleichzeitig ist CyD eine Heteroarylgruppe mit 5 bis 13 aromatischen Ringatomen. Besonders bevorzugt ist CyC eine Aryl- oder Heteroarylgruppe mit 6 bis 10 aromatischen Ringatomen, und gleichzeitig ist CyD eine Heteroarylgruppe mit 5 bis 10 aromatischen Ringatomen. Ganz besonders bevorzugt ist CyC eine Aryl- oder Heteroarylgruppe mit 6 aromatischen Ringatomen und CyD eine Heteroarylgruppe mit 6 bis 10 aromatischen Ringatomen. Dabei können CyC und CyD mit einem oder mehreren Resten R substituiert sein.

Die oben genannten bevorzugten Gruppen (CyC-1) bis (CyC-20) und (CyD-1) bis (CyD-12) können beliebig miteinander kombiniert werden, sofern mindestens eine der Gruppen CyC bzw. CyD eine geeignete Anknüpfungsstelle an die Brücke der Formel (3) aufweist, wobei geeignete Anknüpfungsstellen in den oben genannten Formeln mit "o" gekennzeichnet sind.

Insbesondere bevorzugt ist es, wenn die oben als besonders bevorzugt genannten Gruppen CyC und CyD, also die Gruppen der Formeln (CyC-1a) bis (CyC-20a) und die Gruppen der Formeln (CyD1-a) bis (CyD-14b) miteinander kombiniert werden, sofern mindestens eine der bevorzugten Gruppen CyC bzw. CyD eine geeignete Anknüpfungsstelle an die Brücke der Formel (3) aufweist, wobei geeignete Anknüpfungsstellen in den oben genannten Formeln mit "o" gekennzeichnet sind. Kombinationen, in denen weder CyC noch CyD eine solche geeignete Anknüpfungsstelle für die Brücke der Formel (3) aufweist, sind daher nicht bevorzugt.

Ganz besonders bevorzugt ist es, wenn eine der Gruppen (CyC-1), (CyC-3), (CyC-8), (CyC-10), (CyC-12), (CyC-13) und (CyC-16), und insbesondere die Gruppen (CyC-1a), (CyC-3a), (CyC-8a), (CyC-10a), (CyC-12a), (CyC-13a) und (CyC-16a), mit einer der Gruppen (CyD-1), (CyD-2) und (CyD-3), und insbesondere mit einer der Gruppen (CyD-1a), (CyD-2a) und (CyD-3a), kombiniert wird.

Bevorzugte Teilliganden (L-1) sind die Strukturen der Formeln (L-1-1) und (L-1-2), und bevorzugte Teilliganden (L-2) sind die Strukturen der Formeln (L-2-1) bis (L-2-4), 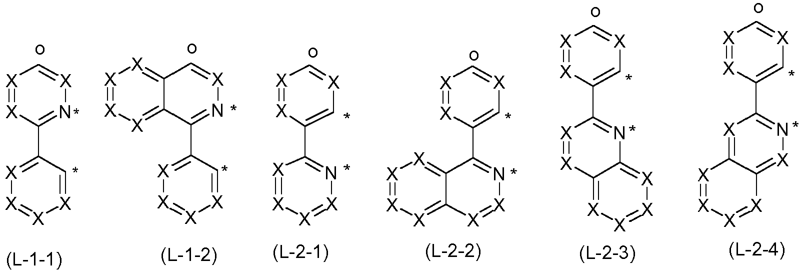 wobei die verwendeten Symbole die oben genannten Bedeutungen aufweisen und "o" die Position der Bindung an die Brücke der Formel (3) darstellt.

Besonders bevorzugte Teilliganden (L-1) sind die Strukturen der Formeln (L-1-1a) und (L-1-2b), und besonders bevorzugte Teilliganden (L-2) sind die Strukturen der Formeln (L-2-1a) bis (L-2-4a), 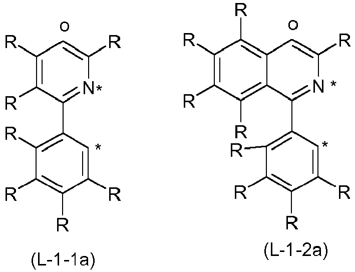 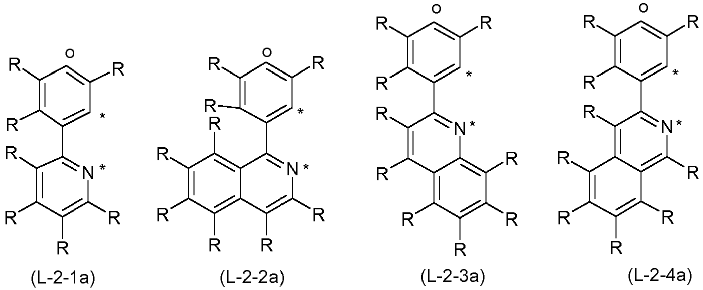 wobei die verwendeten Symbole die oben genannten Bedeutungen aufweisen und "o" die Position der Bindung an die Brücke der Formel (3) darstellt.

Wenn zwei Reste R, von denen einer an CyC und der andere an CyD gebunden sind, miteinander ein aromatisches Ringsystem bilden, können sich überbrückte Teilliganden und beispielsweise auch Teilliganden ergeben, die insgesamt eine einzige größere Heteroarylgruppe darstellen, wie beispielsweise Benzo[h]chinolin, etc.. Die Ringbildung zwischen den Substituenten an CyC und CyD erfolgt dabei bevorzugt durch eine Gruppe gemäß einer der folgenden Formeln (34) bis (43), 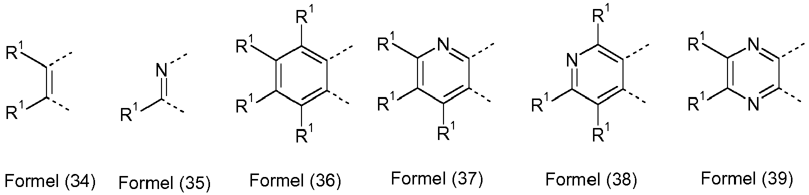 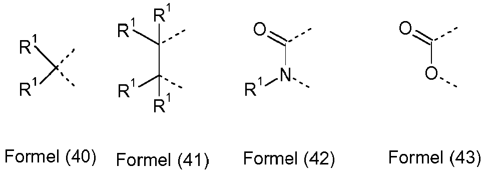 wobei R¹ die oben genannten Bedeutungen aufweist und die gestrichelten Bindungen die Bindungen an CyC bzw. CyD andeuten. Dabei können die unsymmetrischen der oben genannten Gruppen in jeder der beiden Möglichkeiten eingebaut werden, beispielsweise kann bei der Gruppe der Formel (43) das Sauerstoffatom an die Gruppe CyC und die Carbonylgruppe an die Gruppe CyD binden, oder das Sauerstoffatom kann an die Gruppe CyD und die Carbonylgruppe an die Gruppe CyC binden.

Dabei ist die Gruppe der Formel (40) besonders dann bevorzugt, wenn sich dadurch die Ringbildung zu einem Sechsring ergibt, wie beispielsweise unten durch die Formeln (L-21) und (L-22) dargestellt.

Bevorzugte Liganden, die durch Ringbildung zweier Reste R an den unterschiedlichen Cyclen entstehen, sind die im Folgenden aufgeführten Strukturen der Formeln (L-3) bis (L-30), 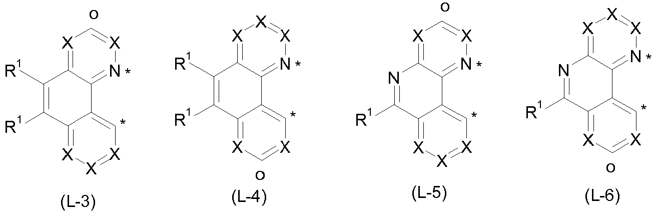 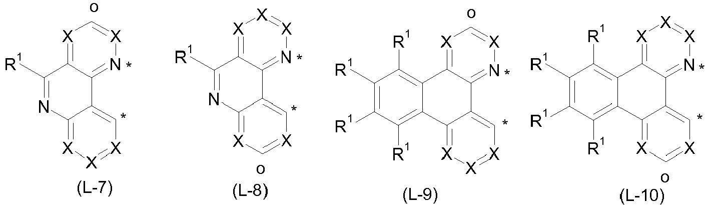 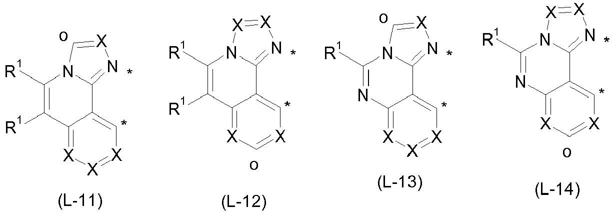 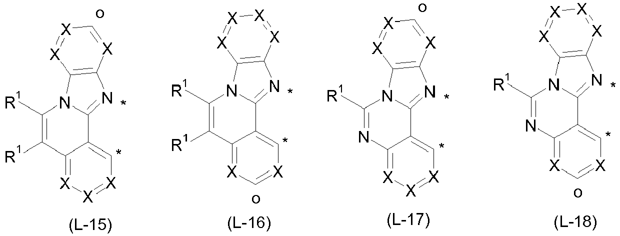 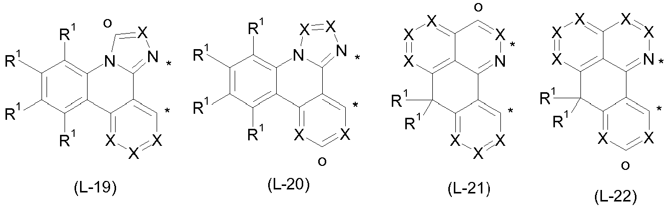 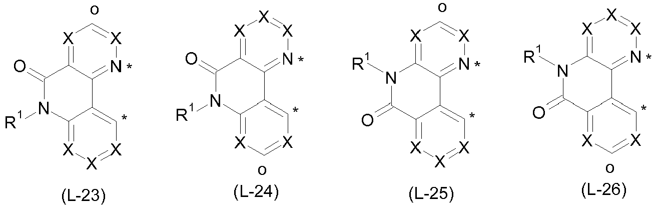 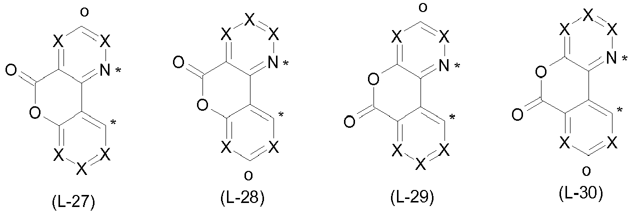 wobei die verwendeten Symbole die oben genannten Bedeutungen aufweisen und "o" die Position angibt, an denen dieser Teilligand mit der Gruppe der Formel (3) verknüpft ist.

In einer bevorzugten Ausführungsform der Teilliganden der Formeln (L-5) bis (L-32) steht insgesamt ein Symbol X für N und die anderen Symbole X stehen für CR, oder alle Symbole X stehen für CR.

In einer weiteren Ausführungsform der Erfindung ist es bevorzugt, falls in den Gruppen (CyC-1) bis (CyC-20) oder (CyD-1) bis (CyD-14) oder in den Teilliganden (L-5) bis (L-32) eines der Atome X für N steht, wenn benachbart zu diesem Stickstoffatom eine Gruppe R als Substituent gebunden ist, welche ungleich Wasserstoff oder Deuterium ist. Dies gilt analog für die bevorzugten Strukturen (CyC-1a) bis (CyC-20a) oder (CyD-1a) bis (CyD-14b), in denen bevorzugt benachbart zu einem nicht koordinierenden Stickstoffatom eine Gruppe R als Substituent gebunden ist, welche ungleich Wasserstoff oder Deuterium ist.

Dabei ist dieser Substituent R bevorzugt eine Gruppe, ausgewählt aus CF₃, OCF₃, Alkylgruppen mit 1 bis 10 C-Atomen, insbesondere verzweigten oder cyclischen Alkylgruppen mit 3 bis 10 C-Atomen, OR¹, wobei R¹ für eine Alkylgruppe mit 1 bis 10 C-Atomen, insbesondere eine verzweigte oder cyclische Alkylgruppe mit 3 bis 10 C-Atomen steht, einer Dialkylaminogruppe mit 2 bis 10 C-Atomen, aromatischen bzw. heteroaromatischen Ringsystemen oder Aralkyl- bzw. Heteroaralkylgruppen. Es handelt sich bei diesen Gruppen um sterisch anspruchsvolle Gruppen. Weiterhin bevorzugt kann dieser Rest R auch mit einem benachbarten Rest R einen Cyclus bilden.

Ein weiterer geeigneter bidentater Teilligand für Metallkomplexe, in denen das Metall ein Übergangsmetall ist, ist ein Teilligand der folgenden Formel (L-31) oder (L-32), 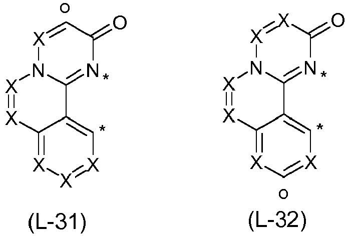 wobei R die oben genannten Bedeutungen aufweist, * die Position der Koordination an das Metall darstellt, "o" die Position der Verknüpfung des Teilliganden mit der Gruppe der Formel (3) darstellt und für die weiteren verwendeten Symbole gilt:
- X: ist bei jedem Auftreten gleich oder verschieden CR oder N mit der Maßgabe, dass maximal ein Symbol X pro Cyclus für N steht.

Wenn zwei Reste R, die in den Teilliganden (L-31) bzw. (L-32) an benachbarten Kohlenstoffatomen gebunden sind, miteinander einen aromatischen Cyclus bilden, so ist dieser zusammen mit den beiden benachbarten Kohlenstoffatomen bevorzugt eine Struktur der Formel (44), 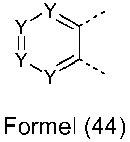 wobei die gestrichelten Bindungen die Verknüpfung dieser Gruppe im Teilliganden symbolisieren und Y gleich oder verschieden bei jedem Auftreten für CR¹ oder N steht und bevorzugt maximal ein Symbol Y für N steht.

In einer bevorzugten Ausführungsform des Teilliganden (L-31) bzw. (L-32) ist maximal eine Gruppe der Formel (44) vorhanden. Es handelt sich also bevorzugt um Teilliganden der folgenden Formeln (L-33) bis (L-38), 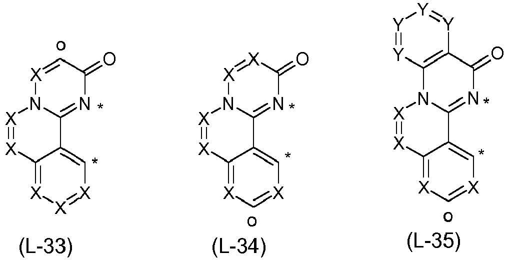 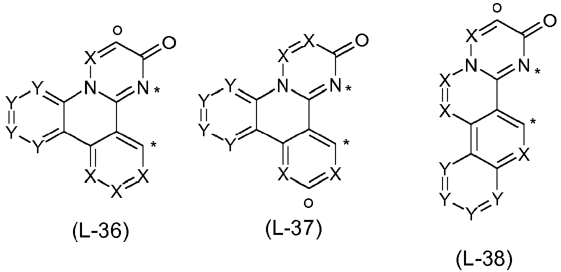 wobei X bei jedem Auftreten gleich oder verschieden für CR oder N steht, jedoch die Reste R nicht miteinander ein aromatisches oder heteroaromatisches Ringsystem bilden und die weiteren Symbole die oben genannten Bedeutungen aufweisen.

In einer bevorzugten Ausführungsform der Erfindung stehen im Teilliganden der Formel (L-31) bis (L-38) insgesamt 0, 1 oder 2 der Symbole X und, falls vorhanden, Y für N. Besonders bevorzugt stehen insgesamt 0 oder 1 der Symbole X und, falls vorhanden, Y für N.

Bevorzugte Ausführungsformen der Formeln (L-33) bis (L-38) sind die Strukturen der folgenden Formeln (L-33a) bis (L-38f), 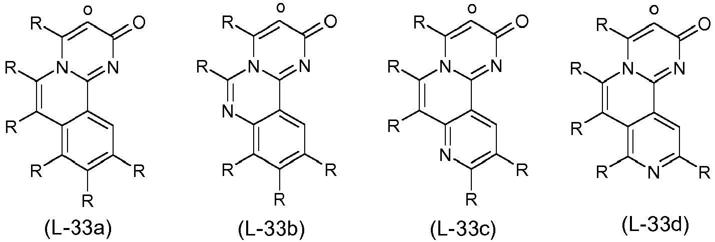 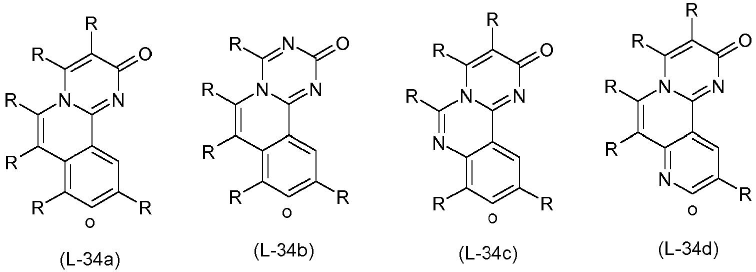 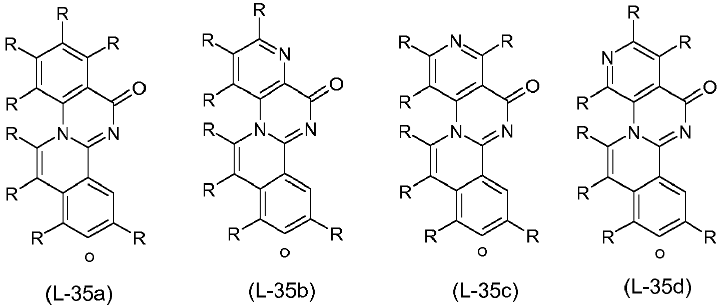 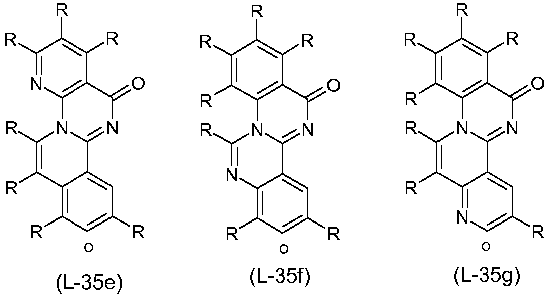 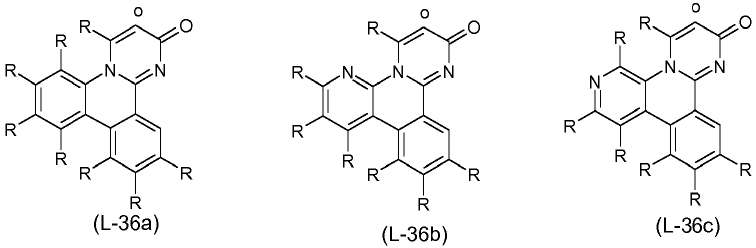 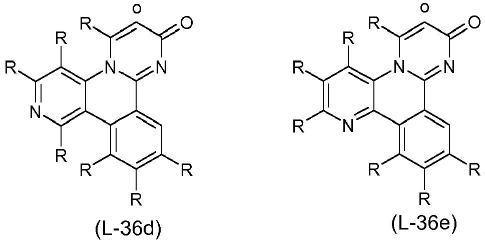 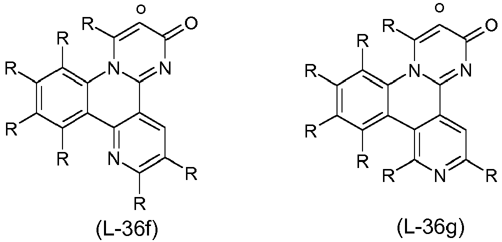 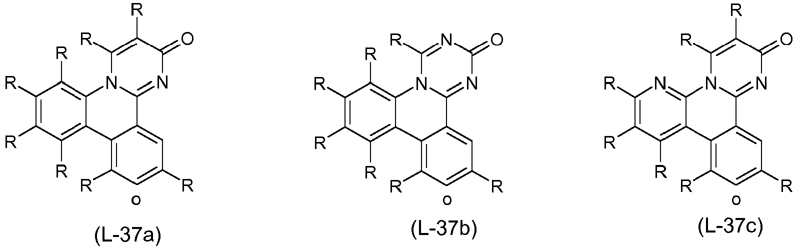 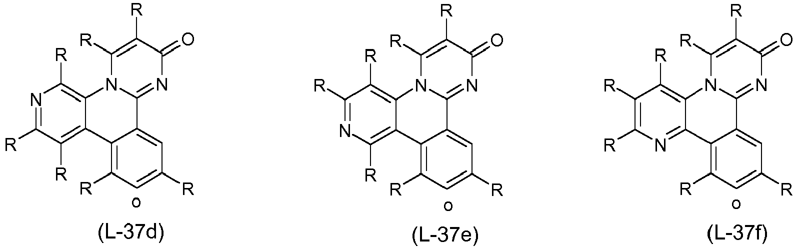 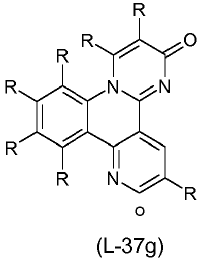 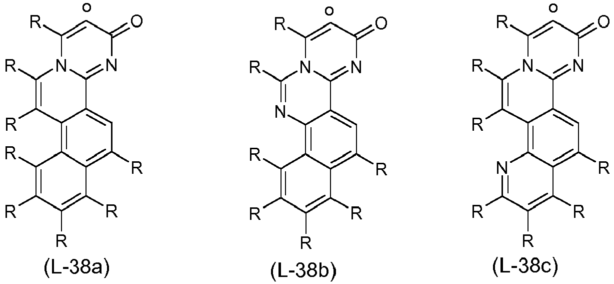 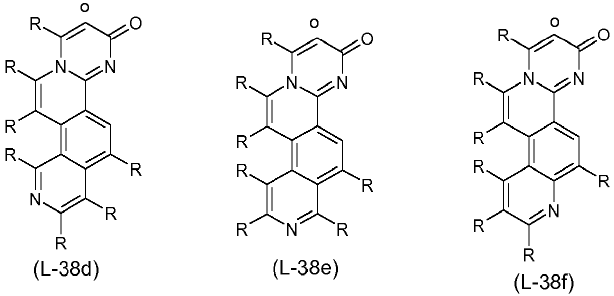 wobei die verwendeten Symbole die oben genannten Bedeutungen aufweisen und "o" die Position der Verknüpfung mit der Gruppe der Formel (3) anzeigt.

In einer bevorzugten Ausführungsform der Erfindung steht die Gruppe X, die in ortho-Position zur Koordination an das Metall vorliegt, für CR. Dabei ist in dieser Rest R, der in ortho-Position zur Koordination an das Metall gebunden ist, bevorzugt ausgewählt aus der Gruppe bestehend aus H, D, F und Methyl.

In einer weiteren Ausführungsform der Erfindung ist es bevorzugt, falls eines der Atome X oder, wenn vorhanden, Y für N steht, wenn benachbart zu diesem Stickstoffatom eine Gruppe R als Substituent gebunden ist, welche ungleich Wasserstoff oder Deuterium ist.

Dabei ist dieser Substituent R bevorzugt eine Gruppe, ausgewählt aus CF₃, OCF₃, Alkylgruppen mit 1 bis 10 C-Atomen, insbesondere verzweigten oder cyclischen Alkylgruppen mit 3 bis 10 C-Atomen, OR¹, wobei R¹ für eine Alkylgruppe mit 1 bis 10 C-Atomen, insbesondere eine verzweigte oder cyclische Alkylgruppe mit 3 bis 10 C-Atomen steht, einer Dialkylaminogruppe mit 2 bis 10 C-Atomen, aromatischen bzw. heteroaromatischen Ringsystemen oder Aralkyl- bzw. Heteroaralkylgruppen. Es handelt sich bei diesen Gruppen um sterisch anspruchsvolle Gruppen. Weiterhin bevorzugt kann dieser Rest R auch mit einem benachbarten Rest R einen Cyclus bilden.

Im Folgenden werden bevorzugte Substituenten beschrieben, wie sie an den oben beschriebenen Teilliganden L² und L³, aber auch an der bivalenten Arylen- oder Heteroarylengruppe in der Struktur der Formeln (3) bis (7), also in der Struktur der Formel (8), vorliegen können.

In einer bevorzugten Ausführungsform der Erfindung enthält der erfindungsgemäße Metallkomplex zwei Substituenten R oder zwei Substituenten R¹, die an benachbarte Kohlenstoffatome gebunden sind und die miteinander einen aliphatischen Ring gemäß einer der nachfolgend beschriebenen Formeln bilden. Dabei können die beiden Substituenten R, die diesen aliphatischen Ring bilden, an der Brücke der Formel (3) vorliegen und/oder an einem oder mehreren der bidentaten Teilliganden vorliegen. Der aliphatische Ring, der durch die Ringbildung von zwei Substituenten R miteinander oder von zwei Substituenten R¹ miteinander gebildet wird, wird bevorzugt durch eine der folgenden Formeln (45) bis (51) beschrieben, 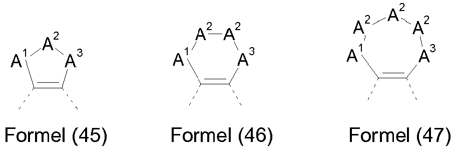 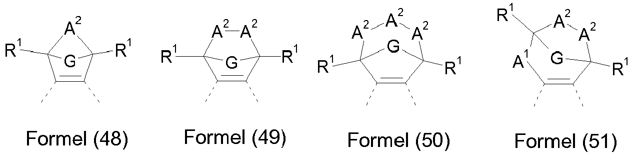 wobei R¹ und R² die oben genannten Bedeutungen aufweisen, die gestrichelten Bindungen die Verknüpfung der beiden Kohlenstoffatome im Liganden andeuten und weiterhin gilt:
- A¹, A³: ist gleich oder verschieden bei jedem Auftreten C(R³)₂, O, S, NR³ oder C(=O);
- A²: ist C(R¹)₂, O, S, NR³ oder C(=O);
- G: ist eine Alkylengruppe mit 1, 2 oder 3 C-Atomen, welche mit einem oder mehreren Resten R² substituiert sein kann, -CR²=CR²- oder eine ortho-verknüpfte Arylen- oder Heteroarylengruppe mit 5 bis 14 aromatischen Ringatomen, welche durch einen oder mehrere Reste R² substituiert sein kann;
- R³: ist gleich oder verschieden bei jedem Auftreten H, F, eine geradkettige Alkyl- oder Alkoxygruppe mit 1 bis 10 C-Atomen, eine verzweigte oder cyclische Alkyl- oder Alkoxygruppe mit 3 bis 10 C-Atomen, wobei die Alkyl- oder Alkoxygruppe jeweils mit einem oder mehreren Resten R² substituiert sein kann, wobei eine oder mehrere nicht benachbarte CH₂-Gruppen durch R²C=CR², C=C, Si(R²)₂, C=O, NR², O, S oder CONR² ersetzt sein können, oder ein aromatisches oder heteroaromatisches Ringsystem mit 5 bis 24 aromatischen Ringatomen, das jeweils durch einen oder mehrere Reste R² substituiert sein kann, oder eine Aryloxy- oder Heteroaryloxygruppe mit 5 bis 24 aromatischen Ringatomen, die durch einen oder mehrere Reste R² substituiert sein kann; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem benachbarten Rest R oder R¹ ein aliphatisches Ringsystem bilden;
mit der Maßgabe, dass in diesen Gruppen nicht zwei Heteroatome direkt aneinander gebunden sind und nicht zwei Gruppen C=O direkt aneinander gebunden sind.

In den oben abgebildeten Strukturen der Formeln (45) bis (51) sowie den weiteren als bevorzugt genannten Ausführungsformen dieser Strukturen wird formal eine Doppelbindung zwischen den zwei Kohlenstoffatomen abgebildet. Dies stellt eine Vereinfachung der chemischen Struktur dar, wenn diese beiden Kohlenstoffatome in ein aromatisches oder heteroaromatisches System eingebunden sind und somit die Bindung zwischen diesen beiden Kohlenstoffatomen formal zwischen dem Bindungsgrad einer Einfachbindung und dem einer Doppelbindung liegt. Das Einzeichnen der formalen Doppelbindung ist somit nicht limitierend für die Struktur auszulegen, sondern es ist für den Fachmann offensichtlich, dass es sich hier um eine aromatische Bindung handelt.

Wenn benachbarte Reste in den erfindungsgemäßen Strukturen ein aliphatisches Ringsystem bilden, dann ist es bevorzugt, wenn dieses keine aziden benzylischen Protonen aufweist. Unter benzylischen Protonen werden Protonen verstanden, die an ein Kohlenstoffatom binden, welches direkt an den Liganden gebunden sind. Dies kann dadurch erreicht werden, dass die Kohlenstoffatome des aliphatischen Ringsystems, die direkt an eine Aryl- oder Heteroarylgruppe binden, vollständig substituiert sind und keine Wasserstoffatome gebunden enthalten. So wird die Abwesenheit von aziden benzylischen Protonen in den Formeln (45) bis (47) dadurch erreicht, dass A¹ und A³, wenn diese für C(R³)₂ stehen, so definiert sind, dass R³ ungleich Wasserstoff ist. Dies kann weiterhin auch dadurch erreicht werden, dass die Kohlenstoffatome des aliphatischen Ringsystems, die direkt an eine Aryl- oder Heteroarylgruppe binden, die Brückenköpfe einer bi- oder polycyclischen Struktur sind. Die an Brückenkopfkohlenstoffatome gebundenen Protonen sind aufgrund der räumlichen Struktur des Bi- oder Polycyclus wesentlich weniger azide als benzylische Protonen an Kohlenstoffatomen, die nicht in einer bi- oder polycyclischen Struktur gebunden sind, und werden im Sinne der vorliegenden Erfindung als nicht-azide Protonen angesehen. So wird die Abwesenheit von aziden benzylischen Protonen ist in Formeln (48) bis (51) dadurch erreicht, dass es sich dabei um eine bicyclische Struktur handelt, wodurch R¹, wenn es für H steht, deutlich weniger azide als benzylische Protonen, da das korrespondierende Anion der bicyclischen Struktur nicht mesomeriestabilisiert ist. Auch wenn R¹ in Formeln (48) bis (51) für H steht, handelt es sich dabei daher um ein nicht-azides Proton im Sinne der vorliegenden Anmeldung.

In einer bevorzugten Ausführungsform der Erfindung ist R³ ungleich H. In einer bevorzugten Ausführungsform der Struktur gemäß Formel (45) bis (51) steht maximal eine der Gruppen A¹, A² und A³ für ein Heteroatom, insbesondere für O oder NR³, und die anderen Gruppen stehen für C(R³)₂ bzw. C(R¹)₂ oder A¹ und A³ stehen gleich oder verschieden bei jedem Auftreten für O oder NR³ und A² steht für C(R¹)₂. In einer besonders bevorzugten Ausführungsform der Erfindung stehen A¹ und A³ gleich oder verschieden bei jedem Auftreten für C(R³)₂ und A² steht für C(R¹)₂ und besonders bevorzugt für C(R³)₂ oder CH₂.

Bevorzugte Ausführungsformen der Formel (45) sind somit die Strukturen der Formel (45-A), (45-B), (45-C) und (45-D), und eine besonders bevorzugte Ausführungsform der Formel (45-A) sind die Strukturen der Formel (45-E) und (45-F), 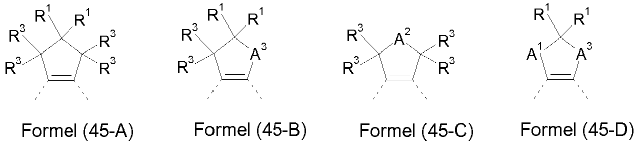 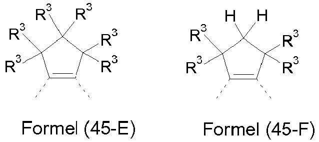 wobei R¹ und R³ die oben genannten Bedeutungen aufweisen und A¹, A² und A³ gleich oder verschieden bei jedem Auftreten für O oder NR³ steht.

Bevorzugte Ausführungsformen der Formel (46) sind die Strukturen der folgenden Formeln (46-A) bis (46-F), 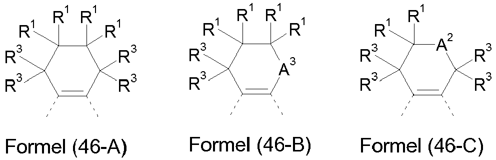 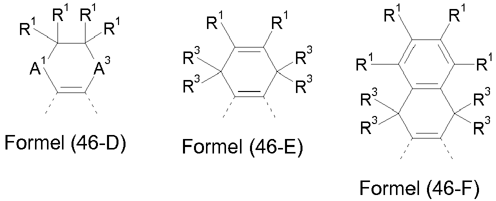 wobei R¹ und R³ die oben genannten Bedeutungen aufweisen und A¹, A² und A³ gleich oder verschieden bei jedem Auftreten für O oder NR³ steht.

Bevorzugte Ausführungsformen der Formel (47) sind die Strukturen der folgenden Formeln (47-A) bis (47-E), 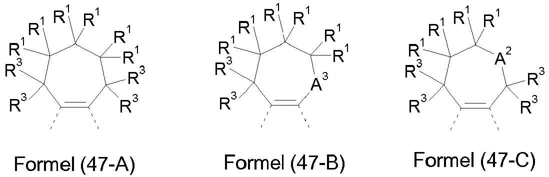 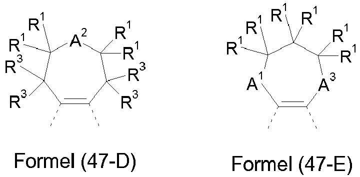 wobei R¹ und R³ die oben genannten Bedeutungen aufweisen und A¹, A² und A³ gleich oder verschieden bei jedem Auftreten für O oder NR³ steht.

In einer bevorzugten Ausführungsform der Struktur gemäß Formel (48) stehen die Reste R¹, die an den Brückenkopf gebunden sind, für H, D, F oder CH₃. Weiterhin bevorzugt steht A² für C(R¹)₂ oder O, und besonders bevorzugt für C(R³)₂. Bevorzugte Ausführungsformen der Formel (48) sind somit eine Strukturen der Formel (48-A) und (48-B), und eine besonders bevorzugte Ausführungsform der Formel (48-A) ist eine Struktur der Formel (48-C), 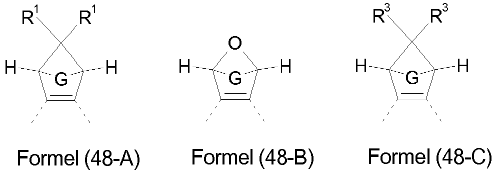 wobei die verwendeten Symbole die oben genannten Bedeutungen aufweisen.

In einer bevorzugten Ausführungsform der Struktur gemäß Formel (49), (50) und (51) stehen die Reste R¹, die an den Brückenkopf gebunden sind, für H, D, F oder CH₃. Weiterhin bevorzugt steht A² für C(R¹)₂. Bevorzugte Ausführungsformen der Formel (49), (50) und (51) sind somit die Strukturen der Formeln (49-A), (50-A) und (51-A), 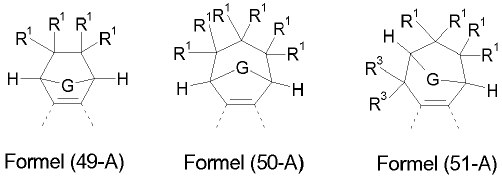 wobei die verwendeten Symbole die oben genannten Bedeutungen aufweisen.

Weiterhin bevorzugt steht die Gruppe G in den Formeln (48), (48-A), (48-B), (48-C), (49), (49-A), (50), (50-A), (51) und (51-A) für eine 1,2-Ethylengruppe, welche mit einem oder mehreren Resten R² substituiert sein kann, wobei R² bevorzugt gleich oder verschieden bei jedem Auftreten für H oder eine Alkylgruppe mit 1 bis 4 C-Atomen steht, oder eine ortho-Arylengruppe mit 6 bis 10 C-Atomen, welche mit einem oder mehreren Resten R² substituiert sein kann, bevorzugt aber unsubstituiert ist, insbesondere eine ortho-Phenylengruppe, welche mit einem oder mehreren Resten R² substituiert sein kann, bevorzugt aber unsubstituiert ist.

In einer weiteren bevorzugten Ausführungsform der Erfindung steht R³ in den Gruppen der Formel (45) bis (51) und in den bevorzugten Ausführungsformen gleich oder verschieden bei jedem Auftreten für F, eine geradkettige Alkylgruppe mit 1 bis 10 C-Atomen oder eine verzweigte oder cyclische Alkylgruppe mit 3 bis 20 C-Atomen, wobei jeweils eine oder mehrere nicht benachbarte CH₂-Gruppen durch R²C=CR² ersetzt sein können und ein oder mehrere H-Atome durch D oder F ersetzt sein können, oder ein aromatisches oder heteroaromatisches Ringsystem mit 5 bis 14 aromatischen Ringatomen, das jeweils durch einen oder mehrere Reste R² substituiert sein kann; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem benachbarten Rest R oder R¹ ein aliphatisches Ringsystem bilden.

In einer besonders bevorzugten Ausführungsform der Erfindung steht R³ in den Gruppen der Formeln (45) bis (51) und in den bevorzugten Ausführungsformen gleich oder verschieden bei jedem Auftreten für F, eine geradkettige Alkylgruppe mit 1 bis 3 C-Atomen, insbesondere Methyl, oder ein aromatisches oder heteroaromatisches Ringsystem mit 5 bis 12 aromatischen Ringatomen, das jeweils durch einen oder mehrere Reste R² substituiert sein kann, bevorzugt aber unsubstituiert ist; dabei können zwei Reste R³, welche an dasselbe Kohlenstoffatom gebunden sind, miteinander ein aliphatisches oder aromatisches Ringsystem bilden und so ein Spirosystem aufspannen; weiterhin kann R³ mit einem benachbarten Rest R oder R¹ ein aliphatisches Ringsystem bilden.

Beispiele für besonders geeignete Gruppen der Formel (45) sind die im Folgenden aufgeführten Strukturen:

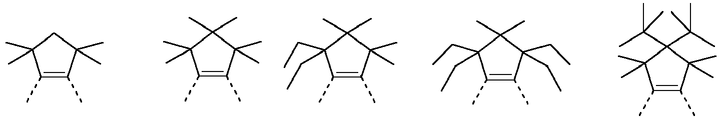

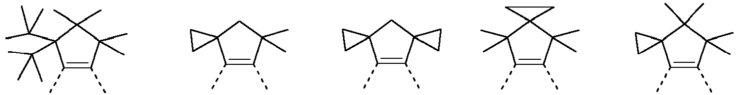

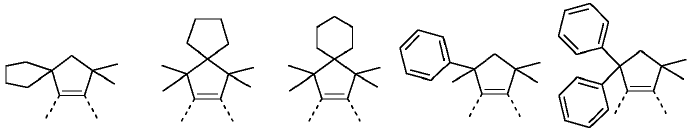

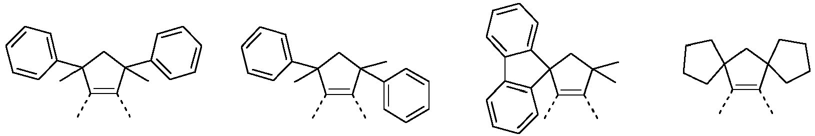

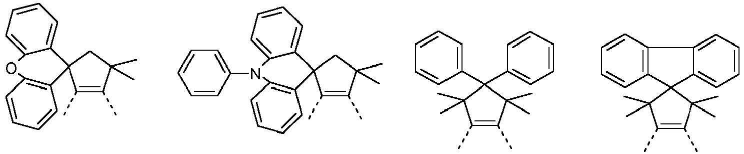

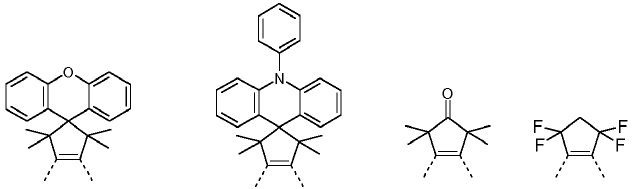

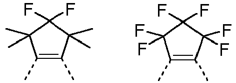

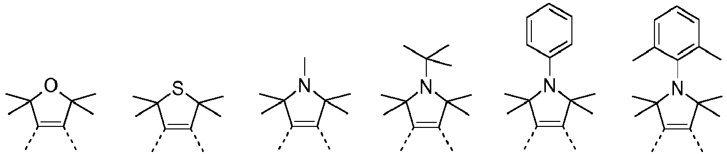

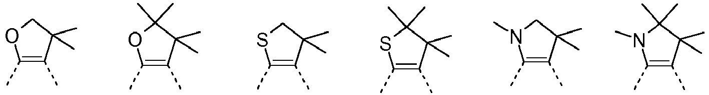

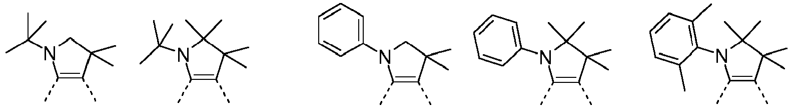

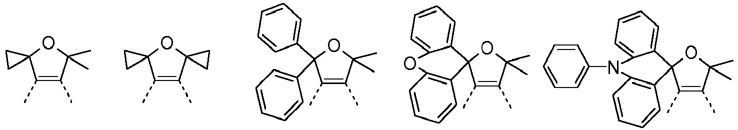

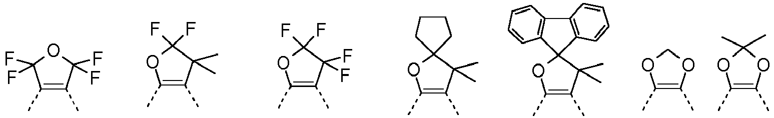

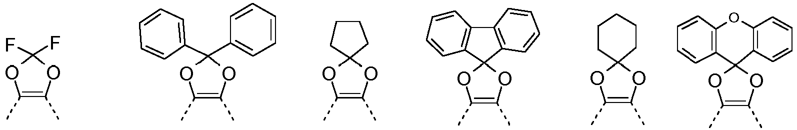

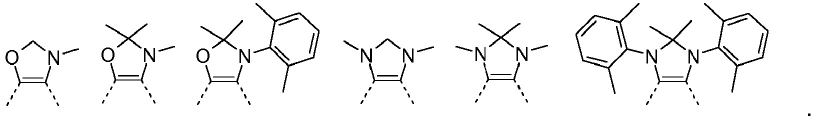

Beispiele für besonders geeignete Gruppen der Formel (46) sind die im Folgenden aufgeführten Strukturen:

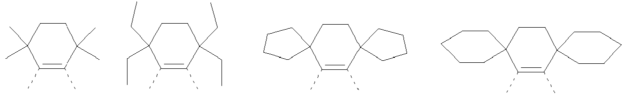

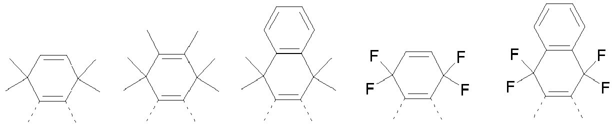

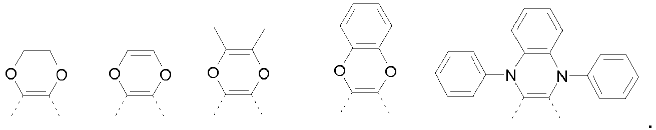

Beispiele für besonders geeignete Gruppen der Formel (47), (49) und (50) sind die im Folgenden aufgeführten Strukturen:

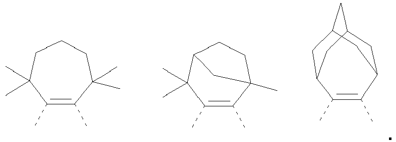

Beispiele für besonders geeignete Gruppen der Formel (48) sind die im Folgenden aufgeführten Strukturen:

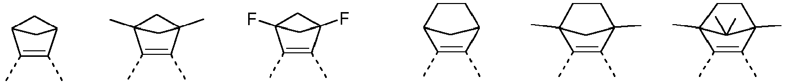

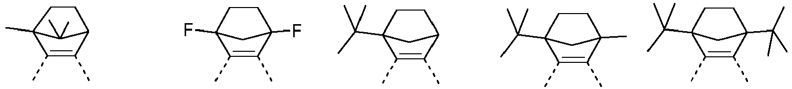

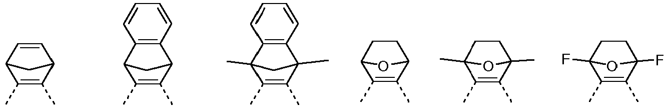

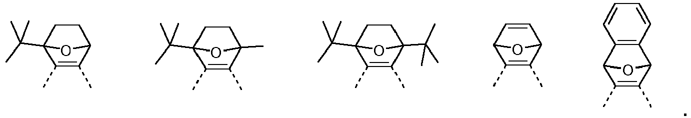

Beispiele für besonders geeignete Gruppen der Formel (49) sind die im Folgenden aufgeführten Strukturen:

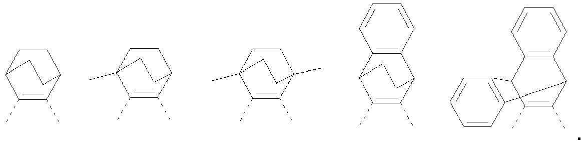

Wenn in den bidentaten Teilliganden bzw. in den bivalenten Arylen- bzw. Heteroarylengruppen der Formel (8), welche in den Formeln (3) bis (7) bzw. den bevorzugten Ausführungsformen gebunden sind, Reste R gebunden sind, so sind diese Reste R bei jedem Auftreten gleich oder verschieden bevorzugt ausgewählt aus der Gruppe bestehend aus H, D, F, Br, I, N(R¹)₂, CN, Si(R¹)₃, B(OR¹)₂, C(=O)R¹, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl oder Alkenylgruppe jeweils mit einem oder mehreren Resten R¹ substituiert sein kann, oder einem aromatischen oder heteroaromatischen Ringsystem mit 5 bis 30 aromatischen Ringatomen, das jeweils durch einen oder mehrere Reste R¹ substituiert sein kann; dabei können zwei benachbarte Rest R oder R mit R¹ auch miteinander ein mono- oder polycyclisches, aliphatisches oder aromatisches Ringsystem bilden. Besonders bevorzugt sind diese Reste R bei jedem Auftreten gleich oder verschieden ausgewählt aus der Gruppe bestehend aus H, D, F, N(R¹)₂, einer geradkettigen Alkylgruppe mit 1 bis 6 C-Atomen oder einer verzweigten oder cyclischen Alkylgruppe mit 3 bis 10 C-Atomen, wobei ein oder mehrere H-Atome durch D oder F ersetzt sein können, oder einem aromatischen oder heteroaromatischen Ringsystem mit 5 bis 24 aromatischen Ringatomen, das jeweils durch einen oder mehrere Reste R¹ substituiert sein kann; dabei können zwei benachbarte Reste R oder R mit R¹ auch miteinander ein mono- oder polycyclisches, aliphatisches oder aromatisches Ringsystem bilden.

Bevorzugte Reste R¹, die an R gebunden sind, sind bei jedem Auftreten gleich oder verschieden H, D, F, N(R²)₂, CN, eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, wobei die Alkylgruppe jeweils mit einem oder mehreren Resten R² substituiert sein kann, oder ein aromatisches oder heteroaromatisches Ringsystem mit 5 bis 24 aromatischen Ringatomen, das jeweils durch einen oder mehrere Reste R² substituiert sein kann; dabei können zwei oder mehrere benachbarte Reste R¹ miteinander ein mono- oder polycyclisches, aliphatisches Ringsystem bilden. Besonders bevorzugte Reste R¹, die an R gebunden sind, sind bei jedem Auftreten gleich oder verschieden H, F, CN, eine geradkettige Alkylgruppe mit 1 bis 5 C-Atomen oder eine verzweigte oder cyclische Alkylgruppe mit 3 bis 5 C-Atomen, die jeweils mit einem oder mehreren Resten R² substituiert sein kann, oder ein aromatisches oder heteroaromatisches Ringsystem mit 5 bis 13 aromatischen Ringatomen, das jeweils durch einen oder mehrere Reste R² substituiert sein kann; dabei können zwei oder mehrere benachbarte Reste R¹ miteinander ein mono- oder polycyclisches, aliphatisches Ringsystem bilden.

Bevorzugte Reste R² sind bei jedem Auftreten gleich oder verschieden H, F oder ein aliphatischer Kohlenwasserstoffrest mit 1 bis 5 C-Atomen oder ein aromatischer Kohlenwasserstoffrest mit 6 bis 12 C-Atomen; dabei können zwei oder mehrere Substituenten R² auch miteinander ein mono- oder polycyclisches, aliphatisches Ringsystem bilden.

Die oben genannten bevorzugten Ausführungsformen sind beliebig miteinander kombinierbar. In einer besonders bevorzugten Ausführungsform der Erfindung gelten die oben genannten bevorzugten Ausführungsformen gleichzeitig.

Beispiele für geeignete erfindungsgemäße Strukturen sind die nachfolgend abgebildeten Verbindungen.

| | | |
|---|---|---|
| 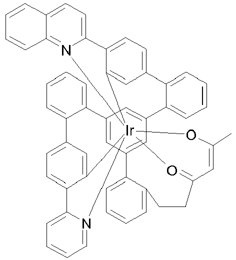 | 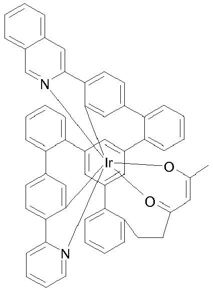 | 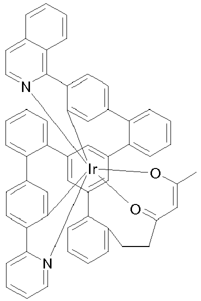 |
| 1 | 2 | 3 |
| 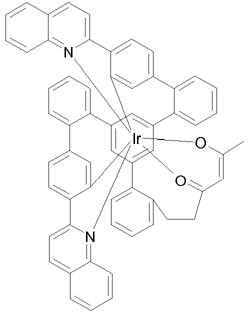 | 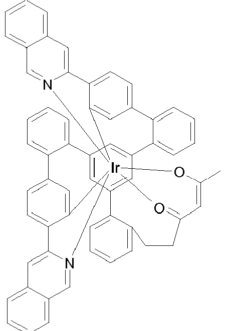 | 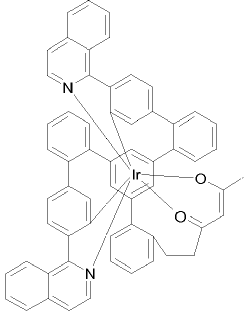 |
| 4 | 5 | 6 |
| 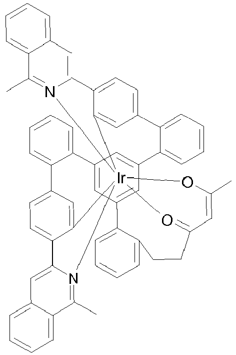 | 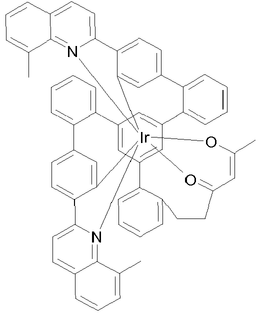 | 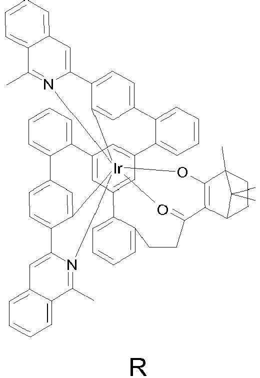 |
| 7 | 8 | 9 |
| 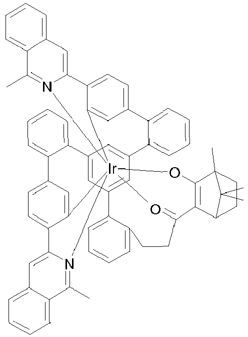 | 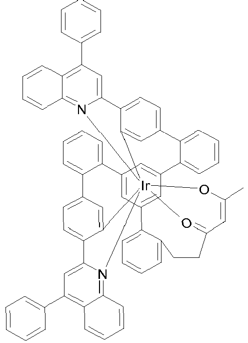 | 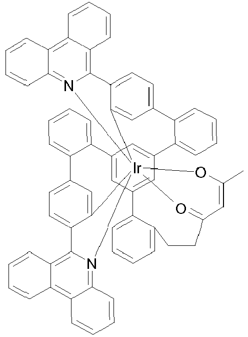 |
| 10 | 11 | 12 |
| 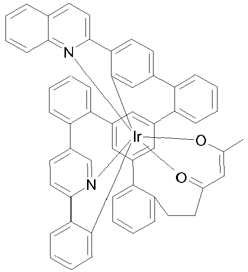 | 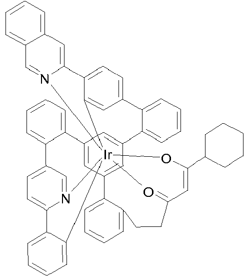 | 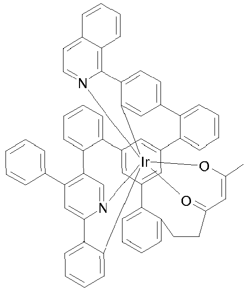 |
| 13 | 14 | 15 |
| 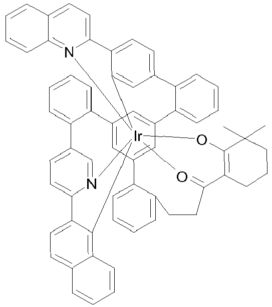 | 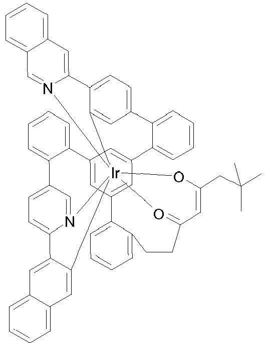 | 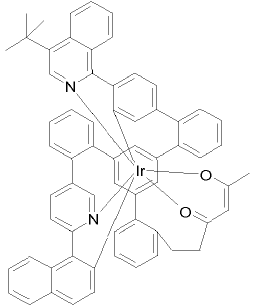 |
| 16 | 17 | 18 |
| 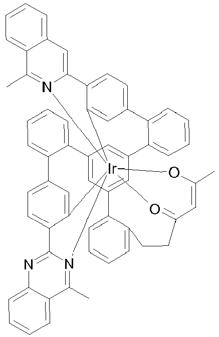 | 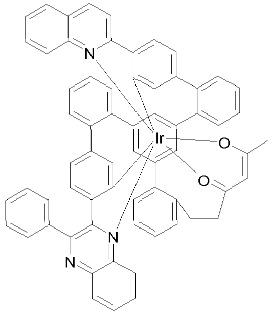 | 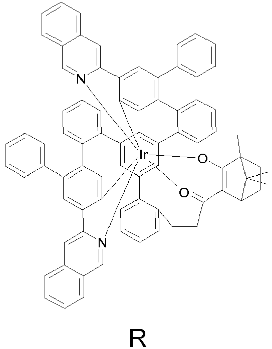 |
| 19 | 20 | 21 |
| 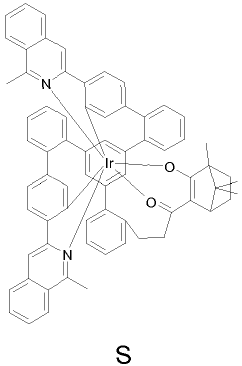 | 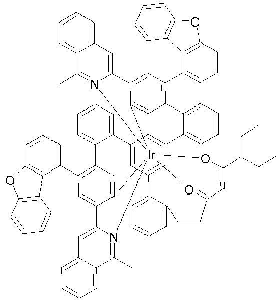 | 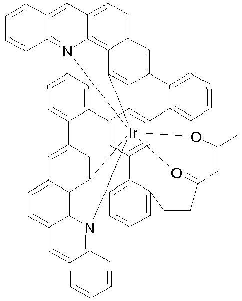 |
| 22 | 23 | 24 |
| 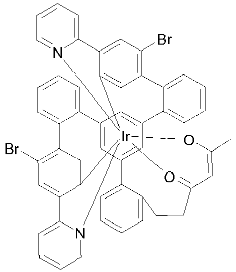 | 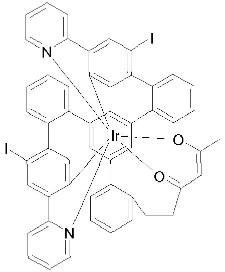 | 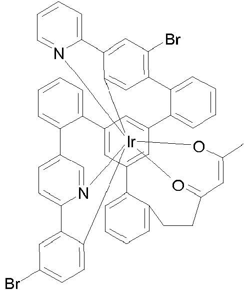 |
| 25 | 26 | 27 |
| 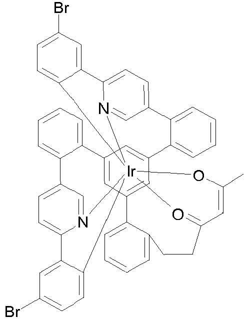 | 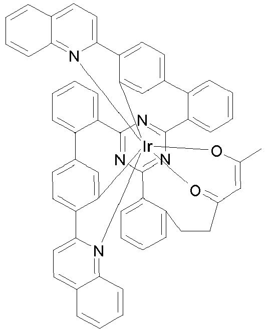 | 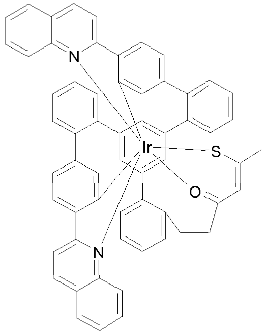 |
| 28 | 29 | 30 |
| 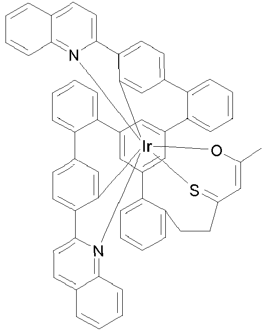 | 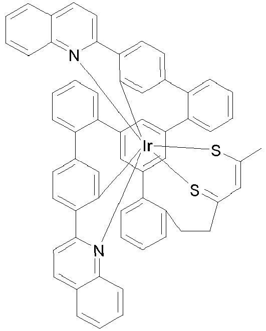 | 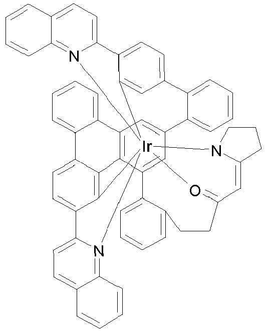 |
| 31 | 32 | 33 |
| 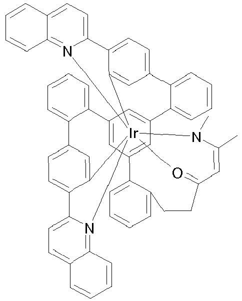 | 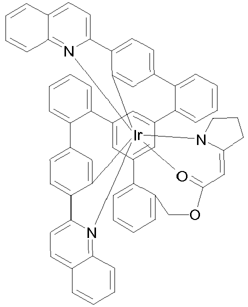 | 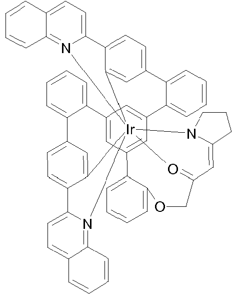 |
| 34 | 35 | 36 |
| 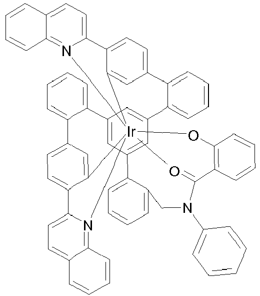 | 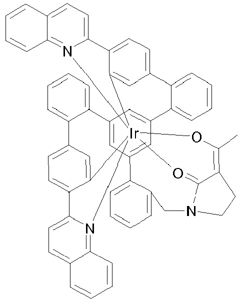 | 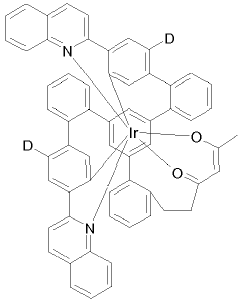 |
| 37 | 38 | 39 |
| 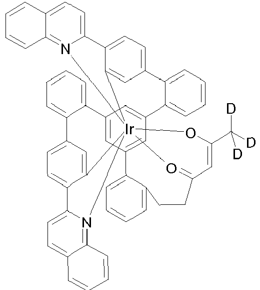 | 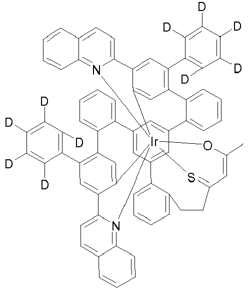 | 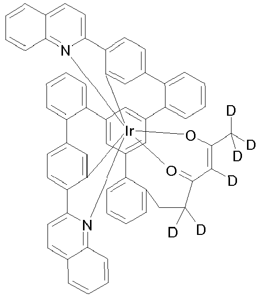 |
| 40 | 41 | 42 |
| 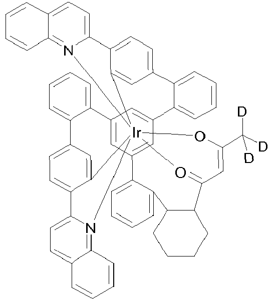 | 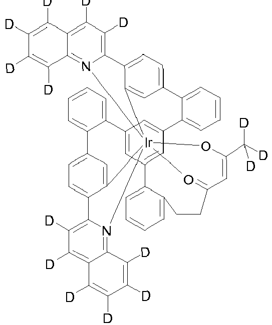 | 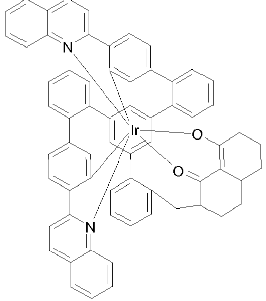 |
| 43 | 44 | 45 |
| 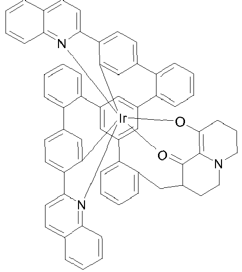 | 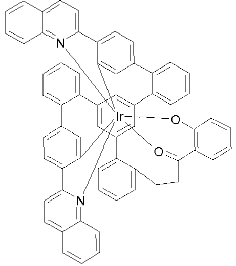 | 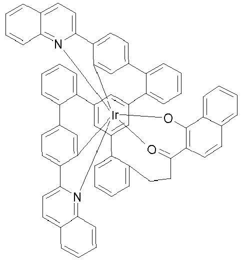 |
| 46 | 47 | 48 |
| 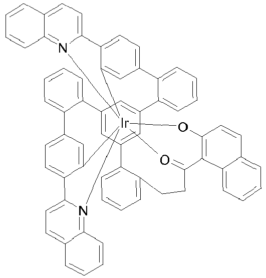 | 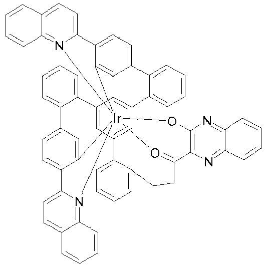 | 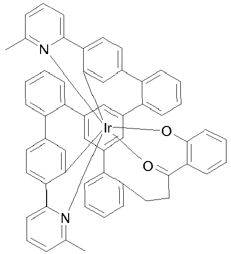 |
| 49 | 50 | 51 |
| 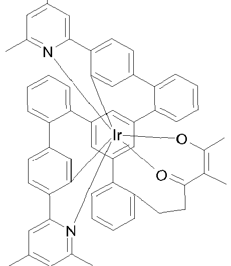 | 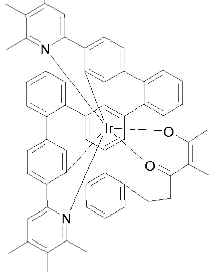 | 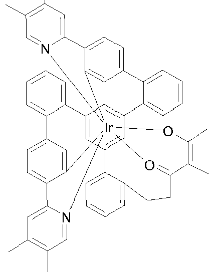 |
| 52 | 53 | 54 |
| 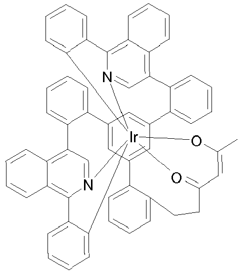 | 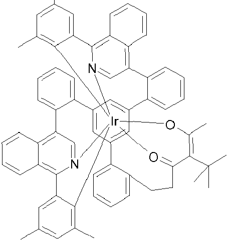 | 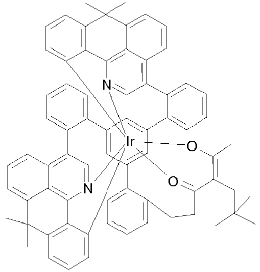 |
| 55 | 56 | 57 |
| 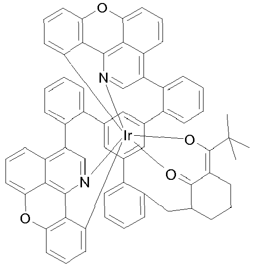 | 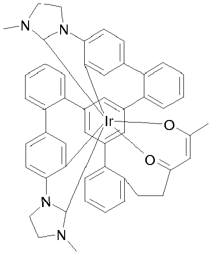 | 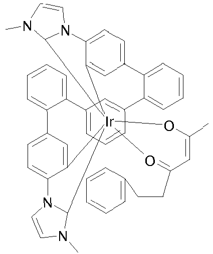 |
| 58 | 59 | 60 |
| 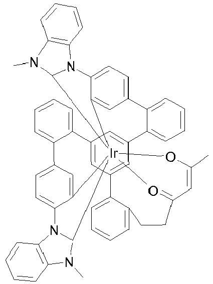 | 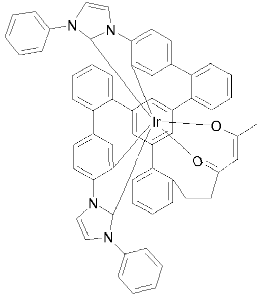 | 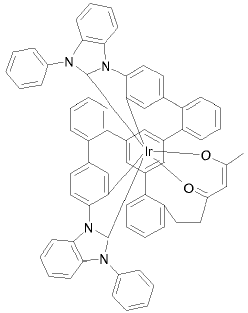 |
| 61 | 62 | 63 |
| 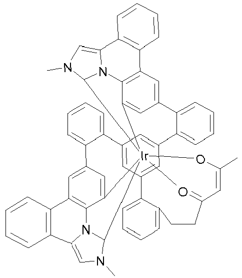 | 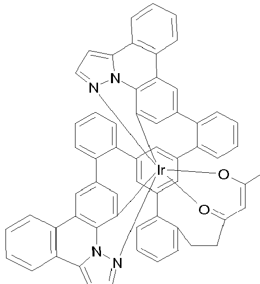 | 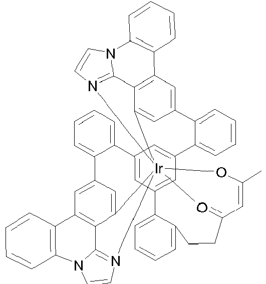 |
| 64 | 65 | 66 |
| 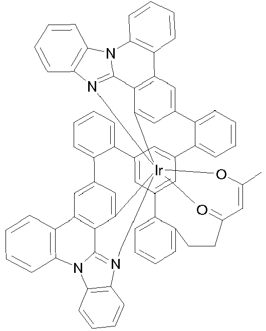 | 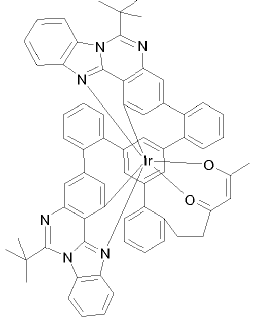 | 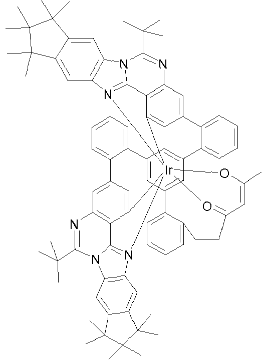 |
| 67 | 68 | 69 |
| 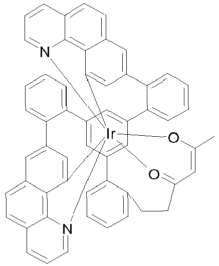 | 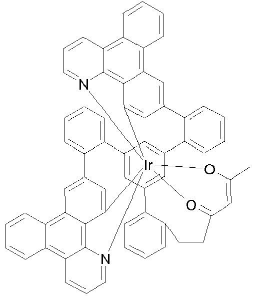 | 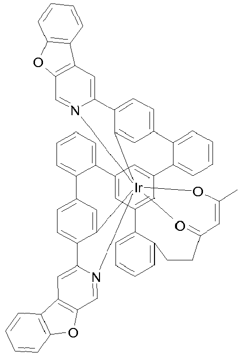 |
| 70 | 71 | 72 |
| 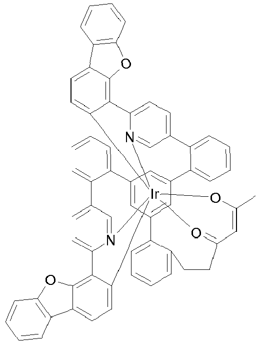 | 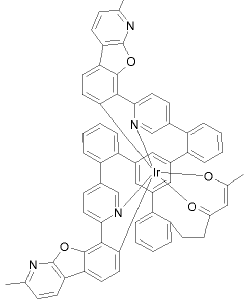 | 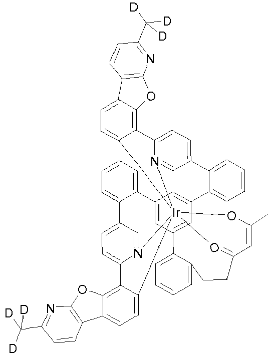 |
| 73 | 74 | 75 |
| 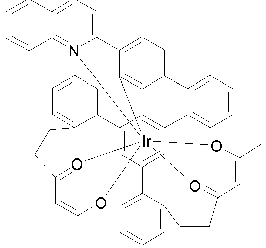 | 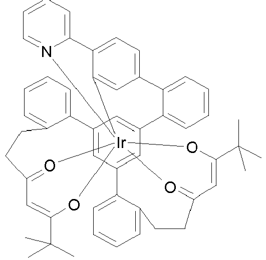 | 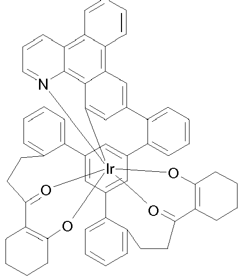 |
| 76 | 77 | 78 |

Bei den erfindungsgemäßen Iridiumkomplexen handelt es sich um chirale Strukturen. Wenn zusätzlich auch der tripodale Ligand der Komplexe chiral ist, ist die Bildung von Diastereomeren und mehreren Enantiomerenpaaren möglich. Die erfindungsgemäßen Komplexe umfassen dann sowohl die Mischungen der verschiedenen Diastereomere bzw. die entsprechenden Racemate wie auch die einzelnen isolierten Diastereomere bzw. Enantiomere.

Werden in der Komplexierungsreaktion Cₛ-symmetrische Liganden mit zwei identischen Teilliganden eingesetzt, fällt üblicherweise ein racemisches Gemisch der C₁-symmetrischen Komplexe, also des Δ- und des Λ-Enantiomers, an. Diese können durch gängige Methoden (Chromatographie an chiralen Materialien / Säulen oder Racemattrennung durch Kristallisation) getrennt werden. Dies ist im folgenden Schema am Beispiel eines Cₛ-symmetrischen Liganden, der zwei identische Phenylpyridin-Teilliganden trägt, gezeigt und gilt in analoger Form auch für alle anderen Cₛ-symmetrischen Liganden.

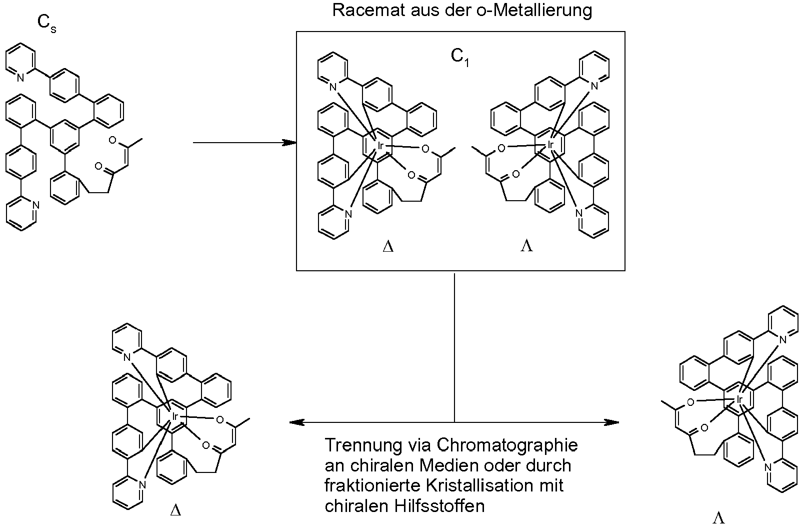

Die Racemattrennung via fraktionierter Kristallisation von diastereomeren Salzpaaren kann nach üblichen Methoden erfolgen. Hierzu bietet es sich an, die neutralen Ir(III)-Komplexe zu oxidieren (z. B. mit Peroxiden, H₂O₂ oder elektrochemisch), die so erzeugten kationischen Ir(IV)-Komplexe mit dem Salz einer enantiomerenreinen, monoanionischen Base (chirale Base) zu versetzen, die so erzeugten diasteromeren Salze durch fraktionierte Kristallisation zu trennen und diese dann mit Hilfe eines Reduktionsmittels (z. B. Zink, Hydrazinhydrat, Ascorbinsäure, etc.) zu den enantiomerenreinen neutralen Komplex zu reduzieren, wie im Folgenden schematisch dargestellt.

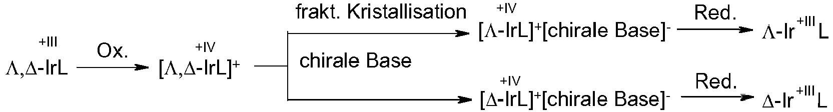

Daneben ist eine enantiomerenreine bzw. enantiomerenanreichernde Synthese durch Komplexierung in einem chiralen Medium (z. B. R- oder S-1,1-Binaphthol) möglich.

Analoge Verfahren können auch mit Komplexen C₁- bzw. Cₛ-symmetrischer Liganden durchgeführt werden.

Werden in der Komplexierung Ci-symmetrische Liganden eingesetzt, fällt üblicherweise ein Diastereomerengemisch der Komplexe an, das durch gängige Methoden (Chromatographie, Kristallisation) getrennt werden kann.

Die erfindungsgemäßen Verbindungen sind prinzipiell durch verschiedene Verfahren darstellbar. Generell wird hierzu ein Iridiumsalz mit dem entsprechenden freien Liganden umgesetzt.

Daher ist ein weiterer Gegenstand der vorliegenden Erfindung ein Verfahren zur Herstellung der erfindungsgemäßen Verbindungen durch Umsetzung der entsprechenden freien Liganden mit Iridiumalkoholaten der Formel (52), mit Iridiumketoketonaten der Formel (53), mit Iridiumhalogeniden der Formel (54) oder mit Iridiumcarboxylaten der Formel (55), 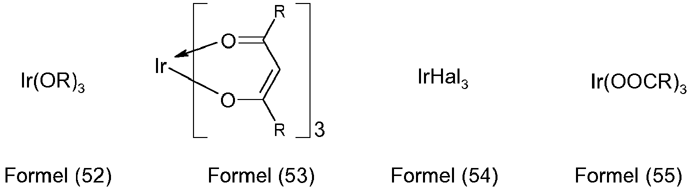 wobei R die oben angegebenen Bedeutungen hat, Hal = F, Cl, Br oder I ist und die Iridiumedukte auch als die entsprechenden Hydrate vorliegen können. Dabei steht R bevorzugt für eine Alkylgruppe mit 1 bis 4 C-Atomen.

Es können ebenfalls Iridiumverbindungen, die sowohl Alkoholat- und/oder Halogenid- und/oder Hydroxy- wie auch Ketoketonatreste tragen, verwendet werden. Diese Verbindungen können auch geladen sein. Entsprechende Iridiumverbindungen, die als Edukte besonders geeignet sind, sind in WO 2004/085449 offenbart. Besonders geeignet sind [lrCl₂(acaC)₂]⁻, beispielsweise Na[lrCl₂(acac)₂], Metallkomplexe mit Acetylacetonat-Derivaten als Ligand, beispielsweise Ir(acac)₃ oder Tris(2,2,6,6-Tetramethylheptan-3,5-dionato)iridium, und IrCl_{3·X}H₂O, wobei x üblicherweise für eine Zahl zwischen 2 und 4 steht.

Die Synthese der Komplexe wird bevorzugt durchgeführt wie in WO 2002/060910 und in WO 2004/085449 beschrieben. Dabei kann die Synthese beispielsweise auch thermisch, photochemisch und/oder durch Mikrowellenstrahlung aktiviert werden. Weiterhin kann die Synthese auch im Autoklaven bei erhöhtem Druck und/oder erhöhter Temperatur durchgeführt werden.

Die Reaktionen können ohne Zusatz von Lösemitteln oder Schmelzhilfen in einer Schmelze der entsprechenden zu o-metallierenden Liganden durchgeführt werden. Gegebenenfalls können auch Lösemittel oder Schmelzhilfen zugesetzt werden. Geeignete Lösemittel sind protische oder aprotische Lösemittel, wie aliphatische und / oder aromatische Alkohle (Methanol, Ethanol, iso-Propanol, t-Butanol, etc.), Oligo- und Polyalkohole (Ethylenglykol, 1,2-Propandiol, Glycerin, etc.), Alkoholether (Ethoxyethanol, Diethylenglykol, Triethylenglycol, Polyethylenglykol, etc.), Ether (Di- und Triethylenglykoldimethylether, Diphenylether, etc.), aromatische, heteroaromatische und oder aliphatische Kohlenwasserstoffe (Toluol, Xylol, Mesitylen, Chlorbenzol, Pyridin, Lutidin, Chinolin, Isochinolin, Tridecan, Hexadecan, etc.), Amide (DMF, DMAC, etc.), Lactame (NMP), Sulfoxide (DMSO) oder Sulfone (Dimethylsulfon, Sulfolan, etc.). Geeignete Schmelzhilfen sind Verbindungen, die bei Rautemperatur fest vorliegen, jedoch beim Erwärmen der Reaktionsmischung schmelzen und die Reaktanden lösen, so dass eine homogene Schmelze entsteht. Besonders geeignet sind Biphenyl, m-Terphenyl, Triphenylen, R- oder S-Binaphthol oder auch das entsprechende Racemat, 1,2-, 1,3-, 1,4-Bisphenoxybenzol, Triphenylphosphinoxid, 18-Krone-6, Phenol, 1-Naphthol, Hydrochinon, etc.. Dabei ist die Verwendung von Hydrochinon besonders bevorzugt.

Durch diese Verfahren, gegebenenfalls gefolgt von Aufreinigung, wie z. B. Umkristallisation oder Sublimation, lassen sich die erfindungsgemäßen Verbindungen gemäß Formel (1) in hoher Reinheit, bevorzugt mehr als 99 % (bestimmt mittels ¹H-NMR und/oder HPLC) erhalten.

Die erfindungsgemäßen Verbindungen können auch durch geeignete Substitution, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen, löslich gemacht werden. Insbesondere auch die Verwendung von ankondensierten aliphatischen Gruppen, wie sie beispielsweise durch die oben offenbarten Formeln (45) bis (51) dargestellt werden, führt zu einer deutlichen Verbesserung der Löslichkeit der Metallkomplexe. Solche Verbindungen sind dann in gängigen organischen Lösemitteln, wie beispielsweise Toluol oder Xylol bei Raumtemperatur in ausreichender Konzentration löslich, um die Komplexe aus Lösung verarbeiten zu können. Diese löslichen Verbindungen eignen sich besonders gut für die Verarbeitung aus Lösung, beispielsweise durch Druckverfahren.

Für die Verarbeitung der erfindungsgemäßen Iridiumkomplexe aus flüssiger Phase, beispielsweise durch Spin-Coating oder durch Druckverfahren, sind Formulierungen der erfindungsgemäßen Iridiumkomplexe erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung, enthaltend mindestens eine erfindungsgemäßen Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch eine weitere organische oder anorganische Verbindung sein, die ebenfalls in der elektronischen Vorrichtung eingesetzt wird, beispielsweise ein Matrixmaterial. Diese weitere Verbindung kann auch polymer sein.

Die erfindungsgemäße Verbindung kann in der elektronischen Vorrichtung als aktive Komponente, bevorzugt als Emitter in der emissiven Schicht oder als Loch- oder Elektronentransportmaterial in einer loch- bzw. elektronentransportierenden Schicht, oder als Sauerstoff-Sensibilisatoren oder als Photoinitiator oder Photokatalysator verwendet werden. Ein weiterer Gegenstand der vorliegenden Erfindung ist somit die Verwendung einer erfindungsgemäßen Verbindung in einer elektronischen Vorrichtung oder als Sauerstoff-Sensibilisator oder als Photoinitiator oder Photokatalysator. Enantiomerenreine erfindungsgemäße Iridiumkomplexe eignen sich als Photokatalysatoren für chirale photoinduzierte Synthesen.

Nochmals ein weiterer Gegenstand der vorliegenden Erfindung ist eine elektronische Vorrichtung enthaltend mindestens eine erfindungsgemäße Verbindung.

Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße elektronische Vorrichtung enthält also Anode, Kathode und mindestens eine Schicht, welche mindestens einen erfindungsgemäßen Iridiumkomplex enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), wobei hierunter sowohl rein organische Solarzellen wie auch farbstoffsensibilisierte Solarzellen verstanden werden, organischen optischen Detektoren, organischen Photorezeptoren, organischen Feld-Quench-Devices (O-FQDs), lichtemittierenden elektrochemischen Zellen (LECs), Sauerstoff-Sensoren oder organischen Laserdioden (O-Laser), enthaltend in mindestens einer Schicht mindestens eine erfindungsgemäße Verbindung. Verbindungen, die im Infraroten emittieren, eignen sich für den Einsatz in organischen Infrarot-Elektrolumineszenzvorrichtungen und Infrorot-Sensoren. Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien. Die erfindungsgemäßen Verbindungen zeigen besonders gute Eigenschaften als Emissionsmaterial in organischen Elektrolumineszenzvorrichtungen. Eine bevorzugte Ausführungsform der Erfindung sind daher organische Elektrolumineszenzvorrichtungen. Weiterhin können die erfindungsgemäßen Verbindungen zur Erzeugung von Singulett-Sauerstoff oder in der Photokatalyse eingesetzt werden.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoO₃ oder WO₃, oder mit (per)fluorierten elektronenarmen Aromaten oder mit elektronenarmen Cyano-substituieren Heteroaromaten (z. B. gemäß JP 4747558, JP 2006-135145, US 2006/0289882, WO 2012/095143), oder mit chinoiden Systemen (z. B. gemäß EP1336208) oder mit LewisSäuren, oder mit Boranen (z. B. gemäß US 2003/0006411,
WO 2002/051850, WO 2015/049030) oder mit Carboxylaten der Elemente der 3. , 4. oder 5. Hauptgruppe (WO 2015/018539) und/oder dass eine oder mehrere Elektronentransportschichten n-dotiert sind.

Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern und/oder Ladungen erzeugen (Charge-Generation-Layer, z. B. in Schichtsystemen mit mehreren emittierenden Schichten, z. B. in weiß emittierenden OLED-Bauteilen) . Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren. Eine bevorzugte Ausführungsform sind Tandem-OLEDs. Weiß emittierende organische Elektrolumineszenzvorrichtungen können für Beleuchtungsanwendungen oder mit Farbfilter auch für Vollfarb-Displays verwendet werden.

In einer bevorzugten Ausführungsform der Erfindung enthält die organische Elektrolumineszenzvorrichtung den erfindungsgemäßen Iridiumkomplex als emittierende Verbindung in einer oder mehreren emittierenden Schichten.

Wenn der erfindungsgemäße Iridiumkomplex als emittierende Verbindung in einer emittierenden Schicht eingesetzt wird, wird er bevorzugt in Kombination mit einem oder mehreren Matrixmaterialien eingesetzt. Die Mischung aus dem erfindungsgemäßen Iridiumkomplex und dem Matrixmaterial enthält zwischen 0.1 und 99 Vol.-%, vorzugsweise zwischen 1 und 90 Vol.-%, besonders bevorzugt zwischen 3 und 40 Vol.-%, insbesondere zwischen 5 und 15 Vol.-% des erfindungsgemäßen Iridiumkomplexes bezogen auf die Gesamtmischung aus Emitter und Matrixmaterial. Entsprechend enthält die Mischung zwischen 99.9 und 1 Vol.-%, vorzugsweise zwischen 99 und 10 Vol.-%, besonders bevorzugt zwischen 97 und 60 Vol.-%, insbesondere zwischen 95 und 85 Vol.-% des Matrixmaterials bezogen auf die Gesamtmischung aus Emitter und Matrixmaterial.

Als Matrixmaterial können generell alle Materialien eingesetzt werden, die gemäß dem Stand der Technik hierfür bekannt sind. Bevorzugt ist das Triplett-Niveau des Matrixmaterials höher als das Triplett-Niveau des Emitters.

Geeignete Matrixmaterialien für die erfindungsgemäßen Verbindungen sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl), m-CBP oder die in WO 2005/039246, US 2005/0069729, JP 2004/288381, EP 1205527, WO 2008/086851 oder US 2009/0134784 offenbarten Carbazolderivate, Biscarbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, Dibenzofuranderivate, z. B. gemäß WO 2009/148015 oder WO 2015/169412, oder verbrückte Carbazolderivate, z. B. gemäß US 2009/0136779, WO 2010/050778, WO 2011/042107 oder WO 2011/088877. Für lösungsprozessierte OLEDs eignen sich als Matrixmaterialien auch Polymere, z. B. gemäß WO 2012/008550 oder WO 2012/048778, Oligomere oder Dendrimere, z. B. gemäß Journal of Luminescence 183 (2017), 150-158.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Eine bevorzugte Kombination ist beispielsweise die Verwendung eines aromatischen Ketons, eines Triazin-Derivats oder eines Phosphinoxid-Derivats mit einem Triarylamin-Derivat oder einem Carbazol-Derivat als gemischte Matrix für den erfindungsgemäßen Metallkomplex. Ebenso bevorzugt ist die Verwendung einer Mischung aus einem ladungstransportierenden Matrixmaterial und einem elektrisch inerten Matrixmaterial (so genannter "wide bandgap host"), welches nicht bzw. nicht in wesentlichem Maße am Ladungstransport beteiligt ist, wie z. B. in WO 2010/108579 oder WO 2016/184540 beschrieben. Ebenso bevorzugt ist die Verwendung von zwei elektronentransportierenden Matrixmaterialien, beispielsweise Triazinderivaten und Lactamderivaten, wie z. B. in WO 2014/094964 beschrieben.

Nachfolgend sind Beispiele für Verbindungen abgebildet, die sich als Matrixmaterialien für die erfindungsgemäßen Verbindungen eignen.

Beispiele für Triazine und Pyrimidine, welche als elektronentransportierende Matrixmaterialien eingesetzt werden können, sind die folgenden Strukturen:

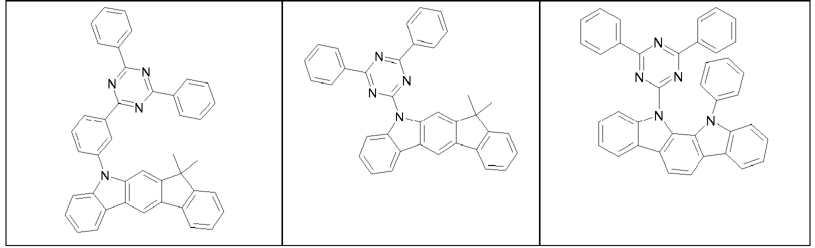

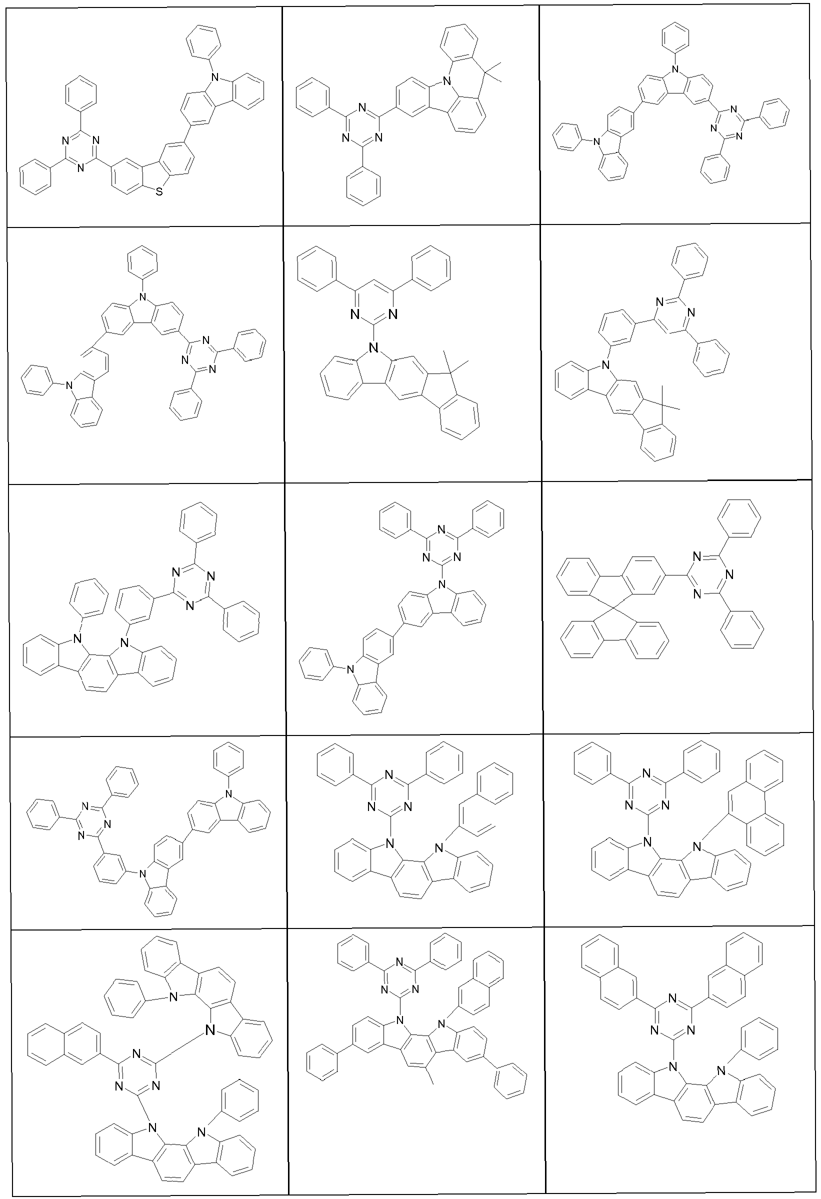

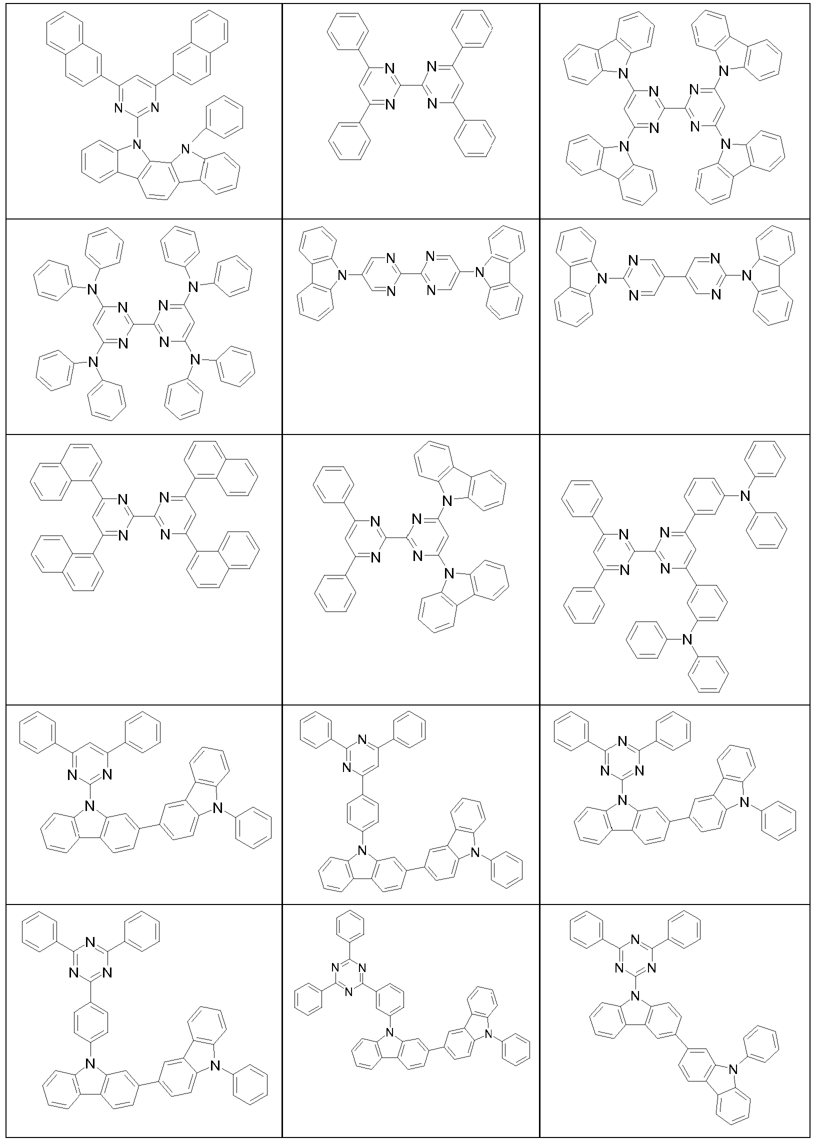

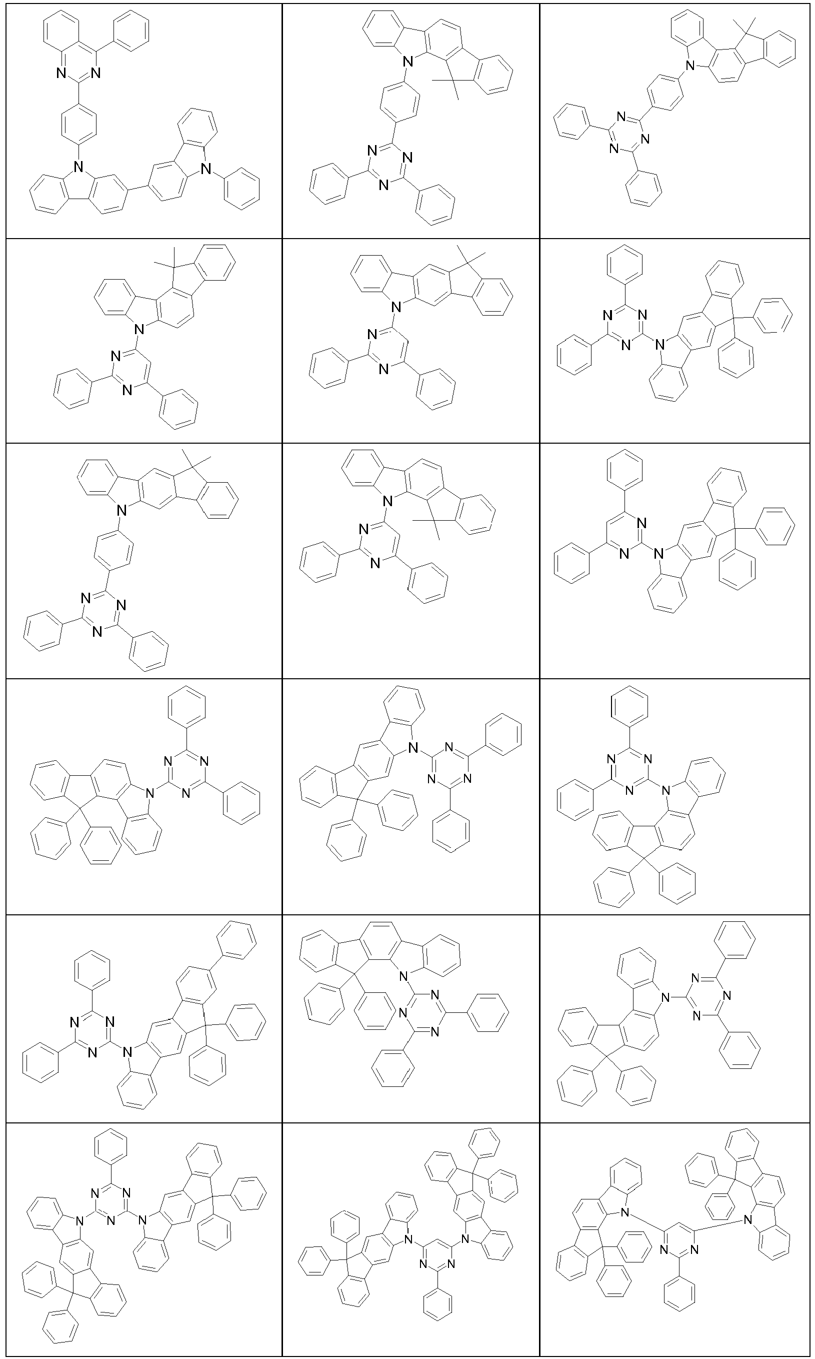

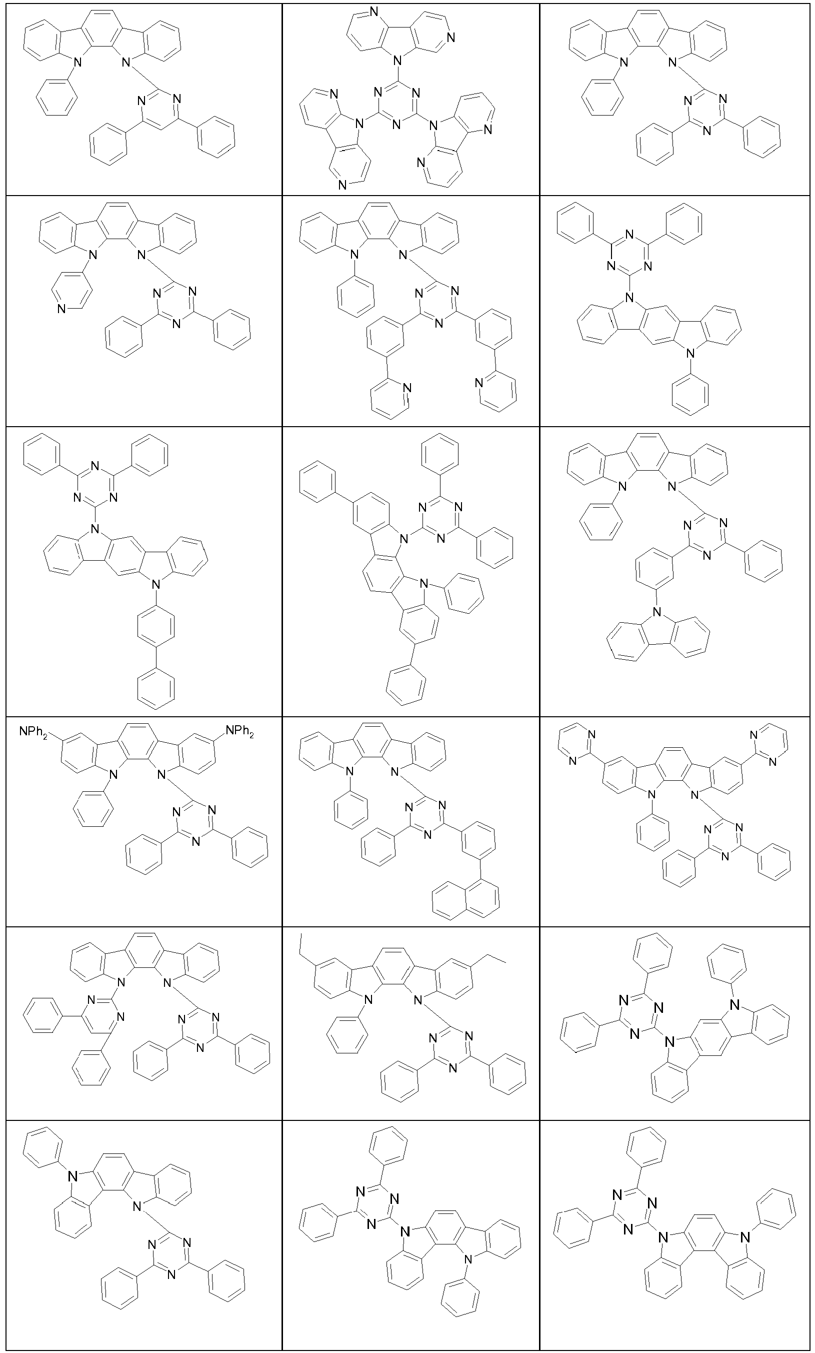

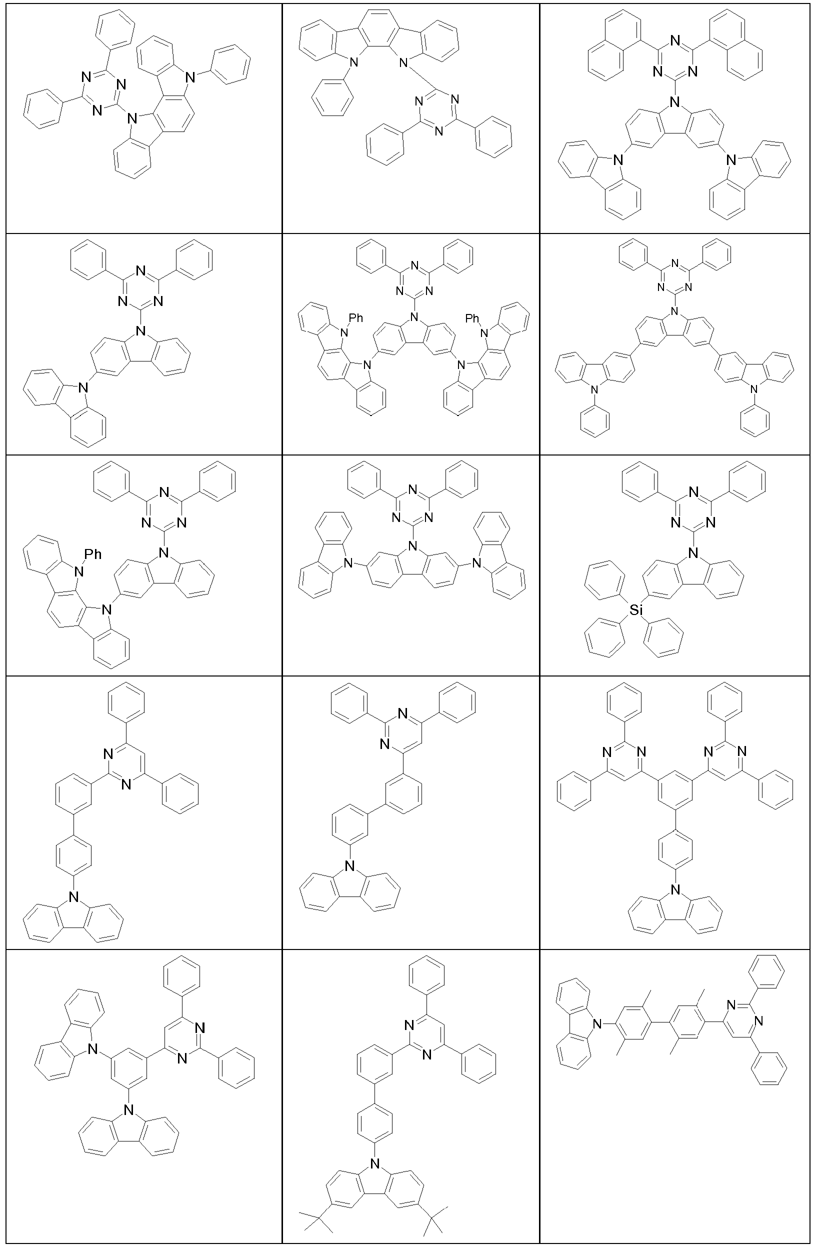

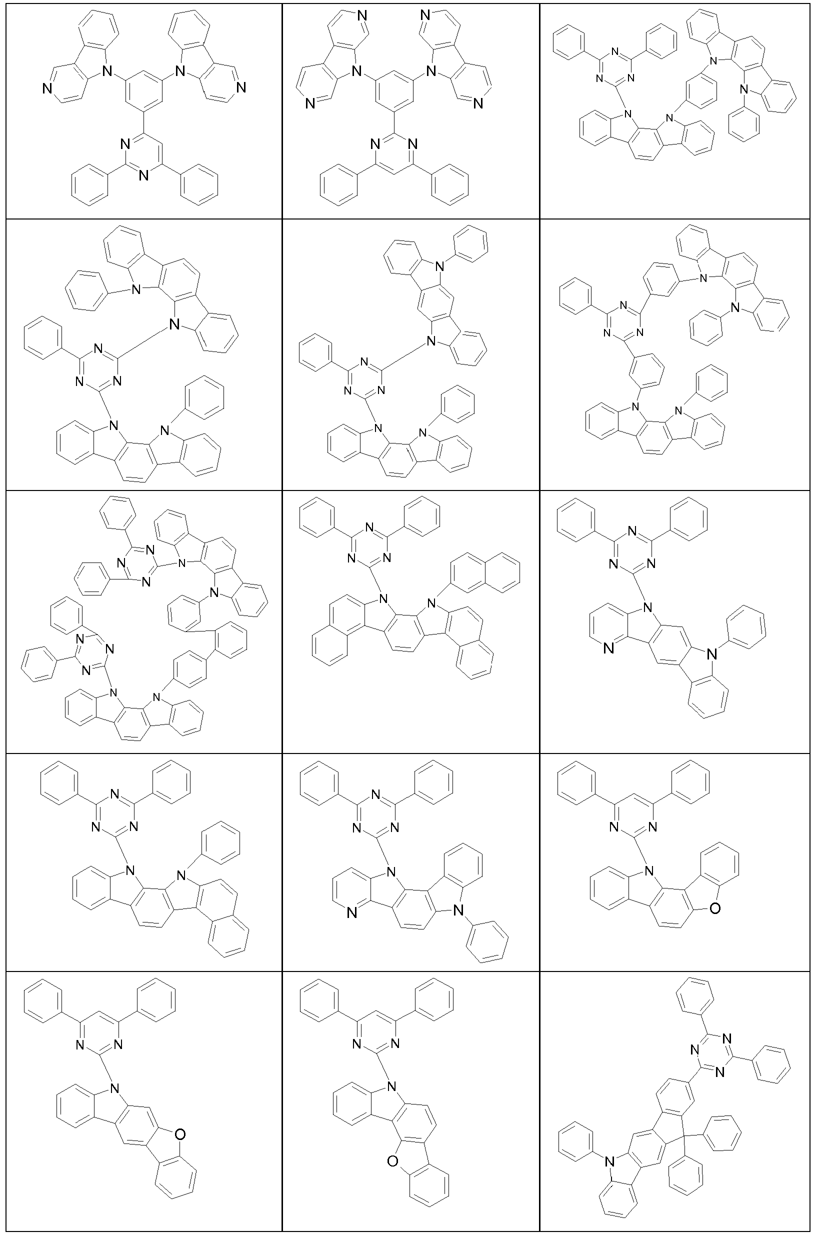

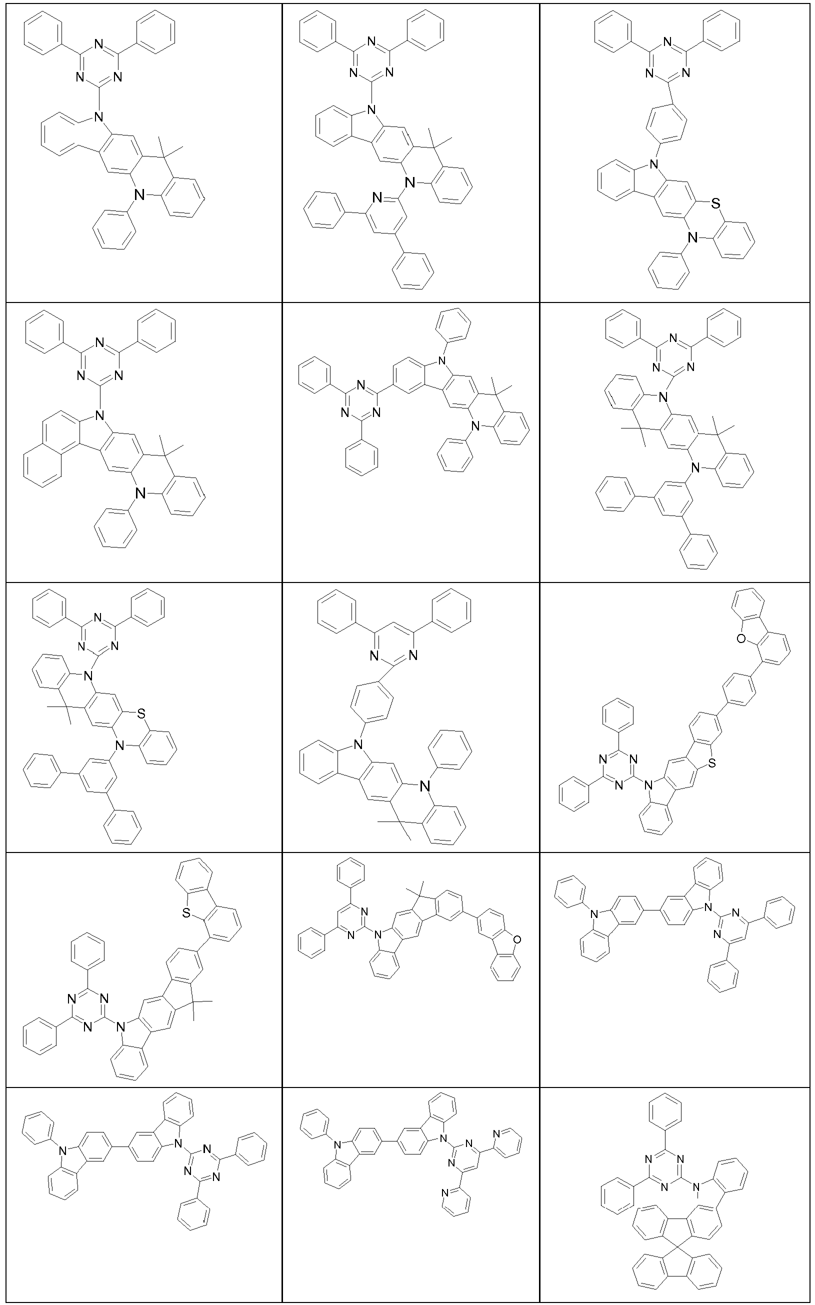

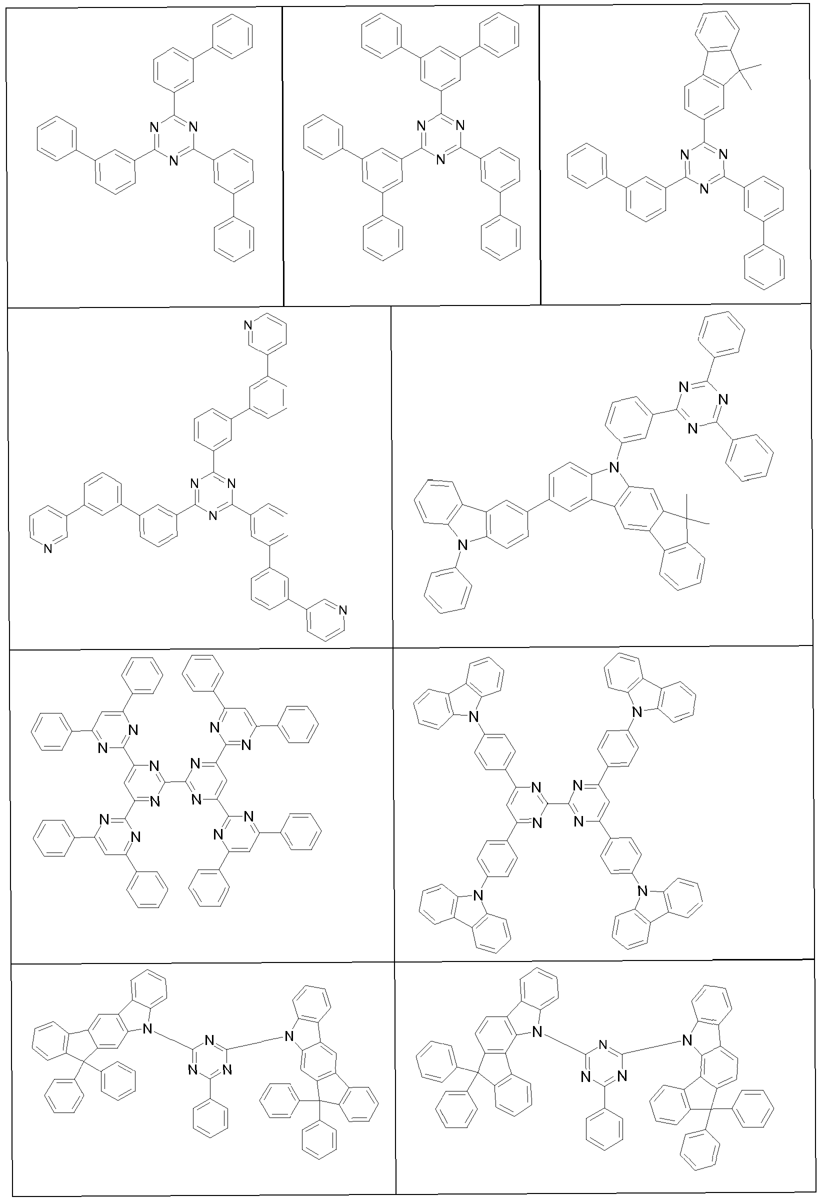

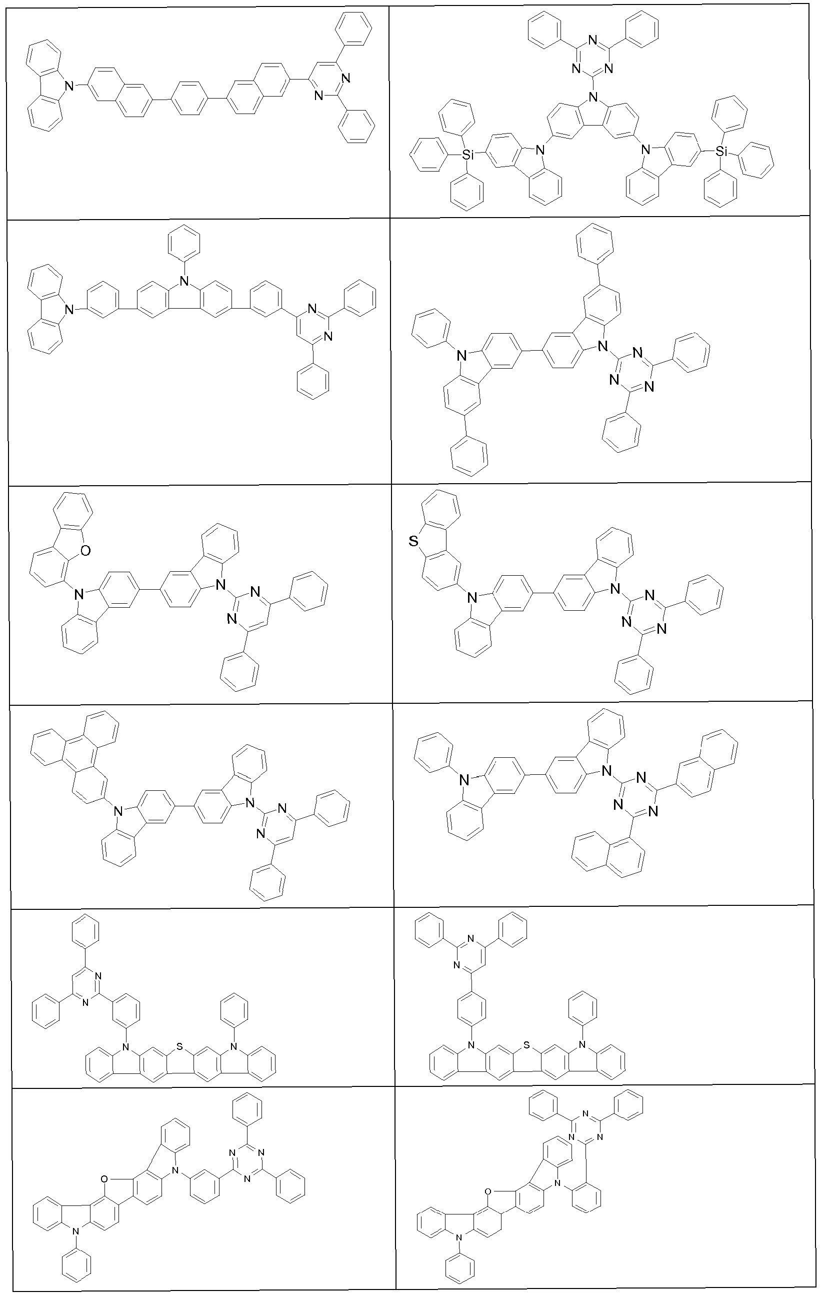

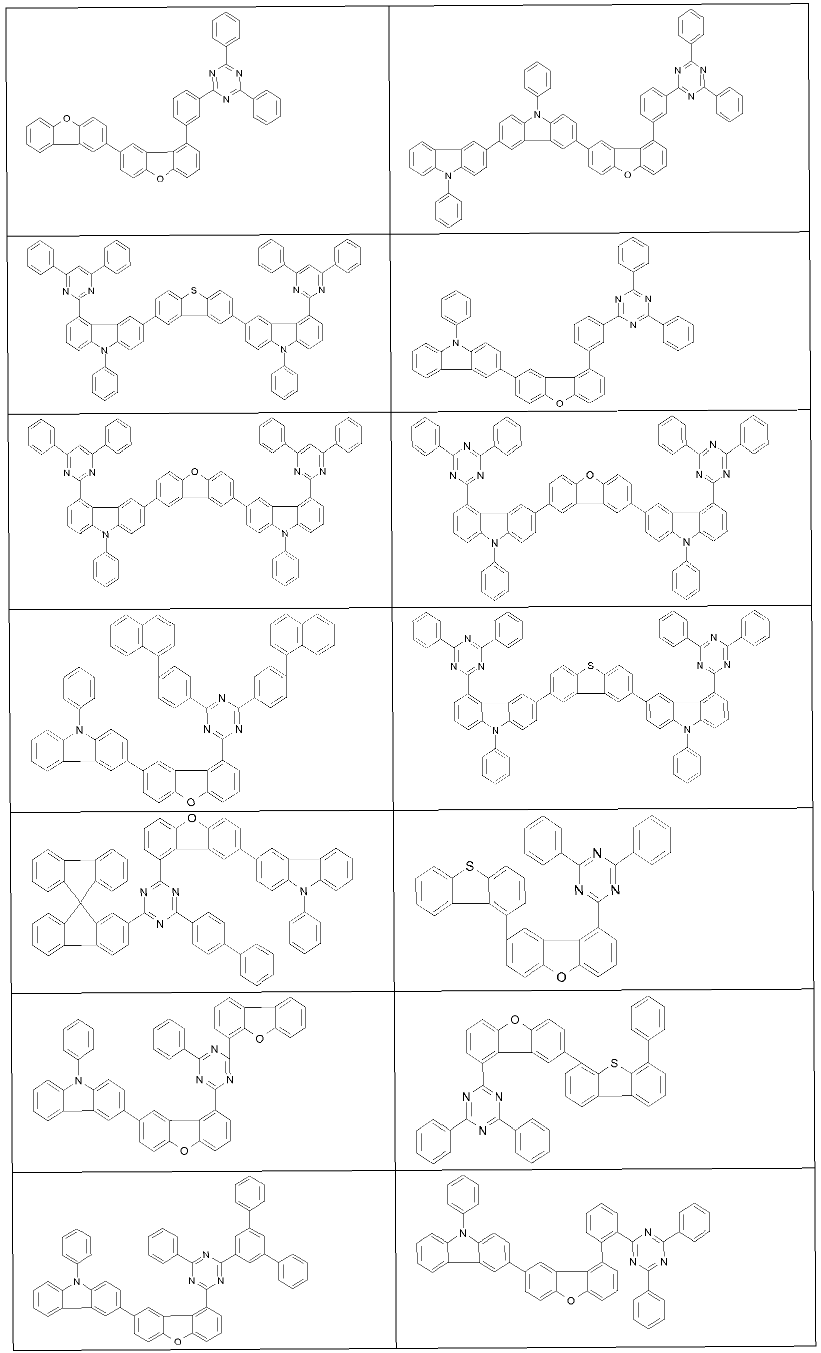

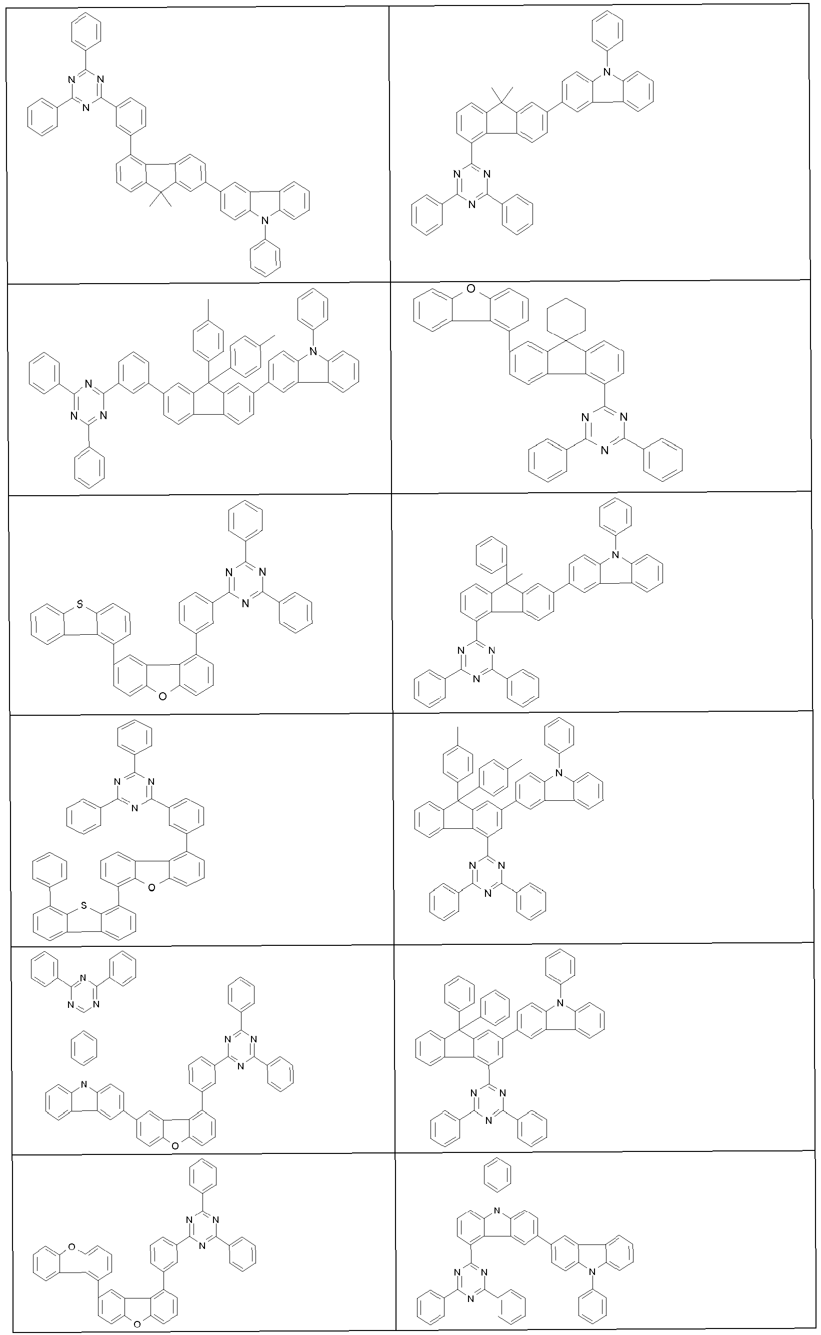

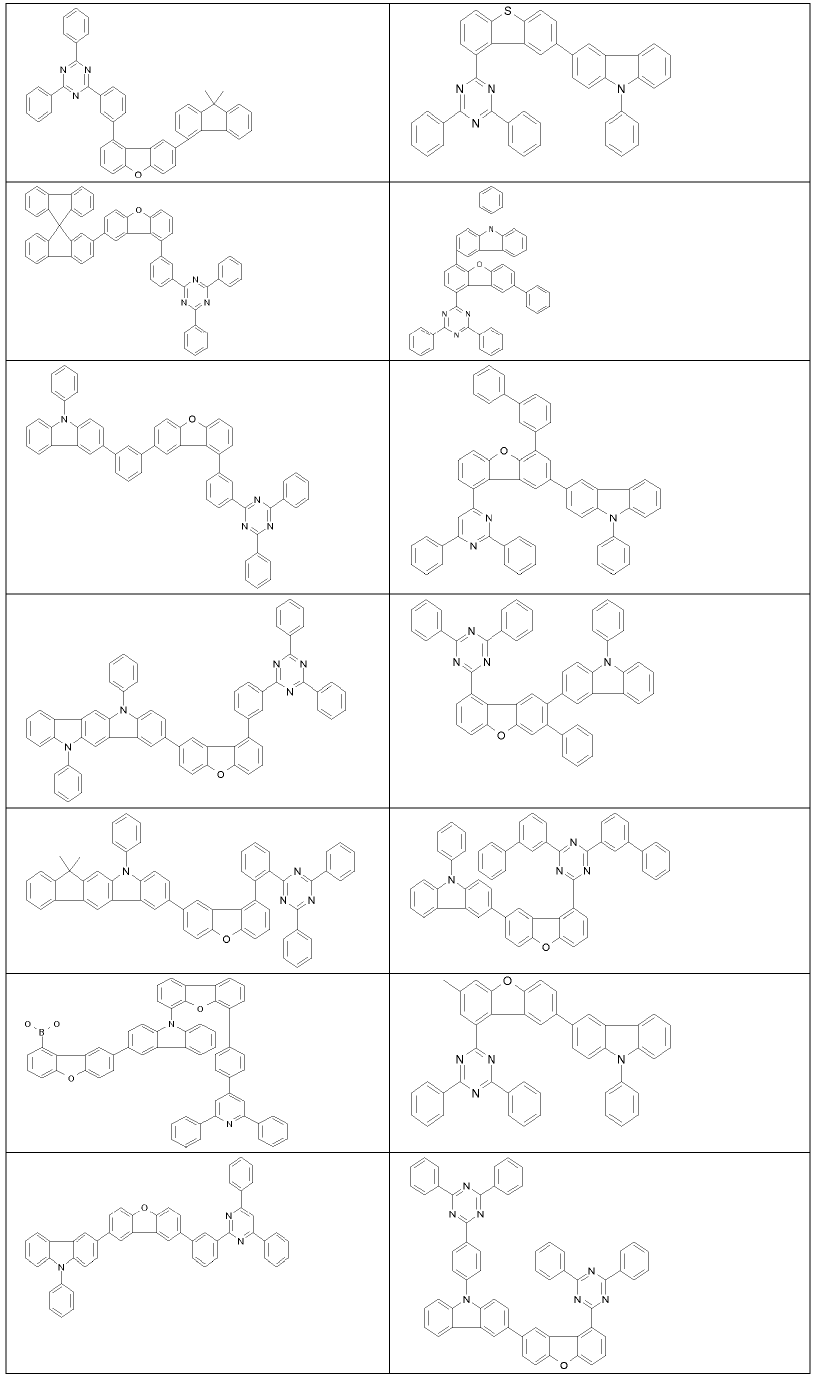

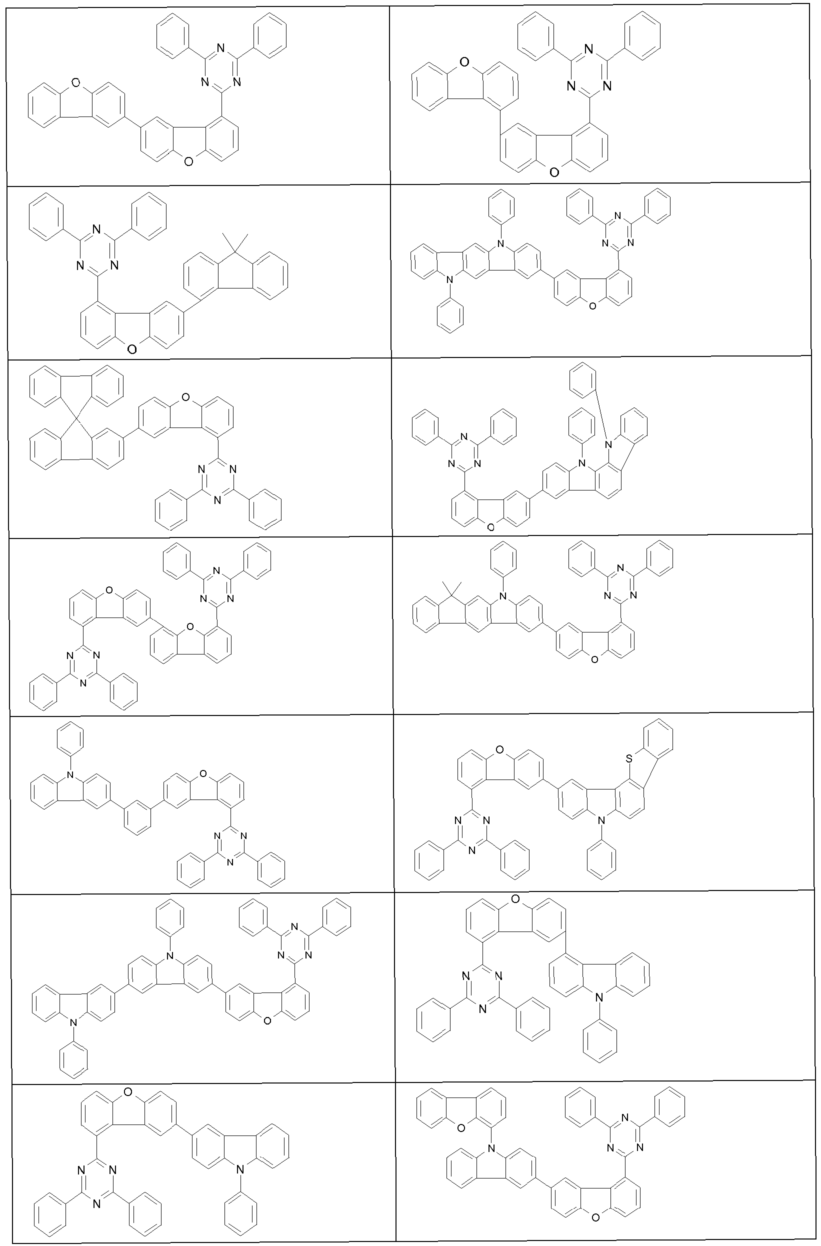

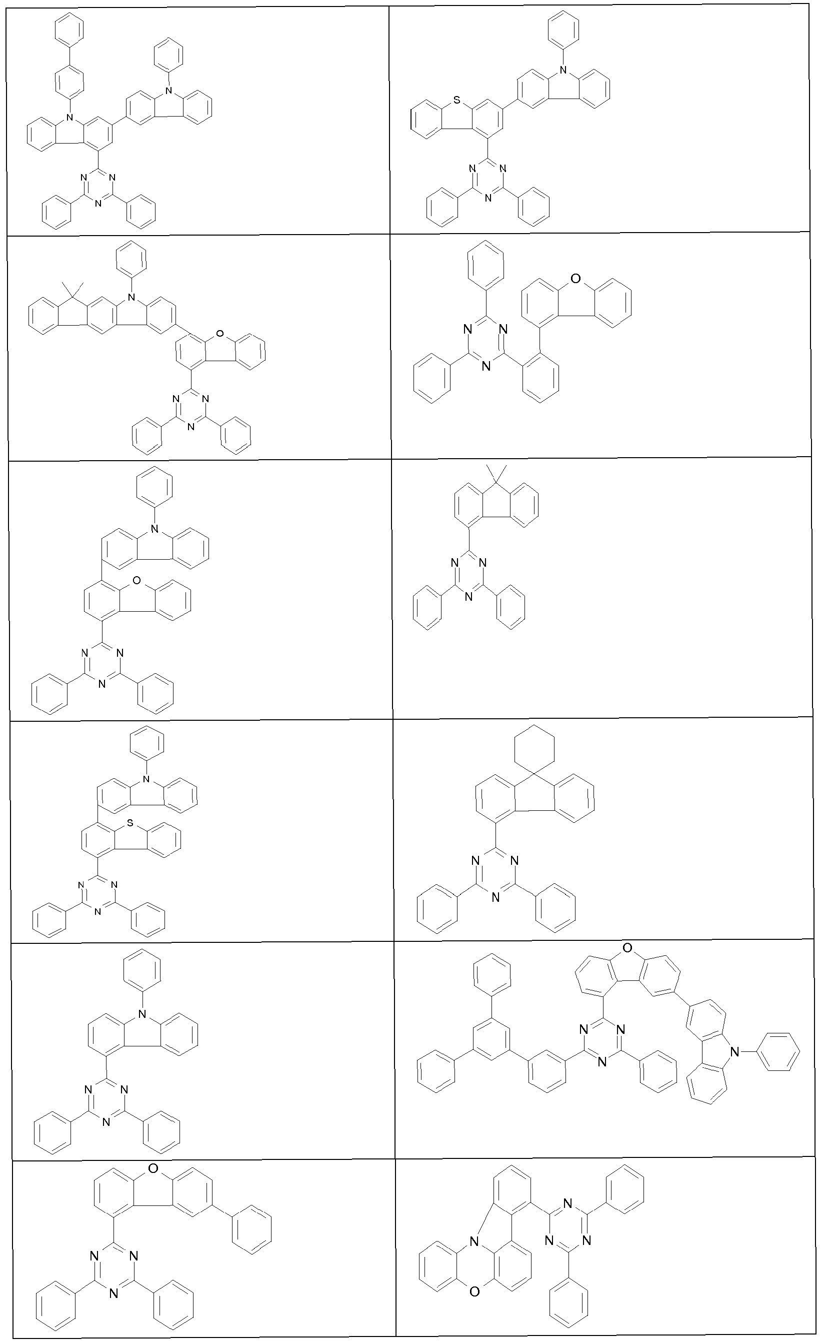

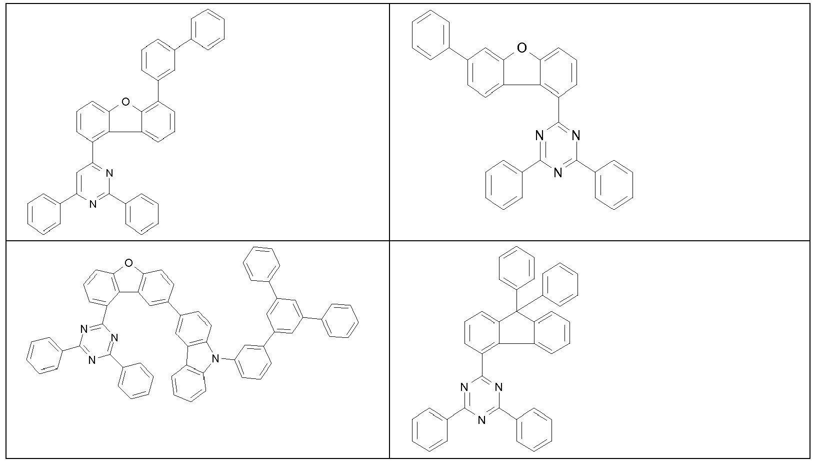

Beispiele für Lactame, welche als elektronentransportierende Matrixmaterialien eingesetzt werden können, sind die folgenden Strukturen:

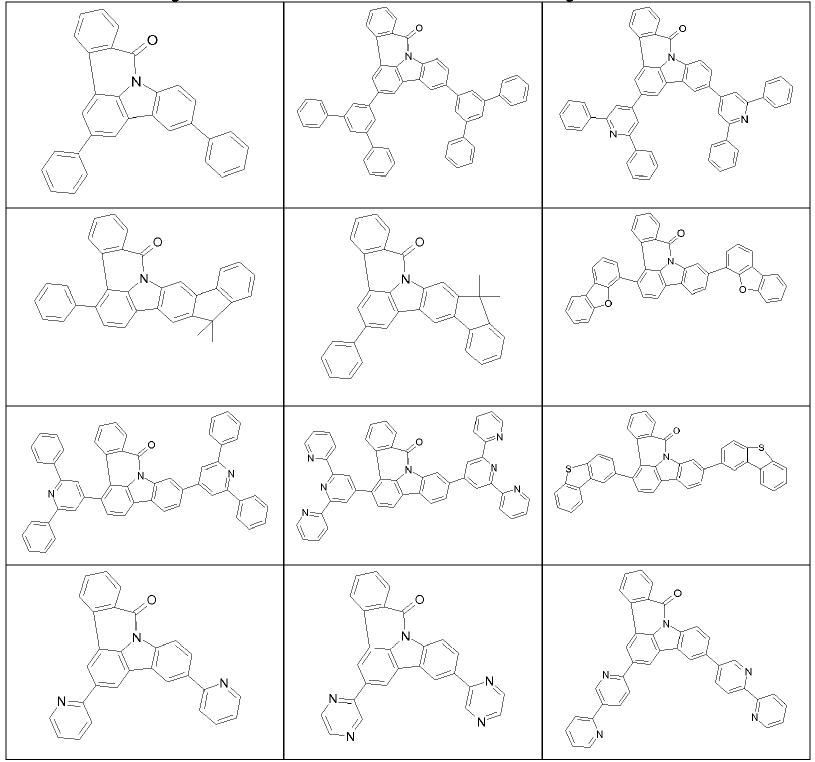

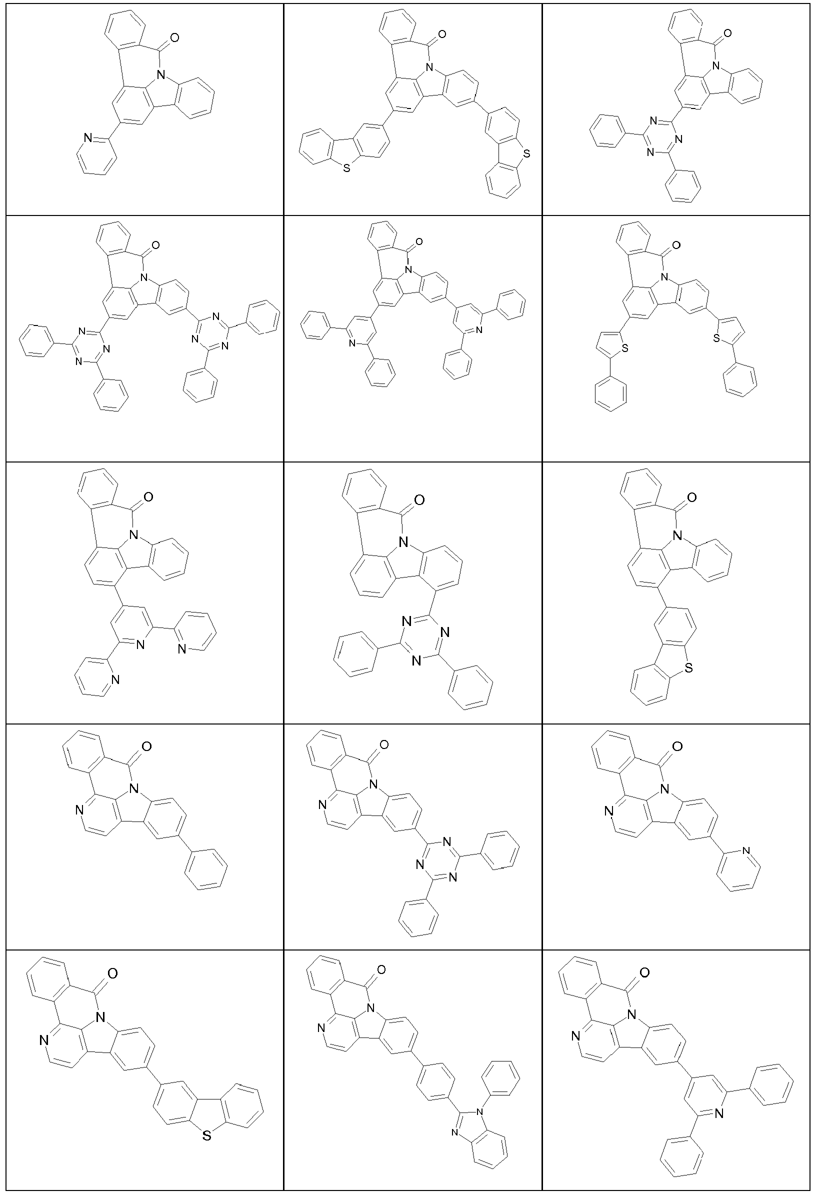

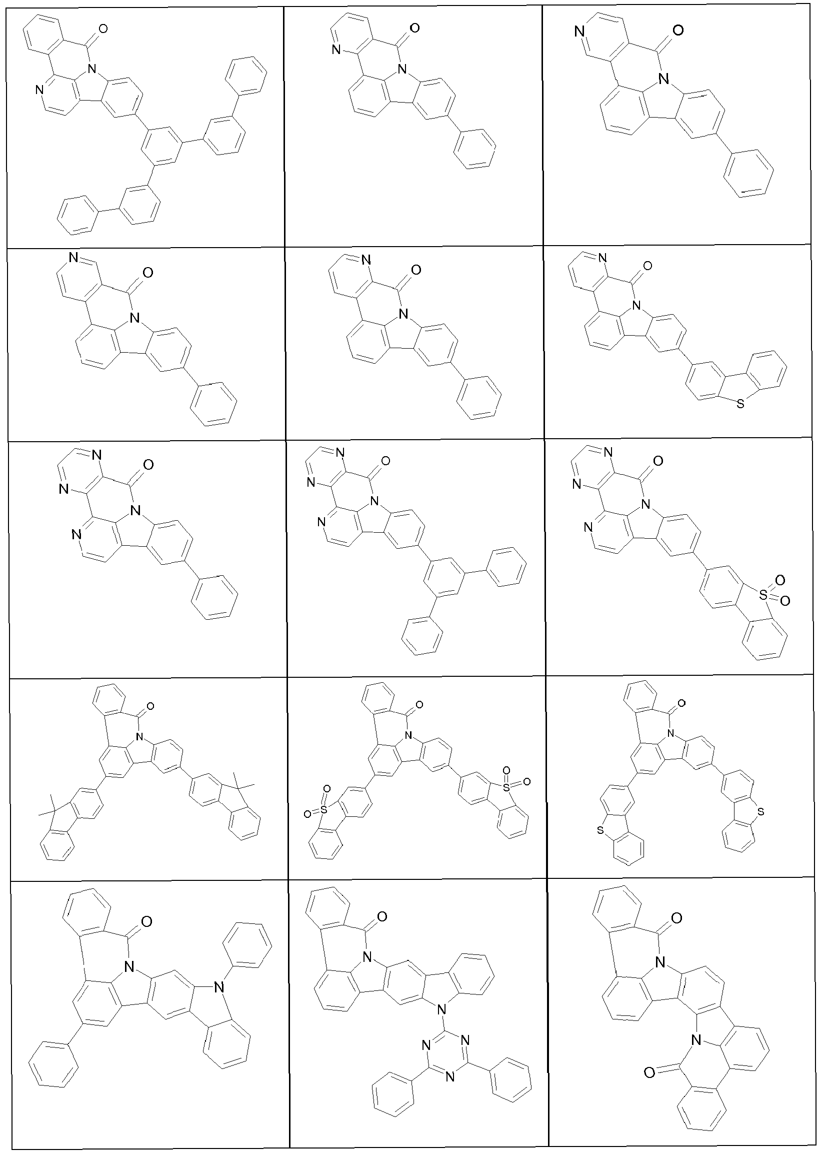

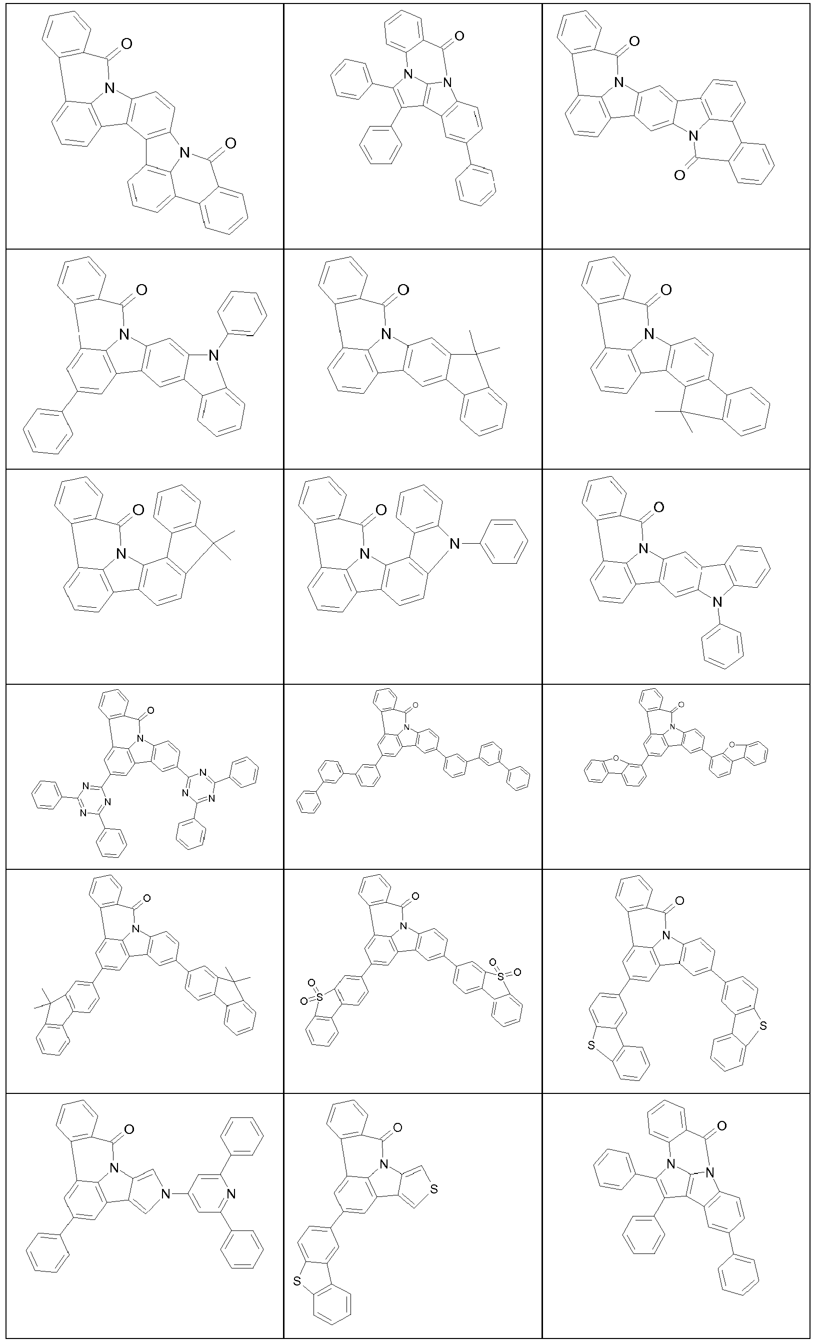

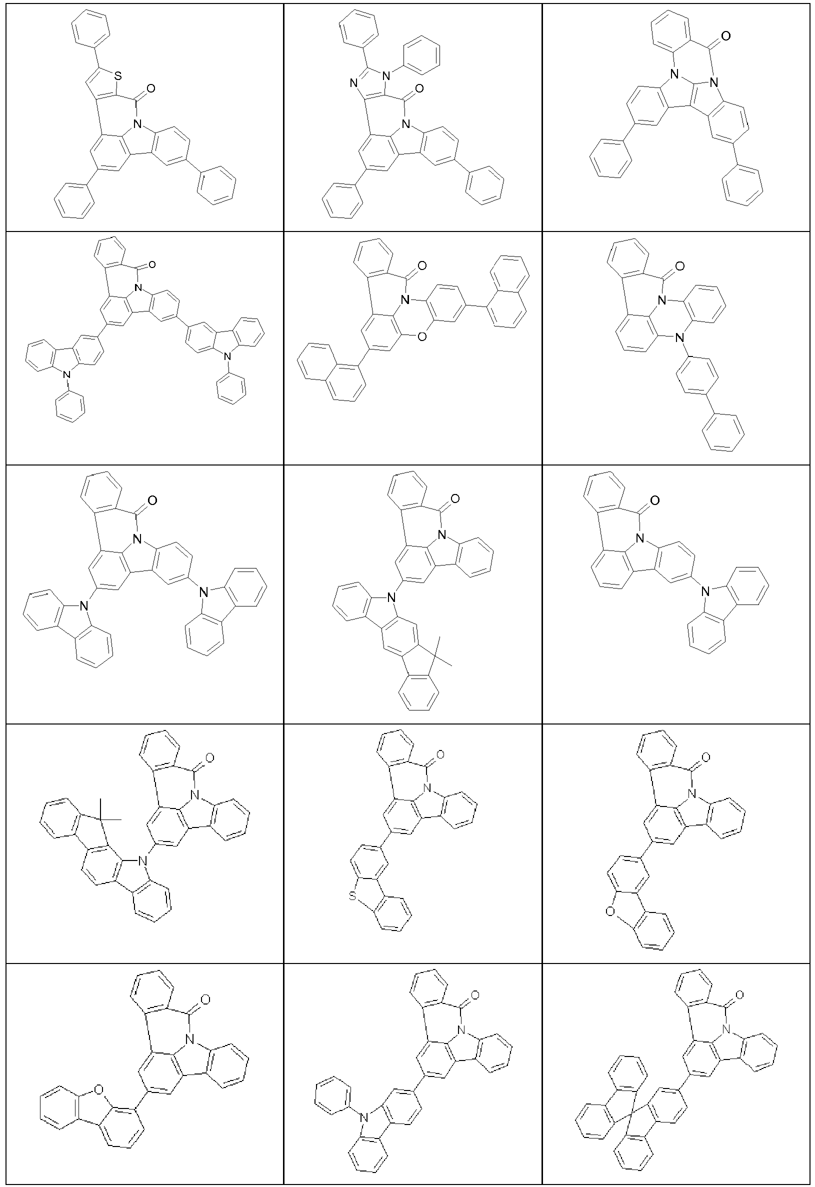

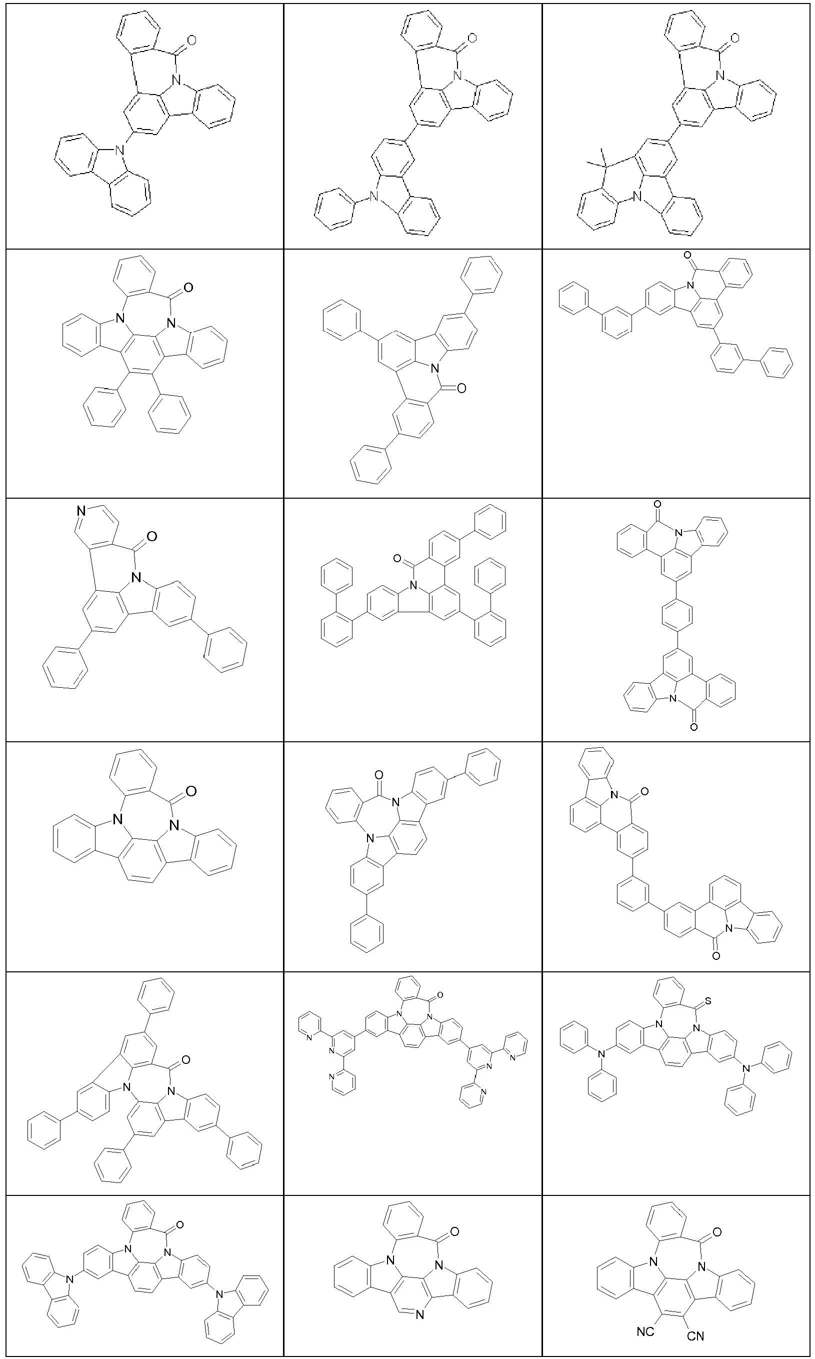

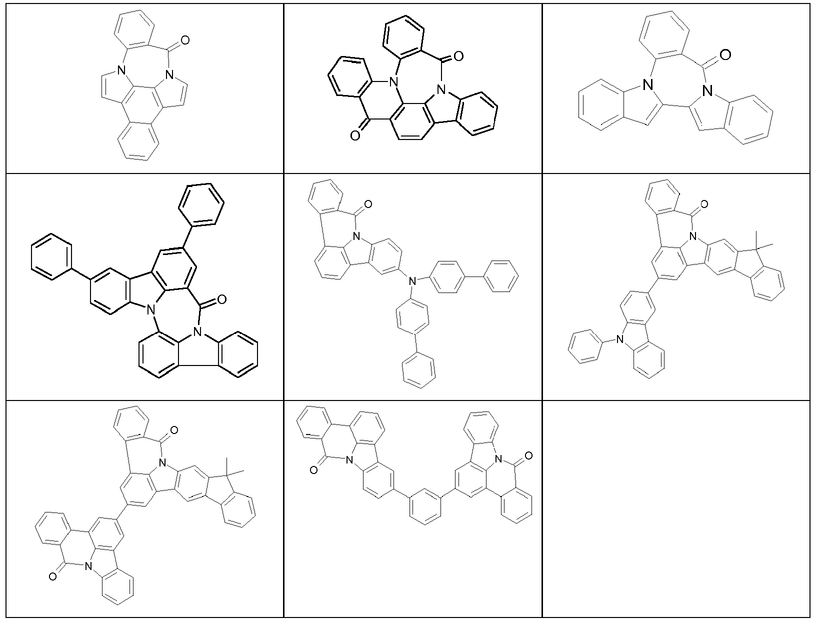

Beispiele für Ketonderivate, welche als elektronentransportierende Matrixmaterialien eingesetzt werden können sind die folgenden Strukturen:

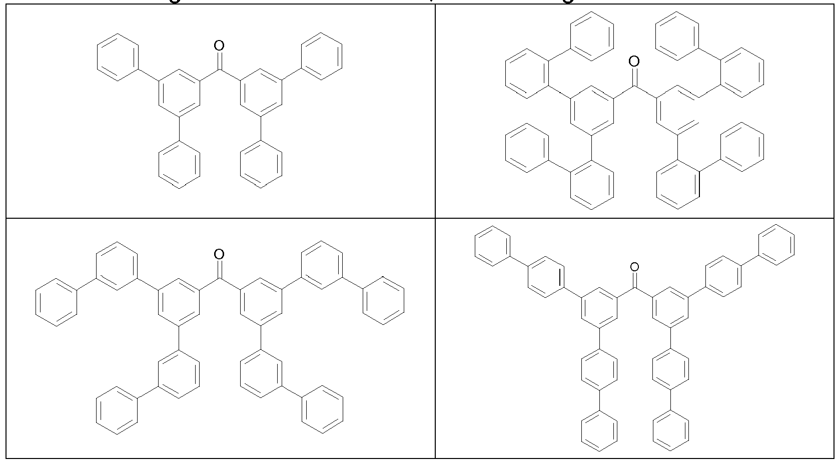

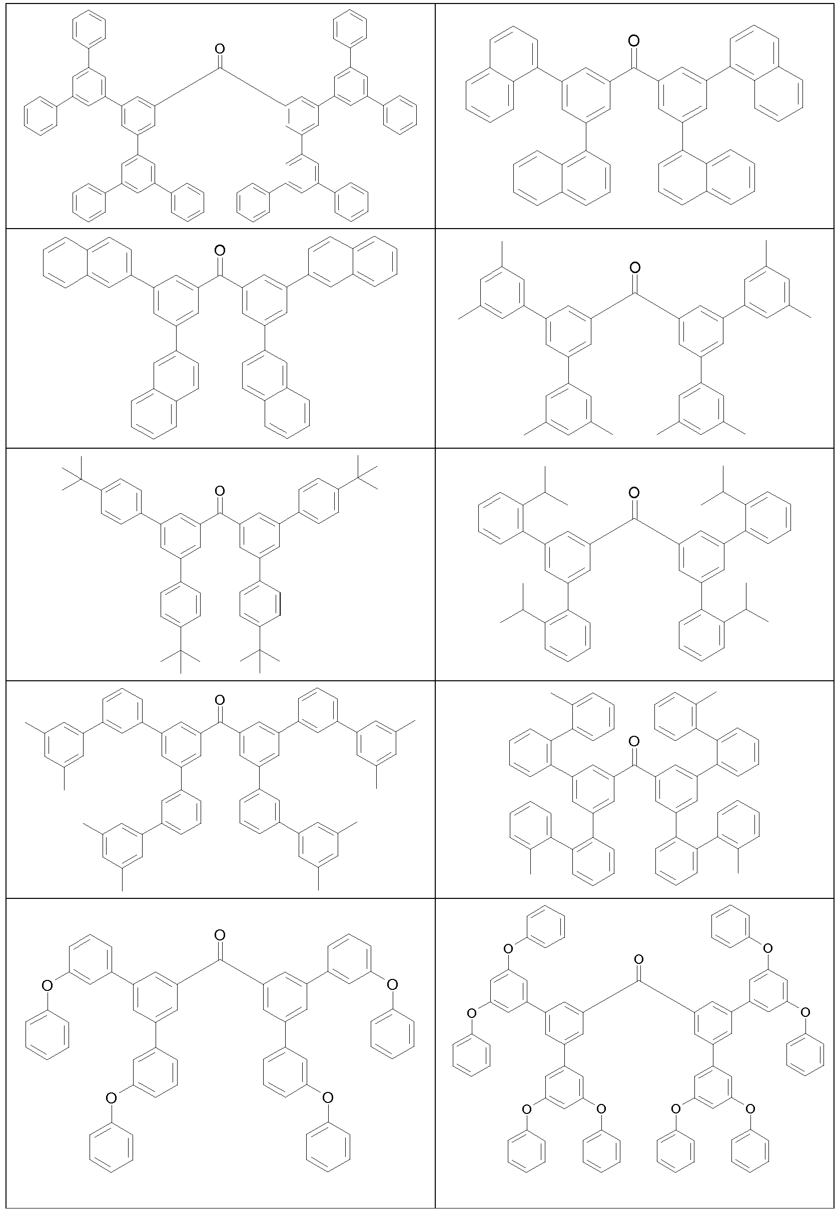

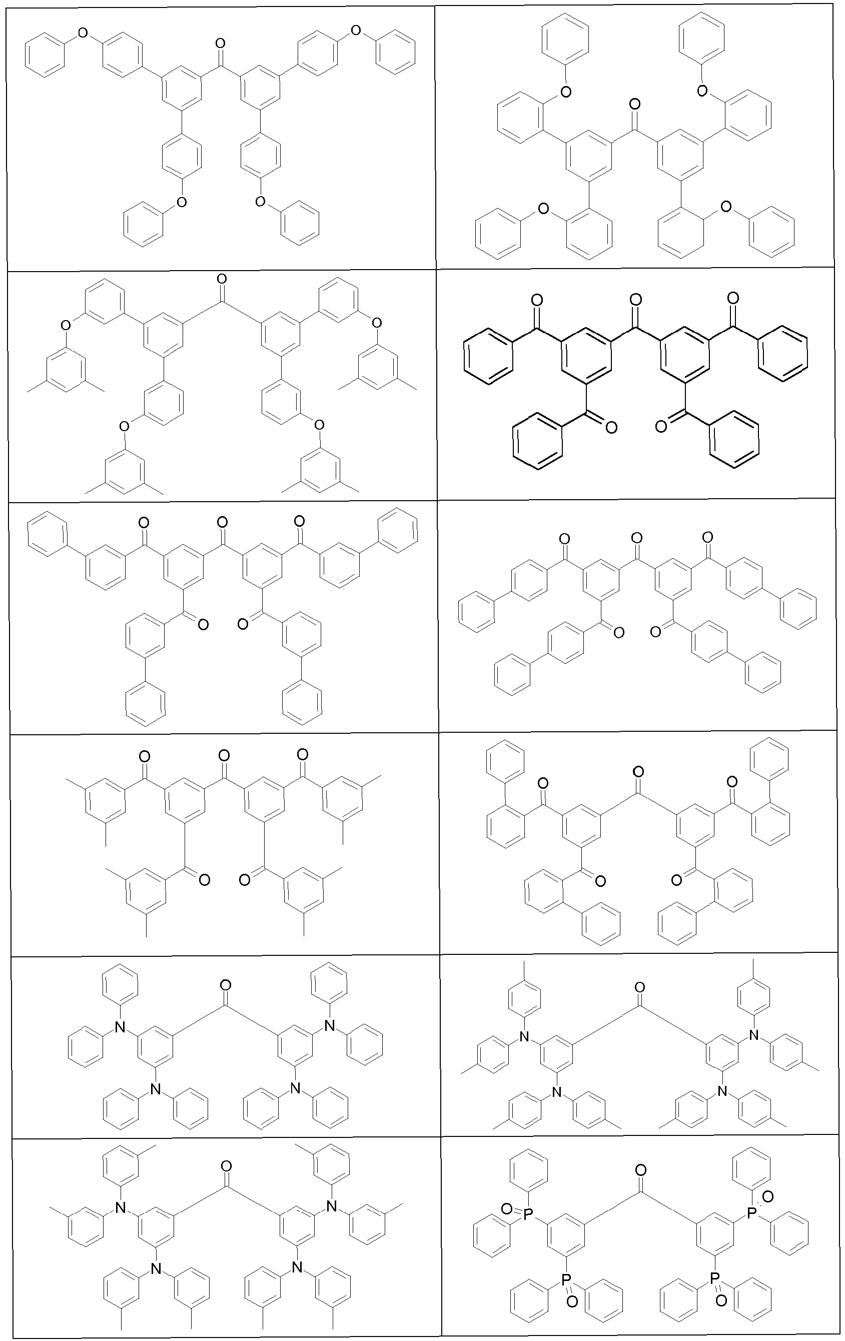

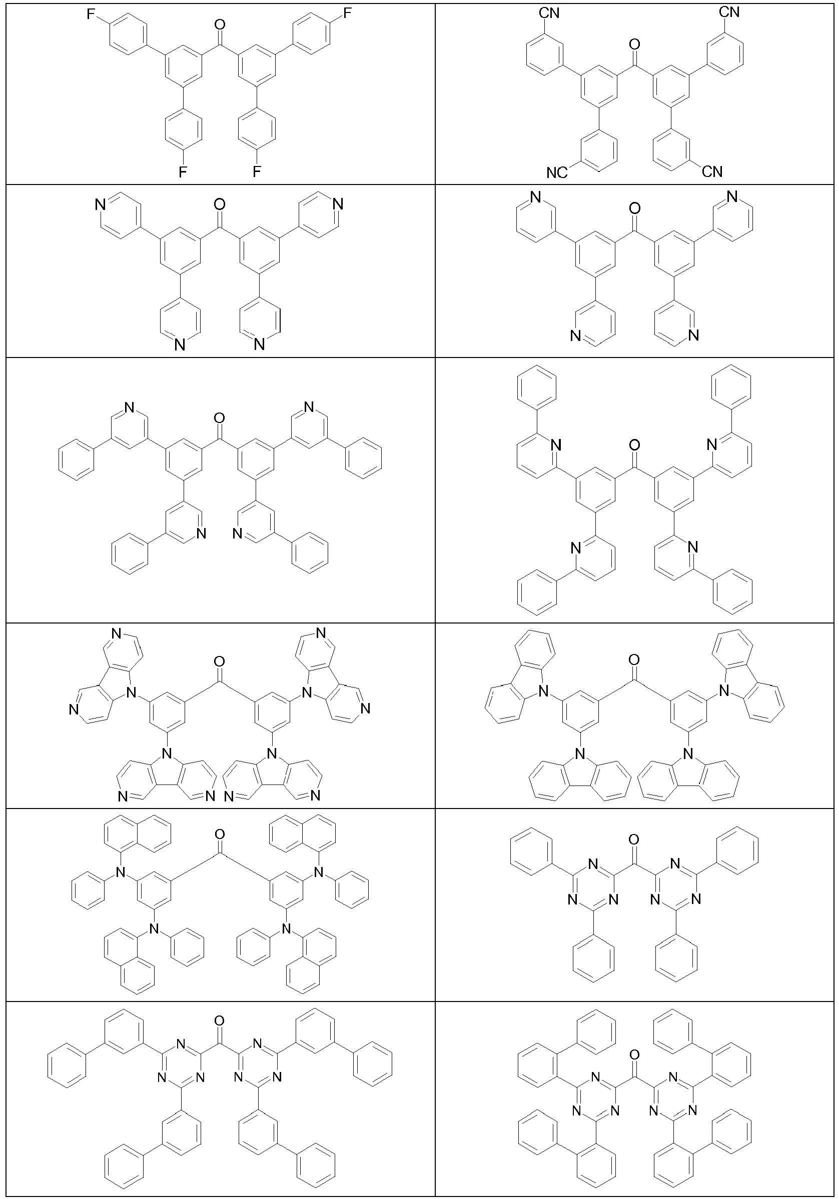

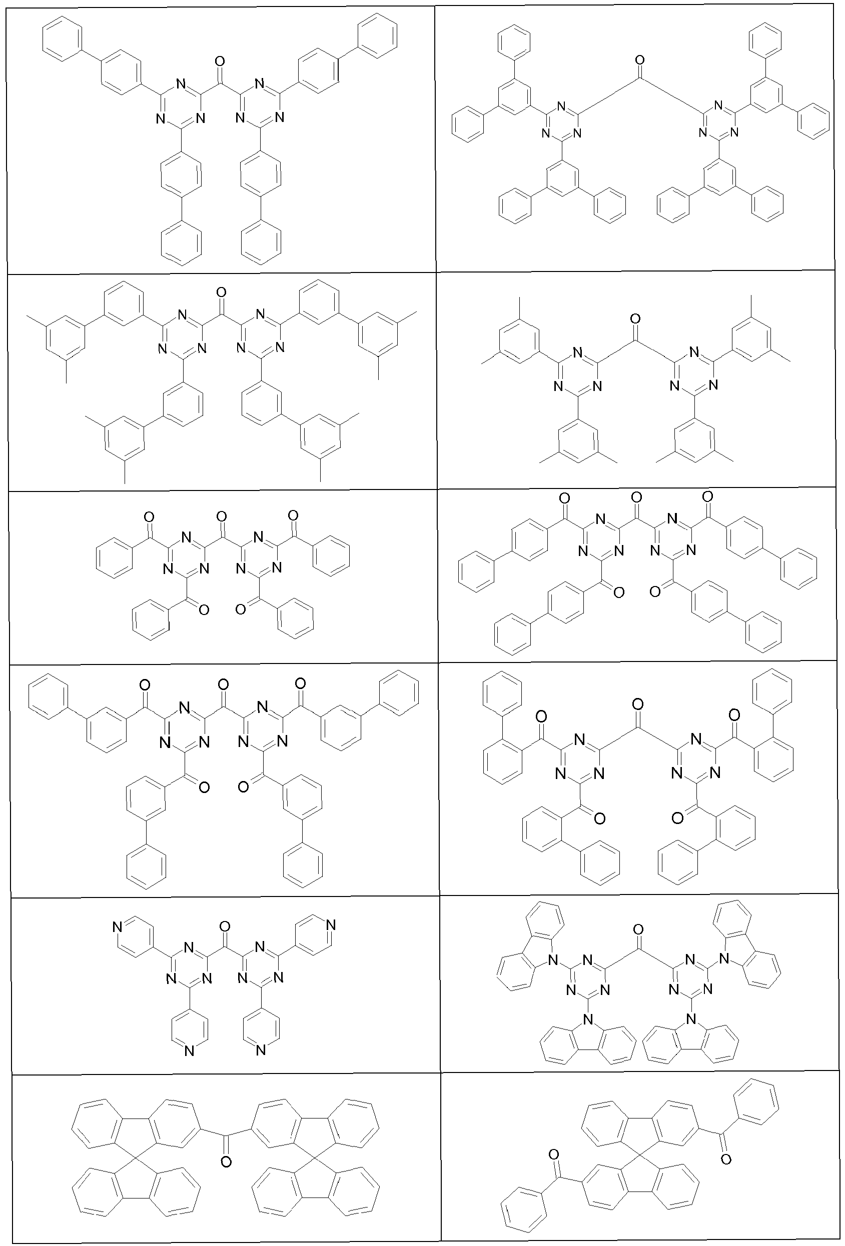

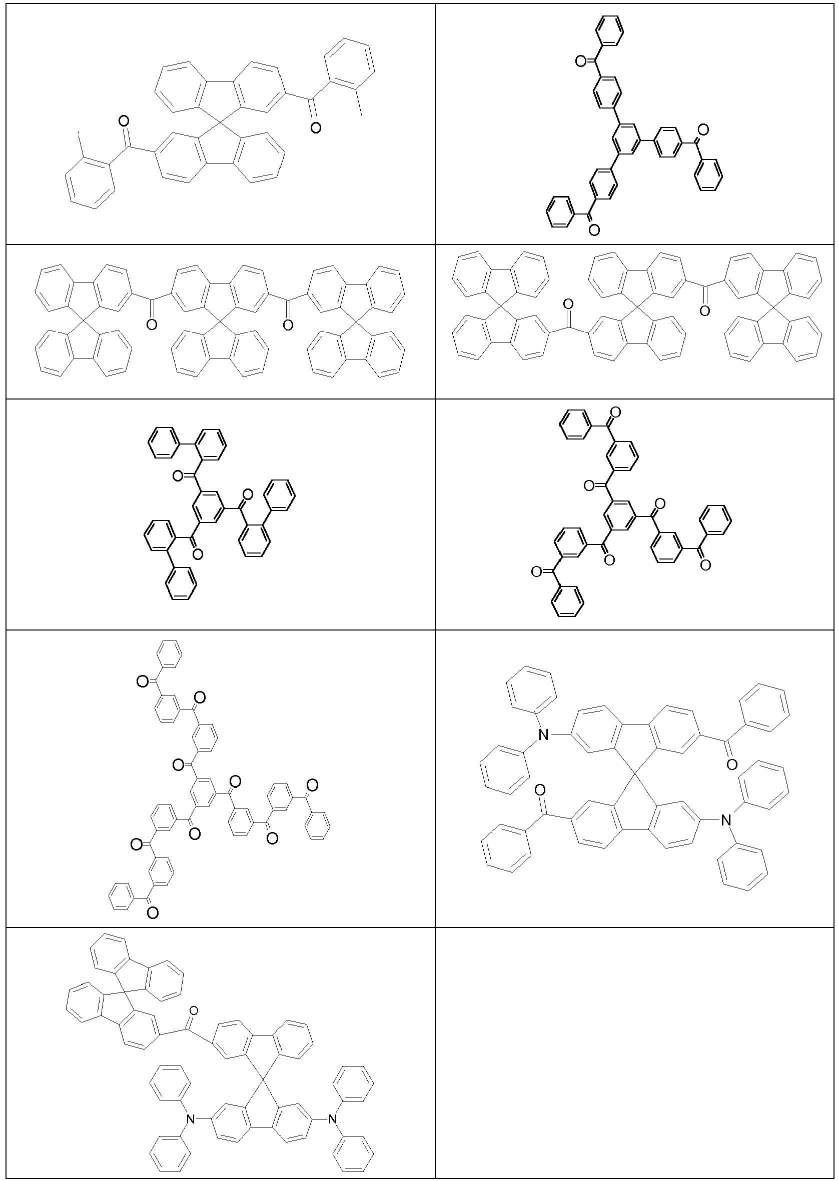

Beispiele für Metallkomplexe, welche als elektronentransportierende Matrixmaterialien eingesetzt werden können, sind die folgenden Strukturen:

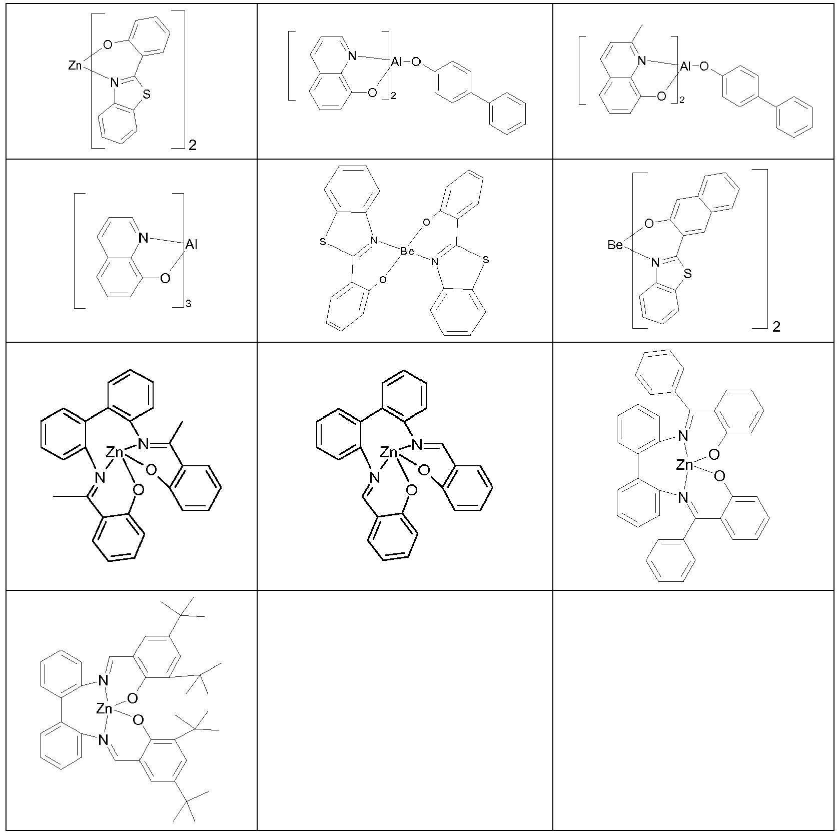

Beispiele für Phosphinoxide, welche als elektronentransportierende Matrixmaterialien eingesetzt werden können:

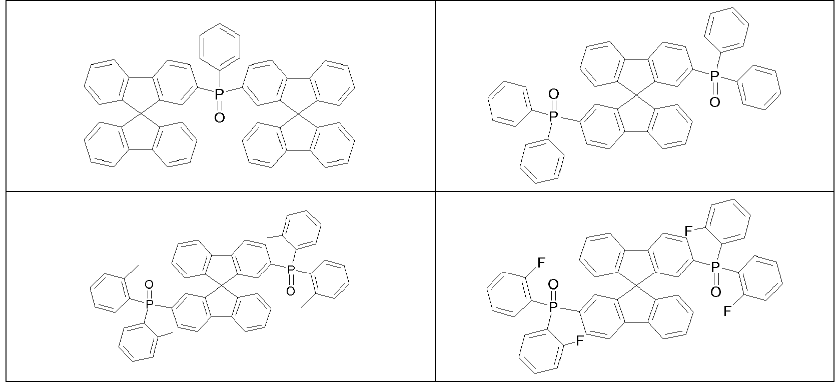

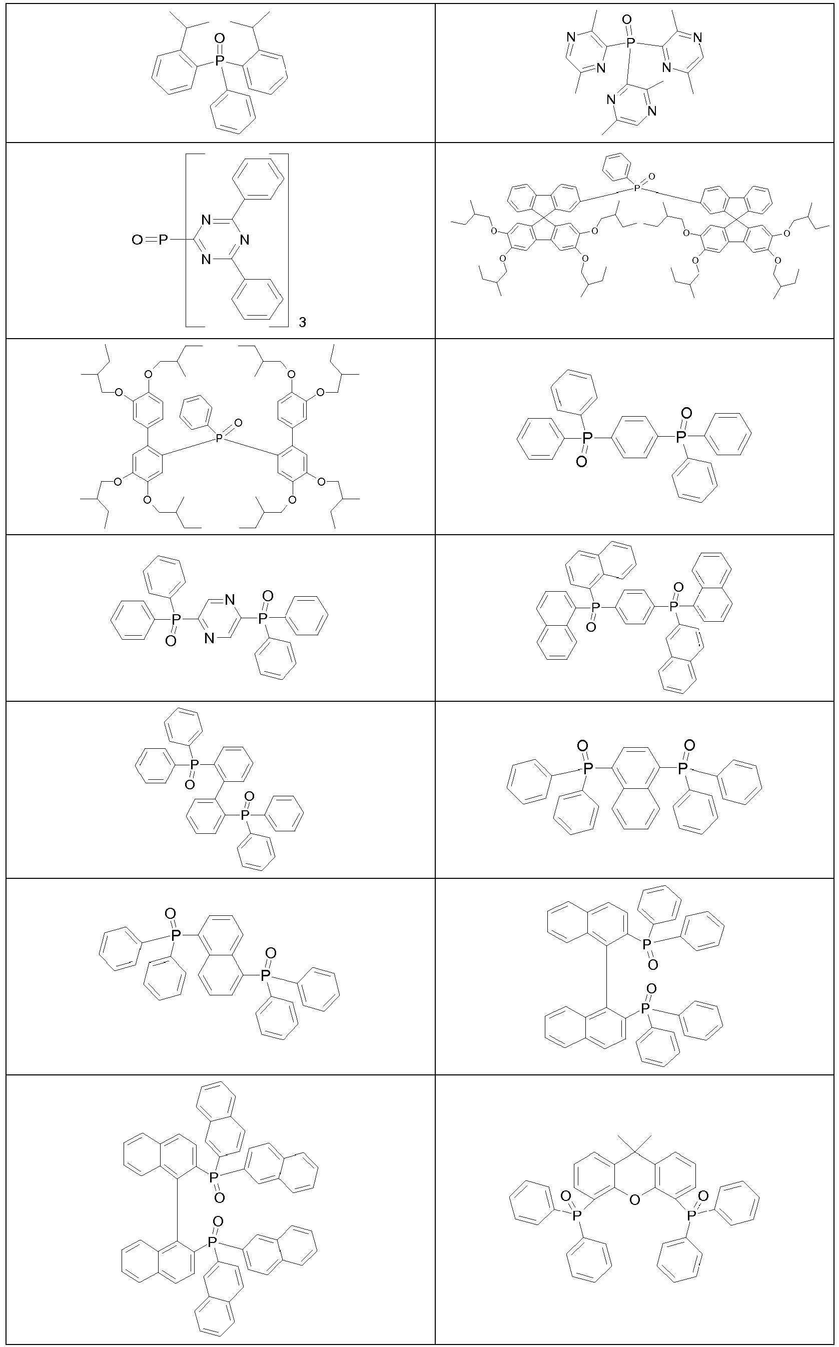

Beispiele für Indolo- und Indenocarbazolderivate im weitesten Sinn, welche je nach Substitutionsmuster als loch- oder elektronentransportierende Matrixmaterialien eingesetzt werden können, sind die folgenden Strukturen:

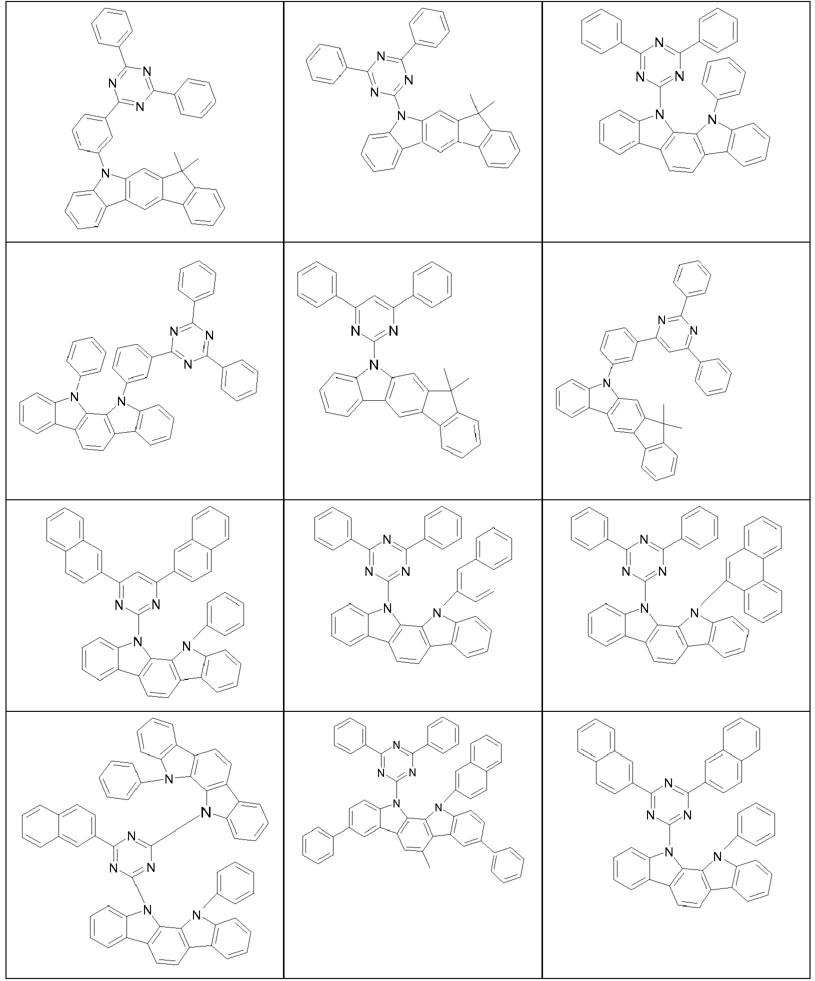

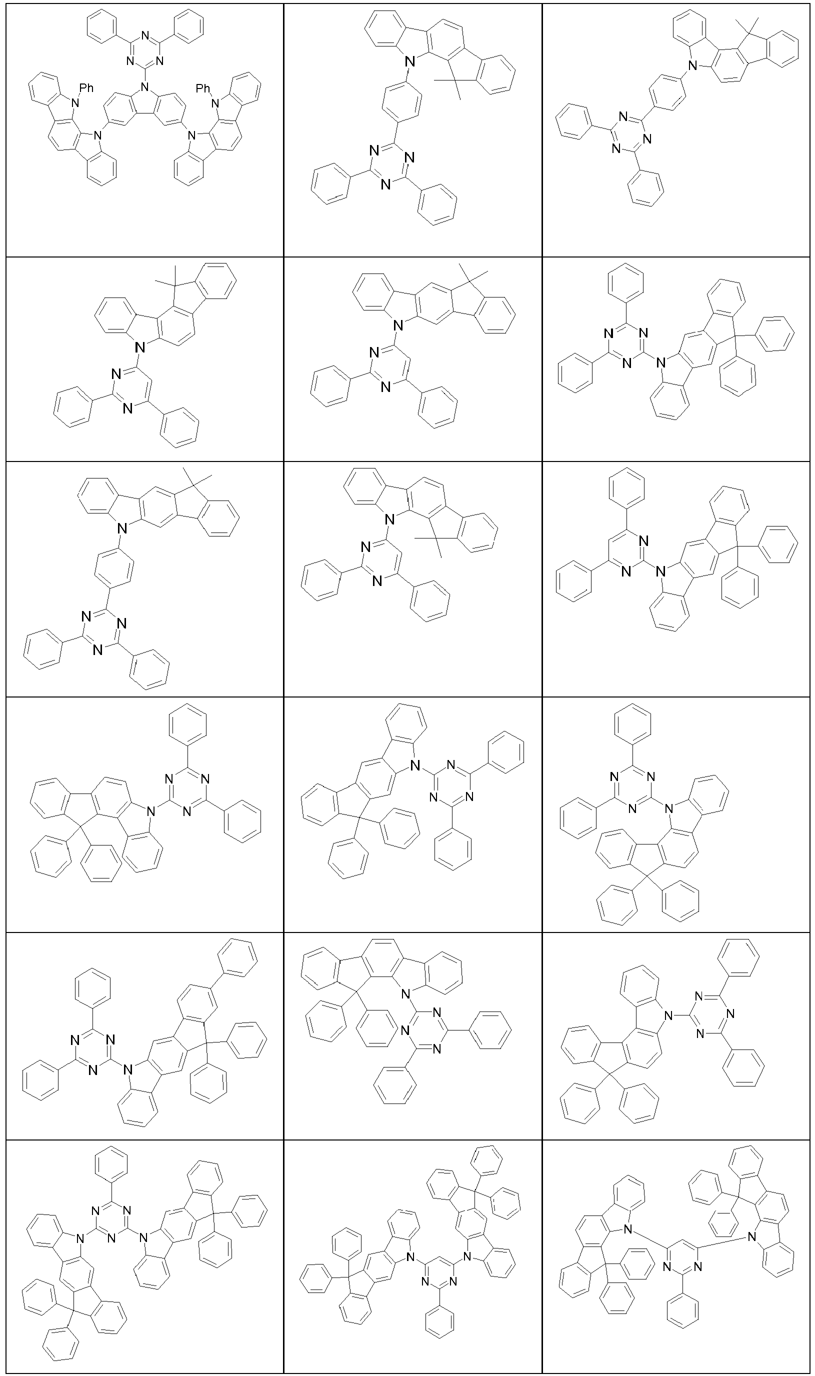

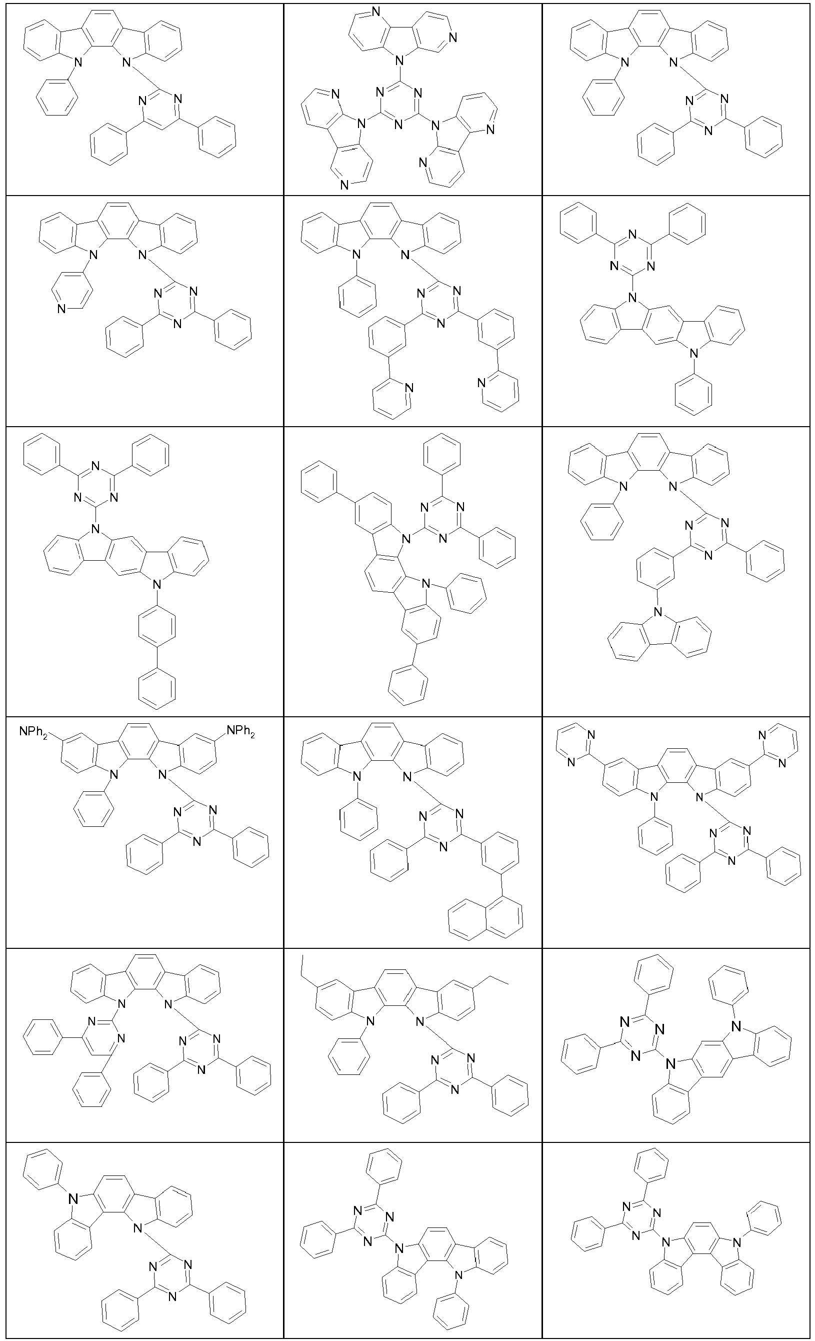

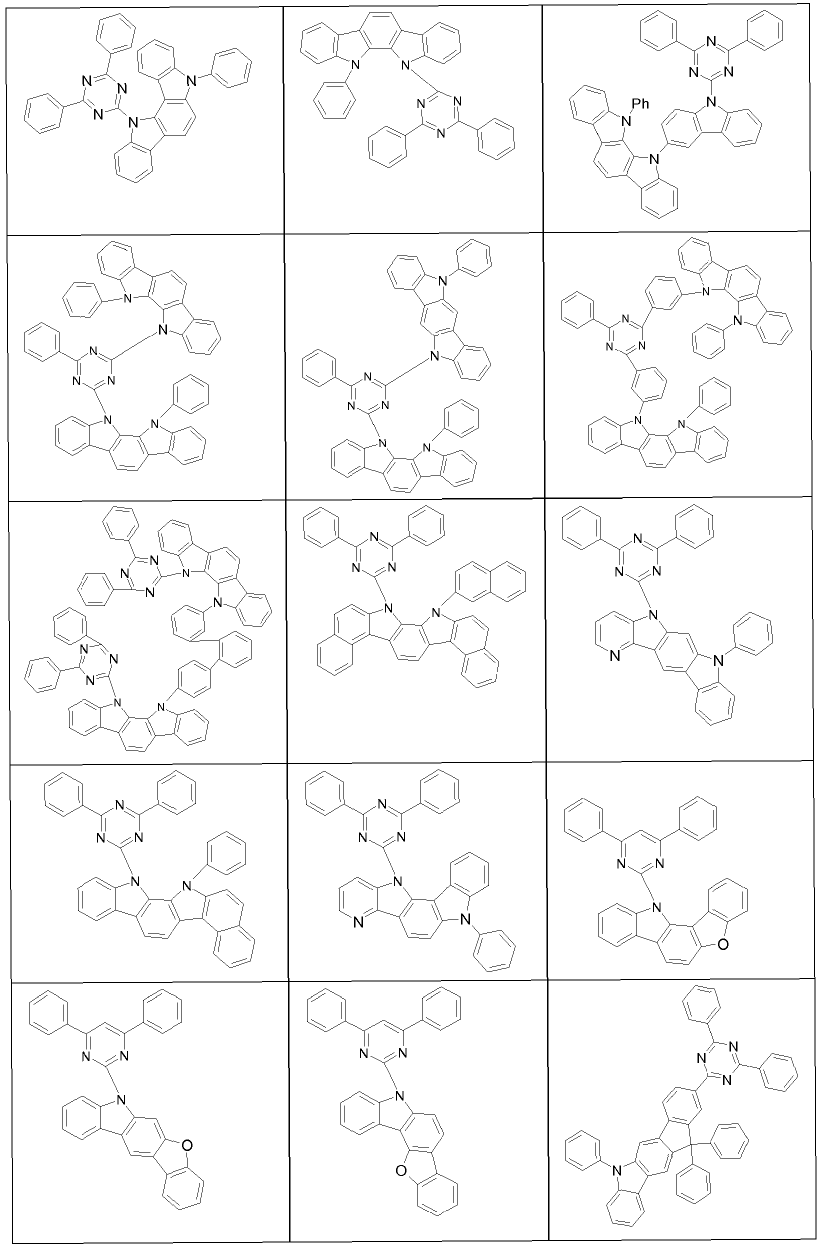

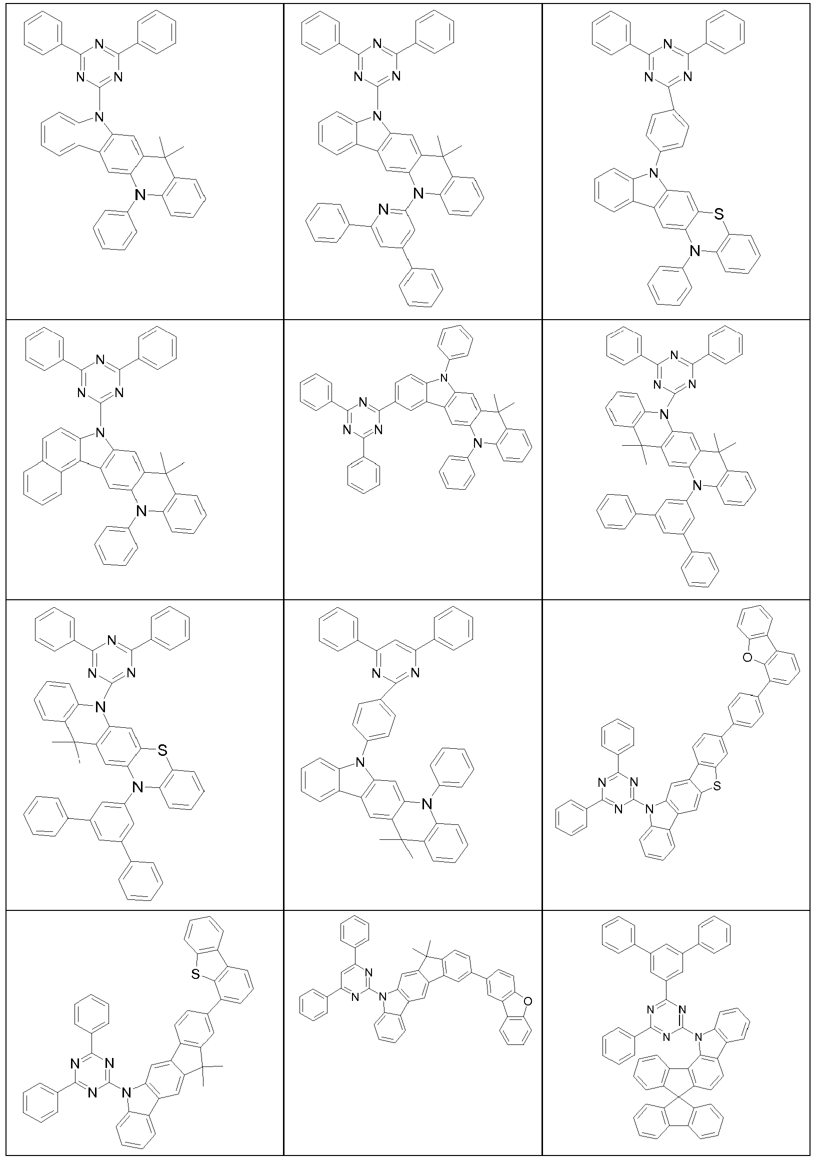

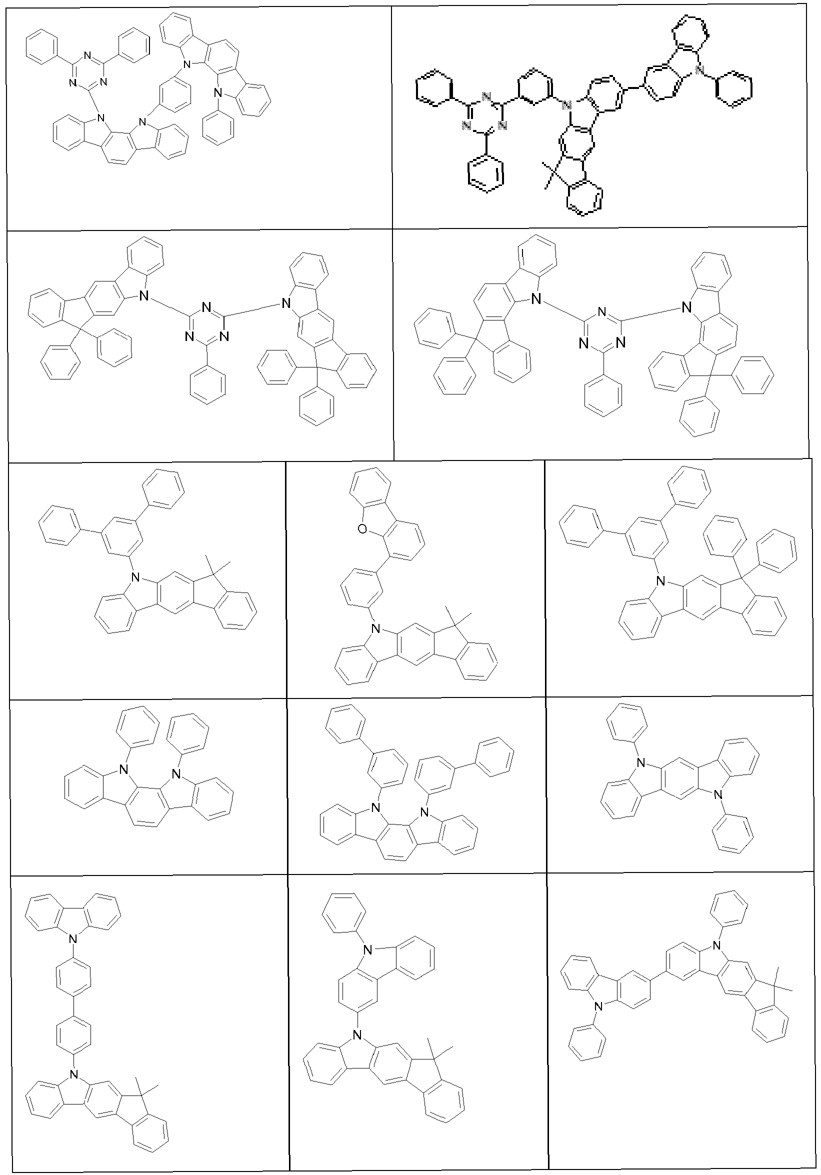

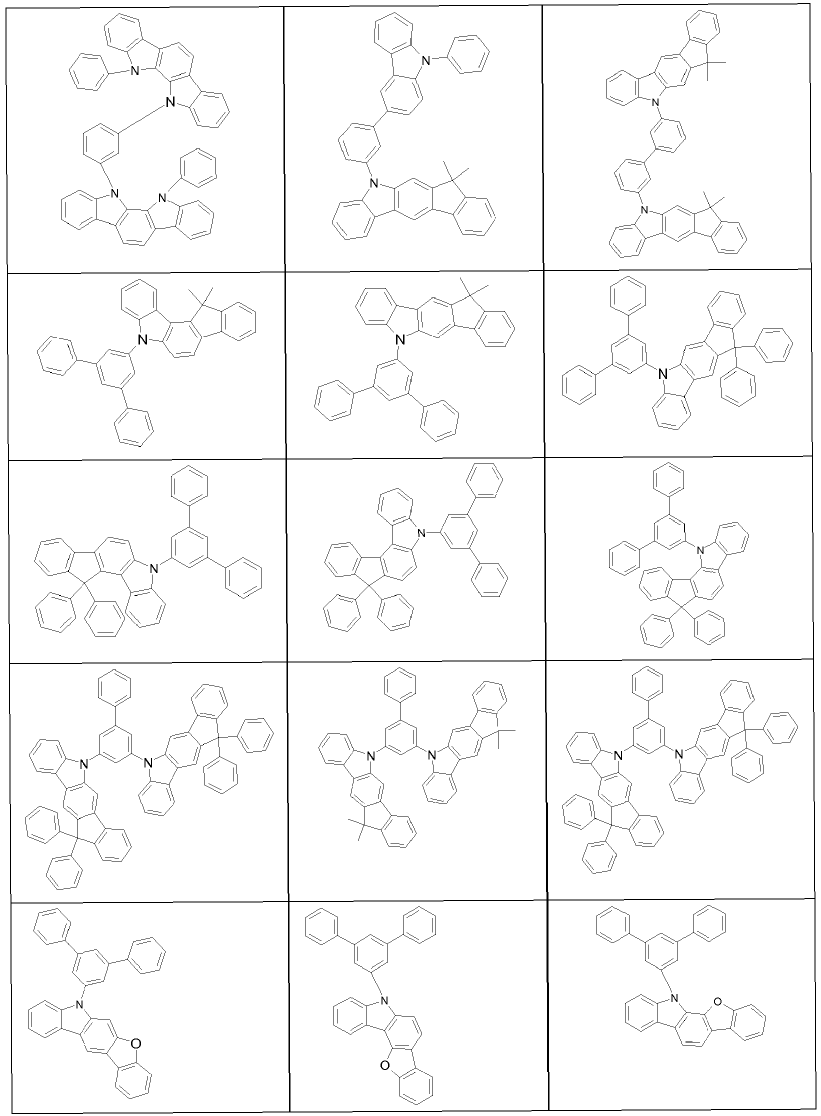

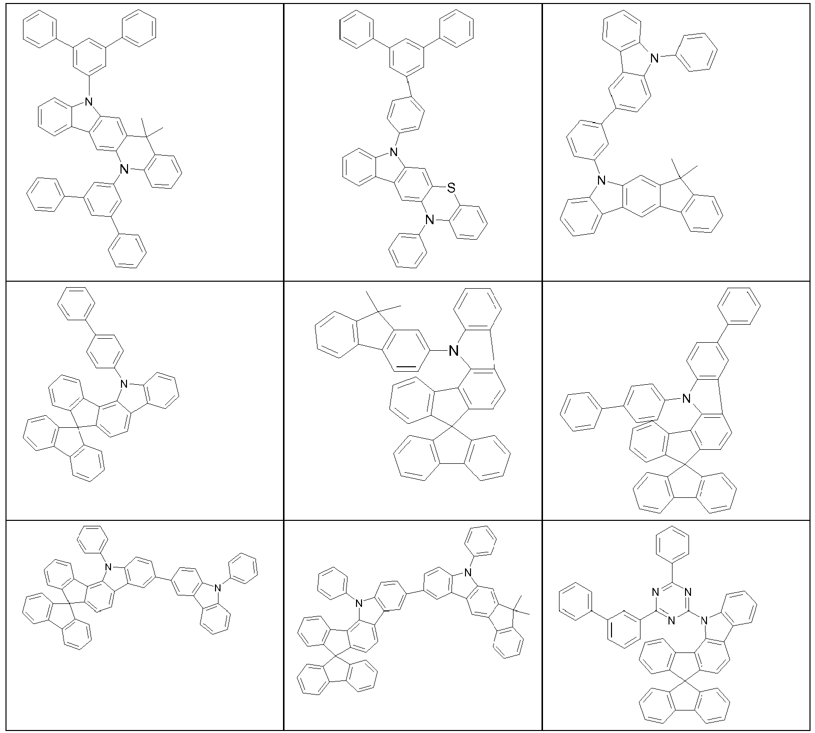

Beispiele für Carbazolderivate, welche je nach Substitutionsmuster als loch- oder elektronentransportierende Matrixmaterialien eingesetzt werden können sind die folgenden Strukturen:

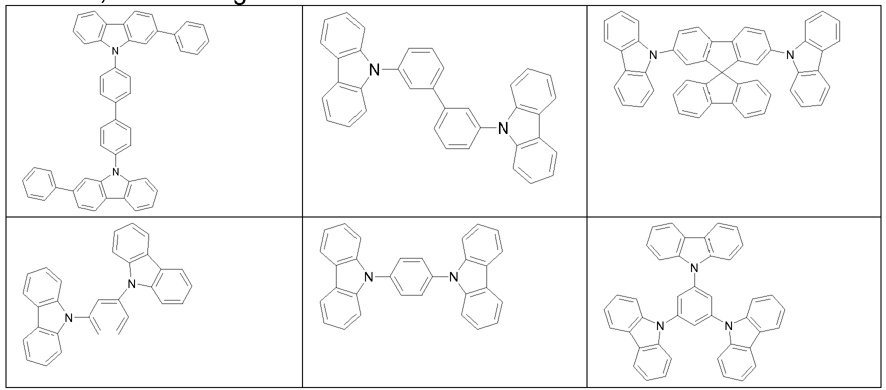

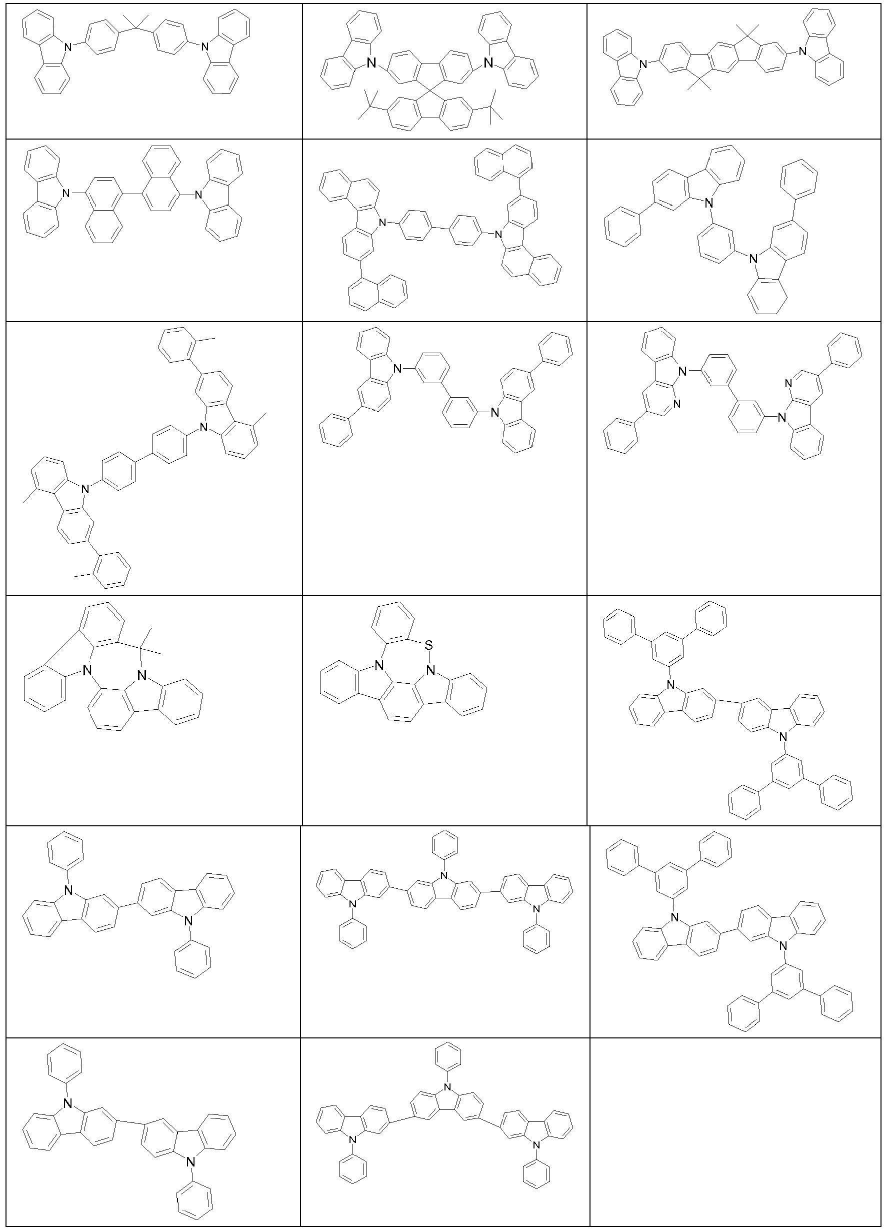

Beispiele für verbrückte Carbazolderivate, welche als lochtransportierende Matrixmaterialien eingesetzt werden können:

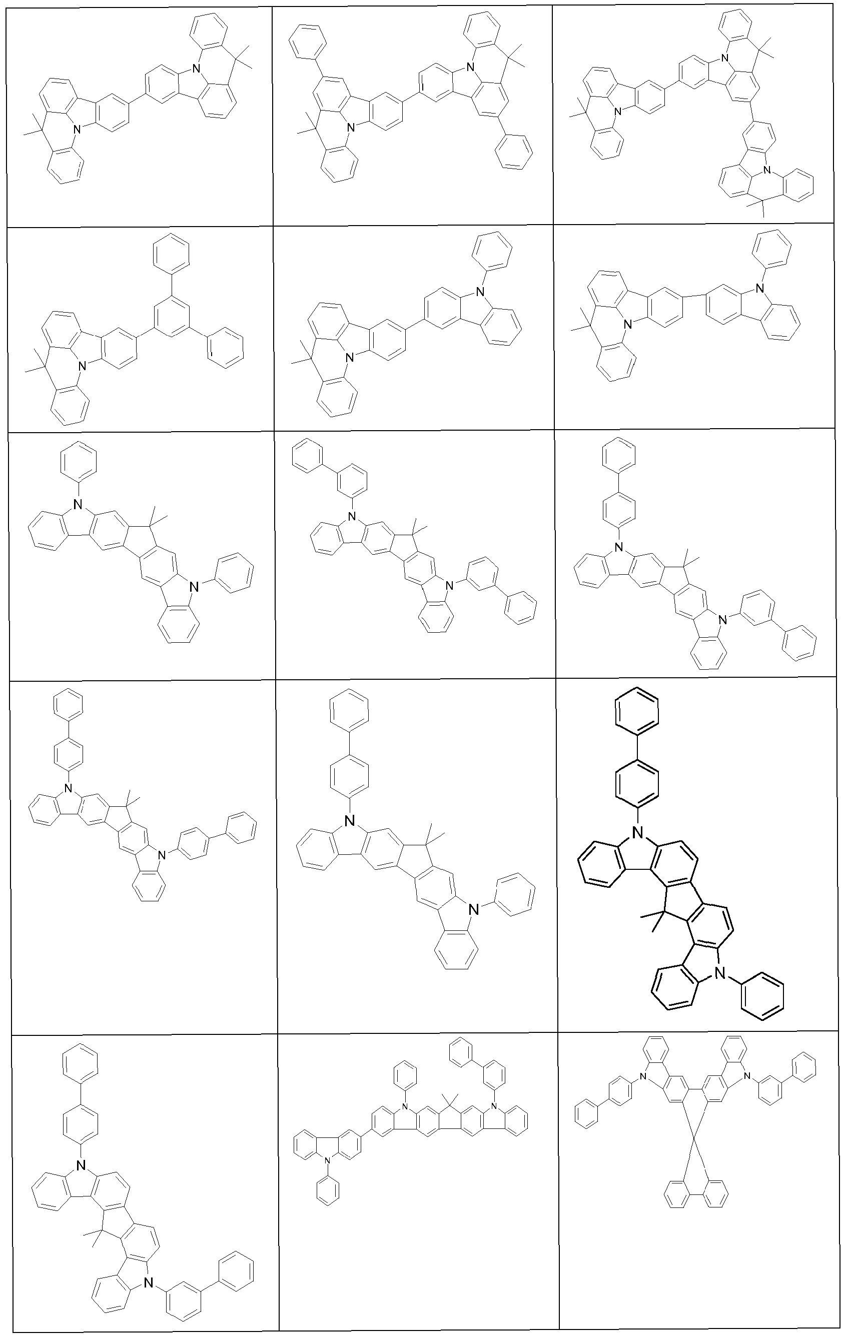

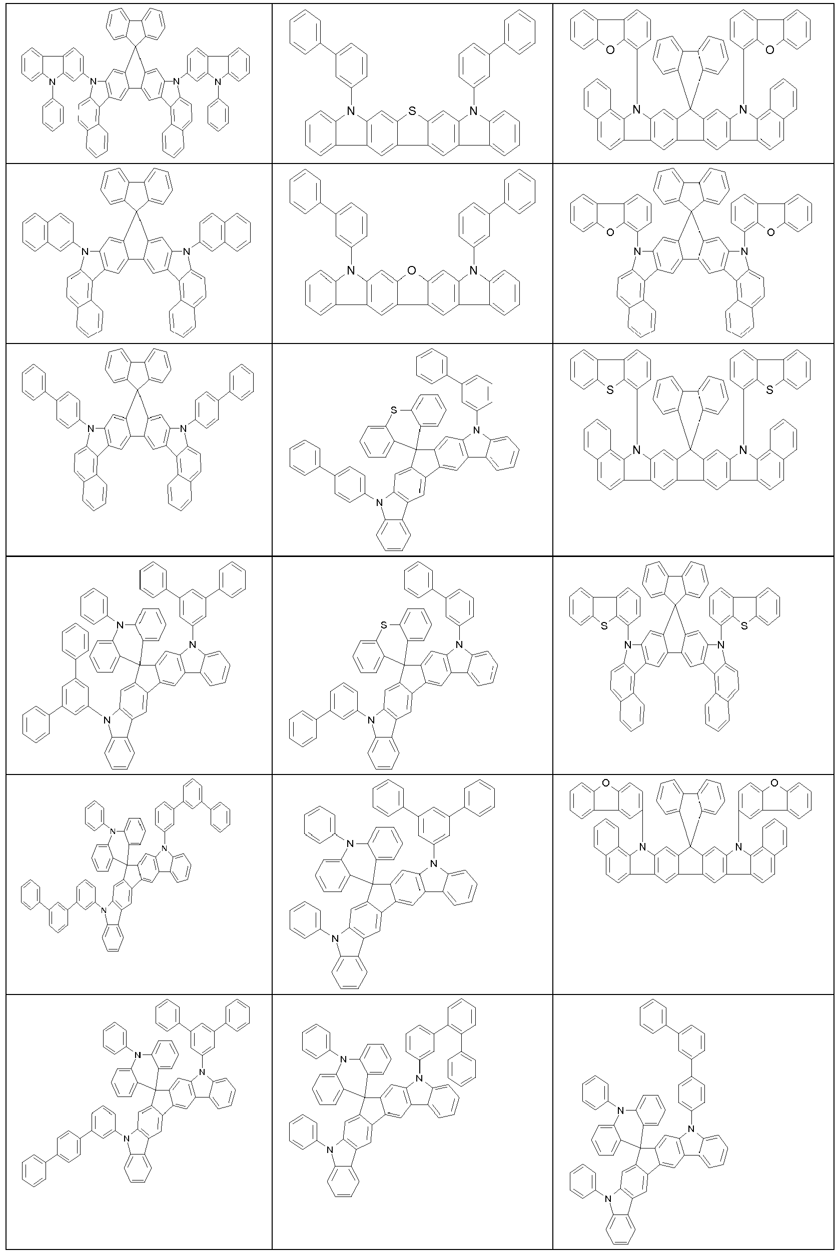

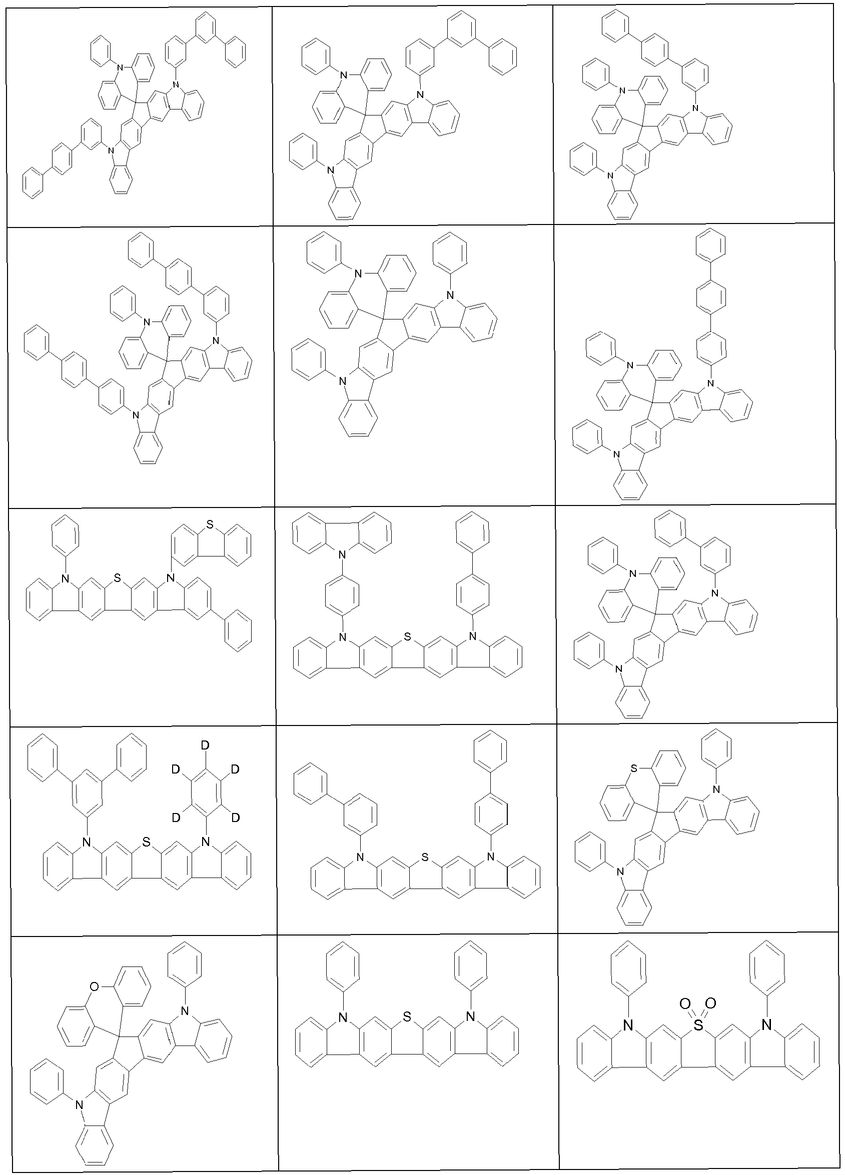

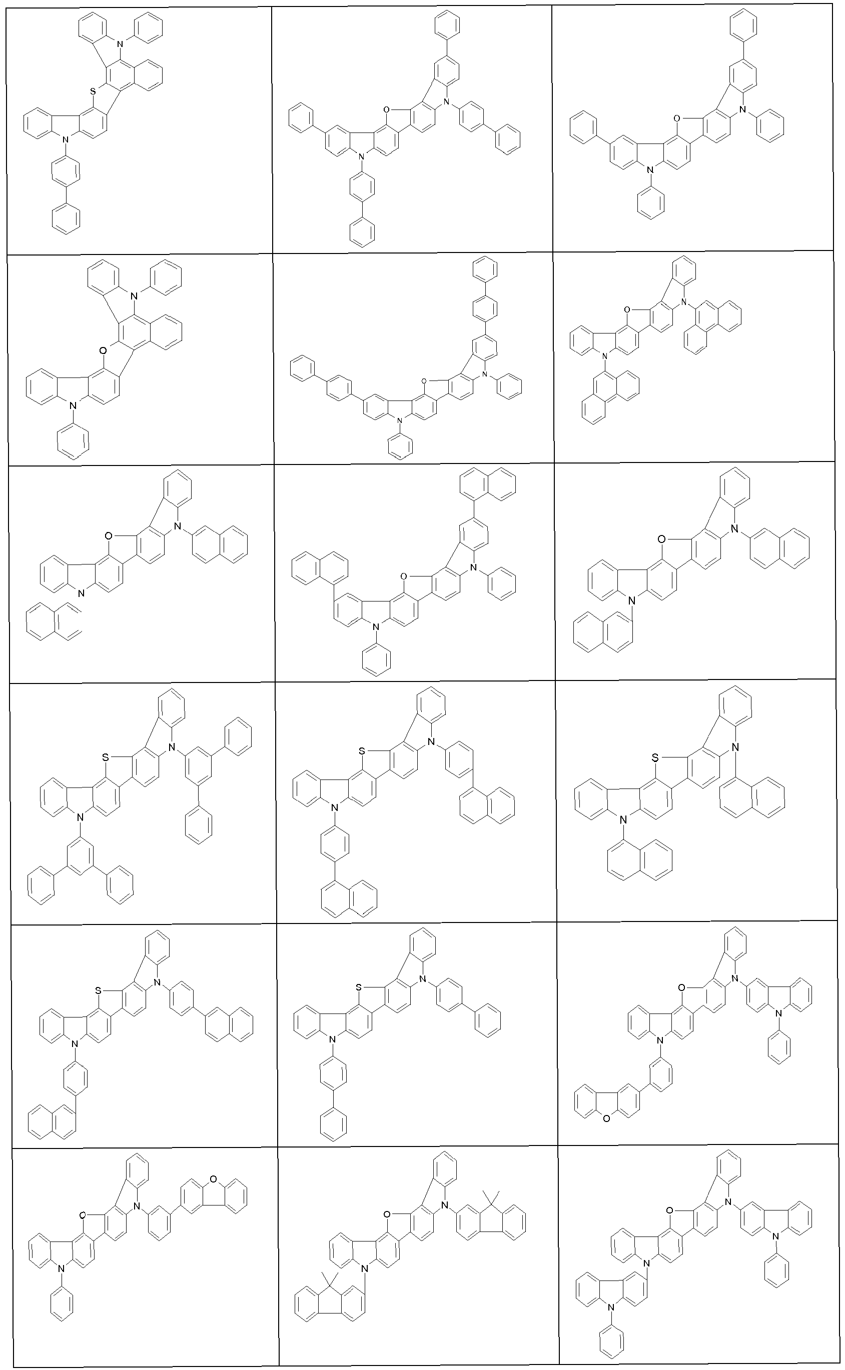

Beispiele für Biscarbazolderivate, welche als lochtransportierende Matrixmaterialien eingesetzt werden können:

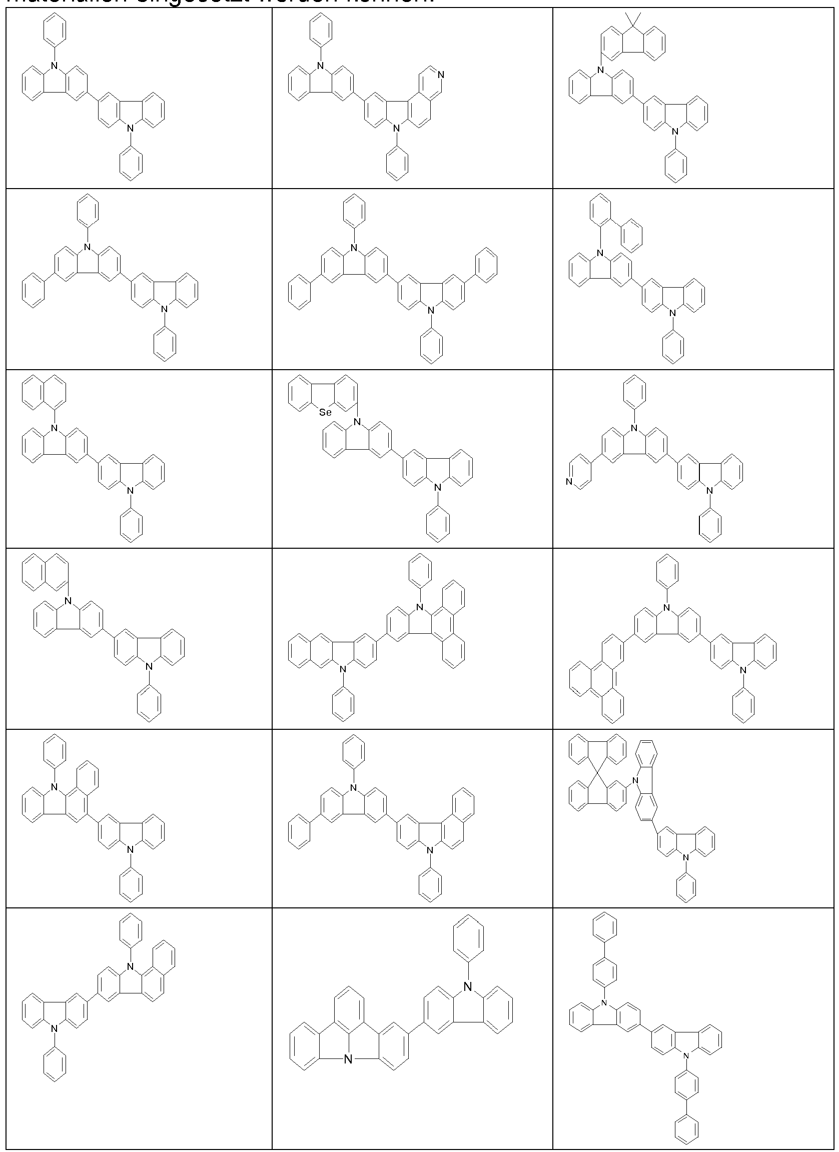

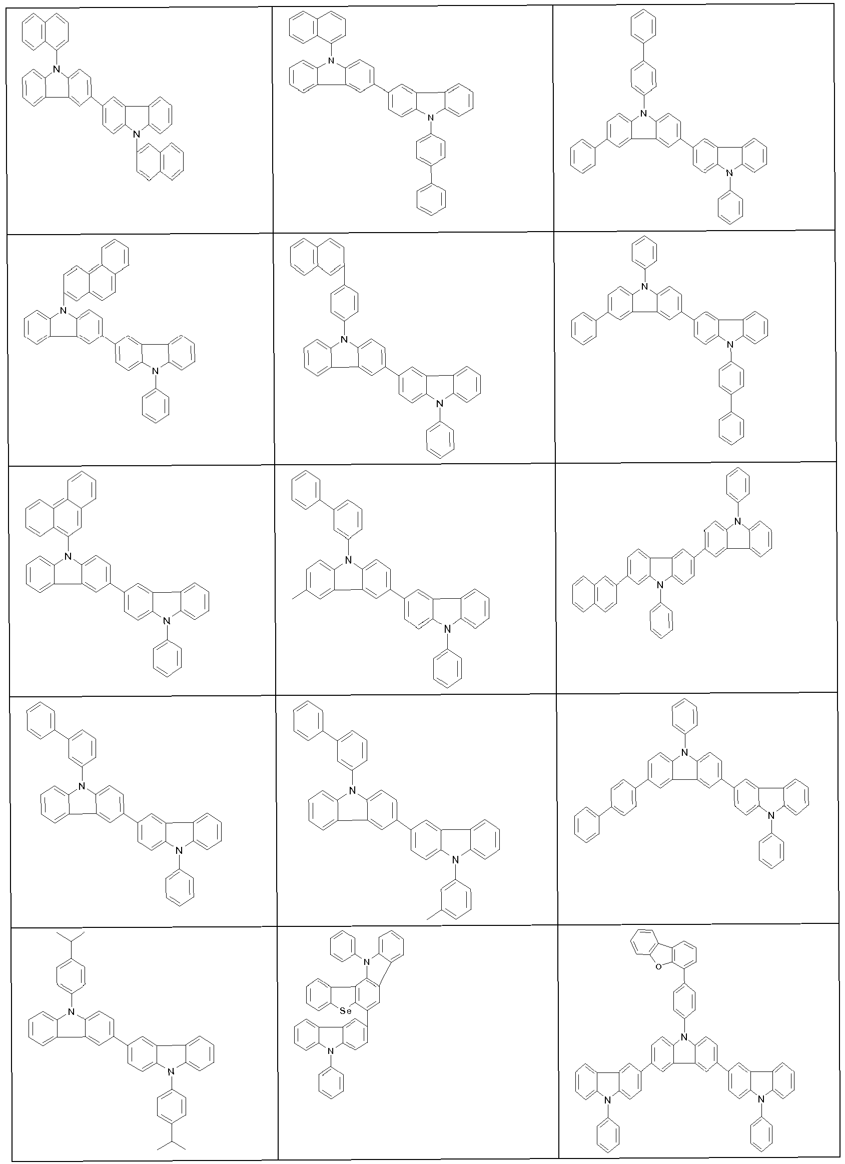

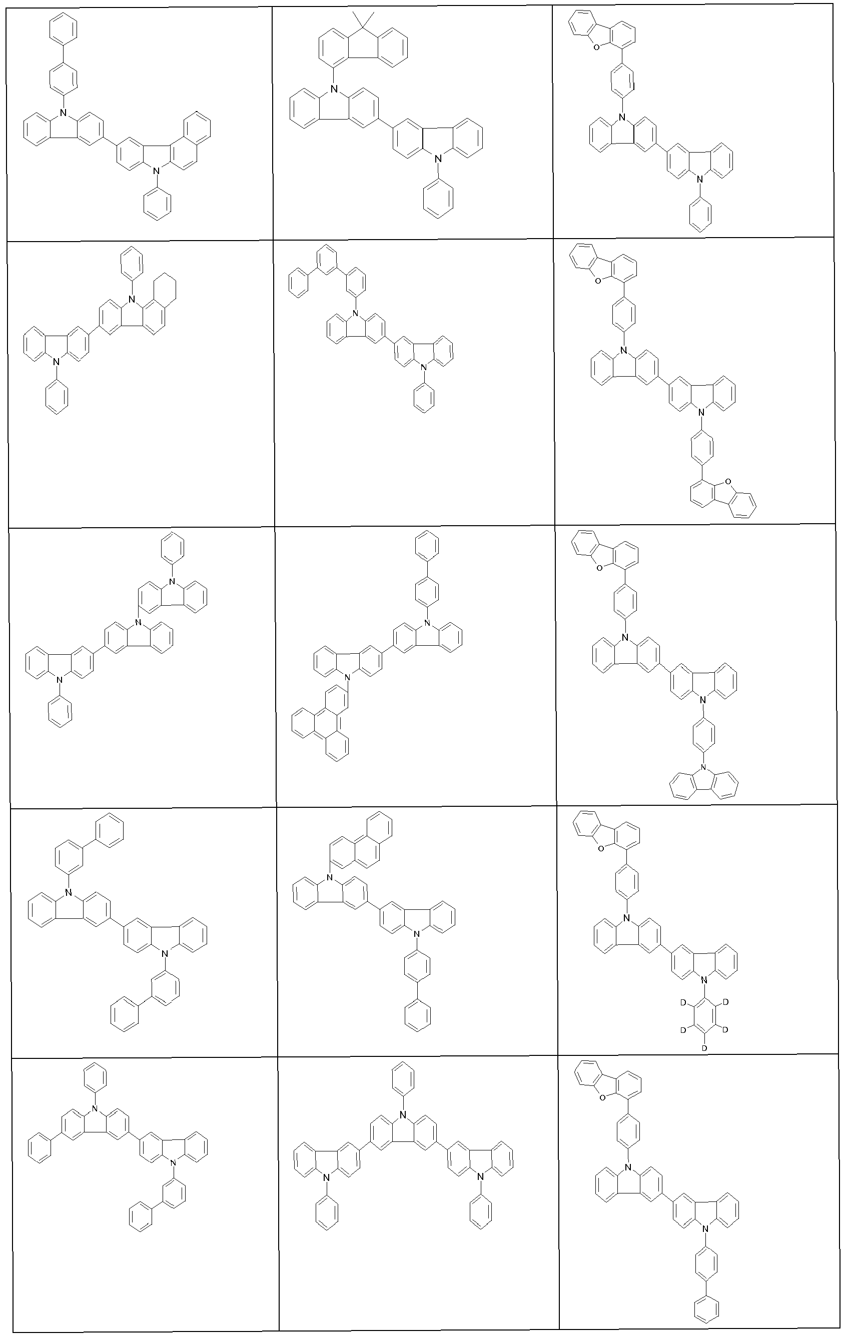

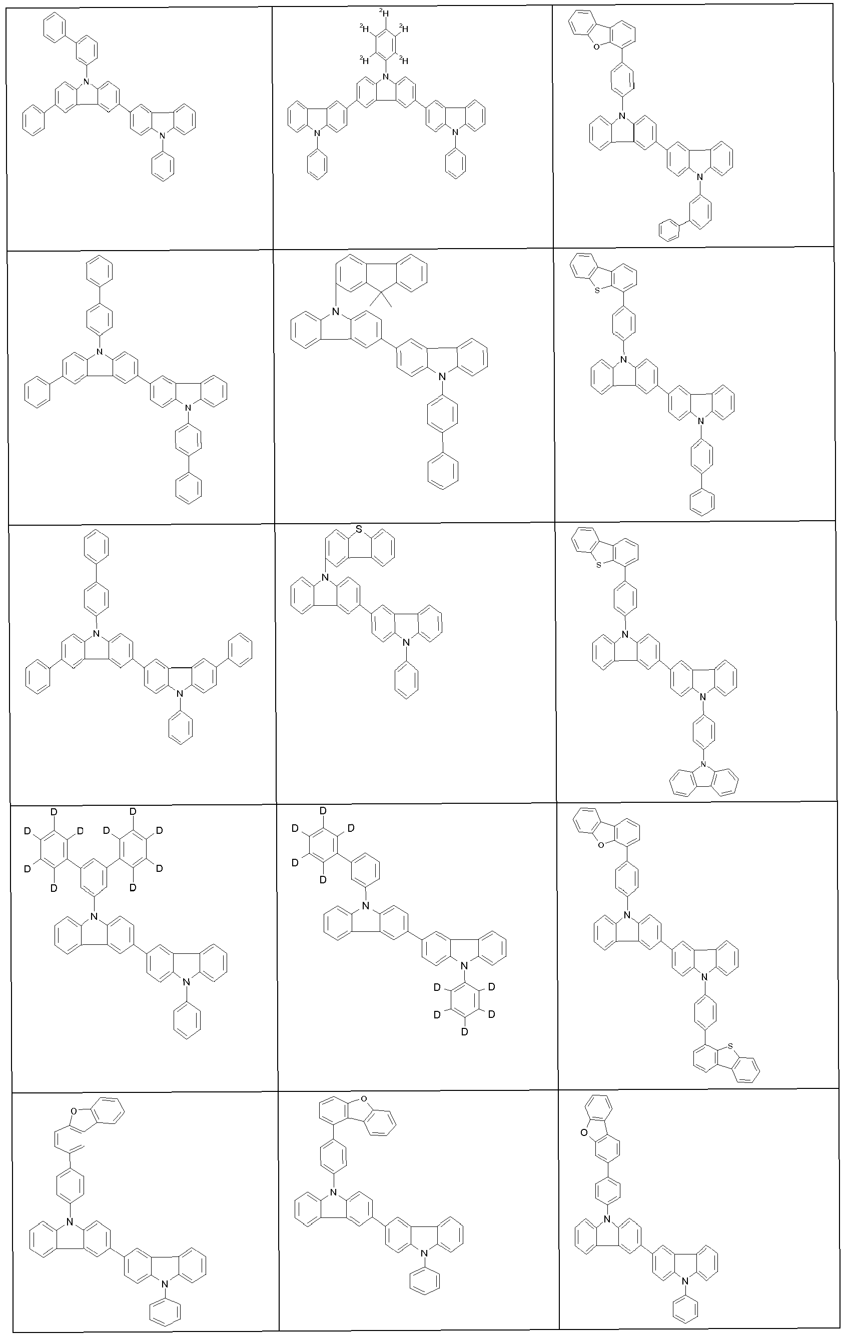

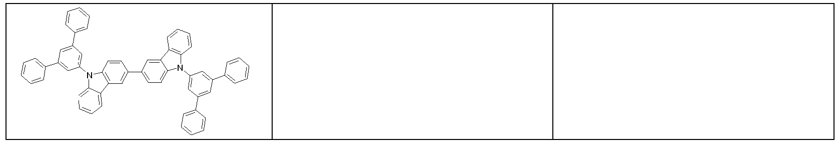

Beispiele für Amine, welche als lochtransportierende Matrixmaterialien eingesetzt werden können:

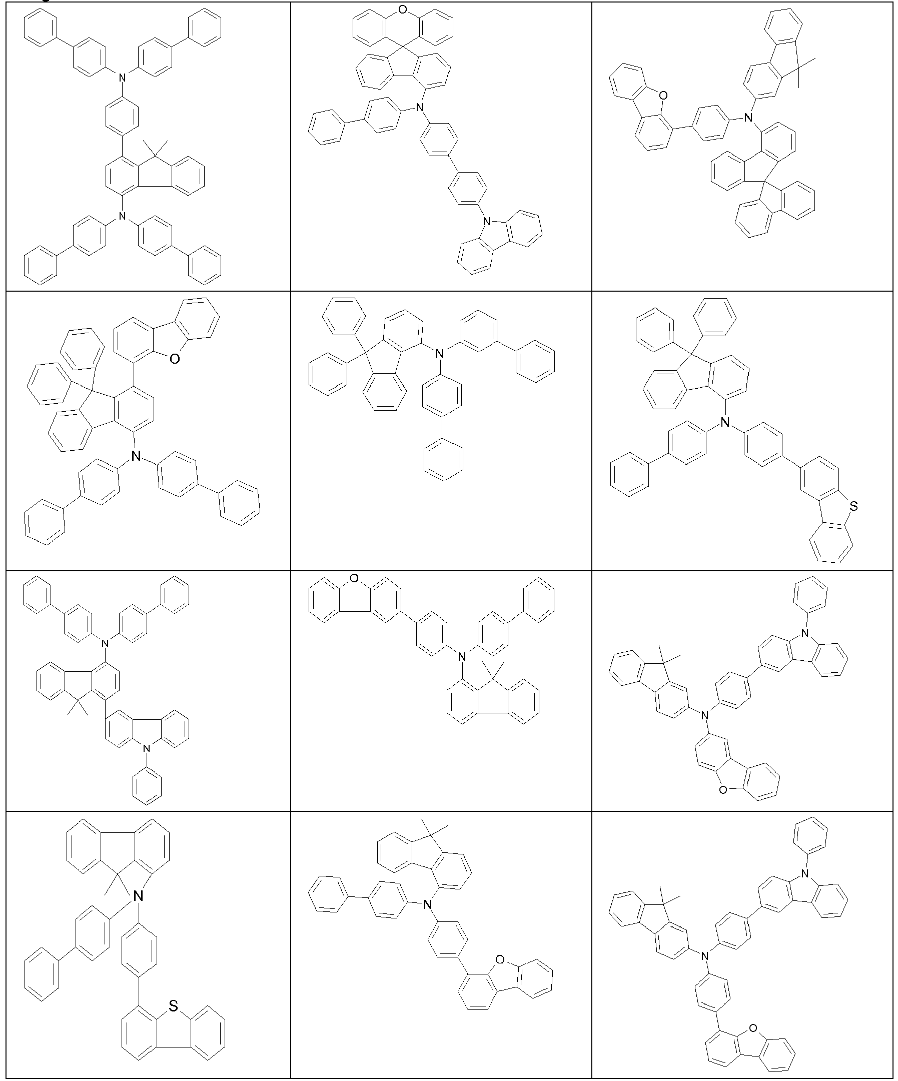

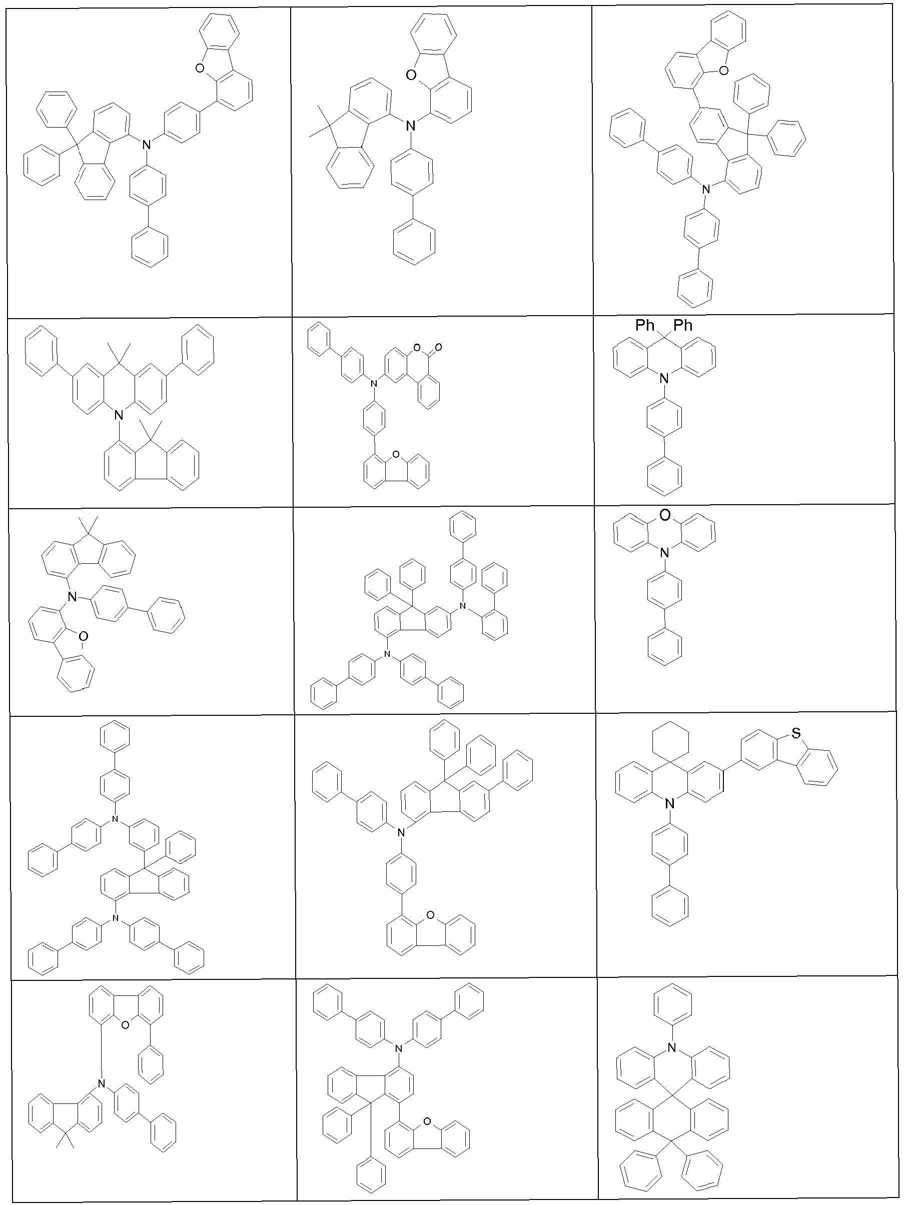

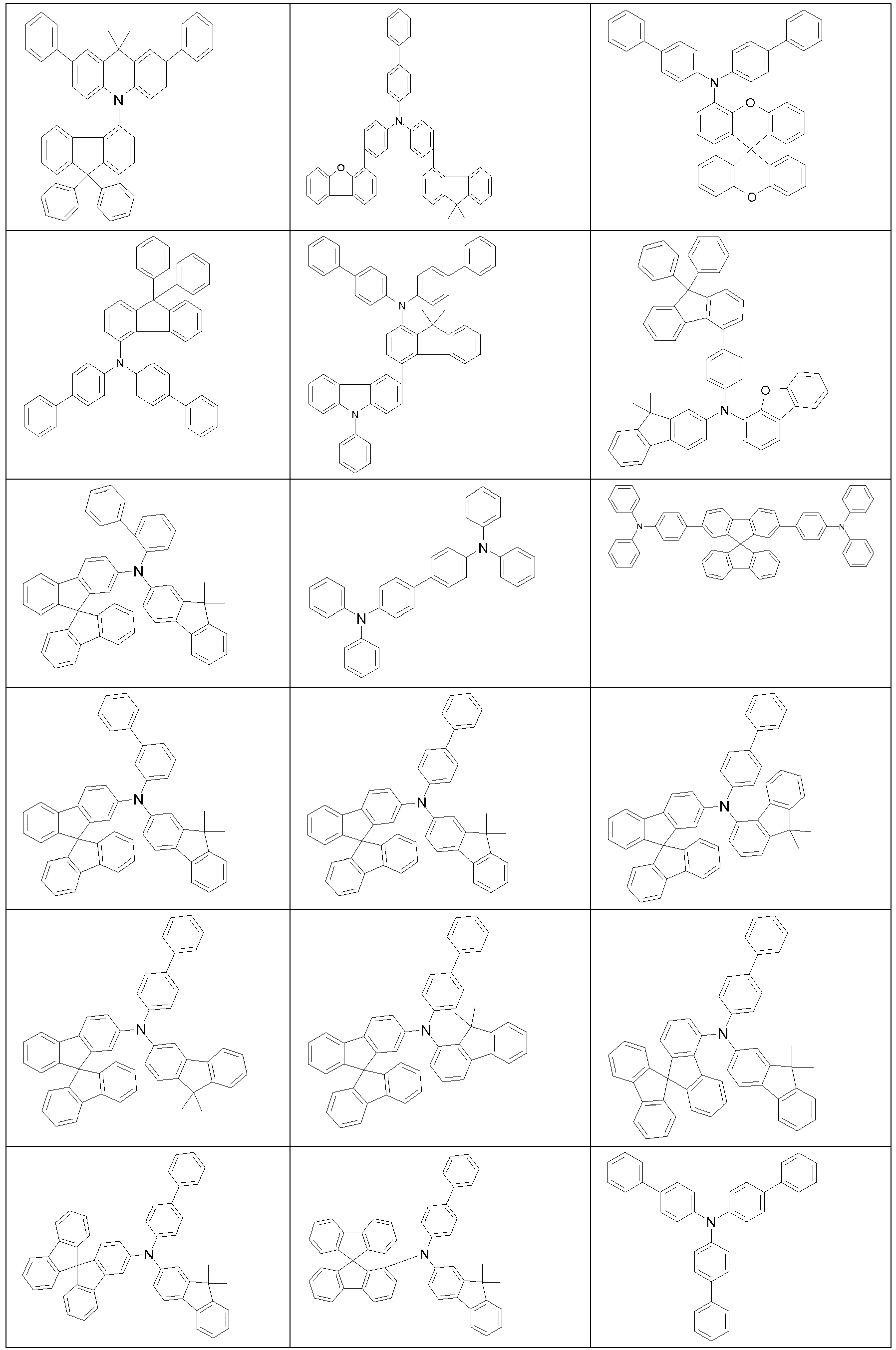

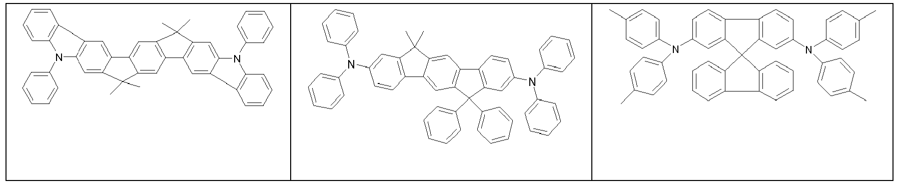

Beispiele für Materialien, welche als Wide Bandgap Matrixmaterialien eingesetzt werden können:

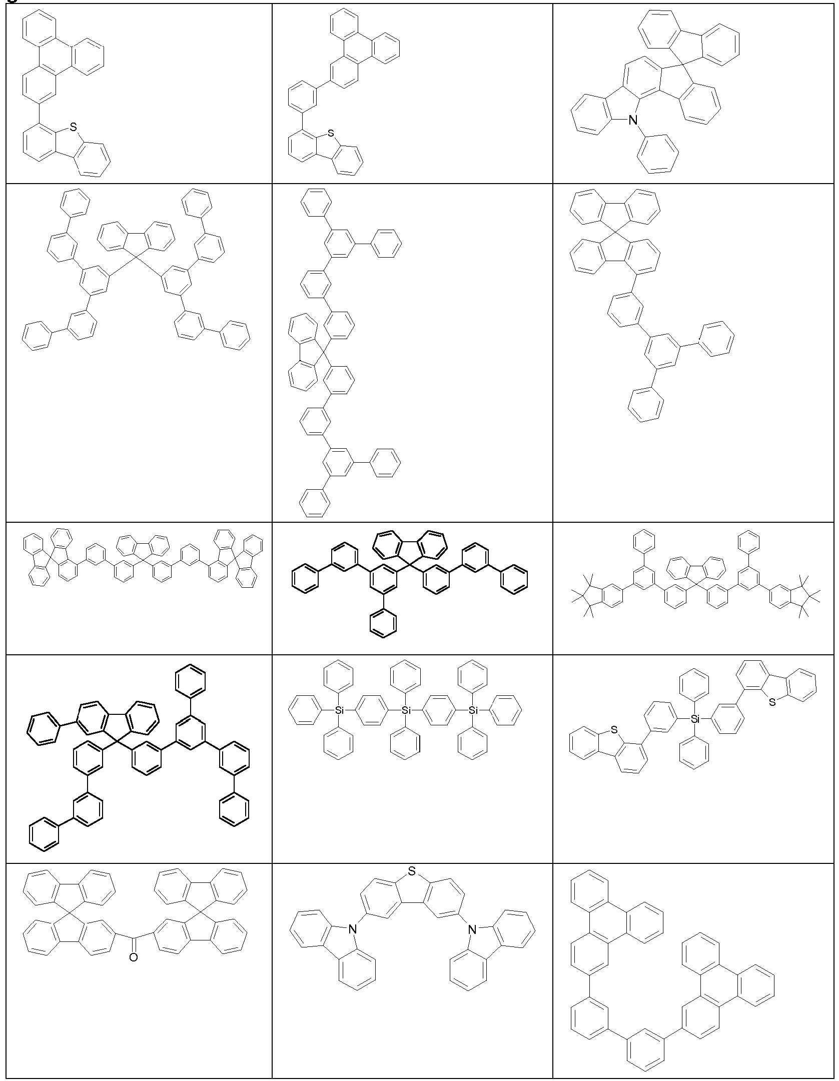

Weiterhin bevorzugt ist es, eine Mischung aus zwei oder mehr Triplett-Emittern, insbesondere zwei oder drei Triplettemittern, zusammen mit einem oder mehreren Matrixmaterialien einzusetzen. Dabei dient der Triplett-Emitter mit dem kürzerwelligen Emissionsspektrum als Co-Matrix für den Triplett-Emitter mit dem längerwelligen Emissionsspektrum. So können beispielsweise die erfindungsgemäßen Metallkomplexe mit einem kürzerwelligen, z.B. blau, grün oder gelb emittierenden, Metallkomplex als Co-Matrix kombiniert werden. Es können beispislweise auch erfindungsgemäße Metallkomplexe als Co-Matrix für längerwellig emittierende Triplettemitter, beispielsweise für rot emittierende Triplettemitter, eingesetzt werden. Dabei kann es auch bevorzugt sein, wenn sowohl der kürzerwellig wie auch der längerwellig emittierende Metallkomplex eine erfindungsgemäße Verbindung ist. Eine bevorzugte Ausführungsform bei Verwendung einer Mischung aus drei Triplett-Emittern ist, wenn zwei als Co-Host und einer als emittierendes Material eingesetzt werden. Dabei weisen diese Triplett-Emitter bevorzugt die Emissionsfarben Grün, Gelb und Rot oder Blau, Grün und Orange auf.

Eine bevorzugte Mischung in der emittierenden Schicht enthält ein elektronentransportierendes Hostmaterial, ein so genanntes "wide bandgap" Hostmaterial, das aufgrund seiner elektronischen Eigenschaften nicht oder nicht in wesentlichem Umfang am Ladungstransport in der Schicht beteiligt ist, einen Co-Dotanden, welcher ein Triplettemitter ist, welcher bei einer kürzeren Wellenlänge als die erfindungsgemäße Verbindung emittiert, sowie eine erfindungsgemäße Verbindung.

Eine weitere bevorzugte Mischung in der emittierenden Schicht enthält ein elektronentransportierendes Hostmaterial, ein so genanntes "wide band gap" Hostmaterial, das aufgrund seiner elektronischen Eigenschaften nicht oder nicht in wesentlichem Umfang am Ladungstransport in der Schicht beteiligt ist, ein lochtransportierendes Hostmaterial, einen Co-Dotanden, welcher ein Triplettemitter ist, welcher bei einer kürzeren Wellenlänge als die erfindungsgemäße Verbindung emittiert, sowie eine erfindungsgemäße Verbindung.

Die erfindungsgemäßen Verbindungen lassen sich auch in anderen Funktionen in der elektronischen Vorrichtung einsetzen, beispielsweise als Lochtransportmaterial in einer Lochinjektions- oder -transportschicht, als Ladungserzeugungsmaterial, als Elektronenblockiermaterial, als Lochblockiermaterial oder als Elektronentransportmaterial, beispielsweise in einer Elektronentransportschicht. Ebenso lassen sich die erfindungsgemäßen Verbindungen als Matrixmaterial für andere phosphoreszierende Metallkomplexe in einer emittierenden Schicht einsetzen.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, CS₂CO₃, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoO₃ oder WO₃, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der Technik für die Schichten verwendet werden, und der Fachmann kann ohne erfinderisches Zutun jedes dieser Materialien in einer elektronischen Vorrichtung mit den erfindungsgemäßen Materialien kombinieren.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden. Bevorzugt sind als Lochtransportmaterialien, die in einer Lochtransport-, Lochinjektions- oder Elektronenblockierschicht in der erfindungsgemäßen Elektrolumineszenzvorrichtung verwendet werden können, Indenofluorenamin-Derivate (z. B. gemäß WO 06/122630 oder WO 06/100896), die in
EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627, WO2014/056565), Fluoren-Amine (z. B. gemäß EP 2875092, EP 2875699 und EP 2875004), Spiro-Dibenzopyran-Amine (z. B. EP 2780325) und Dihydroacridin-Derivate (z. B. gemäß WO 2012/150001).

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner 10⁻⁵ mbar, bevorzugt kleiner 10⁻⁶ mbar aufgedampft. Es ist auch möglich, dass der Anfangsdruck noch geringer oder noch höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend einen erfindungsgemäßen Metallkomplex und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf organische Elektrolumineszenzvorrichtungen enthaltend Verbindungen gemäß Formel (1) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Die erfindungsgemäßen Metallkomplexe lassen sich in sehr hoher Ausbeute und sehr hoher Reinheit bei außergewöhnlich kurzen Reaktionszeiten und vergleichsweise geringen Reaktionstemperaturen synthetisieren.
2. Die erfindungsgemäßen Metallkomplexe weisen eine hervorragende thermische Stabilität auf, was sich auch bei der Sublimation der Komplexe zeigt. Insbesondere zeigen die erfindungsgemäßen Komplexe eine geringere Sublimationstemperatur als ähnliche polypodale Komplexe, welche drei ortho-metallierte Liganden aufweisen. Die erfindungsgemäßen Verbindungen eignen sich daher sehr gut für die Verarbeitung durch Vakuumaufdampfung.
3. Die erfindungsgemäßen Metallkomplexe weisen eine sehr gute Hydrolysestabilität auf. Insbesondere ist die Hydrolysestabilität deutlich besser als bei Komplexen, welche Acetylacetonat-Derivate als Liganden enthalten, in welchen die Liganden aber nicht polypodal überbrückt sind. Die erfindungsgemäßen Verbindungen eignen sich daher auch sehr gut für die Verarbeitung aus Lösung.
4. Organische Elektrolumineszenzvorrichtungen enthaltend die erfindungsgemäßen Metallkomplexe als emittierende Materialien weisen eine sehr gute Lebensdauer auf. Dies gilt insbesondere auch in einfachen OLEDs in denen der erfindungsgemäße Metallkomplex in eine SingleMatrix - also ein Matrix- bzw. Host-Material - eingebracht ist.
5. Organische Elektrolumineszenzvorrichtungen enthaltend die erfindungsgemäßen Metallkomplexe als emittierende Materialien weisen eine hervorragende Effizienz auf und zeigen orientierte Emission.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße elektronische Vorrichtungen herstellen und somit die Erfindung im gesamten beanspruchten Bereich ausführen.

### Beispiele:

Die nachfolgenden Synthesen werden, sofern nicht anders angegeben, unter einer Schutzgasatmosphäre in getrockneten Lösungsmitteln durchgeführt. Die Metallkomplexe werden zusätzlich unter Ausschluss von Licht bzw. unter Gelblicht gehandhabt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen. Bei Verbindungen, die mehrere tautomere Formen aufweisen können, wird stellvertretend eine tautomere Form dargestellt.

### A: Synthese der Synthone S - Teil 1:

### Beispiel S1:

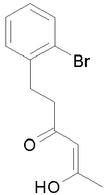

Darstellung analog zu Casey, Brian M. et al., Beilstein Journal of Organic Chemistry, 9, 1472-1479, 2013.

Eine auf 0 °C gekühlte Suspension von 2.6 g (110 mmol) NaH in 300 ml THF wird tropfenweise unter gutem Rühren mit 10.3 g (100 mmol) Acetylacetcon [123-54-6] versetzt (Achtung: Wasserstoffentwicklung) und 15 min nachgerührt. Dann tropft man 42.0 ml (105 mmol) n-BuLi, 2.5 M in n-Hexan, zu und rührt erneut 15 min. nach. Dann gibt man eine auf 0 °C gekühlte Lösung von 25.0 g (100 mmol) 2-Brombenzylbromid [3433-80-5] in 25 ml THF unter sehr gutem Rühren auf ein Mal zu. Man rührt 10 min. nach, entfernt das Eisbad, lässt während 30 min. auf 15 °C erwärmen und hydrolysiert durch tropfenweise Zugabe von 110 ml 2N wässriger HCl. Man trennt die wässrige Phase ab, und extrahiert diese dreimal mit je 200 ml Ethylacetat. Die vereinigten organischen Phasen werden zweimal mit je 300 ml gesättigter Kochsalzlösung gewaschen und über Magnesiumsulfat getrocknet. Nach Entfernen des Lösungsmittels im Vakuum wird der ölige Rückstand an einem Säulenautomaten (CombiFlash Torrent der Fr. A. Semrau) chromatographiert. Ausbeute: 12.4 g (46 mmol), 46%. Reinheit: ca. 97%ig nach ¹H-NMR.

Analog können folgende Verbindungen dargestellt werden.

| Bsp. | Edukte | Produkt | Ausbeute |
|---|---|---|---|
| S2 | 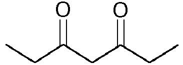 | 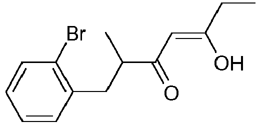 | 39% |
| | [7307-03-1] | | |
| | [3433-80-5] | | |
| S3 | 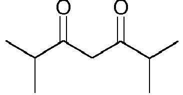 | 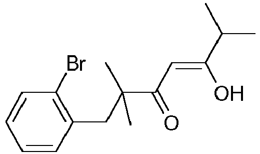 | 37 % |
| | [18362-64-4] | | |
| | [3433-80-51 | | |
| S4 | 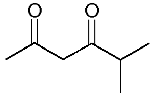 | 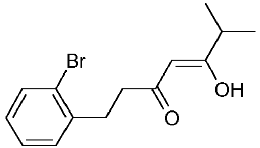 | 48% |
| | [7307-03-1] | | |
| | [3433-80-5] | | |
| S5 | 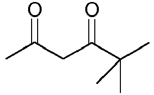 | 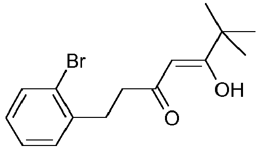 | 64% |
| | [7307-04-2] | | |
| | [3433-80-51 | | |
| S6 | 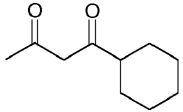 | 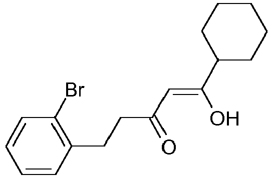 | 50% |
| | [15972-15-3] | | |
| | [3433-80-51 | | |
| S7 | 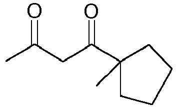 | 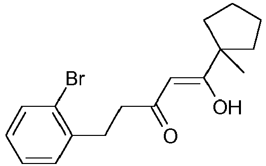 | 61% |
| | [15867-34-2] | | |
| | [3433-80-5] | | |
| S8 | 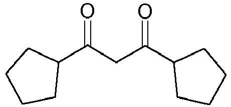 | 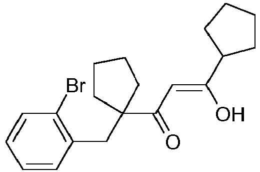 | 48% |
| | [1821143-85-4] | | |
| | [3433-80-5] | | |
| S9 | 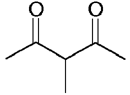 | 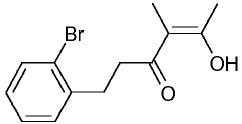 | 50% |
| | [815-57-6] | | |
| | [3433-80-51 | | |
| S10 | 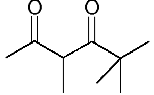 | 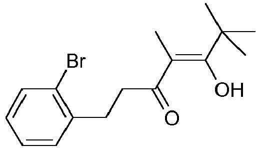 | 63% |
| | [60600-51-3] | | |
| | [3433-80-5] | | |
| S11 | 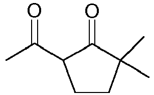 | 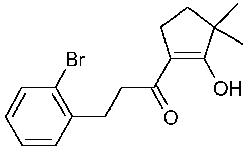 | 59% |
| | [2570-70-8] | | |
| | [3433-80-5] | | |
| S12 | 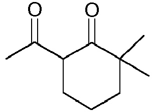 | 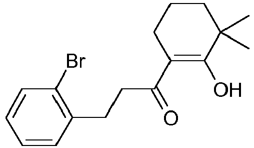 | 63% |
| | [111239-32-8] | | |
| | [3433-80-5] | | |
| S13 | 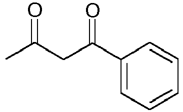 | 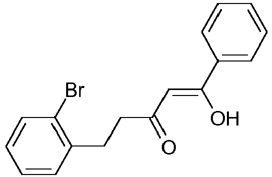 | 60% |
| | [3318-61-4] | | |
| | [3433-80-5] | | |
| S14 | 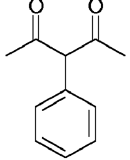 | 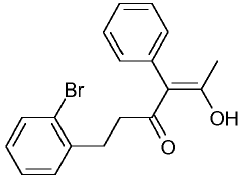 | 55% |
| | [5910-25-8] | | |
| | [3433-80-5] | | |
| S15 | 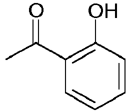 | 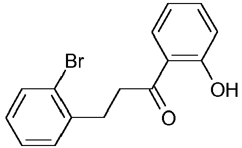 | 49% |
| | [118-93-4] | | |
| | [3433-80-5] | | |
| S16 | 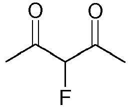 | 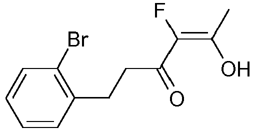 | 29% |
| | [759-02-4] | | |
| | [3433-80-5] | | |
| S17 | 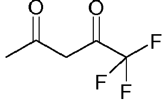 | 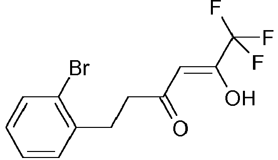 | 46% |
| | [367-57-7] | | |
| | [3433-80-5] | | |
| S18 | 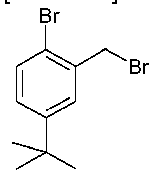 [123-54-6] | 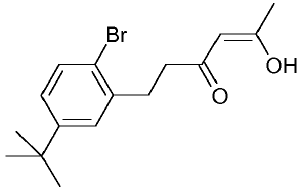 | 55% |
| | [875664-32-7] | | |
| S19 | 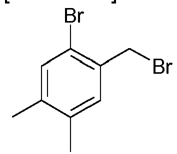 [123-54-6] | 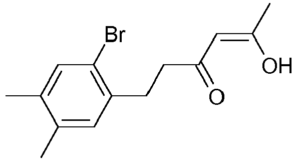 | 57% |
| | [2090556-71-9] | | |
| S20 | [123-54-6] | 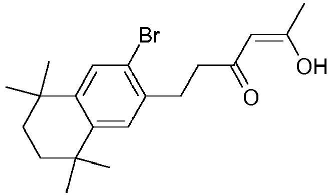 | 61% |
| | 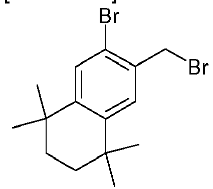 | | |
| | [410528-62-0] | | |
| S21 | [123-54-6] | 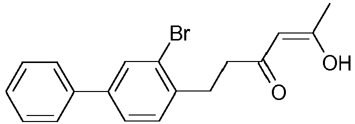 | 56% |
| | 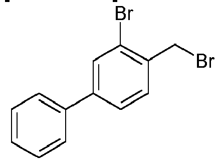 | | |
| | [1396865-04-5] | | |
| S22 | [123-54-6] | 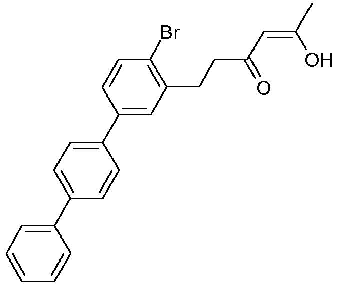 | 53% |
| | 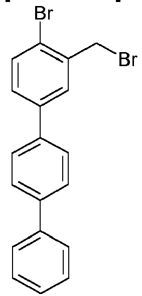 | | |
| | [1422181-28-9] | | |
| S23 | [123-54-6] | 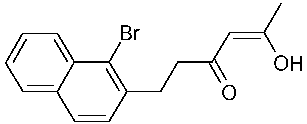 | 46% |
| | 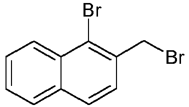 | | |
| | [37763-43-2] | | |

### Beispiel S50:

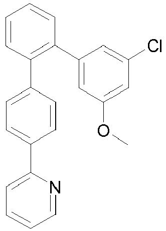

Ein Gemisch aus 26.9 g (100 mmol) 2-(3-Chlor-5-methoxyphenyl)-4,4,5,5-tetramethyl-1,3,2-dioxaborolan [929626-16-4], 31.0 g (100 mmol) 2-(2'-brom-[1,1'-biphenyl]-4-yl)-pyridin [1374202-35-3], 21.2 g (200 mmol) Natriumcarbonat, 788 mg (3 mmol) Triphenylphosphin, 225 mg (1 mmol) Palladium(ll)acetat, 300 ml Toluol, 100 ml Ethanol und 300 ml Wasser wird 48 h unter Rückfluss erhitzt. Nach Erkalten wird mit 300 ml Toluol erweitert, die organische Phase wird abgetrennt, einmal mit 500 ml Wasser und einmal mit 500 ml gesättigter Kochsalzlösung gewaschen und über Magnesiumsulfat getrocknet. Nach Entfernen des Lösungsmittels wird der Rückstand an Kieselgel (n-Heptan/Ethylacetat 2:1 vv) chromatographiert. Ausbeute: 28.4 g (76 mmol), 76 %. Reinheit: ca. 97 %ig nach ¹H-NMR.

Analog können folgende Verbindungen dargestellt werden.

| Bsp. | Edukte | Produkt | Ausbeute |
|---|---|---|---|
| S51 | 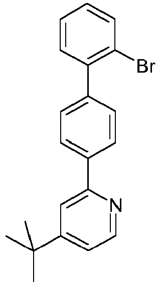 | 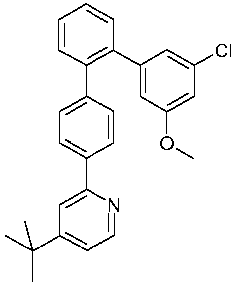 | 80% |
| | [1989597-33-2] | | |
| S52 | 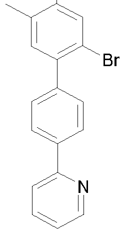 | 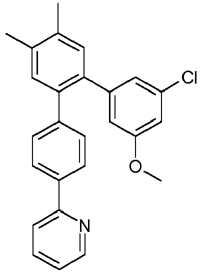 | 73% |
| | [1989597-43-4] | | |
| S53 | 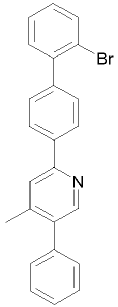 | 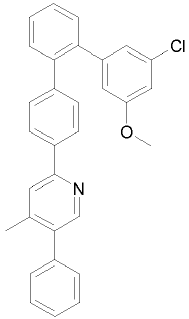 | 75% |
| | [1989597-34-3] | | |
| S54 | 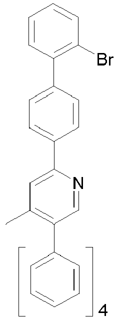 | 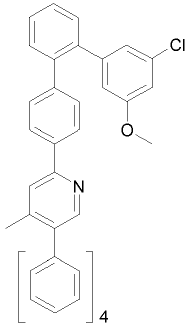 | 80% |
| | [1989597-41-2] | | |
| S55 | 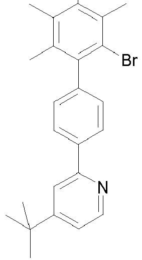 | 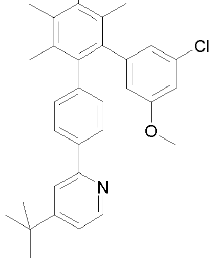 | 77% |
| | [1989597-44-5] | | |
| S56 | 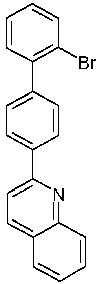 | 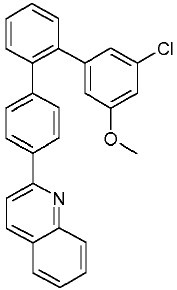 | 71% |
| | [2088182-36-7] | | |

### Beispiel S100:

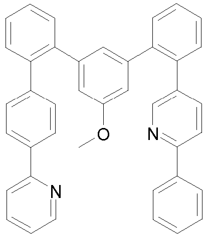

Ein Gemisch aus 37.2 g (100 mmol) S50, 31.0 g (100 mmol) 5-(2-Bromphenyl)-2-phenyl-pyridin [1989597-29-6], 21.2 g (200 mmol) Natriumcarbonat, 1.23 g (3 mmol) SPhos, 449 mg (2 mmol) Palladium(ll)acetat, 300 ml Toluol, 100 ml Ethanol und 300 ml Wasser wird 16 h unter Rückfluss erhitzt. Nach Erkalten wird mit 300 ml Toluol erweitert, die organsiche Phase wird abgetrennt, einmal mit 500 ml Wasser und einmal mit 500 ml gesättigter Kochsalzlösung gewaschen und über Magnesiumsulfat getrocknet. Nach Entfernen des Lösungsmittels wird der Rückstand an Kieselgel (n-Heptan/Ethylacetat 2:1 vv) chromatographiert. Ausbeute: 40.3 g (71 mmol), 71 %. Reinheit: ca. 97 %ig nach ¹H-NMR.

Analog können folgende Verbindungen dargestellt werden.

| Bsp. | Edukte | Produkt | Ausbeute |
|---|---|---|---|
| S101 | 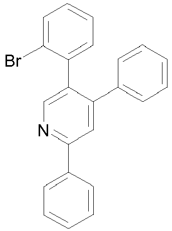 | 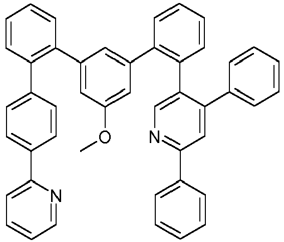 | 68% |
| | [1989597-32-1] S50 | | |
| S102 | 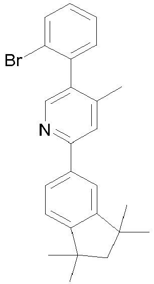 | 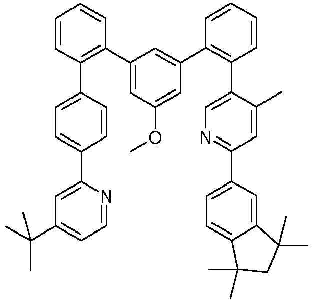 | 70% |
| | [2088182-35-6] S51 | | |
| S103 | 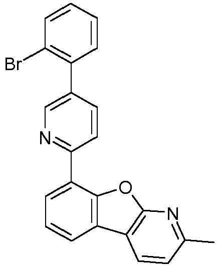 | 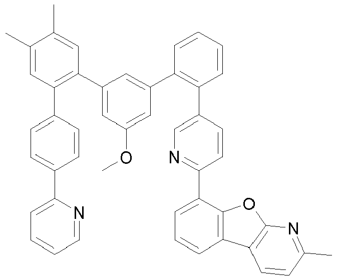 | 74% |
| | [1989597-42-3] S52 | | |
| S104 | 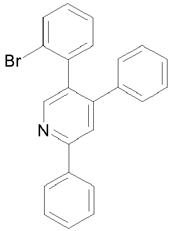 | 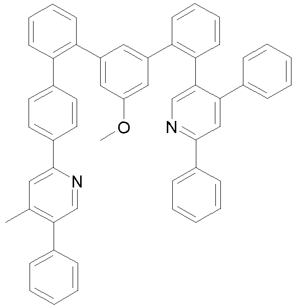 | 69% |
| | [1989597-32-1] S53 | | |
| S105 | 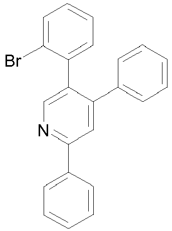 | 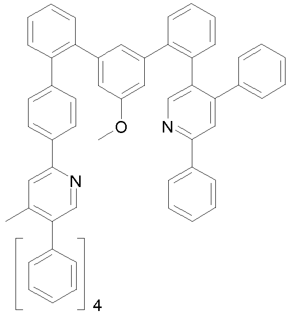 | 75% |
| | [1989597-32-1] S54 | | |
| S106 | 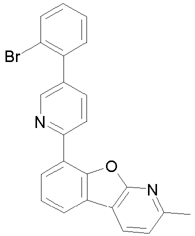 | 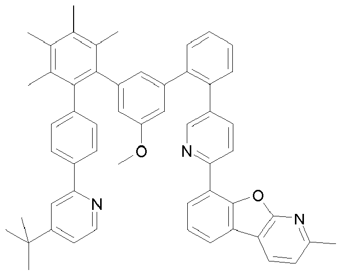 | 73% |
| | [1989597-42-3] S55 | | |
| S107 | 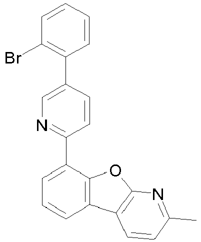 | 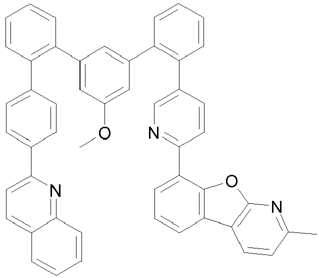 | 67% |
| | [1989597-42-3] S56 | | |
| S108 | 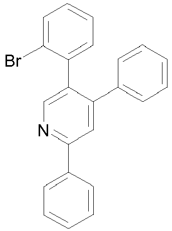 | 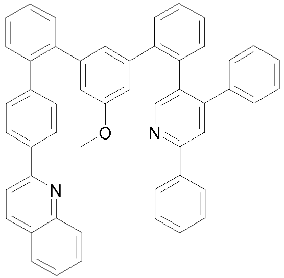 | 70% |
| | [1989597-32-1] S56 | | |

**Beispiel S200:** 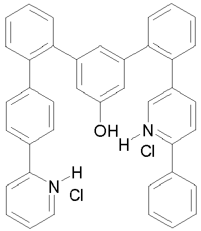 Ein Gemisch aus 55.6 g (100 mmol) S100 und 115.6 g (1 mol) Pyridiniumhydrochlorid [628-13-7] wird 3 h am Wasserabscheider auf 200 °C erhitzt, wobei das Destillat von Zeit zu Zeit abgelassen wird. Nach Erkalten versetzt man die Reaktionsmischung mit 1000 ml Eiswasser, wobei das Produkt kristallisiert. Man lässt über Nacht im Kühlschank stehen, saugt ab, wäscht mit wenig Eiswasser und trocknet im Vakuum. Ausbeute: 55.0 g (87 mmol), 87 %; Reinheit: ca. 97 %ig nach ¹H-NMR.

Analog können folgende Verbindungen dargestellt werden.

| Bsp. | Edukt | Produkt | Ausbeute |
|---|---|---|---|
| S201 | S101 | 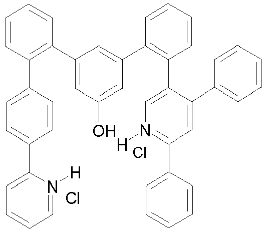 | 90% |
| S202 | S102 | 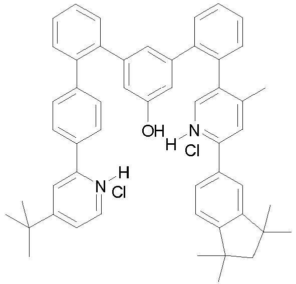 | 88% |
| S203 | S103 | 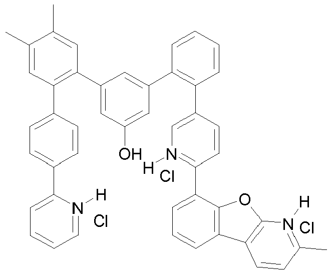 | 85% |
| S204 | S104 | 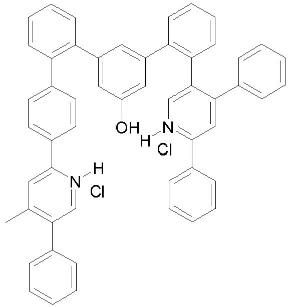 | 85% |
| S205 | S105 | 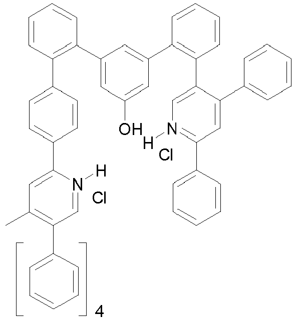 | 91% |
| S206 | S106 | 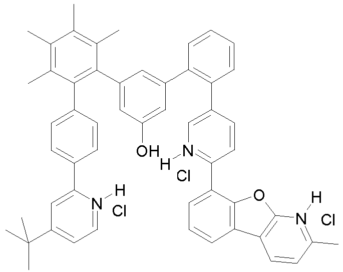 | 90% |
| S207 | S107 | 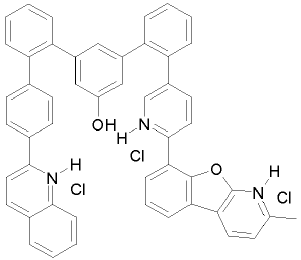 | 86% |
| S208 | S108 | 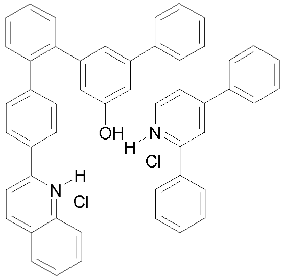 | 88% |

### Beispiel S300:

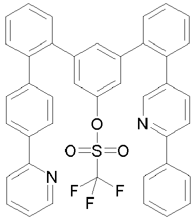

Eine auf 0 °C gekühlte Lösung von 55.6 g (100 mmol) S200 in einem Gemisch aus 500 ml Dichlormethan und 100 ml Pyridin wird unter gutem Rühren tropfenweise mit 34 ml (200 mmol) Trifluormethansulfonsäureanhydrid [358-23-6] versetzt. Man lässt die Reaktionsmischung auf Raumtemperatur erwärmen, rührt 16 h nach, gießt unter Rühren auf 1000 ml Eiswasser, rührt 10 min. nach, trennt die organische Phase ab und extrahiert die wässrige Phase dreimal mit je 300 ml Dichlormethan. Die vereinigten organischen Phasen werden zweimal mit je 300 ml Eiswasser und einmal mit 500 ml gesättigter NaCl-Lösung gewaschen und über Natriumsulfat getrocknet. Das nach Entfernen des Dichlormethans im Vakuum erhaltene Wachs wird aus Acetonitril umkristallisiert. Ausbeute: 60.5 g (88 mmol), 88 %; Reinheit: ca. 95 %ig nach ¹H-NMR.

Analog können folgende Verbindungen dargestellt werden.

| Bsp. | Edukt | Produkt | Ausbeute |
|---|---|---|---|
| S301 | S201 | 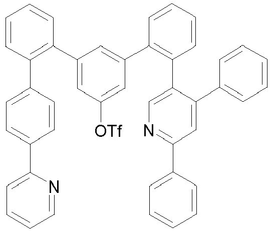 | 90% |
| S302 | S202 | 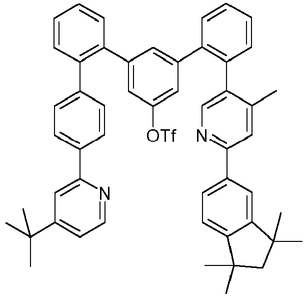 | 81% |
| S303 | S203 | 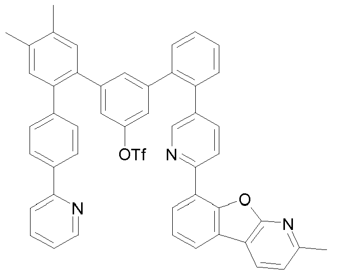 | 83% |
| S304 | S204 | 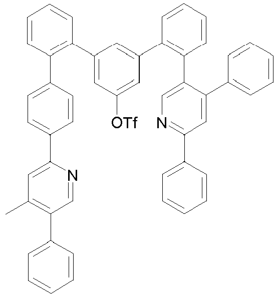 | 81% |
| S305 | S205 | 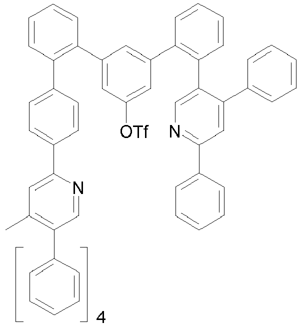 | 83% |
| S306 | S206 | 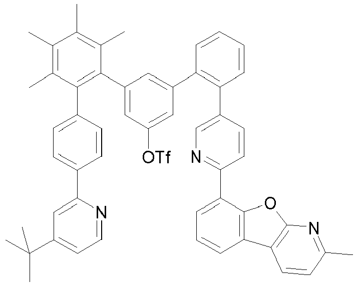 | 77% |
| S307 | S207 | 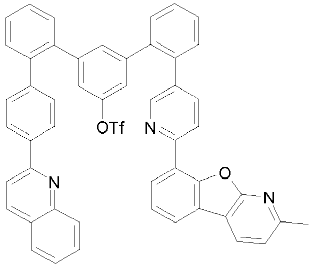 | 79% |
| S308 | S208 | 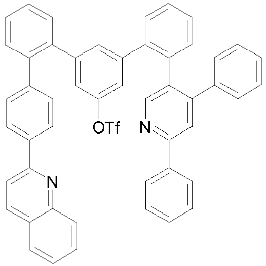 | 85% |

### Beispiel S400:

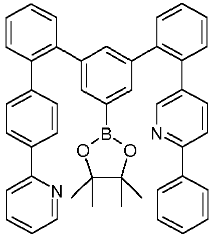

Eine Lösung aus 68.5 g (100 mmol) S300 und Bis(diphenylphosphino)-palladium(II)dichlorid x DCM in 500 ml Dioxan wird unter gutem Rühren mit 41.8 ml (300 mmol) Triethylamin und dann tropfenweise mit 29.0 ml (200 mmol) 4,4,5,5-Tetramethyl-[1,3,2]-dioxaborolan versetzt und 16 h unter Rückfluss erhitzt. Nach Erkalten engt man im Vakuum zur Trockene ein, nimmt das Öl in 500 ml Ethylacetat auf, wäscht dreimal mit je 300 ml Wasser, einmal mit 300 ml gesättigter Kochsalzlösung, trocknet über Magnesiumsulfat und flitriert über ein mit Ethylacetat vorgeschlämmtes Kieselgel-Bett vom Trockenmittel ab. Man entfernt das Lösungsmittel im Vakuum und kristallisiert den Rückstand zweimal aus Acetonitril unter Zugabe von etwas Ethylacetat um. Ausbeute: 49.7 g (75 mmol), 75 %; Reinheit: ca. 95 %ig nach ¹H-NMR.

Analog können folgende Verbindungen dargestellt werden.

| Bsp. | Edukt | Produkt | Ausbeute |
|---|---|---|---|
| S401 | S301 | 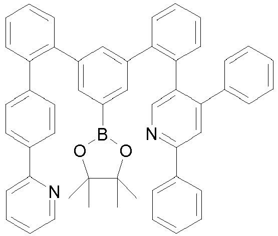 | 73% |
| S402 | S302 | 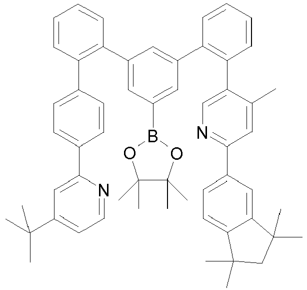 | 69% |
| S403 | S303 | 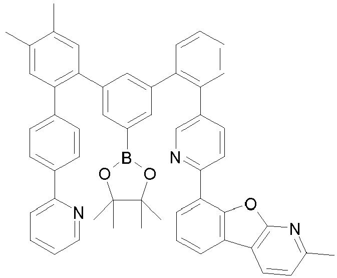 | 67% |
| S404 | S304 | 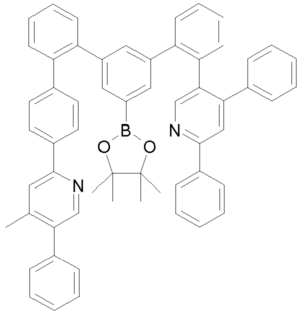 | 70% |
| S405 | S305 | 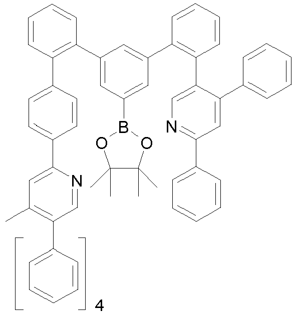 | 74% |
| S406 | S306 | 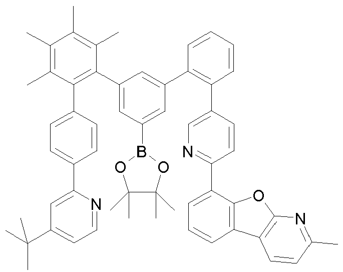 | 68% |
| S407 | S307 | 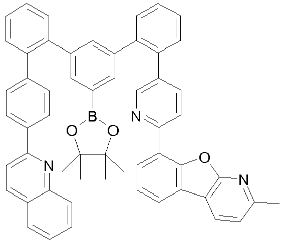 | 70% |
| S408 | S308 | 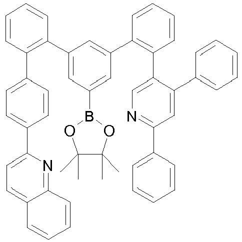 | 67% |

### B: Synthese der Liganden L:

### Beispiel L1:

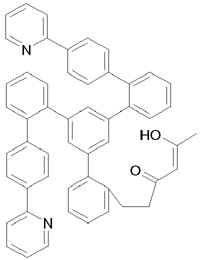

Ein Gemisch aus 66.3 g (100 mmol) 2,2'-[5"-(4,4,5,5-Tetramethyl-1,3,2-dioxaborolan-2-yl)-[1,1':2',1":3",1"':2"',1""-quinquephenyl]-4,4""-diyl]bis-pyridin [1989597-72-9], 29.6 g (110 mmol) S1, 31.8 g (300 mmol) Natriumcarbonat, 1.23 g (3 mmol) SPhos, 449 mg (2 mmol) Palladium(ll)acetat, 300 ml Toluol, 100 ml Ethanol und 300 ml Wasser wird 18 h unter Rückfluss erhitzt. Nach Erkalten wird mit Essigsäure auf pH 6-7 eingestellt, die organische Phase wird abgetrennt, die wässrige Phase wird dreimal mit je 100 ml Toluol extrahiert, die vereinigten organischen Phasen werden einmal mit 300 ml Wasser und einmal mit 500 ml gesättigter Kochsalzlösung gewaschen und über Magnesiumsulfat getrocknet. Nach Entfernen des Lösungsmittels wird der Rückstand chromatographiert (CombiFlash Torrent der Fa. A. Semrau). Ausbeute: 50.0 g (69 mmol), 69 %; Reinheit: ca. 97 %ig nach ¹H-NMR.

Analog können folgende Verbindungen dargestellt werden.

| Bsp. | Edukte | Produkt | Ausbeute |
|---|---|---|---|
| L-Ref.1 | 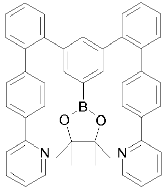 | 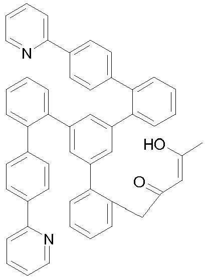 | 58% |
| | [1989597-72-91 | | |
| | 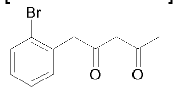 | | |
| | [473758-02-0] | | |
| L2 | 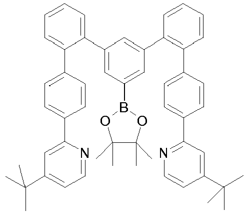 | 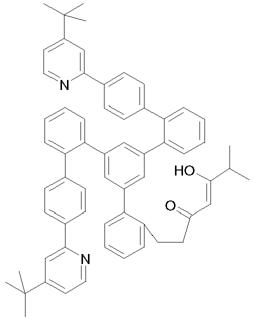 | 70% |
| | [1989597-75-2] S4 | | |
| L3 | [1989597-72-9] S22 | 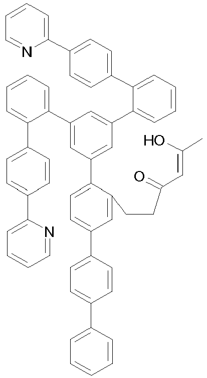 | 64% |

### Beispiel L100:

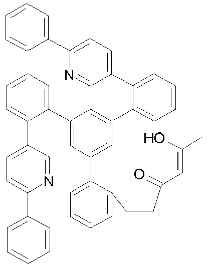

Durchführung analog zu Beispiel L1, wobei anstelle 2,2'-[5"-(4,4,5,5-Tetramethyl-1,3,2-dioxaborolan-2-yl)-[1,1':2',1":3",1"':2"',1""-quinquephenyl]-4, 4""-diyl]bis-pyridin [1989597-72-9] 3,3'-[5'-(4,4,5,5-Tetramethyl-1,3,2-dioxaborolan-2-yl)-[1,1':3',1"-terphenyl]-2,2"-diyl]bis[6-phenyl-pyridin] [1989597-70-7] eingesetzt wird. Ausbeute: 53.2 g (73 mmol), 73 %; Reinheit: ca. 97 %ig nach ¹H-NMR.

Analog können folgende Verbindungen dargestellt werden.

| Bsp. | Edukte | Produkt | Ausbeute |
|---|---|---|---|
| L-Ref.2 | 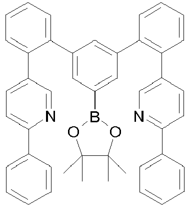 | 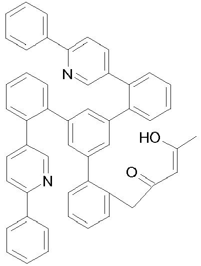 | 54% |
| | [1989597-70-7] | | |
| | 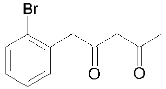 | | |
| | [473758-02-0] | | |
| L101 | [1989597-70-7] | 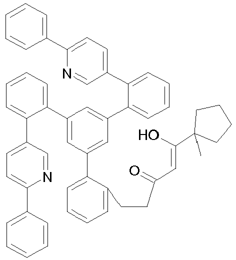 | 65% |
| | S7 | | |
| L102 | 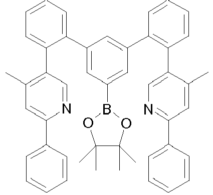 | 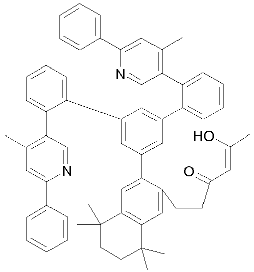 | 68% |
| | [1989597-71-8] | | |
| | S20 | | |
| L103 | 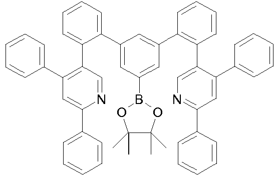 | 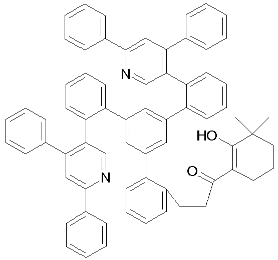 | 67% |
| | [1989597-74-1] | | |
| | S12 | | |
| L104 | 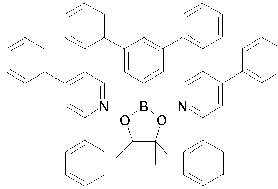 | 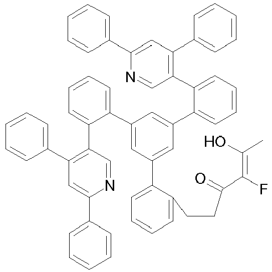 | 61% |
| | [1989597-74-1] | | |
| | S16 | | |
| L105 | 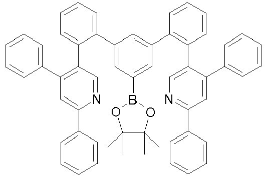 | 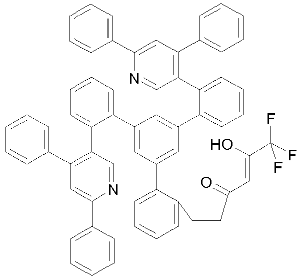 | 58% |
| | [1989597-74-1] | | |
| | S17 | | |

### Beispiel L200:

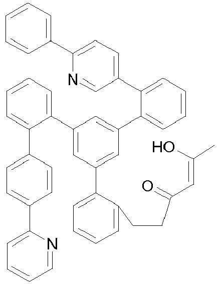

Durchführung analog Beispiel L1, wobei anstelle von 2,2'-[5"-(4,4,5,5-Tetramethyl-1,3,2-dioxaborolan-2-yl)-[1,1':2',1":3",1"':2"',1""-quinque-phenyl]-4,4""-diyl]bis-pyridin [1989597-72-9] S400 eingesetzt wird. Ausbeute: 53.0 g (72 mmol), 72 %; Reinheit: ca. 97 %ig nach ¹H-NMR.

Analog können folgende Verbindungen dargestellt werden.

| Bsp. | Edukte | Produkt | Ausbeute |
|---|---|---|---|
| L-Ref.3 | S400 | 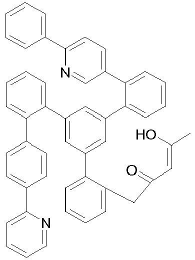 | 56% |
| | [473758-02-0] | | |
| L201 | S401 | 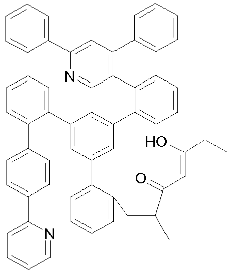 | 54% |
| | S2 | | |
| L202 | S401 | 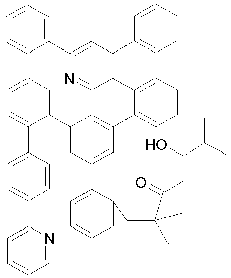 | 57% |
| | S3 | | |
| L203 | S402 | 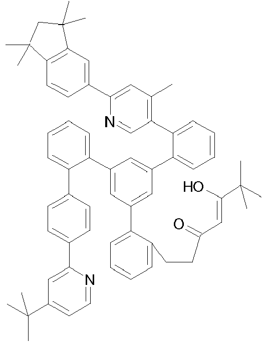 | 65% |
| | S5 | | |
| L204 | S402 | 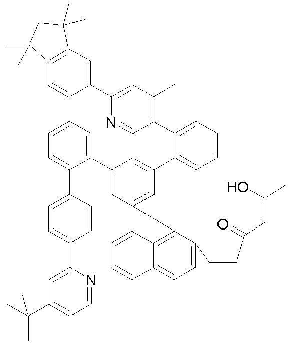 | 67% |
| | S23 | | |
| L205 | S403 | 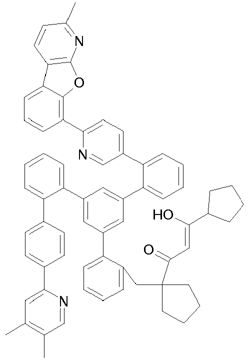 | 60% |
| | S8 | | |
| L206 | S403 | 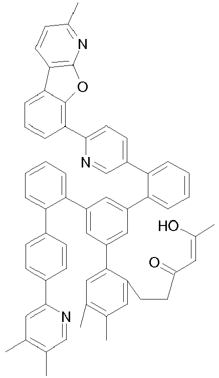 | 62% |
| | S19 | | |
| L207 | S403 | 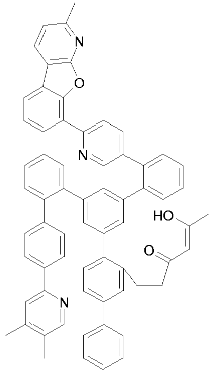 | 66% |
| | S21 | | |
| L208 | S404 | 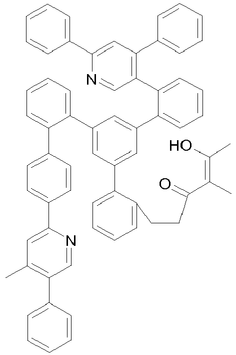 | 65% |
| | S9 | | |
| L209 | S404 | 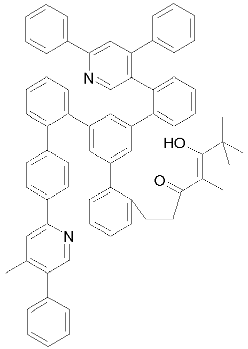 | 60% |
| | S10 | | |
| L210 | S405 | 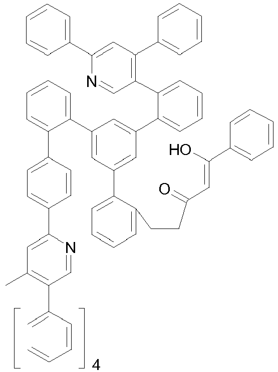 | 57% |
| | S13 | | |
| L211 | S405 | 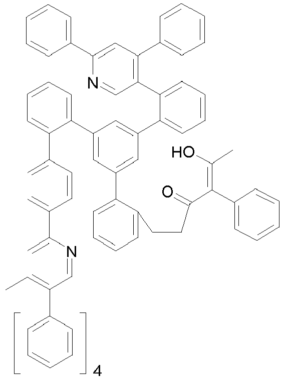 | 62% |
| | S14 | | |
| L212 | S406 | 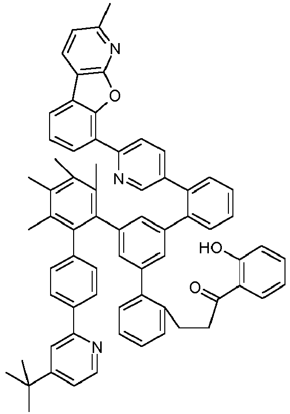 | 70% |
| | S15 | | |
| L213 | S407 | 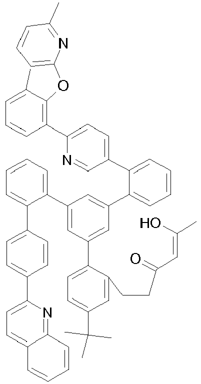 | 67% |
| | S18 | | |
| L214 | S408 | 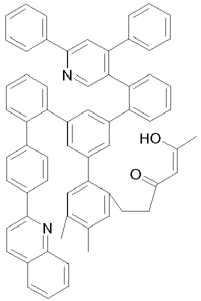 | 59% |
| | S19 | | |

### C: Darstellung der Metallkomplexe

### Beispiel Ir(L1):

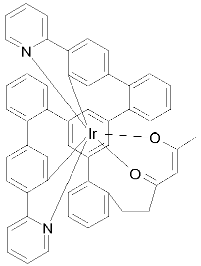

### Variante A:

Ein Gemisch aus 7.25 g (10 mmol) des Liganden L1, 4.90 g (10 mmol) Tris-acetylacetonato-iridium(III) [15635-87-7] und 120 g Hydrochinon [123-31-9] werden in einem 1000 mL Zweihalsrundkolben mit einem glasummantelten Magnetkern vorgelegt. Der Kolben wird mit einem Wasserabscheider (für Medien geringerer Dichte als Wasser) und einem Luftkühler mit Argonüberlagerung versehen. Der Kolben wird in einer Metallheizschale platziert. Die Apparatur wird über die Argonüberlagerung von oben her 15 min. mit Argon gespült, wobei man das Argon aus dem Seitenhals des Zweihalskolbens ausströmen lässt. Über den Seitenhals des Zweihalskolbens führt man ein glasummanteltes Pt-100 Thermoelement in den Kolben ein und platziert das Ende kurz oberhalb des Magnetrührkerns. Dann wird die Apparatur mit mehreren lockeren Wicklungen von Haushaltsaluminiumfolie thermisch isoliert, wobei die Isolation bis zur Mitte des Steigrohres des Wasserabscheiders geführt wird. Dann wird die Apparatur schnell mit einem Laborheizrührwerk auf 250-255 °C, gemessen am Pt-100 Thermofühler, der in die aufgeschmolzene, gerührte Reaktionsmischung eintaucht, erhitzt. Während der nächsten 1 h wird das Reaktionsgemisch bei 250-255 °C gehalten, wobei wenig Kondensat abdestilliert und sich im Wasserabscheider sammelt. Nach 1 h lässt man auf 190 °C abkühlen, entfernt die Heizschale und tropft dann 100 ml Ethylenglycol zu. Nach Erkalten auf 100 °C tropft man langsam 400 ml Methanol zu. Die so erhaltene beige Suspension wird über eine Umkehrfritte filtriert, der beige Feststoff wird dreimal mit 50 ml Methanol gewaschen und dann im Vakuum getrocknet. Rohausbeute: quantitativ. Der so erhaltene Feststoff wird in 200 ml Dichlormethan gelöst und über ca. 1 kg mit Dichlormethan vorgeschlämmtes Kieselgel (Säulendurchmesser ca. 18 cm) unter Luft- und Lichtausschluss filtriert, wobei dunkle Anteile am Start liegen bleiben. Die Kernfraktion wird herausgeschnitten und am Rotationsverdampfer eingeengt, wobei gleichzeitig kontinuierlich MeOH bis zur Kristallisation zugetropft wird. Nach Absaugen, Waschen mit wenig MeOH und Trocknen im Vakuum erfolgt die weitere Reinigung des orangenfarbenen Produkts durch fünfmalige kontinuierliche Heißextraktion mit Dichlormethan/Acetonitril 1:1 (vv) (Vorlagemenge jeweils ca. 200 ml, Extraktionshülse: Standard Soxhletthülsen aus Cellulose der Fa. Whatman) unter sorgfältigem Luft- und Lichtausschluss. Über das Verhältnis Dichlormethan (Niedersieder und Gutlöser) : Acetonitril (Hochsieder und Schlechtlöser) kann der Verlust in die Mutterlauge eingestellt werden. Typischerweise sollte er 3-6 Gew.-% der eingesetzten Menge betragen. Es können zur Heißextration auch andere Lösungsmittel wie Toluol, Xylol, Essigester, Butylacetat, etc. verwendet werden. Abschließend wird das Produkt im Hochvakuum bei 390 °C sublimiert. Ausbeute: 5.95 g (6.1 mmol), 61 %; Reinheit: > 99.9 %ig nach HPLC.

### Variante B:

Durchführung analog Ir(L1) Variante A, jedoch werden statt 120 g Hydrochinon 300 ml Ethylenglycol [111-46-6] verwendet, und es wird 16 h bei 190 °C gerührt. Nach Erkalten auf 70 °C wird mit 300 ml Ethanol verdünnt, der Feststoff wird abgesaugt (P3), dreimal mit je 100 ml Ethanol gewaschen und dann im Vakuum getrocknet. Die weitere Reinigung erfolgt wie unter Variante A beschrieben. Ausbeute: 6.35 g (6.5 mmol), 65 %; Reinheit: > 99.9 %ig nach HPLC.

### Variante C:

Durchführung analog Ir(L1) Variante B, jedoch werden statt 4.90 g (10 mmol) Tris-acetylacetonato-iridium(III) [15635-87-7] 3.53 g (10 mmol) Iridium(III)chlorid x n H₂O (n ca. 3) und statt 120 g Hydrochinon 300 ml 2-Ethoxyethanol/Wasser (3:1, vv) verwendet, und es wird 30 h unter Rückfluss gerührt. Nach Erkalten wird der Feststoff abgesaugt (P3), dreimal mit je 30 ml Ethanol gewaschen und dann im Vakuum getrocknet. Die weitere Reinigung erfolgt wie unter Variante B beschrieben. Ausbeute: 4.67 g (5.1 mmol), 51 %; Reinheit: > 99.9 %ig nach HPLC.

Die Metallkomplexe fallen üblicherweise als 1:1 Mischung der A- und Δ-Isomere/Enantiomere an. Die im Folgenden aufgeführte Abbildungen von Komplexen zeigen üblicherweise nur ein Isomer. Werden Liganden mit drei verschiedenen Teilliganden eingesetzt, bzw. werden chirale Liganden als Racemat eingesetzt, fallen die abgeleiteten Metallkomplexe als Diastereomerenmischung an. Diese können durch fraktionierte Kristallisation oder chromatographisch getrennt werden, z. B. mit einem Säulenautomaten (CombiFlash der Fa. A. Semrau). Werden chirale Liganden enantiomerenrein eingesetzt, fallen die abgeleiteten Metallkomplexe als Diastereomerenmischung an, deren Trennung durch fraktionierte Kristallisation oder Chromatographie zu reinen Enantiomeren führt. Die getrennten Diastereomeren bzw. Enantiomeren können wie oben beschrieben z. B. durch Heißextraktion weiter gereinigt werden.

Im Folgenden wird als Diastereomer1 dasjenige Diasteromer bezeichnet, das auf Dünnschichtchromatographieplatten (DC Kieselgel 60 F₂₅₄ der Fa. Merck) mit dem Elutionsmittel Ethylacetat den größeren Rf-Wert zeigt; als Diastereomer2 wird dasjenige Diasteromer bezeichnet, das den kleineren Rf-Wert zeigt.

Analog können folgende Verbindungen dargestellt werden:

| Bsp. | Ligand | Produkt Variante / Extraktionsmittel* | Ausbeute |
|---|---|---|---|
| Ir(L-Ref.1) | L-Ref.1 | Ir(L-Ref.1) | 30% |
| | | B | |
| Ir(L2) | L2 | Ir(L2) | 63% |
| | | B | |
| Ir(L3) | L3 | Ir(L3) | 65% |
| | | A / Toluol | |
| Ir(100) | L100 | 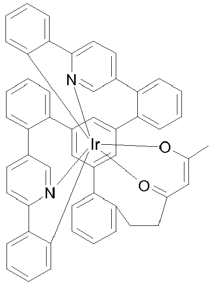 | 60% |
| Ir(L-Ref.2) | L-Ref.2 | Ir(L-Ref.2) | 24% |
| | | B | |
| Ir(L101) | L101 | Ir(L101) | 57% |
| | | A | |
| Ir(L102) | L102 | Ir(L102) | 60% |
| | | B / Toluol | |
| Ir(L103) | L103 | Ir(L103) | 66% |
| | | C / Toluol | |
| Ir(L104) | L104 | Ir(L104) | 62% |
| | | A | |
| Ir(L105) | L105 | Ir(L105) | 58% |
| | | A | |
| Ir1(L200) | L200 | 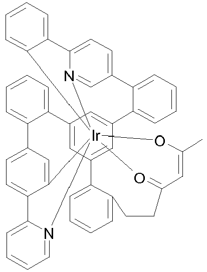 | 24% |
| | | Ir1(L200) | |
| | | A | |
| | | Diastereomer1 | |
| Ir2(L200) | L200 | Ir2(L200) | 27% |
| | | A | |
| | | Diastereomer2 | |
| Ir1(L-Ref.3) | L-Ref.3 | Ir1(L-Ref.3) | 10% |
| | | B | |
| | | Diastereomer1 | |
| Ir2(L-Ref.3) | L-Ref.3 | Ir2(L-Ref.3) | 13% |
| | | B | |
| | | Diastereomer2 | |
| Ir(L201) | L201 | Ir(L201) | 68% |
| | | A | |
| | | Isomerengemisch | |
| Ir1(L202) | L202 | Ir1(L202) | 30% |
| | | B | |
| | | Diastereomer1 | |
| Ir2(L202) | L202 | Ir2(L202) | 28% |
| | | B | |
| | | Diastereomer2 | |
| Ir1(L203) | L203 | Ir1(L203) | 25% |
| | | C | |
| | | Diastereomer1 | |
| Ir2(L203) | L203 | Ir2(L203) | 29% |
| | | C | |
| | | Diastereomer2 | |
| Ir1(L204) | L204 | Ir1(L204) | 35% |
| | | C | |
| | | Diastereomer1 | |
| Ir2L204) | L204 | Ir2L204) | 23% |
| | | C | |
| | | Diastereomer2 | |
| Ir1(L205) | L205 | Ir1(L205) | 31% |
| | | A | |
| | | Diastereomer1 | |
| Ir2L205) | L205 | Ir2L205) | 33% |
| | | A | |
| | | Diastereomer2 | |
| Ir1(L206) | L206 | Ir1(L206) | 27% |
| | | A | |
| | | Diastereomer1 | |
| Ir2L206) | L206 | Ir2L206) | 34% |
| | | A | |
| | | Diastereomer2 | |
| Ir1(L207) | L207 | Ir1(L207) | 30% |
| | | B | |
| | | Diastereomer1 | |
| Ir2L207) | L207 | Ir2L207) | 35% |
| | | B | |
| | | Diastereomer2 | |
| Ir1(L208) | L208 | Ir1(L208) | 28% |
| | | B | |
| | | Diastereomer1 | |
| Ir2L208) | L208 | Ir2L208) | 31% |
| | | B | |
| | | Diastereomer2 | |
| Ir1(L209) | L209 | Ir1(L209) | 30% |
| | | B | |
| | | Diastereomer1 | |
| Ir2L209) | L209 | Ir2L209) | 30% |
| | | B | |
| | | Diastereomer2 | |
| Ir1(L210) | L210 | Ir1(L210) | 32% |
| | | A | |
| | | Diastereomer1 | |
| Ir2L210) | L210 | Ir2L210) | 27% |
| | | A | |
| | | Diastereomer2 | |
| Ir1(L211) | L211 | Ir1(L211) | 33% |
| | | A | |
| | | Diastereomer1 | |
| Ir2L211) | L211 | Ir2L211) | 30% |
| | | A | |
| | | Diastereomer2 | |
| Ir1(L212) | L212 | Ir1(L212) | 33% |
| | | C | |
| | | Diastereomer1 | |
| Ir2L212) | L212 | Ir2L212) | 29% |
| | | C | |
| | | Diastereomer2 | |
| Ir1(L213) | L213 | Ir1(L213) | 35% |
| | | A | |
| | | Diastereomer1 | |
| Ir2L213) | L213 | Ir2L213) | 31% |
| | | A | |
| | | Diastereomer2 | |
| Ir1(L214) | L214 | Ir1(L214) | 27% |
| | | B | |
| | | Diastereomer1 | |
| Ir2L214) | L214 | Ir2L214) | 23% |
| | | B | |
| | | Diastereomer2 | |

| | | | |
|---|---|---|---|
| * falls abweichend | | | |

### Sublimationstemperaturen und -raten:

Im Vergleich zu den tripodalen Komplexen mit drei Phenylpyridin-artigen Teilliganden sublimieren die erfindungsgemäßen Verbindungen bei geringeren Temperaturen und mit höheren Sublimationsraten (g/h) bei einer gegebenen Sublimationstemperatur, wie in der nachfolgenden Tabelle aufgeführt. Die exakten Temperaturen und Sublimationsraten hängen dabei immer vom genauen Druck und der jeweiligen Geometrie der verwendeten Sublimationsapparatur ab. Die Temperaturen und Sublimationsraten werden bei einem Basisdruck von ca. 10⁻⁵ mbar in jeweils derselben Apparatur bestimmt.

| **Komplex** | **Sublimationstemperatur [°C]** | **Sublimationsrate bei der angegebenen Sublimationstemperatur [g/h]** |
|---|---|---|
| Ir-Ref.1 | ∼ 440 | 0.6 |
| Ir-Ref.2 | ∼ 440 | 0.5 |
| Ir-Ref.3 | ∼ 420 | 1.0 |
| Ir(L1) | ∼ 390 | 1.4 |
| Ir(L100) | ∼ 390 | 1.3 |
| Ir(L200) | ∼ 380 | 1.8 |

### Beispiel: Herstellung der OLEDs

### 1) Vakuum-prozessierte Devices:

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Gereinigte Glasplättchen (Reinigung in Miele Laborspülmaschine, Reiniger Merck Extran), die mit strukturiertem ITO (Indium Zinn Oxid) der Dicke 50 nm beschichtet sind, werden 25 Minuten mit UV-Ozon vorbehandelt (UV-Ozon Generator PR-100, Firma UVP), innerhalb 30 min. zur verbesserten Prozessierung mit 20 nm PEDOT:PSS beschichtet (Poly-(3,4-ethylendioxythiophen)poly(styrolsulfonat), bezogen als CLEVIOS™ P VP AI 4083 von Heraeus Precious Metals GmbH Deutschland, aus wässriger Lösung aufgeschleudert) und anschließend bei 180 °C 10 min. lang ausgeheizt. Diese beschichteten Glasplättchen bilden die Substrate, auf welche die OLEDs aufgebracht werden. Die OLEDs haben prinzipiell folgenden Schichtaufbau: Substrat / Lochtransportschicht 1 (HTL1) bestehend aus HTM dotiert mit 5 % NDP-9 (kommerziell erhältlich von der Fa. Novaled), 20 nm / Lochtransportschicht 2 (HTL2) / optionale Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet.

Zunächst werden vakuum-prozessierte OLEDs beschrieben. Hierfür werden alle Materialien in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Co-Verdampfung in einem bestimmten Volumenanteil beigemischt wird. Eine Angabe wie M1:M2:Ir(L2) (55%:35%:10%) bedeutet hierbei, dass das Material M1 in einem Volumenanteil von 55%, M2 in einem Volumenanteil von 35% und Ir(L1) in einem Volumenanteil von 10% in der Schicht vorliegt. Analog kann auch die Elektronentransportschicht aus einer Mischung zweier Materialien bestehen. Der genaue Aufbau der OLEDs ist Tabelle 2 zu entnehmen. Die zur Herstellung der OLEDs verwendeten Materialien sind in Tabelle 4 gezeigt.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Als Lebensdauer LD80 wird die Zeit definiert, nach der die Leuchtdichte bei Betrieb mit einem konstantem Strom von 40 mA/cm² auf 80% der Startleuchtdichte absinkt.

### Verwendung von erfindungsgemäßen Verbindungen als Emittermaterialien in phosphoreszierenden OLEDs

Die erfindungsgemäßen Verbindungen lassen sich unter anderem als phosphoreszierende Emittermaterialien in der Emissionsschicht in OLEDs einsetzen. Als Vergleich gemäß dem Stand der Technik werden die Iridiumverbindungen gemäß Tabelle 4 verwendet. Die Ergebnisse der OLEDs sind in Tabelle 2 zusammengefasst.

**Tabelle 1: Aufbau der OLEDs**

| **Bsp.** | **HTL2 Dicke** | **EBL Dicke** | **EML Dicke** | **HBL Dicke** | **ETL Dicke** |
|---|---|---|---|---|---|
| Ref.-D1 | HTM 40 nm | --- | M1:Ir-Ref.1 (90%:10%) 30 nm | ETM1 10nm | ETM1:ETM2 (50%:50%) 30 nm |
| Ref.-D2 | HTM 40 nm | --- | M1: Ir-Ref.2 (90%:10%) 30 nm | ETM1 10nm | ETM1:ETM2 (50%:50%) 30 nm |
| Ref.-D3 | HTM 40 nm | --- | M1:I Ir-Ref.3 (90%:10%) 30 nm | ETM1 10nm | ETM1:ETM2 (50%:50%) 30 nm |
| Ref.-D4 | HTM 40 nm | --- | M1:M2: Ir-Ref.1 (60%:30%:10%) 30 nm | ETM1 10nm | ETM1:ETM2 (50%:50%) 30 nm |
| Ref.-D5 | HTM 40 nm | --- | M1:M2: Ir-Ref.2 (60%:30%:10%) 30 nm | ETM1 10nm | ETM1:ETM2 (50%:50%) 30 nm |
| Ref.-D6 | HTM 40 nm | --- | M1:Ir(L-Ref.1) (90%:10%) 30 nm | ETM1 10nm | ETM1:ETM2 (50%:50%) 30 nm |
| Ref.-D7 | HTM 40 nm | --- | M1: Ir(L-Ref.2) (90%:10%) 30 nm | ETM1 10nm | ETM1:ETM2 (50%:50%) 30 nm |
| Ref.-D8 | HTM 40 nm | --- | M1:I Ir1(L-Ref.3) (90%:10%) 30 nm | ETM1 10nm | ETM1:ETM2 (50%:50%) 30 nm |
| D1 | HTM 40 nm | --- | M1: Ir(L1) (90%:10%) 30 nm | ETM1 10nm | ETM1:ETM2 (50%:50%) 30 nm |
| D2 | HTM 40 nm | --- | M1:Ir(L100) (90%:10%) 30 nm | ETM1 10nm | ETM1:ETM2 (50%:50%) 30 nm |
| D3 | HTM 40 nm | --- | M1:Ir(L200) (90%:10%) 30 nm | ETM1 10nm | ETM1:ETM2 (50%:50%) 30 nm |
| D4 | HTM 40 nm | --- | M1:M2: Ir(I1) (60%:30%:10%) 30 nm | ETM1 10nm | ETM1:ETM2 (50%:50%) 30 nm |
| D5 | HTM 40 nm | --- | M1:M2: Ir(L100) (60%:30%:10%) 30 nm | ETM1 10nm | ETM1:ETM2 (50%:50%) 30 nm |
| D6 | HTM 40 nm | --- | M1:M2: Ir(L200) (60%:30%:10%) 30 nm | ETM1 10nm | ETM1:ETM2 (50%:50%) 30 nm |
| D7 | HTM 30 nm | EBM 10 nm | M1:M2: Ir(L200) (60%:30%:10%) 30 nm | ETM1 10nm | ETM1:ETM2 (50%:50%) 30 nm |

**Tabelle 2: Ergebnisse der vakuum-prozessierten OLEDs**

| **Bsp.** | **EQE (%) @ 1000 cd/m²** | **Spannung (V) @ 1000 cd/m²** | **CIE x/y @ 1000 cd/m²** | **LD80 (h) @ 40 mA/cm²** |
|---|---|---|---|---|
| Ref.-D1 | 20.3 | 3.1 | 0.34/0.62 | 220 |
| Ref.-D2 | 20.7 | 3.0 | 0.40/0.58 | 240 |
| Ref.-D3 | 16.0 | 3.1 | 0.38/0.57 | 250 |
| Ref.-D4 | 20.6 | 3.1 | 0.34/0.62 | 250 |
| Ref.-D5 | 21.0 | 3.1 | 0.39/0.59 | 270 |
| Ref.-D6 | 19.9 | 3.2 | 0.37/0.60 | 150 |
| Ref.-D7 | 20.1 | 3.3 | 0.45/0.52 | 130 |
| Ref.-D8 | 20.4 | 3,2 | 0.43/0.53 | 160 |
| D1 | 22.6 | 2.9 | 0.39/0.59 | 220 |
| D2 | 22.9 | 3.0 | 0.43/0.55 | 250 |
| D3 | 23.4 | 3.0 | 0.44/0.54 | 280 |
| D4 | 22.2 | 2.9 | 0.39/0.59 | 280 |
| D5 | 22.5 | 3.0 | 0.43/0.55 | 310 |
| D6 | 23.0 | 3.0 | 0.44/0.54 | 300 |
| D7 | 22.9 | 3.0 | 0.44/0.54 | 320 |

### Lösungs-prozessierte Devices:

### Aus niedermolekularen löslichen Funktionsmaterialien

Die erfindungsgemäßen Verbindungen können auch aus Lösung verarbeitet werden und führen dort zu prozesstechnisch wesentlich einfacheren OLEDs im Vergleich zu vakuumprozessierten OLEDs mit dennoch guten Eigenschaften. Die Herstellung solcher Bauteile lehnt sich an die Herstellung polymerer Leuchtdioden (PLEDs) an, die in der Literatur bereits vielfach beschrieben ist (z. B. in der WO 2004/037887). Der Aufbau setzt sich aus Substrat / ITO / Lochinjektionsschicht (60 nm) / Interlayer (20 nm) / Emissionsschicht (60 nm) / Lochblockierschicht (10 nm) / Elektronentransportschicht (40 nm) / Kathode zusammen. Dazu werden Substrate der Firma Technoprint (Sodalimeglas) verwendet, auf welche die ITO-Struktur (Indium-Zinn-Oxid, eine transparente, leitfähige Anode) aufgebracht wird. Die Substrate werden im Reinraum mit DI Wasser und einem Detergens (Deconex 15 PF) gereinigt und dann durch eine UV/Ozon-Plasmabehandlung aktiviert. Danach wird ebenfalls im Reinraum eine 20 nm Lochinjektionsschicht durch Spin-Coating aufgebracht. Die benötigte Spinrate hängt vom Verdünnungsgrad und der spezifischen Spin-Coater-Geometrie ab. Um Restwasser aus der Schicht zu entfernen, werden die Substrate für 30 Minuten bei 200 °C auf einer Heizplatte ausgeheizt. Die verwendete Interlayer dient dem Lochtransport, in diesem Fall wird HL-X092 von Merck verwendet. Die Interlayer kann alternativ auch durch eine oder mehrere Schichten ersetzt werden, die lediglich die Bedingung erfüllen müssen, durch den nachgelagerten Prozessierungsschritt der EML-Abscheidung aus Lösung nicht wieder abgelöst zu werden. Zur Herstellung der Emissionsschicht werden die erfindungsgemäßen Triplettemitter zusammen mit den Matrixmaterialien in Toluol oder Chlorbenzol gelöst. Der typische Feststoffgehalt solcher Lösungen liegt zwischen 16 und 25 g/L, wenn, wie hier, die für eine Device typische Schichtdicke von 60 nm mittels Spincoating erzielt werden soll. Die lösungsprozessierten Devices vom Typ1 enthalten eine Emissionsschicht aus M3:M4:IrL (20%:60%:20%), die vom Typ2 enthalten eine Emissionsschicht aus M3:M4:IrLa:IrLb (30%:34%:30%:6%), d. h. sie enthalten zwei verschiedene Iridiumkomplexe. Die Emissionsschicht wird in einer Inertgasatmosphäre, im vorliegenden Fall Argon, aufgeschleudert und 10 min bei 160 °C ausgeheizt. Darüber wird die Lochblockierschicht (10 nm ETM1) und die Elektronentransportschicht (40 nm ETM1 (50%) / ETM2 (50%)) aufgedampft (Aufdampfanlagen von Lesker o.a., typischer Aufdampfdruck 5 x 10⁻⁶ mbar). Zuletzt wird eine Kathode aus Aluminium (100 nm) (hochreines Metall von Aldrich) aufgedampft. Um das Device vor Luft und Luftfeuchtigkeit zu schützen, wird die Vorrichtung abschließend verkapselt und dann charakterisiert. Die genannten OLED-Beispiele sind noch nicht optimiert, Tabelle 3 fasst die erhaltenen Daten zusammen.

**Tabelle 3: Ergebnisse mit aus Lösung prozessierten Materialien**

| **Bsp.** | **Emitter Device** | **EQE (%) 1000 cd/m²** | **Spannung (V) 1000 cd/m²** | **CIE x/y** | **LD50 (h) 1000 cd/m²** |
|---|---|---|---|---|---|
| Sol-Ref.1 | Ir-Ref.4 | 20.6 | 5.2 | 0.36/0.61 | 220000 |
| | Typ1 | | | | |
| Sol-Ref.2 | Ir-Ref.5 | 21.4 | 5.0 | 0.31/0.62 | 11000 |
| | Typ1 | | | | |
| Sol-D1 | Ir(L2) | 22.3 | 5.1 | 0.38/0.59 | 230000 |
| | Typ1 | | | | |
| Sol-D2 | Ir(L202) | 21.4 | 4.7 | 0.68/0.32 | 320000 |
| | Ir(L213) | | | | |
| | Typ2 | | | | |

**Tabelle 4: Strukturformeln der verwendeten Materialien**

| | |
|---|---|
| 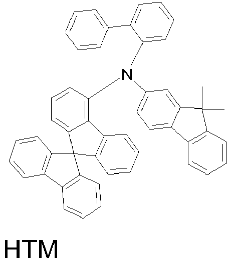 | 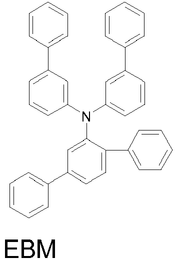 |
| [1450933-44-4] | [1443540-48-4] |
| 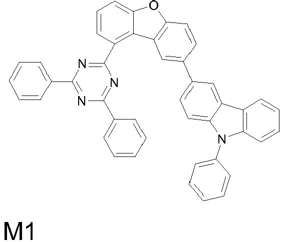 | 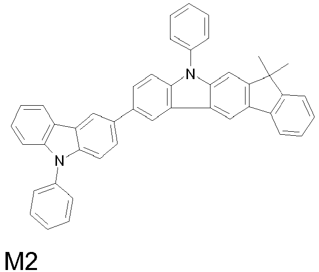 |
| [1822310-78-0] | [1357150-54-9] |
| 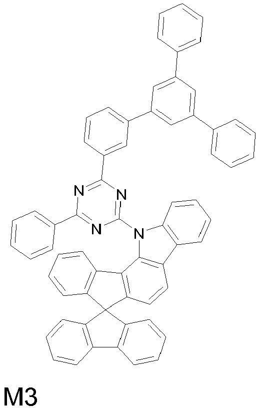 | 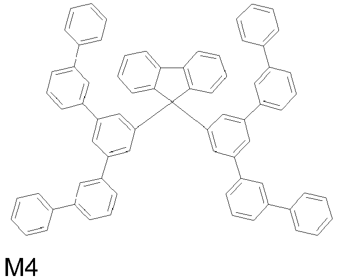 |
| [1616231-60-7] | [1246496-85-4] |
| 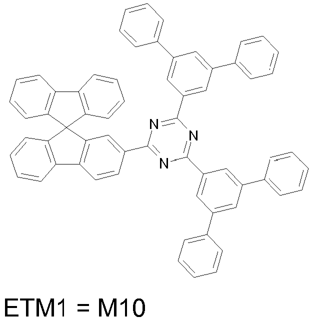 | 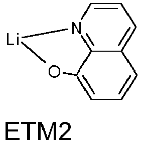 |
| [1233200-52-6] | [25387-93-3] |
| 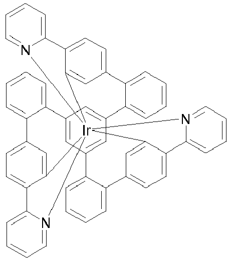 | 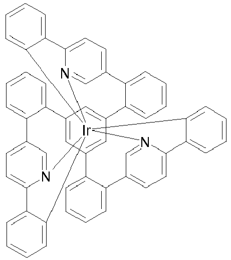 |
| [1989600-78-3] | [1989600-75-0] |
| Ir-Ref.1 | Ir-Ref.2 |
| 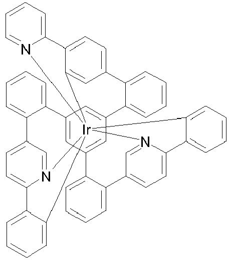 | 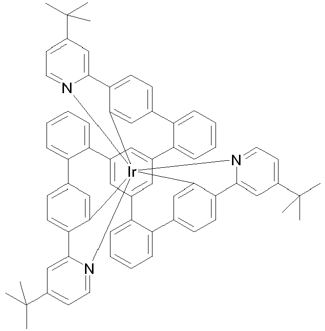 |
| [1989600-79-4] | [1989606-01-0] |
| Ir-Ref.3 | Ir-Ref.4 |
| 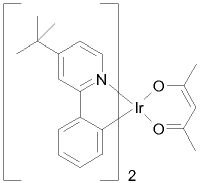 | |
| Ir-Ref.5 | |
| [1375601-05-0] | |

## Patentansprüche

1. Verbindung der Formel (1), 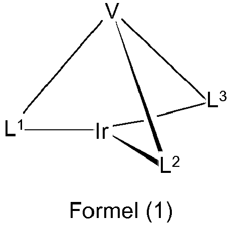 wobei für die verwendeten Symbole gilt:
L¹ ist ein Teilligand der Formel (2), der über die beiden Gruppen Z an das Iridium koordiniert und der über die gestrichelte Bindung an V gebunden ist, 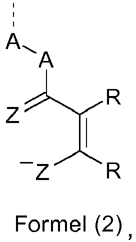
A ist bei jedem Auftreten gleich oder verschieden CR₂, O, S oder NR, wobei mindestens eine Gruppe A für CR₂ steht;
Z ist bei jedem Auftreten gleich oder verschieden O, S oder NR;
L² ist ein bidentater, monoanionischer Teilligand, der über ein Kohlenstoffatom und ein Stickstoffatom oder über zwei Kohlenstoffatome an das Iridium koordiniert;
L³ ist ein bidentater, monoanionischer Teilligand, der über ein Kohlenstoffatom und ein Stickstoffatom oder über zwei Kohlenstoffatome an das Iridium koordiniert, oder ist ein Teilligand der Formel (2), der gleich oder verschieden L¹ sein kann;
V ist eine Gruppe der Formel (3), wobei die gestrichelten Bindungen jeweils die Verknüpfung der Teilliganden L¹, L² und L³ darstellen, 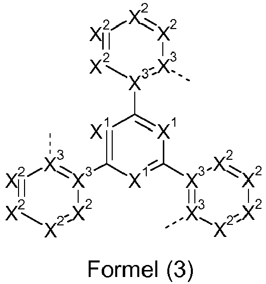
X¹ ist bei jedem Auftreten gleich oder verschieden CR oder N;
X² ist bei jedem Auftreten gleich oder verschieden CR oder N, oder zwei benachbarte Gruppen X² stehen zusammen für NR, O oder S, so dass ein Fünfring entsteht; oder zwei benachbarte Gruppen X² stehen zusammen für CR oder N, wenn in dem Cyclus eine der Gruppen X³ für N steht, so dass ein Fünfring entsteht; mit der Maßgabe, dass maximal zwei benachbarte Gruppen X² in jedem Ring für N stehen;
X³ ist bei jedem Auftreten in einem Cyclus C oder eine Gruppe X³ steht für N und die andere Gruppe X³ in demselben Cyclus steht für C, wobei die Gruppen X³ in den drei Cyclen unabhängig voneinander gewählt werden können; mit der Maßgabe, dass zwei benachbarte Gruppen X² zusammen für CR oder N stehen, wenn in dem Cyclus eine der Gruppen X³ für N steht;
R ist bei jedem Auftreten gleich oder verschieden H, D, F, Cl, Br, I, N(R¹)₂, OR¹, SR¹, CN, NO₂, COOH, C(=O)N(R¹)₂, Si(R¹)₃, B(OR¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann und wobei eine oder mehrere nicht benachbarte CH₂-Gruppen durch Si(R¹)₂, C=O, NR¹, O, S oder CONR¹ ersetzt sein können, oder ein aromatisches oder heteroaromatisches Ringsystem mit 5 bis 40 aromatischen Ringatomen, das jeweils durch einen oder mehrere Reste R¹ substituiert sein kann; dabei können zwei Reste R auch miteinander ein Ringsystem bilden;
R¹ ist bei jedem Auftreten gleich oder verschieden H, D, F, Cl, Br, I, N(R²)₂, OR², SR², CN, NO₂, Si(R²)₃, B(OR²)₂, C(=O)R², P(=O)(R²)₂, S(=O)R², S(=O)₂R², OSO₂R², eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R² substituiert sein kann und wobei eine oder mehrere nicht benachbarte CH₂-Gruppen durch Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann; dabei können zwei oder mehrere Reste R¹ miteinander ein Ringsystem bilden;
R² ist bei jedem Auftreten gleich oder verschieden H, D, F oder ein aliphatischer, aromatischer und/oder heteroaromatischer organischer Rest, insbesondere ein Kohlenwasserstoffrest, mit 1 bis 20 C-Atomen, in dem auch ein oder mehrere H-Atome durch F ersetzt sein können;
dabei können die drei bidentaten Liganden L¹, L² und L³ außer durch die Brücke V auch noch durch eine weitere Brücke zu einem Kryptat geschlossen sein.

2. Verbindung nach Anspruch 1, **dadurch gekennzeichnet, dass** V ausgewählt ist aus den Formeln (4a) bis (7a), 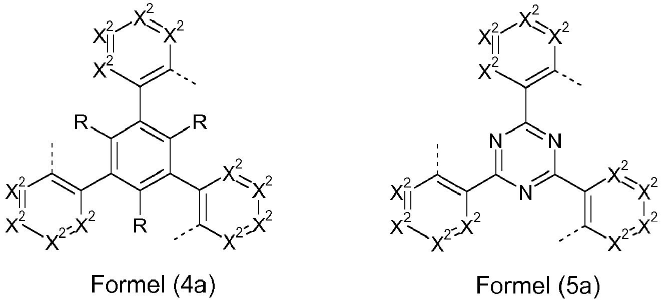 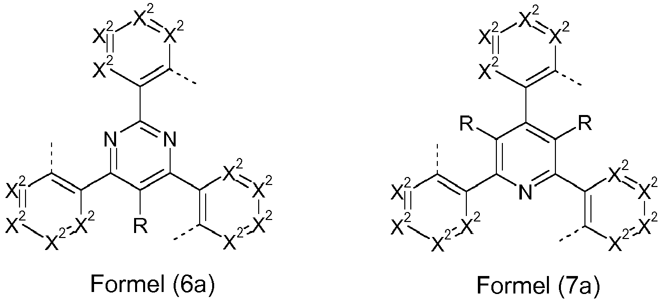 wobei die Symbole die in Anspruch 1 aufgeführten Bedeutungen aufweisen.

3. Verbindung nach Anspruch 1 oder 2, **dadurch gekennzeichnet, dass** V eine Struktur der Formel (4b'), (4c) oder (5c) aufweist, 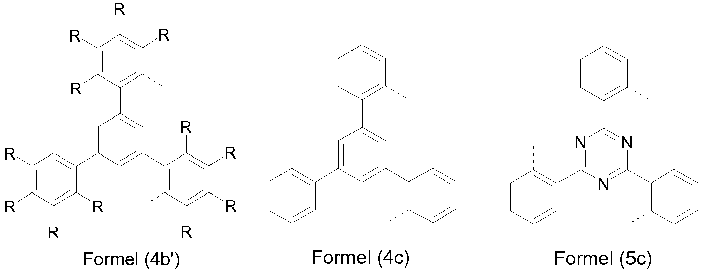 wobei die Symbole die in Anspruch 1 genannten Bedeutungen aufweisen.

4. Verbindung nach einem oder mehreren der Ansprüche 1 bis 3, **dadurch gekennzeichnet, dass** beide Gruppen A im Teilliganden L¹ für CR₂ stehen.

5. Verbindung nach einem oder mehreren der Ansprüche 1 bis 4, **dadurch gekennzeichnet, dass** beide Gruppen Z im Teilliganden L¹ für O stehen.

6. Verbindung nach einem oder mehreren der Ansprüche 1 bis 5, **dadurch gekennzeichnet, dass** L¹ eine Struktur der Formel (2c) aufweist, 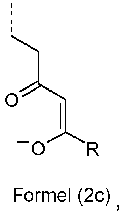 wobei die gestrichelte Bindung die Bindung an V darstellt und R gleich oder verschieden bei jedem Auftreten ausgewählt ist aus der Gruppe bestehend aus H, D, OR¹, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R¹ substituiert sein kann, oder einem aromatischen oder heteroaromatischen Ringsystem mit 5 bis 24 aromatischen Ringatomen, das jeweils durch einen oder mehrere Reste R¹ substituiert sein kann, und R¹ die in Anspruch 1 genannten Bedeutungen aufweist.

7. Verbindung nach einem oder mehreren der Ansprüche 1 bis 6, **dadurch gekennzeichnet, dass** beide Teilliganden L² und L³ jeweils ein Kohlenstoffatom und ein Stickstoffatom als koordinierende Atome aufweisen.

8. Verbindung nach einem oder mehreren der Ansprüche 1 bis 7, **dadurch gekennzeichnet, dass** die Teilliganden L² und L³ gleich oder verschieden bei jedem Auftreten für eine Struktur gemäß Formel (L-1) oder (L-2) stehen, 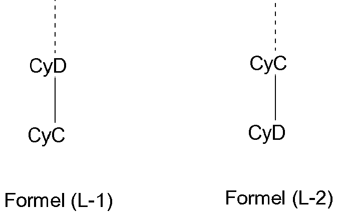 wobei die gestrichelte Bindung die Bindung des Teilliganden an V darstellt und für die weiteren Symbole gilt:
CyC ist gleich oder verschieden bei jedem Auftreten eine substituierte oder unsubstituierte Aryl- oder Heteroarylgruppe mit 5 bis 14 aromatischen Ringatomen, welche jeweils über ein Kohlenstoffatom an das Metall koordiniert und welche über eine kovalente Bindung mit CyD verbunden ist;
CyD ist gleich oder verschieden bei jedem Auftreten eine substituierte oder unsubstituierte Heteroarylgruppe mit 5 bis 14 aromatischen Ringatomen, welche über ein Stickstoffatom oder über ein Carben-Kohlenstoffatom an das Metall koordiniert und welche über eine kovalente Bindung mit CyC verbunden ist;
dabei können mehrere der optionalen Substituenten miteinander ein Ringsystem bilden.

9. Verbindung nach Anspruch 8, **dadurch gekennzeichnet, dass** einer der Teilliganden L² und L³ eine Struktur der Formel (L-1) aufweist und der andere der Teilliganden L² und L³ eine Struktur der Formel (L-2) aufweist.

10. Verbindung nach Anspruch 8 oder 9, **dadurch gekennzeichnet, dass** CyC ausgewählt ist aus den Strukturen der Formeln (CyC-1) bis (CyC-19), wobei die Gruppe CyC jeweils an der durch # gekennzeichneten Position an CyD bindet und an der durch * gekennzeichneten Position an das Iridium koordiniert, und dass die Gruppe CyD ausgewählt ist aus den Strukturen der Formeln (CyD-1) bis (CyD-12), wobei die Gruppe CyD jeweils an der durch # gekennzeichneten Position an CyC bindet und an der durch * gekennzeichneten Position an das Iridium koordiniert, 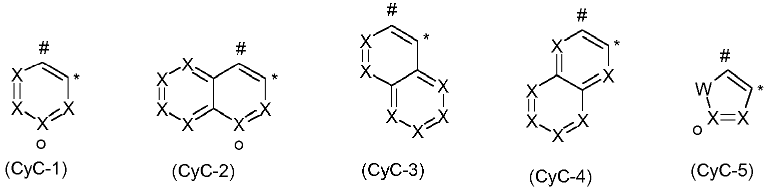 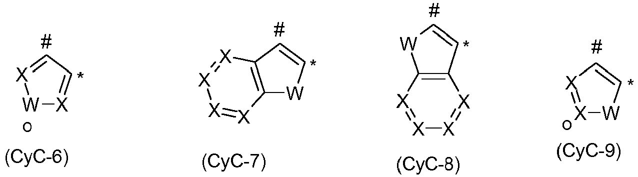 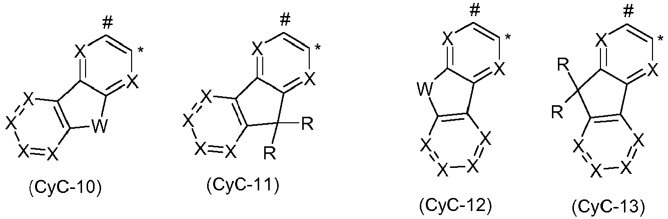 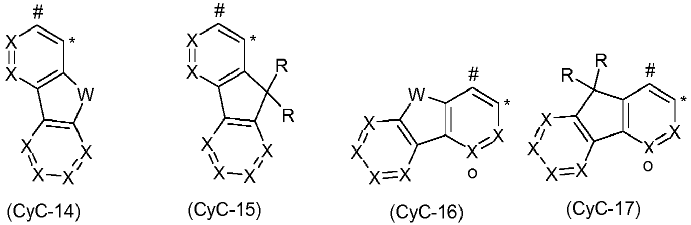 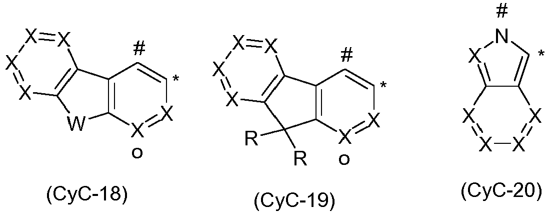 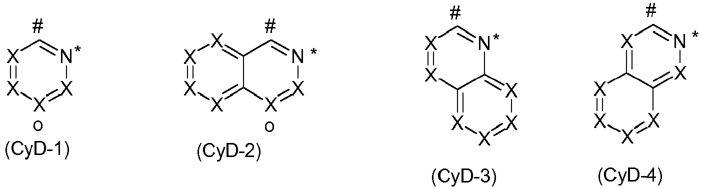 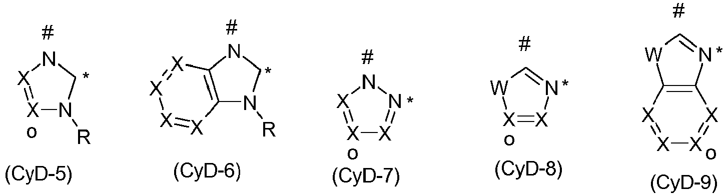 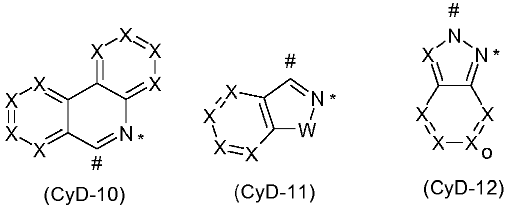 wobei R die in Anspruch 1 genannten Bedeutungen aufweist und für die weiteren Symbole gilt:
X ist bei jedem Auftreten gleich oder verschieden CR oder N mit der Maßgabe, dass maximal zwei Symbole X pro Cyclus für N stehen;
W ist bei jedem Auftreten gleich oder verschieden NR, O oder S;
mit der Maßgabe, dass, wenn die Brücke V an CyC gebunden ist, ein Symbol X in der entsprechenden Gruppe CyC für C steht und die Brücke V an dieses Kohlenstoffatom gebunden ist, und wenn die Brücke V an CyD gebunden ist, ein Symbol X in der entsprechenden Gruppe CyD für C steht und die Brücke V an dieses Kohlenstoffatom gebunden ist; dabei erfolgt die Bindung an die Brücke V über eine Position, die mit "o" markiert ist.

11. Verfahren zur Herstellung einer Verbindung nach einem oder mehreren der Ansprüche 1 bis 10 durch Umsetzung des freien Liganden mit Iridiumalkoholaten der Formel (52), mit Iridiumketoketonaten der Formel (53), mit Iridiumhalogeniden der Formel (54) oder mit Iridiumcarboxylaten der Formel (55), 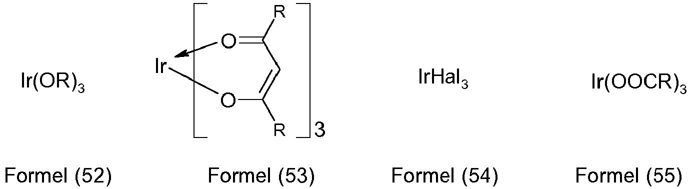 wobei R die in Anspruch 1 angegebenen Bedeutungen hat, Hal = F, Cl, Br oder I ist und die Iridiumedukte der Formeln (52) bis (55) auch als Hydrate vorliegen können.

12. Formulierung, enthaltend mindestens eine Verbindung nach einem oder mehreren der Ansprüche 1 bis 10 und mindestens eine weitere Verbindung, wobei die weitere Verbindung bevorzugt ausgewählt ist aus einem Matrixmaterial und/oder einem Lösemittel.

13. Verwendung einer Verbindung nach einem oder mehreren der Ansprüche 1 bis 10 in einer elektronischen Vorrichtung oder als Sauerstoff-Sensibilisator oder als Photoinitiator oder als Photokatalysator.

14. Elektronische Vorrichtung enthaltend mindestens eine Verbindung nach einem oder mehreren der Ansprüche 1 bis 10, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen, Infrarot-Sensoren, Sauerstoff-Sensoren oder organischen Laserdioden.

15. Elektronische Vorrichtung nach Anspruch 14, wobei es sich um eine organische Elektrolumineszenzvorrichtung handelt, **dadurch gekennzeichnet, dass** die Verbindung nach einem oder mehreren der Ansprüche 1 bis 10 in einer oder mehreren emittierenden Schichten eingesetzt wird.

## Claims

1. Compound of the formula (1) 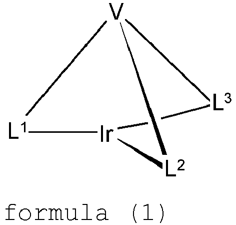 where the symbols used are as follows:
L¹ is a sub-ligand of the formula (2) which coordinates to the iridium via the two Z groups and which is bonded to V via the dotted bond, 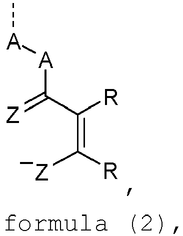
A is the same or different at each instance and is CR₂, O, S or NR, where at least one A group is CR₂;
Z is the same or different at each instance and is O, S or NR;
L² is a bidentate, monoanionic sub-ligand which coordinates to the iridium via one carbon atom and one nitrogen atom or via two carbon atoms;
L³ is a bidentate, monoanionic sub-ligand which coordinates to the iridium via one carbon atom and one nitrogen atom or via two carbon atoms, or is a sub-ligand of the formula (2) which may be the same as or different from L¹;
V is a group of the formula (3), where the dotted bonds each represent the linkage of the sub-ligands L¹, L² and L³, 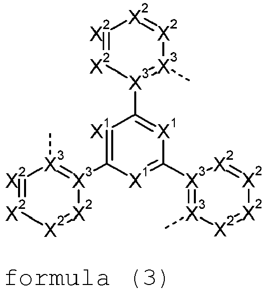
X¹ is the same or different at each instance and is CR or N;
X² is the same or different at each instance and is CR or N, or two adjacent X² groups together are NR, O or S, thus forming a five-membered ring; or two adjacent X² groups together are CR or N when one of the X³ groups in the cycle is N, thus forming a five-membered ring; with the proviso that not more than two adjacent X² groups in each ring are N;
X³ is C at each instance in one cycle or one X³ group is N and the other X³ group in the same cycle is C, where the X³ groups in the three cycles may be selected independently, with the proviso that two adjacent X² groups together are CR or N when one of the X³ groups in the cycle is N;
R is the same or different at each instance and is H, D, F, Cl, Br, I, N(R¹)₂, OR¹, SR¹, CN, NO₂, COOH, C(=O)N(R¹)₂, Si (R¹)₃, B(OR¹)₂, C(=O)R¹, P(=O) (R¹)₂, S(=O)R¹, S(=O)₂R¹, OSO₂R¹, a straight-chain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may in each case be substituted by one or more R¹ radicals and where one or more nonadjacent CH₂ groups may be replaced by Si (R¹)₂, C=O, NR¹, O, S or CONR¹, or an aromatic or heteroaromatic ring system which has 5 to 40 aromatic ring atoms and may be substituted in each case by one or more R¹ radicals; at the same time, two R radicals together may also form a ring system;
R¹ is the same or different at each instance and is H, D, F, Cl, Br, I, N(R²)₂, OR², SR², CN, NO₂, Si (R²)₃, B(OR²)₂, C(=O)R², P(=O) (R²)₂, S(=O)R², S(=O)₂R², OSO₂R², a straight-chain alkyl group having 1 to 20 carbon atoms or an alkenyl or alkynyl group having 2 to 20 carbon atoms or a branched or cyclic alkyl group having 3 to 20 carbon atoms, where the alkyl, alkenyl or alkynyl group may in each case be substituted by one or more R² radicals, and where one or more nonadjacent CH₂ groups may be replaced by Si(R²)₂, C=O, NR², O, S or CONR², or an aromatic or heteroaromatic ring system which has 5 to 40 aromatic ring atoms and may be substituted in each case by one or more R² radicals; at the same time, two or more R¹ radicals together may form a ring system;
R² is the same or different at each instance and is H, D, F or an aliphatic, aromatic and/or heteroaromatic organic radical, especially a hydrocarbyl radical, having 1 to 20 carbon atoms, in which one or more hydrogen atoms may also be replaced by F;
at the same time, the three bidentate ligands L¹, L² and L³, apart from by the bridge V, may also be closed by a further bridge to form a cryptate.

2. Compound according to Claim 1, **characterized in that** V is selected from the formulae (4a) to (7a) 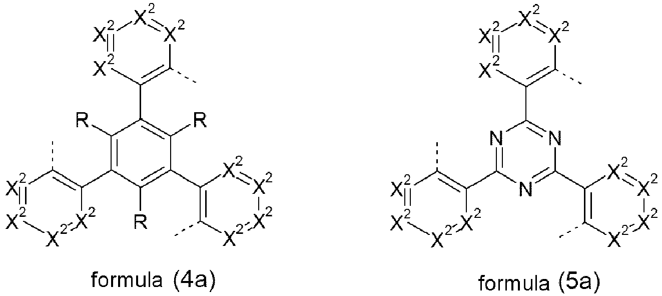 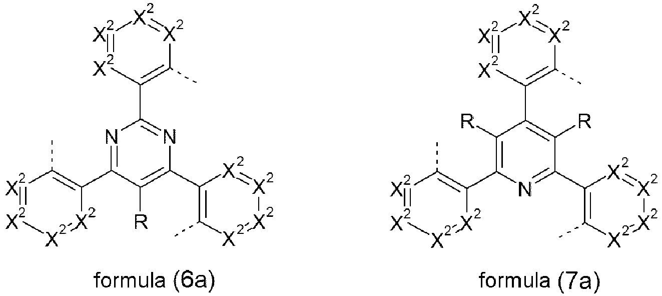 where the symbols have the definitions detailed in Claim 1.

3. Compound according to Claim 1 or 2, **characterized in that** V has a structure of the formula (4b'), (4c) or (5c) 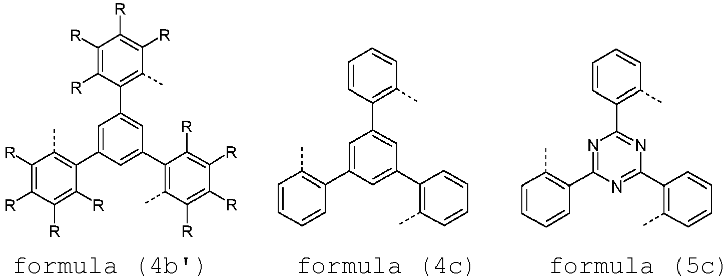 where the symbols have the definitions given in Claim 1.

4. Compound according to one or more of Claims 1 to 3, **characterized in that** both A groups in the sub-ligands L¹ are CR₂.

5. Compound according to one or more of Claims 1 to 4, **characterized in that** both Z groups in the sub-ligands L¹ are O.

6. Compound according to one or more of Claims 1 to 5, **characterized in that** L¹ has a structure of the formula (2c) 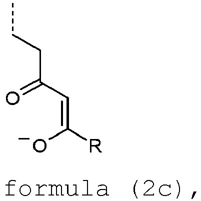 where the dotted bond represents the bond to V, and R is the same or different at each instance and is selected from the group consisting of H, D, OR¹, a straight-chain alkyl group having 1 to 10 carbon atoms or a branched or cyclic alkyl group which has 3 to 10 carbon atoms and may be substituted in each case by one or more R¹ radicals, or an aromatic or heteroaromatic ring system which has 5 to 24 aromatic ring atoms and may be substituted in each case by one or more R¹ radicals, and R¹ has the definitions given in Claim 1.

7. Compound according to one or more of Claims 1 to 6, **characterized in that** the two sub-ligands L² and L³ each have one carbon atom and one nitrogen atom as coordinating atoms.

8. Compound according to one or more of Claims 1 to 7, **characterized in that** the sub-ligands L² and L³ are the same or different at each instance and are a structure of the formula (L-1) or (L-2) 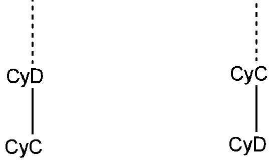 formula (L-1) formula (L-2) where the dotted bond represents the bond of the sub-ligand to V and the other symbols are as follows:
CyC is the same or different at each instance and is a substituted or unsubstituted aryl or heteroaryl group which has 5 to 14 aromatic ring atoms and coordinates in each case to the metal via a carbon atom and which is bonded to CyD via a covalent bond;
CyD is the same or different at each instance and is a substituted or unsubstituted heteroaryl group which has 5 to 14 aromatic ring atoms and coordinates to the metal via a nitrogen atom or via a carbene carbon atom and which is bonded to CyC via a covalent bond;
at the same time, two or more of the optional substituents together may form a ring system.

9. Compound according to Claim 8, **characterized in that** one of the sub-ligands L² and L³ has a structure of the formula (L-1) and the other of the sub-ligands L² and L³ has a structure of the formula (L-2).

10. Compound according to Claim 8 or 9, **characterized in that** CyC is selected from the structures of the formulae (CyC-1) to (CyC-19), where the CyC group binds to CyD in each case at the position identified by # and coordinates to the iridium at the position identified by *, and **in that** the CyD group is selected from the structures of the formulae (CyD-1) to (CyD-12), where the CyD group binds to CyC in each case at the position identified by # and coordinates to the iridium at the position identified by *, 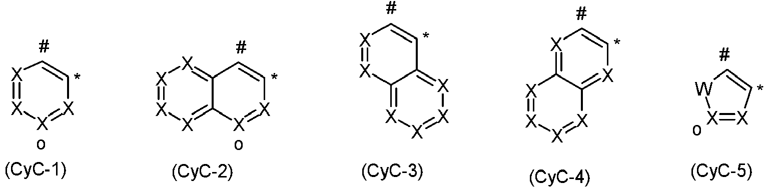 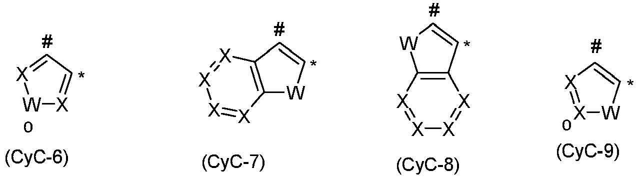 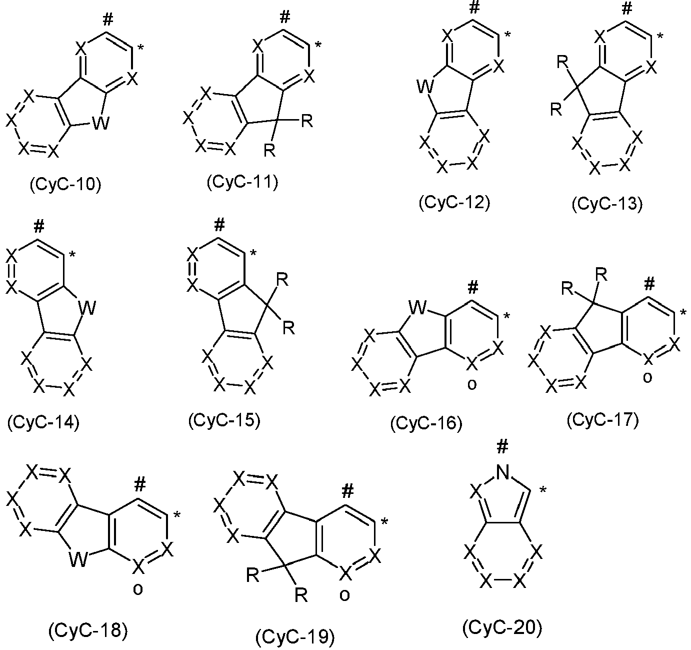 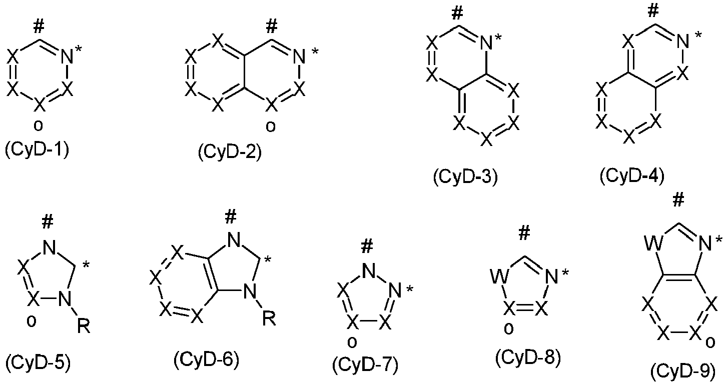 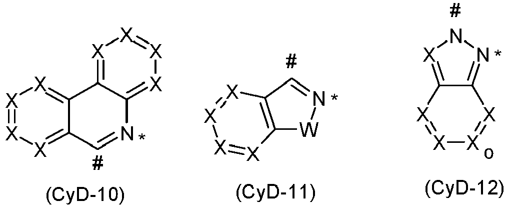 where R has the definitions given in Claim 1 and the other symbols are as follows:
X is the same or different at each instance and is CR or N, with the proviso that not more than two symbols X per cycle are N;
W is the same or different at each instance and is NR, O or S;
with the proviso that when the bridge V is bonded to CyC, one symbol X in the corresponding CyC group is C and the bridge V is bonded to this carbon atom, and, when the bridge V is bonded to CyD, one symbol X in the corresponding CyD group is C and the bridge V is bonded to this carbon atom; in this case, the bond to the bridge V is via a position marked by "o".

11. Process for preparing a compound according to one or more of Claims 1 to 10 by reacting the free ligand with iridium alkoxides of the formula (52), with iridium ketoketonates of the formula (53), with iridium halides of the formula (54) or with iridium carboxylates of the formula (55) 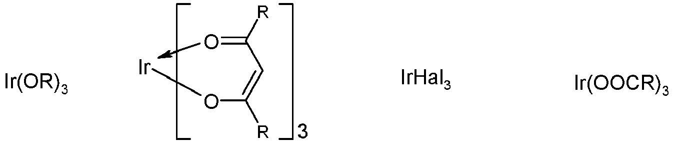 formula (52) formula (53) formula (54) formula (55) where R has the definitions given in Claim 1, Hal = F, Cl, Br or I and the iridium reactants of the formulae (52) to (55) may also be in the form of hydrates.

12. Formulation comprising at least one compound according to one or more of Claims 1 to 10 and at least one further compound, wherein the further compound is preferably selected from a matrix material and/or a solvent.

13. Use of a compound according to one or more of Claims 1 to 10 in an electronic device or as oxygen sensitizer or as photoinitiator or as photocatalyst.

14. Electronic device comprising at least one compound according to one or more of Claims 1 to 10, wherein the electronic device is preferably selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells, infrared sensors, oxygen sensors and organic laser diodes.

15. Electronic device according to Claim 14 which is an organic electroluminescent device, **characterized in that** the compound according to one or more of Claims 1 to 10 is used in one or more emitting layers.

## Revendications

1. Composé de formule (1), 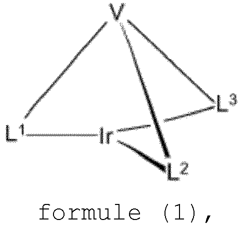 ce qui suit s'appliquant aux symboles utilisés :
L¹ est un ligand partiel de formule (2), qui coordine à l'iridium par l'intermédiaire des deux groupes Z et qui est lié à V par l'intermédiaire de la liaison en pointillés, 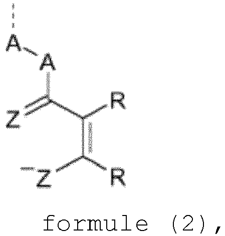
A, identique ou différent en chaque occurrence, est CR₂, O, S ou NR, dans lequel au moins un groupe A représente CR₂ ;
Z, identique ou différent en chaque occurrence, est O, S ou NR ;
L² est un ligand partiel bidentate, monoanionique qui coordine à l'iridium par l'intermédiaire d'un atome de carbone et d'un atome d'azote ou par l'intermédiaire de deux atomes de carbone ;
L³ est un ligand partiel bidentate, monoanionique qui coordine à l'iridium par l'intermédiaire d'un atome de carbone et d'un atome d'azote ou par l'intermédiaire de deux atomes de carbone, ou est un ligand partiel de formule (2), qui peut être identique ou différent de L¹ ;
V est un groupe de formule (3), les liaisons en pointillés représentant à chaque fois le lien des ligands partiels L¹, L² et L³, 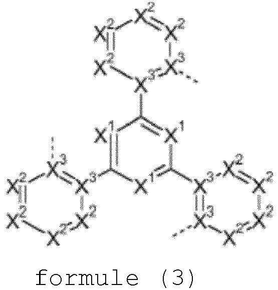
X¹, identique ou différent en chaque occurrence, est CR ou N ;
X², identique ou différent en chaque occurrence, est CR ou N, ou deux groupes X² voisins représentent ensemble NR, O ou S, de sorte qu'un cycle à 5 chaînons est généré ; ou deux groupes X² voisins représentent ensemble CR ou N, lorsque l'un des groupes X³ dans le cycle représente N, de sorte qu'un cycle à 5 chaînons est généré ; à la condition qu'au maximum deux groupes X² voisins dans chaque cycle représentent N ;
X³ est, en chaque occurrence dans un cycle, C ou un groupe X³ représente N et l'autre groupe X³ dans le même cycle représente C, les groupes X³ dans les trois cycles pouvant être choisis indépendamment les uns des autres ; à la condition que deux groupes voisins X² représentent ensemble CR ou N, lorsque, dans le cycle, l'un des groupes X³ représente N ;
R, identique ou différent en chaque occurrence, est H, D, F, Cl, Br, I, N(R¹)₂, OR¹, SR¹, CN, NO₂, COOH, C(=O)N(R¹)₂, Si (R¹)₃, B(OR¹)₂, C(=O)R¹, P(=O) (R¹)₂, S(=O)R¹, S(=O)₂R¹, OSO₂R¹, un groupe alkyle à chaîne linéaire possédant 1 à 20 atomes de C ou un groupe alcényle ou alcynyle comportant 2 à 20 atomes de C ou un groupe alkyle ramifié ou cyclique comportant 3 à 20 atomes de C, le groupe alkyle, alcényle ou alcynyle pouvant être à chaque fois substitué par un ou plusieurs radicaux R¹ et un ou plusieurs groupes non adjacents CH₂ pouvant être remplacés par Si (R¹)₂, C=O, NR¹, O, S ou CONR¹, ou un système cyclique aromatique ou hétéroaromatique comportant 5 à 40 atomes de cycle aromatiques, qui peut être à chaque fois substitué par un ou plusieurs radicaux R¹ ; à cet égard, deux radicaux R peuvent aussi former ensemble un système cyclique ;
R¹, identique ou différent en chaque occurrence, est H, D, F, Cl, Br, I, N(R²)₂, OR², SR², CN, NO₂, Si (R²)₃, B(OR²)₂, C(=O)R², P(=O) (R²)₂, S(=O)R², S(=O)₂R², OSO₂R², un groupe alkyle à chaîne linéaire possédant 1 à 20 atomes de C ou un groupe alcényle ou alcynyle comportant 2 à 20 atomes de C ou un groupe alkyle ramifié ou cyclique comportant 3 à 20 atomes de C, le groupe alkyle, alcényle ou alcynyle pouvant être à chaque fois substitué par un ou plusieurs radicaux R² et un ou plusieurs groupes non adjacents CH₂ pouvant être remplacés par Si(R²)₂, C=O, NR², O, S ou CONR², ou un système cyclique aromatique ou hétéroaromatique comportant 5 à 40 atomes de cycle aromatiques, qui peut être à chaque fois substitué par un ou plusieurs radicaux R² ; à cet égard, deux radicaux R¹ ou plus peuvent former ensemble un système cyclique ;
R², identique ou différent en chaque occurrence, est H, D, F ou un radical organique aliphatique, aromatique et/ou hétéroaromatique, en particulier un radical hydrocarboné, comportant 1 à 20 atomes de C, dans lequel également un ou plusieurs atomes de H peuvent être remplacés par F ;
à cet égard, les trois ligands bidentates L¹, L² et L³ peuvent être fermés, non seulement par le pont V, mais aussi par un pont supplémentaire pour donner un cryptate.

2. Composé selon la revendication 1, **caractérisé en ce que** V est choisi parmi les formules (4a) à (7a), 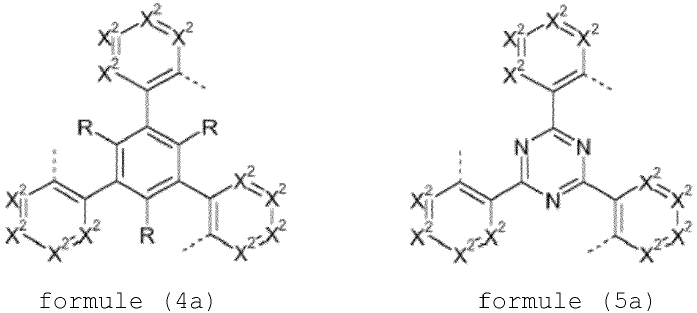 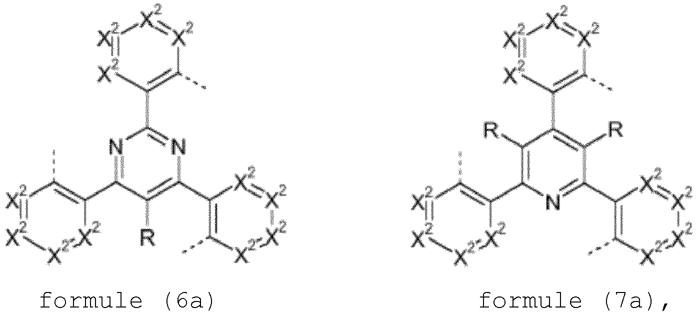 les symboles présentant les significations indiquées dans la revendication 1.

3. Composé selon la revendication 1 ou 2, **caractérisé en ce que** V présente une structure de formule (4b'), (4c) ou (5c), 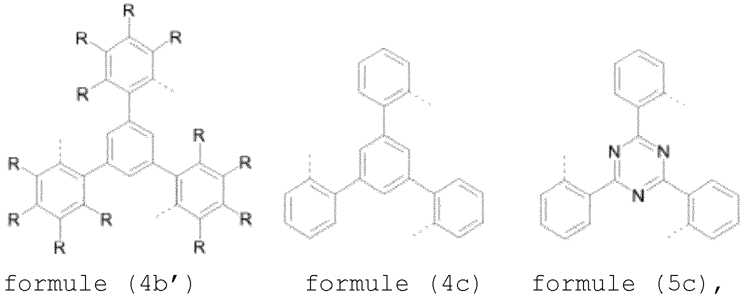 les symboles présentant les significations indiquées dans la revendication 1.

4. Composé selon l'une ou plusieurs des revendications 1 à 3, **caractérisé en ce que** les deux groupes A dans le ligand partiel L¹ représentent CR₂.

5. Composé selon l'une ou plusieurs des revendications 1 à 4, **caractérisé en ce que** les deux groupes Z dans le ligand partiel L¹ représentent O.

6. Composé selon l'une ou plusieurs des revendications 1 à 5, **caractérisé en ce que** L¹ présente une structure de formule (2c), 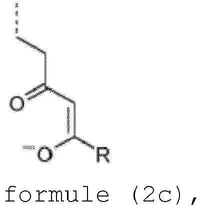 la ligne en pointillés représentant la liaison à V, et R, identique ou différent en chaque occurrence, étant choisi dans le groupe constitué par H, D, OR¹, un groupe alkyle à chaîne linéaire comportant 1 à 10 atomes de C et un groupe alkyle ramifié ou cyclique comportant 3 à 10 atomes de C, qui peut être à chaque fois substitué par un ou plusieurs radicaux R¹, ou un système cyclique aromatique ou hétéroaromatique comportant 5 à 24 atomes de cycle aromatiques, qui peut être à chaque fois substitué par un ou plusieurs radicaux R¹, et R¹ présentant les significations mentionnées dans la revendication 1.

7. Composé selon l'une ou plusieurs des revendications 1 à 6, **caractérisé en ce que** les deux ligands partiels L² et L³ présentent à chaque fois un atome de carbone et un atome d'azote en tant qu'atomes coordinants.

8. Composé selon l'une ou plusieurs des revendications 1 à 7, **caractérisé en ce que** les ligands partiels L² et L³, identiques ou différents en chaque occurrence, représentent une structure selon la formule (L-1) ou (L-2), 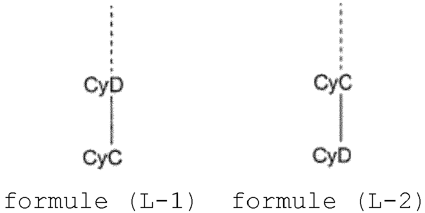 la liaison en pointillés représentant la liaison du ligand partiel à V et ce qui suit s'appliquant aux autres symboles :
CyC, identique ou différent en chaque occurrence, est un groupe aryle ou hétéroaryle substitué ou non substitué comportant 5 à 14 atomes de cycle aromatiques, qui coordine à chaque fois au métal par l'intermédiaire d'un atome de carbone et qui est relié à CyD par l'intermédiaire d'une liaison covalente ;
CyD, identique ou différent en chaque occurrence, est un groupe hétéroaryle substitué ou non substitué comportant 5 à 14 atomes de cycle aromatiques, qui coordine au métal par l'intermédiaire d'un atome d'azote ou par l'intermédiaire d'un atome de carbone de carbène et qui est relié à CyC par l'intermédiaire d'une liaison covalente ;
à cet égard, plusieurs des substituants éventuels peuvent former l'un avec l'autre un système cyclique.

9. Composé selon la revendication 8, **caractérisé en ce que** l'un des ligands partiels L² et L³ présente une structure de formule (L-1) et l'autre des ligands partiels L² et L³ présente une structure de formule (L-2) .

10. Composé selon la revendication 8 ou 9, **caractérisé en ce que** CyC est choisi parmi les structures des formules (CyC-1) à (CyC-19), le groupe CyC étant à chaque fois lié à CyD au niveau de la position marquée par # et coordinant à l'iridium au niveau de la position marquée par *, et **en ce que** le groupe CyD est choisi parmi les structures des formules (CyD-1) à (CyD-12), le groupe CyD étant à chaque fois lié à CyC au niveau de la position marquée par # et coordinant à l'iridium au niveau de la position marquée par *, 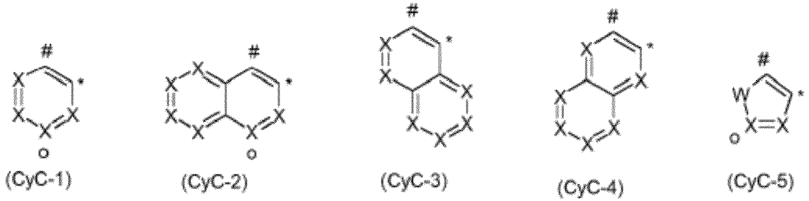 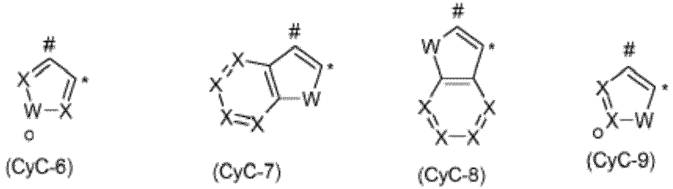 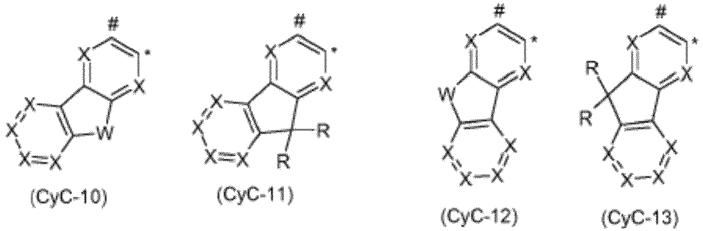 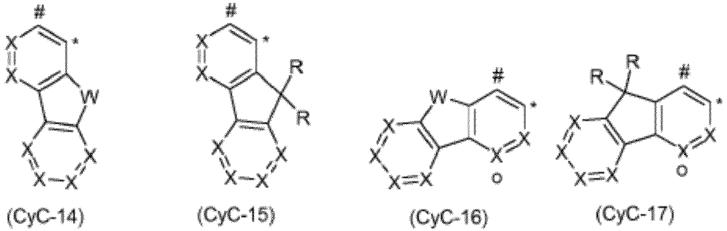 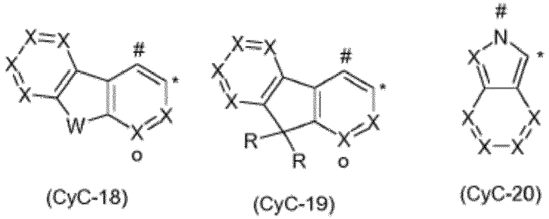 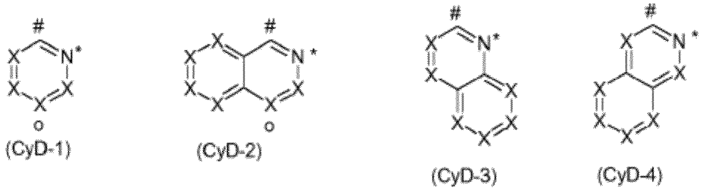 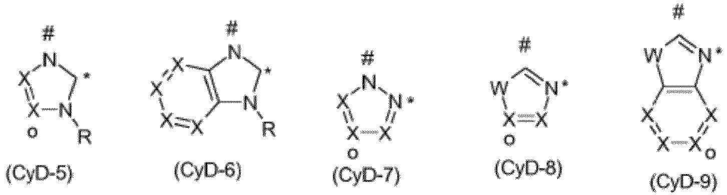 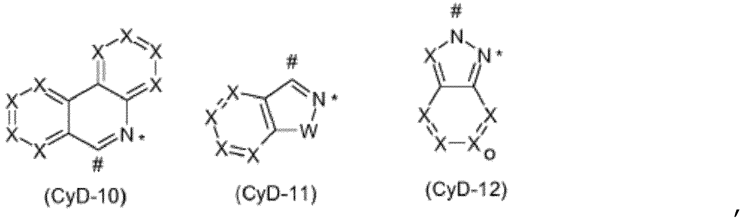 R présentant les significations mentionnées dans la revendication 1 et ce qui suit s'appliquant aux autres symboles :
X, identique ou différent en chaque occurrence, est CR ou N à la condition qu'au maximum deux symboles X par cycle représentent N ;
W, identique ou différent en chaque occurrence, est NR, O ou S ;
à la condition que lorsque le pont V est lié à CyC, un symbole X dans le groupe correspondant CyC représente C et le pont V est lié au niveau de cet atome de carbone, et lorsque le pont V est lié à CyD, un symbole X dans le groupe correspondant CyD représente C et le pont V est lié au niveau de cet atome de carbone ; la liaison au pont V est alors réalisée par l'intermédiaire d'une position qui est marquée par « o ».

11. Procédé pour la préparation d'un composé selon l'une ou plusieurs des revendications 1 à 10 par la transformation du ligand libre avec des alcoolates d'iridium de formule (52), avec des cétocétonates d'iridium de formule (53), avec des halogénures d'iridium de formule (54) ou avec des carboxylates d'iridium de formule (55), 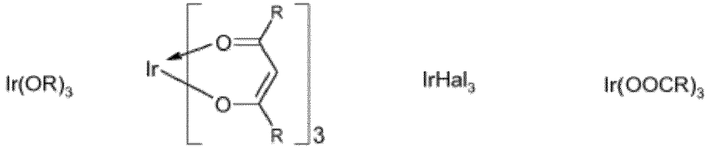 formule (52) formule (53) formule (54) formule (55) R possédant les significations mentionnées dans la revendication 1, Hal = F, Cl, Br ou I et les éduits d'iridium des formules (52) à (55) pouvant également se trouver sous forme d'hydrates.

12. Formulation, contenant au moins un composé selon l'une ou plusieurs des revendications 1 à 10 et au moins un composé supplémentaire, le composé supplémentaire étant préférablement choisi parmi un matériau de matrice et/ou un solvant.

13. Utilisation d'un composé selon l'une ou plusieurs des revendications 1 à 10 dans un dispositif électronique ou en tant que sensibilisateur à l'oxygène ou en tant que photoinitiateur ou en tant que photocatalyseur.

14. Dispositif électronique contenant au moins un composé selon l'une ou plusieurs des revendications 1 à 10, le dispositif électronique étant préférablement choisi dans le groupe constitué par des dispositifs organiques d'électroluminescence, des circuits intégrés organiques, des transistors organiques à effet de champ, des transistors organiques en film mince, des transistors organiques luminescents, des cellules solaires organiques, des détecteurs optiques organiques, des photorécepteurs organiques, des dispositifs organiques d'extinction de champ, des cellules électrochimiques luminescentes, des capteurs infrarouges, des capteurs d'oxygène et des diodes laser organiques.

15. Dispositif électronique selon la revendication 14, qui est un dispositif organique d'électroluminescence, **caractérisé en ce que** le composé selon l'une ou plusieurs des revendications 1 à 10 est utilisé dans une ou plusieurs couches émettrices.
